# EUROPEAN PATENT APPLICATION

(11) **EP 3 988 568 A1**
(43) Date of publication of application: **27.04.2022**
(21) Application number: 20203110.0
(22) Date of filing: 21.10.2020
(51) Int. Cl.: C07K 16/28, C07K 16/30, C07K 16/32, C07K 16/46, A61K 39/395, C07K 16/18

(54) **COMBINATION TREATMENT**

(71) Applicant: Numab Therapeutics AG, 8820 Wädenswil (CH)
(72) Inventor: The designation of the inventor has not yet been filed
(74) Representative: Virnekäs, Bernhard

(57) **Abstract**

The present invention relates to a pharmaceutical composition comprising a first multispecific antibody (MA1) comprising one binding domain, which specifically binds to CD137 (CD137-BD), and one binding domain, which specifically binds to PDL1 (PDL1-BD); a second multispecific antibody (MA2) comprising at least one binding domain, which specifically binds to a tumor cell associated antigen (TAA-BD), and one binding domain, which specifically binds to CD3 (CD3-BD); and a pharmaceutically acceptable carrier. The present invention further relates to a kit comprising said first multispecific antibody MA1 and said second multispecific antibody MA2. The present invention further relates to the use as well as to methods of use of said pharmaceutical composition or said kit. Finally, the present invention relates to a method for treating a patient suffering from cancer, comprising the step of administering a first multispecific antibody (MA1) comprising one binding domain, which specifically binds to CD137 (CD137-BD), and one binding domain, which specifically binds to PDL1 (PDL1-BD), and a second multispecific antibody (MA2) comprising at least one binding domain, which specifically binds to a tumor cell associated antigen (TAA-BD), and one binding domain, which specifically binds to CD3 (CD3-BD).

SEQUENCE LISTING

<110>  Numab Therapeutics AG

<120>  COMBINATION TREATMENT

<130>  116994P877EP

<160>  181

<170>  PatentIn version 3.5

<210>  1
<211>  11
<212>  PRT
<213>  Artificial Sequence

<220>
<223>  artificial antibody-based sequence

<400>  1

Gly Phe Ser Phe Ser Asn Ser Tyr Trp Ile Cys
1               5                   10

<210>  2
<211>  18
<212>  PRT
<213>  Artificial Sequence

<220>
<223>  artificial antibody-based sequence

<400>  2

Cys Thr Phe Val Gly Ser Ser Asp Ser Thr Tyr Tyr Ala Asn Trp Ala
1               5                   10                  15

Lys Gly

<210>  3
<211>  14
<212>  PRT
<213>  Artificial Sequence

<220>
<223>  artificial antibody-based sequence

<400>  3

Arg His Pro Ser Asp Ala Val Tyr Gly Tyr Ala Asn Asn Leu
1               5                   10

<210>  4
<211>  124
<212>  PRT
<213>  Artificial Sequence

<220>
<223>  artificial antibody-based sequence

<400>  4

Gln Val Gln Leu Gln Glu Ser Gly Pro Gly Leu Val Lys Pro Ser Glu
1               5                   10                  15

Thr Leu Ser Leu Thr Cys Lys Val Ser Gly Phe Ser Phe Ser Asn Ser
            20                  25                  30

Tyr Trp Ile Cys Trp Ile Arg Gln Pro Pro Gly Lys Gly Leu Glu Trp
        35                  40                  45

Ile Gly Cys Thr Phe Val Gly Ser Ser Asp Ser Thr Tyr Tyr Ala Asn
    50                  55                  60

Trp Ala Lys Gly Arg Val Thr Ile Ser Val Asp Ser Ser Lys Asn Gln
65                  70                  75                  80

Phe Ser Leu Lys Leu Ser Ser Val Thr Ala Ala Asp Thr Ala Val Tyr
                85                  90                  95

Tyr Cys Ala Arg His Pro Ser Asp Ala Val Tyr Gly Tyr Ala Asn Asn
            100                 105                 110

Leu Trp Gly Gln Gly Thr Leu Val Thr Val Ser Ser
        115                 120


<210>  5
<211>  124
<212>  PRT
<213>  Artificial Sequence

<220>
<223>  artificial antibody-based sequence

<400>  5

Gln Val Gln Leu Gln Glu Ser Gly Pro Gly Leu Val Lys Pro Ser Glu
1               5                   10                  15

Thr Leu Ser Leu Thr Cys Lys Val Ser Gly Phe Ser Phe Ser Asn Ser
            20                  25                  30

Tyr Trp Ile Cys Trp Val Arg Gln Pro Pro Gly Lys Gly Leu Glu Trp
        35                  40                  45

Ile Gly Cys Thr Phe Val Gly Ser Ser Asp Ser Thr Tyr Tyr Ala Asn
    50                  55                  60

Trp Ala Lys Gly Arg Val Thr Ile Ser Val Asp Ser Ser Lys Asn Gln
65                  70                  75                  80

Val Ser Leu Lys Leu Ser Ser Val Thr Ala Ala Asp Thr Ala Val Tyr
                85                  90                  95

Phe Cys Ala Arg His Pro Ser Asp Ala Val Tyr Gly Tyr Ala Asn Asn
            100                 105                 110

Leu Trp Gly Gln Gly Thr Leu Val Thr Val Ser Ser
        115                 120

<210>  6
<211>  124
<212>  PRT
<213>  Artificial Sequence

<220>
<223>  artificial antibody-based sequence

<400>  6

Gln Val Gln Leu Gln Glu Ser Gly Pro Gly Leu Val Lys Pro Ser Glu
1               5                   10                  15

Thr Leu Ser Leu Thr Cys Lys Ala Ser Gly Phe Ser Phe Ser Asn Ser
            20                  25                  30

Tyr Trp Ile Cys Trp Val Arg Gln Pro Pro Gly Lys Gly Leu Glu Trp
        35                  40                  45

Ile Gly Cys Thr Phe Val Gly Ser Ser Asp Ser Thr Tyr Tyr Ala Asn
    50                  55                  60

Trp Ala Lys Gly Arg Val Thr Ile Ser Lys Asp Ser Ser Lys Asn Gln
65                  70                  75                  80

Val Ser Leu Lys Leu Ser Ser Val Thr Ala Ala Asp Thr Ala Val Tyr
                85                  90                  95

Phe Cys Ala Arg His Pro Ser Asp Ala Val Tyr Gly Tyr Ala Asn Asn
            100                 105                 110

Leu Trp Gly Gln Gly Thr Leu Val Thr Val Ser Ser
        115                 120

<210>  7
<211>  124
<212>  PRT
<213>  Artificial Sequence

<220>
<223>  artificial antibody-based sequence

<400>  7

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Ser Asn Ser
            20                  25                  30

Tyr Trp Ile Cys Trp Val Arg Gln Ala Pro Gly Lys Cys Leu Glu Trp
        35                  40                  45

Ile Gly Cys Thr Phe Val Gly Ser Ser Asp Ser Thr Tyr Tyr Ala Asn
    50                  55                  60

Trp Ala Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr
                85                  90                  95

Tyr Cys Ala Arg His Pro Ser Asp Ala Val Tyr Gly Tyr Ala Asn Asn
            100                 105                 110

Leu Trp Gly Gln Gly Thr Leu Val Thr Val Ser Ser
        115                 120


<210>  8
<211>  11
<212>  PRT
<213>  Artificial Sequence

<220>
<223>  artificial antibody-based sequence

<400>  8

Gln Ala Ser Gln Ser Ile Asn Asn Val Leu Ala
1               5                   10


<210>  9
<211>  7
<212>  PRT
<213>  Artificial Sequence

<220>
<223>  artificial antibody-based sequence

<400>  9

Arg Ala Ser Thr Leu Ala Ser
1               5

<210>  10
<211>  9
<212>  PRT
<213>  Artificial Sequence

<220>
<223>  artificial antibody-based sequence

<400>  10

Gln Ser Ser Tyr Gly Asn Tyr Gly Asp
1               5


<210>  11
<211>  108
<212>  PRT
<213>  Artificial Sequence

<220>
<223>  artificial antibody-based sequence

<400>  11

Asp Ile Gln Met Thr Gln Ser Pro Ser Ser Leu Ser Ala Ser Val Gly
1               5                   10                  15


Asp Arg Val Thr Ile Thr Cys Gln Ala Ser Gln Ser Ile Asn Asn Val
            20                  25                  30


Leu Ala Trp Tyr Gln Gln Lys Pro Gly Lys Ala Pro Lys Leu Leu Ile
        35                  40                  45


Tyr Arg Ala Ser Thr Leu Ala Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Ser Ser Tyr Gly Asn Tyr Gly
                85                  90                  95


Asp Phe Gly Thr Gly Thr Lys Val Thr Val Leu Gly
            100                 105


<210>  12
<211>  108
<212>  PRT
<213>  Artificial Sequence

<220>
<223>  artificial antibody-based sequence

<400>  12

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Pro Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Thr Gly Thr Lys Val Thr Val Leu Gly
            100                 105

<210>  13
<211>  108
<212>  PRT
<213>  Artificial Sequence

<220>
<223>  artificial antibody-based sequence

<400>  13

Asp Leu Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Pro Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Thr Gly Thr Lys Val Thr Val Leu Gly
            100                 105

<210> 14
<211> 108
<212> PRT
<213> Artificial Sequence

<220>
<223> artificial antibody-based sequence

<400> 14

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Cys Gly Thr Lys Val Thr Val Leu Gly
            100                 105

<210> 15
<211> 252
<212> PRT
<213> Artificial Sequence

<220>
<223> artificial antibody-based sequence

<400> 15

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Thr Gly Thr Lys Val Thr Val Leu Gly Gly Gly Gly Gly
            100                 105                 110

Ser Gly Gly Gly Gly Ser Gly Gly Gly Gly Ser Gly Gly Gly Gly Ser
        115                 120                 125

Gln Val Gln Leu Gln Glu Ser Gly Pro Gly Leu Val Lys Pro Ser Glu
    130                 135                 140

Thr Leu Ser Leu Thr Cys Lys Val Ser Gly Phe Ser Phe Ser Asn Ser
145                 150                 155                 160

Tyr Trp Ile Cys Trp Ile Arg Gln Pro Pro Gly Lys Gly Leu Glu Trp
                165                 170                 175

Ile Gly Cys Thr Phe Val Gly Ser Ser Asp Ser Thr Tyr Tyr Ala Asn
            180                 185                 190

Trp Ala Lys Gly Arg Val Thr Ile Ser Val Asp Ser Ser Lys Asn Gln
        195                 200                 205

Phe Ser Leu Lys Leu Ser Ser Val Thr Ala Ala Asp Thr Ala Val Tyr
    210                 215                 220

Tyr Cys Ala Arg His Pro Ser Asp Ala Val Tyr Gly Tyr Ala Asn Asn
225                 230                 235                 240

Leu Trp Gly Gln Gly Thr Leu Val Thr Val Ser Ser
                245                 250

<210>  16
<211>  252
<212>  PRT
<213>  Artificial Sequence

<220>
<223>  artificial antibody-based sequence

<400>  16

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Pro Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Thr Gly Thr Lys Val Thr Val Leu Gly Gly Gly Gly Gly
            100                 105                 110

Ser Gly Gly Gly Gly Ser Gly Gly Gly Gly Ser Gly Gly Gly Gly Ser
        115                 120                 125

Gln Val Gln Leu Gln Glu Ser Gly Pro Gly Leu Val Lys Pro Ser Glu
    130                 135                 140

Thr Leu Ser Leu Thr Cys Lys Val Ser Gly Phe Ser Phe Ser Asn Ser
145                 150                 155                 160

Tyr Trp Ile Cys Trp Val Arg Gln Pro Pro Gly Lys Gly Leu Glu Trp
                165                 170                 175

Ile Gly Cys Thr Phe Val Gly Ser Ser Asp Ser Thr Tyr Tyr Ala Asn
            180                 185                 190

Trp Ala Lys Gly Arg Val Thr Ile Ser Val Asp Ser Ser Lys Asn Gln
        195                 200                 205

Val Ser Leu Lys Leu Ser Ser Val Thr Ala Ala Asp Thr Ala Val Tyr
    210                 215                 220

Phe Cys Ala Arg His Pro Ser Asp Ala Val Tyr Gly Tyr Ala Asn Asn
225                 230                 235                 240

Leu Trp Gly Gln Gly Thr Leu Val Thr Val Ser Ser
                245                 250

<210>  17
<211>  252
<212>  PRT
<213>  Artificial Sequence

<220>
<223>  artificial antibody-based sequence

<400>  17

Asp Leu Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Pro Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Thr Gly Thr Lys Val Thr Val Leu Gly Gly Gly Gly Gly
            100                 105                 110

Ser Gly Gly Gly Gly Ser Gly Gly Gly Gly Ser Gly Gly Gly Gly Ser
        115                 120                 125

Gln Val Gln Leu Gln Glu Ser Gly Pro Gly Leu Val Lys Pro Ser Glu
    130                 135                 140

Thr Leu Ser Leu Thr Cys Lys Ala Ser Gly Phe Ser Phe Ser Asn Ser
145                 150                 155                 160

Tyr Trp Ile Cys Trp Val Arg Gln Pro Pro Gly Lys Gly Leu Glu Trp
                165                 170                 175

Ile Gly Cys Thr Phe Val Gly Ser Ser Asp Ser Thr Tyr Tyr Ala Asn
            180                 185                 190

Trp Ala Lys Gly Arg Val Thr Ile Ser Lys Asp Ser Ser Lys Asn Gln
        195                 200                 205

Val Ser Leu Lys Leu Ser Ser Val Thr Ala Ala Asp Thr Ala Val Tyr
    210                 215                 220

Phe Cys Ala Arg His Pro Ser Asp Ala Val Tyr Gly Tyr Ala Asn Asn
225                 230                 235                 240

Leu Trp Gly Gln Gly Thr Leu Val Thr Val Ser Ser
                245                 250

<210>  18
<211>  252
<212>  PRT
<213>  Artificial Sequence

<220>
<223>  artificial antibody-based sequence

<400>  18

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Cys Gly Thr Lys Val Thr Val Leu Gly Gly Gly Gly Gly
            100                 105                 110

Ser Gly Gly Gly Gly Ser Gly Gly Gly Gly Ser Gly Gly Gly Gly Ser
        115                 120                 125

Glu Val Gln Leu Val Glu Ser Gly Gly Gly Leu Val Gln Pro Gly Gly
    130                 135                 140

Ser Leu Arg Leu Ser Cys Ala Ala Ser Gly Phe Ser Phe Ser Asn Ser
145                 150                 155                 160

Tyr Trp Ile Cys Trp Val Arg Gln Ala Pro Gly Lys Cys Leu Glu Trp

                165                 170                 175

Ile Gly Cys Thr Phe Val Gly Ser Ser Asp Ser Thr Tyr Tyr Ala Asn
            180                 185                 190

Trp Ala Lys Gly Arg Phe Thr Ile Ser Arg Asp Asn Ser Lys Asn Thr
        195                 200                 205

Val Tyr Leu Gln Met Asn Ser Leu Arg Ala Glu Asp Thr Ala Val Tyr
    210                 215                 220

Tyr Cys Ala Arg His Pro Ser Asp Ala Val Tyr Gly Tyr Ala Asn Asn
225                 230                 235                 240

Leu Trp Gly Gln Gly Thr Leu Val Thr Val Ser Ser
                245                 250

<210> 19
<211> 11
<212> PRT
<213> Artificial Sequence

<220>
<223> artificial antibody-based sequence

<400> 19

Gly Phe Ser Phe Asn Asn Asp Tyr Asp Met Cys
1               5                   10

<210> 20
<211> 17
<212> PRT
<213> Artificial Sequence

<220>
<223> artificial antibody-based sequence

<400> 20

Cys Ile Asp Thr Gly Asp Gly Ser Thr Tyr Tyr Ala Ser Trp Ala Lys
1               5                   10                  15

Gly

<210> 21
<211> 16
<212> PRT
<213> Artificial Sequence

<220>
<223> artificial antibody-based sequence

<400>  21

Arg Glu Ala Ala Ser Ser Ser Gly Tyr Gly Met Gly Tyr Phe Asp Leu
1               5                   10                  15


<210>  22
<211>  125
<212>  PRT
<213>  Artificial Sequence

<220>
<223>  artificial antibody-based sequence

<400>  22

Gln Val Gln Leu Gln Glu Ser Gly Pro Gly Leu Val Lys Pro Ser Glu
1               5                   10                  15


Thr Leu Ser Leu Thr Cys Lys Val Ser Gly Phe Ser Phe Asn Asn Asp
            20                  25                  30


Tyr Asp Met Cys Trp Ile Arg Gln Pro Pro Gly Lys Gly Leu Glu Trp
        35                  40                  45


Ile Gly Cys Ile Asp Thr Gly Asp Gly Ser Thr Tyr Tyr Ala Ser Trp
    50                  55                  60


Ala Lys Gly Arg Val Thr Ile Ser Val Asp Ser Ser Lys Asn Gln Phe
65                  70                  75                  80


Ser Leu Lys Leu Ser Ser Val Thr Ala Ala Asp Thr Ala Val Tyr Tyr
                85                  90                  95


Cys Ala Arg Glu Ala Ala Ser Ser Ser Gly Tyr Gly Met Gly Tyr Phe
            100                 105                 110


Asp Leu Trp Gly Gln Gly Thr Leu Val Thr Val Ser Ser
        115                 120                 125


<210>  23
<211>  13
<212>  PRT
<213>  Artificial Sequence

<220>
<223>  artificial antibody-based sequence

<400>  23

Gln Ser Ser Gln Ser Val Tyr Asp Asn Asn Trp Leu Ala
1               5                   10


<210>  24

<211>  7
<212>  PRT
<213>  Artificial Sequence

<220>
<223>  artificial antibody-based sequence

<400>  24

Arg Ala Ser Asn Leu Ala Ser
1               5


<210>  25
<211>  12
<212>  PRT
<213>  Artificial Sequence

<220>
<223>  artificial antibody-based sequence

<400>  25

Gln Gly Thr Tyr Leu Ser Ser Asn Trp Tyr Trp Ala
1               5                   10


<210>  26
<211>  113
<212>  PRT
<213>  Artificial Sequence

<220>
<223>  artificial antibody-based sequence

<400>  26

Asp Ile Gln Met Thr Gln Ser Pro Ser Ser Leu Ser Ala Ser Val Gly
1               5                   10                  15


Asp Arg Val Thr Ile Thr Cys Gln Ser Ser Gln Ser Val Tyr Asp Asn
            20                  25                  30


Asn Trp Leu Ala Trp Tyr Gln Gln Lys Pro Gly Lys Ala Pro Lys Leu
        35                  40                  45


Leu Ile Tyr Arg Ala Ser Asn Leu Ala Ser Gly Val Pro Ser Arg Phe
    50                  55                  60


Ser Gly Ser Gly Ser Gly Thr Asp Phe Thr Leu Thr Ile Ser Ser Leu
65                  70                  75                  80


Gln Pro Glu Asp Phe Ala Thr Tyr Tyr Cys Gln Gly Thr Tyr Leu Ser
                85                  90                  95


Ser Asn Trp Tyr Trp Ala Phe Gly Thr Gly Thr Lys Val Thr Val Leu
            100                 105                 110

Gly

<210>  27
<211>  258
<212>  PRT
<213>  Artificial Sequence

<220>
<223>  artificial antibody-based sequence

<400>  27

Asp Ile Gln Met Thr Gln Ser Pro Ser Ser Leu Ser Ala Ser Val Gly
1               5                   10                  15

Asp Arg Val Thr Ile Thr Cys Gln Ser Ser Gln Ser Val Tyr Asp Asn
            20                  25                  30

Asn Trp Leu Ala Trp Tyr Gln Gln Lys Pro Gly Lys Ala Pro Lys Leu
        35                  40                  45

Leu Ile Tyr Arg Ala Ser Asn Leu Ala Ser Gly Val Pro Ser Arg Phe
    50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Gln Gly Thr Tyr Leu Ser
                85                  90                  95

Ser Asn Trp Tyr Trp Ala Phe Gly Thr Gly Thr Lys Val Thr Val Leu
            100                 105                 110

Gly Gly Gly Gly Gly Ser Gly Gly Gly Gly Ser Gly Gly Gly Gly Ser
        115                 120                 125

Gly Gly Gly Gly Ser Gln Val Gln Leu Gln Glu Ser Gly Pro Gly Leu
    130                 135                 140

Val Lys Pro Ser Glu Thr Leu Ser Leu Thr Cys Lys Val Ser Gly Phe
145                 150                 155                 160

Ser Phe Asn Asn Asp Tyr Asp Met Cys Trp Ile Arg Gln Pro Pro Gly
                165                 170                 175

Lys Gly Leu Glu Trp Ile Gly Cys Ile Asp Thr Gly Asp Gly Ser Thr
            180                 185                 190

Tyr Tyr Ala Ser Trp Ala Lys Gly Arg Val Thr Ile Ser Val Asp Ser
        195                 200                 205

Ser Lys Asn Gln Phe Ser Leu Lys Leu Ser Ser Val Thr Ala Ala Asp
    210                 215                 220

Thr Ala Val Tyr Tyr Cys Ala Arg Glu Ala Ala Ser Ser Ser Gly Tyr
225                 230                 235                 240

Gly Met Gly Tyr Phe Asp Leu Trp Gly Gln Gly Thr Leu Val Thr Val
                245                 250                 255

Ser Ser

<210>  28
<211>  11
<212>  PRT
<213>  Artificial Sequence

<220>
<223>  artificial antibody-based sequence

<400>  28

Gly Phe Ser Phe Ser Ala Asn Tyr Tyr Pro Cys
1               5                   10

<210>  29
<211>  17
<212>  PRT
<213>  Artificial Sequence

<220>
<223>  artificial antibody-based sequence

<400>  29

Cys Ile Tyr Gly Gly Ser Ser Asp Ile Thr Tyr Asp Ala Asn Trp Thr
1               5                   10                  15

Lys

<210>  30
<211>  12
<212>  PRT
<213>  Artificial Sequence

<220>
<223>  artificial antibody-based sequence

<400>  30

Arg Ser Ala Trp Tyr Ser Gly Trp Gly Gly Asp Leu
1               5                   10

<210>  31
<211>  122
<212>  PRT
<213>  Artificial Sequence

<220>
<223>  artificial antibody-based sequence

<400>  31

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Ser Ala Asn
            20                  25                  30

Tyr Tyr Pro Cys Trp Val Arg Gln Ala Pro Gly Lys Gly Leu Glu Trp
        35                  40                  45

Ile Gly Cys Ile Tyr Gly Gly Ser Ser Asp Ile Thr Tyr Asp Ala Asn
    50                  55                  60

Trp Thr Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr
                85                  90                  95

Tyr Cys Ala Arg Ser Ala Trp Tyr Ser Gly Trp Gly Gly Asp Leu Trp
            100                 105                 110

Gly Gln Gly Thr Leu Val Thr Val Ser Ser
        115                 120

<210>  32
<211>  122
<212>  PRT
<213>  Artificial Sequence

<220>
<223>  artificial antibody-based sequence

<400>  32

Glu Ser Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Ser Ala Asn
            20                  25                  30

Tyr Tyr Pro Cys Trp Val Arg Gln Ala Pro Gly Lys Gly Leu Glu Trp
        35                  40                  45

Ile Gly Cys Ile Tyr Gly Gly Ser Ser Asp Ile Thr Tyr Asp Ala Asn
    50                  55                  60

Trp Thr Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr
                85                  90                  95

Phe Cys Ala Arg Ser Ala Trp Tyr Ser Gly Trp Gly Gly Asp Leu Trp
            100                 105                 110

Gly Pro Gly Thr Leu Val Thr Val Ser Ser
        115                 120

<210>  33
<211>  122
<212>  PRT
<213>  Artificial Sequence

<220>
<223>  artificial antibody-based sequence

<400>  33

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Ser Ala Asn
            20                  25                  30

Tyr Tyr Pro Cys Trp Val Arg Gln Ala Pro Gly Lys Cys Leu Glu Trp
        35                  40                  45

Ile Gly Cys Ile Tyr Gly Gly Ser Ser Asp Ile Thr Tyr Asp Ala Asn
    50                  55                  60

Trp Thr Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr
                85                  90                  95

Tyr Cys Ala Arg Ser Ala Trp Tyr Ser Gly Trp Gly Gly Asp Leu Trp
            100                 105                 110

Gly Gln Gly Thr Leu Val Thr Val Ser Ser
        115                 120

<210>  34
<211>  11
<212>  PRT
<213>  Artificial Sequence

<220>
<223>  artificial antibody-based sequence

<400>  34

Gln Ala Ser Gln Ser Ile Ser Asn Arg Leu Ala
1               5                   10

<210>  35
<211>  7
<212>  PRT
<213>  Artificial Sequence

<220>
<223>  artificial antibody-based sequence

<400>  35

Ser Ala Ser Thr Leu Ala Ser
1               5

<210>  36
<211>  11
<212>  PRT
<213>  Artificial Sequence

<220>
<223>  artificial antibody-based sequence

<400>  36

Gln Ser Thr Tyr Tyr Gly Asn Asp Gly Asn Ala
1               5                   10

<210>  37
<211>  110
<212>  PRT
<213>  Artificial Sequence

<220>
<223>  artificial antibody-based sequence

<400>  37

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Ser Asn Arg
            20                  25                  30

Leu Ala Trp Tyr Gln Gln Lys Pro Gly Lys Ala Pro Lys Leu Leu Ile
        35                  40                  45

Tyr Ser Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Thr Tyr Tyr Gly Asn Asp
                85                  90                  95

Gly Asn Ala Phe Gly Thr Gly Thr Lys Val Thr Val Leu Gly
            100                 105                 110

<210>  38
<211>  110
<212>  PRT
<213>  Artificial Sequence

<220>
<223>  artificial antibody-based sequence

<400>  38

Asp Phe Gln Leu Thr Gln Ser Pro Ser Ser Leu Ser Ala Ser Val Gly
1               5                   10                  15

Asp Arg Val Thr Ile Thr Cys Gln Ala Ser Gln Ser Ile Ser Asn Arg
            20                  25                  30

Leu Ala Trp Tyr Gln Gln Lys Pro Gly Lys Pro Pro Lys Leu Leu Ile
        35                  40                  45

Tyr Ser Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Thr Tyr Tyr Gly Asn Asp
                85                  90                  95

Gly Asn Ala Phe Gly Thr Gly Thr Lys Val Thr Val Leu Gly
            100                 105                 110

<210>  39
<211>  110
<212>  PRT
<213>  Artificial Sequence

<220>
<223>  artificial antibody-based sequence

<400>  39

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Ser Asn Arg
            20                  25                  30

Leu Ala Trp Tyr Gln Gln Lys Pro Gly Lys Ala Pro Lys Leu Leu Ile
        35                  40                  45

Tyr Ser Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Thr Tyr Tyr Gly Asn Asp
                85                  90                  95

Gly Asn Ala Phe Gly Cys Gly Thr Lys Val Thr Val Leu Gly
            100                 105                 110

<210>  40
<211>  252
<212>  PRT
<213>  Artificial Sequence

<220>
<223>  artificial antibody-based sequence

<400>  40

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Ser Asn Arg
            20                  25                  30

Leu Ala Trp Tyr Gln Gln Lys Pro Gly Lys Ala Pro Lys Leu Leu Ile
        35                  40                  45

Tyr Ser Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Thr Tyr Tyr Gly Asn Asp
                85                  90                  95

Gly Asn Ala Phe Gly Thr Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Val Gln Leu Val Glu Ser Gly Gly Gly Leu Val Gln Pro
    130                 135                 140

Gly Gly Ser Leu Arg Leu Ser Cys Ala Ala Ser Gly Phe Ser Phe Ser
145                 150                 155                 160

Ala Asn Tyr Tyr Pro Cys Trp Val Arg Gln Ala Pro Gly Lys Gly Leu
                165                 170                 175

Glu Trp Ile Gly Cys Ile Tyr Gly Gly Ser Ser Asp Ile Thr Tyr Asp
            180                 185                 190

Ala Asn Trp Thr Lys Gly Arg Phe Thr Ile Ser Arg Asp Asn Ser Lys
        195                 200                 205

Asn Thr Val Tyr Leu Gln Met Asn Ser Leu Arg Ala Glu Asp Thr Ala
    210                 215                 220

Val Tyr Tyr Cys Ala Arg Ser Ala Trp Tyr Ser Gly Trp Gly Gly Asp
225                 230                 235                 240

Leu Trp Gly Gln Gly Thr Leu Val Thr Val Ser Ser
                245                 250

<210>  41
<211>  252
<212>  PRT
<213>  Artificial Sequence

<220>
<223>  artificial antibody-based sequence

<400>  41

Asp Phe Gln Leu Thr Gln Ser Pro Ser Ser Leu Ser Ala Ser Val Gly
1               5                   10                  15

Asp Arg Val Thr Ile Thr Cys Gln Ala Ser Gln Ser Ile Ser Asn Arg
            20                  25                  30

Leu Ala Trp Tyr Gln Gln Lys Pro Gly Lys Pro Pro Lys Leu Leu Ile
        35                  40                  45

Tyr Ser Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Thr Tyr Tyr Gly Asn Asp
                85                  90                  95

Gly Asn Ala Phe Gly Thr Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Ser Gln Leu Val Glu Ser Gly Gly Gly Leu Val Gln Pro
    130                 135                 140

Gly Gly Ser Leu Arg Leu Ser Cys Ala Ala Ser Gly Phe Ser Phe Ser
145                 150                 155                 160

Ala Asn Tyr Tyr Pro Cys Trp Val Arg Gln Ala Pro Gly Lys Gly Leu
                165                 170                 175

Glu Trp Ile Gly Cys Ile Tyr Gly Gly Ser Ser Asp Ile Thr Tyr Asp
            180                 185                 190

Ala Asn Trp Thr Lys Gly Arg Phe Thr Ile Ser Arg Asp Asn Ser Lys
        195                 200                 205

Asn Thr Val Tyr Leu Gln Met Asn Ser Leu Arg Ala Glu Asp Thr Ala
    210                 215                 220

Val Tyr Phe Cys Ala Arg Ser Ala Trp Tyr Ser Gly Trp Gly Gly Asp
225                 230                 235                 240

Leu Trp Gly Pro Gly Thr Leu Val Thr Val Ser Ser
                245                 250

<210>  42
<211>  252
<212>  PRT
<213>  Artificial Sequence

<220>
<223>  artificial antibody-based sequence

<400>  42

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Ser Asn Arg
            20                  25                  30

Leu Ala Trp Tyr Gln Gln Lys Pro Gly Lys Ala Pro Lys Leu Leu Ile
        35                  40                  45

Tyr Ser Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Thr Tyr Tyr Gly Asn Asp
                85                  90                  95

Gly Asn Ala Phe Gly Cys Gly Thr Lys Val Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Val Gln Leu Val Glu Ser Gly Gly Gly Leu Val Gln Pro
    130                 135                 140

Gly Gly Ser Leu Arg Leu Ser Cys Ala Ala Ser Gly Phe Ser Phe Ser
145                 150                 155                 160

Ala Asn Tyr Tyr Pro Cys Trp Val Arg Gln Ala Pro Gly Lys Cys Leu
                165                 170                 175

Glu Trp Ile Gly Cys Ile Tyr Gly Gly Ser Ser Asp Ile Thr Tyr Asp
            180                 185                 190

Ala Asn Trp Thr Lys Gly Arg Phe Thr Ile Ser Arg Asp Asn Ser Lys
        195                 200                 205

Asn Thr Val Tyr Leu Gln Met Asn Ser Leu Arg Ala Glu Asp Thr Ala
    210                 215                 220

Val Tyr Tyr Cys Ala Arg Ser Ala Trp Tyr Ser Gly Trp Gly Gly Asp
225                 230                 235                 240

Leu Trp Gly Gln Gly Thr Leu Val Thr Val Ser Ser
                245                 250

<210>  43
<211>  11
<212>  PRT
<213>  Artificial Sequence

<220>
<223>  artificial antibody-based sequence

<400>  43

Gly Phe Ser Phe Asn Ser Asp Tyr Trp Ile Tyr
1               5                   10

<210>  44
<211>  18
<212>  PRT
<213>  Artificial Sequence

<220>
<223>  artificial antibody-based sequence

<400>  44

Ser Ile Tyr Gly Gly Ser Ser Gly Asn Thr Gln Tyr Ala Ser Trp Ala
1               5                   10                  15

Gln Gly

<210>  45
<211>  12
<212>  PRT
<213>  Artificial Sequence

<220>
<223>  artificial antibody-based sequence

<400>  45

Arg Gly Tyr Val Asp Tyr Gly Gly Ala Thr Asp Leu
1               5                   10

<210>  46
<211>  122
<212>  PRT
<213>  Artificial Sequence

<220>
<223>  artificial antibody-based sequence

<400>  46

Gln Val Gln Leu Gln Glu Ser Gly Pro Gly Leu Val Lys Pro Ser Glu
1               5                   10                  15

Thr Leu Ser Leu Thr Cys Lys Val Ser Gly Phe Ser Phe Asn Ser Asp
            20                  25                  30

Tyr Trp Ile Tyr Trp Ile Arg Gln Pro Pro Gly Lys Gly Leu Glu Trp
        35                  40                  45

Ile Gly Ser Ile Tyr Gly Gly Ser Ser Gly Asn Thr Gln Tyr Ala Ser
    50                  55                  60

Trp Ala Gln Gly Arg Val Thr Ile Ser Val Asp Ser Ser Lys Asn Gln
65                  70                  75                  80

Phe Ser Leu Lys Leu Ser Ser Val Thr Ala Ala Asp Thr Ala Val Tyr
                85                  90                  95

Tyr Cys Ala Arg Gly Tyr Val Asp Tyr Gly Gly Ala Thr Asp Leu Trp
            100                 105                 110

Gly Gln Gly Thr Leu Val Thr Val Ser Ser
        115                 120

<210>  47
<211>  122
<212>  PRT
<213>  Artificial Sequence

<220>
<223>  artificial antibody-based sequence

<400>  47

Gln Val Gln Leu Val Gln Ser Gly Ala Glu Val Lys Lys Pro Gly Ala
1               5                   10                  15

Ser Val Lys Val Ser Cys Lys Ala Ser Gly Phe Ser Phe Asn Ser Asp
            20                  25                  30

Tyr Trp Ile Tyr Trp Val Arg Gln Ala Pro Gly Gln Gly Leu Glu Trp
        35                  40                  45

Met Gly Ser Ile Tyr Gly Gly Ser Ser Gly Asn Thr Gln Tyr Ala Ser
    50                  55                  60

Trp Ala Gln Gly Arg Val Thr Met Thr Arg Asp Thr Ser Ile Ser Thr
65                  70                  75                  80

Ala Tyr Met Glu Leu Ser Ser Leu Arg Ser Glu Asp Thr Ala Val Tyr
                85                  90                  95

Tyr Cys Ala Arg Gly Tyr Val Asp Tyr Gly Gly Ala Thr Asp Leu Trp
            100                 105                 110

Gly Gln Gly Thr Leu Val Thr Val Ser Ser
        115                 120

<210>  48
<211>  122
<212>  PRT
<213>  Artificial Sequence

<220>
<223>  artificial antibody-based sequence

<400>  48

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Asn Ser Asp
            20                  25                  30

Tyr Trp Ile Tyr Trp Val Arg Gln Ala Pro Gly Lys Gly Leu Glu Trp
        35                  40                  45

Ile Ala Ser Ile Tyr Gly Gly Ser Ser Gly Asn Thr Gln Tyr Ala Ser
    50                  55                  60

Trp Ala Gln Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr
                85                  90                  95

Phe Cys Ala Arg Gly Tyr Val Asp Tyr Gly Gly Ala Thr Asp Leu Trp
            100                 105                 110

Gly Gln Gly Thr Leu Val Thr Val Ser Ser
        115                 120

<210>  49
<211>  11
<212>  PRT
<213>  Artificial Sequence

<220>
<223>  artificial antibody-based sequence

<400>  49

Gln Ala Ser Gln Ser Ile Gly Thr Tyr Leu Ala
1               5                   10

<210>  50
<211>  7
<212>  PRT
<213>  Artificial Sequence

<220>
<223>  artificial antibody-based sequence

<400>  50

Arg Ala Phe Ile Leu Ala Ser
1               5


<210>  51
<211>  15
<212>  PRT
<213>  Artificial Sequence

<220>
<223>  artificial antibody-based sequence

<400>  51

Gln Ser Asn Phe Tyr Ser Asp Ser Thr Thr Ile Gly Pro Asn Ala
1               5                   10                  15


<210>  52
<211>  114
<212>  PRT
<213>  Artificial Sequence

<220>
<223>  artificial antibody-based sequence

<400>  52

Asp Ile Gln Met Thr Gln Ser Pro Ser Ser Leu Ser Ala Ser Val Gly
1               5                   10                  15


Asp Arg Val Thr Ile Thr Cys Gln Ala Ser Gln Ser Ile Gly Thr Tyr
            20                  25                  30


Leu Ala Trp Tyr Gln Gln Lys Pro Gly Lys Ala Pro Lys Leu Leu Ile
        35                  40                  45


Tyr Arg Ala Phe Ile Leu Ala Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly

<210>  53
<211>  114
<212>  PRT
<213>  Artificial Sequence

<220>
<223>  artificial antibody-based sequence

<400>  53

Asp Ile Gln Met Thr Gln Ser Pro Ala Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Pro Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly

<210>  54
<211>  256
<212>  PRT
<213>  Artificial Sequence

<220>
<223>  artificial antibody-based sequence

<400>  54

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Ala Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Gly Gly Gly Gly Ser Gly Gly Gly Gly
        115                 120                 125

Ser Gly Gly Gly Gly Ser Gln Val Gln Leu Gln Glu Ser Gly Pro Gly
    130                 135                 140

Leu Val Lys Pro Ser Glu Thr Leu Ser Leu Thr Cys Lys Val Ser Gly
145                 150                 155                 160

Phe Ser Phe Asn Ser Asp Tyr Trp Ile Tyr Trp Ile Arg Gln Pro Pro
                165                 170                 175

Gly Lys Gly Leu Glu Trp Ile Gly Ser Ile Tyr Gly Gly Ser Ser Gly
            180                 185                 190

Asn Thr Gln Tyr Ala Ser Trp Ala Gln Gly Arg Val Thr Ile Ser Val
        195                 200                 205

Asp Ser Ser Lys Asn Gln Phe Ser Leu Lys Leu Ser Ser Val Thr Ala
    210                 215                 220

Ala Asp Thr Ala Val Tyr Tyr Cys Ala Arg Gly Tyr Val Asp Tyr Gly
225                 230                 235                 240

Gly Ala Thr Asp Leu Trp Gly Gln Gly Thr Leu Val Thr Val Ser Ser
                245                 250                 255

<210>  55
<211>  256

<212>  PRT
<213>  Artificial Sequence

<220>
<223>  artificial antibody-based sequence

<400>  55

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Ala Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Gly Gly Gly Gly Ser Gly Gly Gly Gly
        115                 120                 125

Ser Gly Gly Gly Gly Ser Gln Val Gln Leu Val Gln Ser Gly Ala Glu
    130                 135                 140

Val Lys Lys Pro Gly Ala Ser Val Lys Val Ser Cys Lys Ala Ser Gly
145                 150                 155                 160

Phe Ser Phe Asn Ser Asp Tyr Trp Ile Tyr Trp Val Arg Gln Ala Pro
                165                 170                 175

Gly Gln Gly Leu Glu Trp Met Gly Ser Ile Tyr Gly Gly Ser Ser Gly
            180                 185                 190

Asn Thr Gln Tyr Ala Ser Trp Ala Gln Gly Arg Val Thr Met Thr Arg
        195                 200                 205

Asp Thr Ser Ile Ser Thr Ala Tyr Met Glu Leu Ser Ser Leu Arg Ser
    210                 215                 220

Glu Asp Thr Ala Val Tyr Tyr Cys Ala Arg Gly Tyr Val Asp Tyr Gly
225                 230                 235                 240

Gly Ala Thr Asp Leu Trp Gly Gln Gly Thr Leu Val Thr Val Ser Ser
                245                 250                 255

<210>  56
<211>  256
<212>  PRT
<213>  Artificial Sequence

<220>
<223>  artificial antibody-based sequence

<400>  56

Asp Ile Gln Met Thr Gln Ser Pro Ala Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Pro Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Gly Gly Gly Gly Ser Gly Gly Gly Gly
        115                 120                 125

Ser Gly Gly Gly Gly Ser Glu Val Gln Leu Val Glu Ser Gly Gly Gly
    130                 135                 140

Leu Val Gln Pro Gly Gly Ser Leu Arg Leu Ser Cys Ala Ala Ser Gly
145                 150                 155                 160

Phe Ser Phe Asn Ser Asp Tyr Trp Ile Tyr Trp Val Arg Gln Ala Pro
                165                 170                 175

Gly Lys Gly Leu Glu Trp Ile Ala Ser Ile Tyr Gly Gly Ser Ser Gly
                180                 185                 190

Asn Thr Gln Tyr Ala Ser Trp Ala Gln Gly Arg Phe Thr Ile Ser Arg
        195                 200                 205

Asp Asn Ser Lys Asn Thr Val Tyr Leu Gln Met Asn Ser Leu Arg Ala
    210                 215                 220

Glu Asp Thr Ala Val Tyr Phe Cys Ala Arg Gly Tyr Val Asp Tyr Gly
225                 230                 235                 240

Gly Ala Thr Asp Leu Trp Gly Gln Gly Thr Leu Val Thr Val Ser Ser
                245                 250                 255

<210>  57
<211>  11
<212>  PRT
<213>  Artificial Sequence

<220>
<223>  artificial antibody-based sequence

<400>  57

Gly Phe Ser Phe Ser Ser Gly Tyr Asp Met Cys
1               5                   10

<210>  58
<211>  18
<212>  PRT
<213>  Artificial Sequence

<220>
<223>  artificial antibody-based sequence

<400>  58

Cys Val Val Ala Gly Ser Val Asp Ile Thr Tyr Tyr Ala Ser Trp Ala
1               5                   10                  15

Lys Gly

<210>  59
<211>  11
<212>  PRT
<213>  Artificial Sequence

<220>
<223>  artificial antibody-based sequence

<400>  59

Arg Lys Asp Ala Tyr Ser Asp Ala Phe Asn Leu

1               5                   10


<210>  60
<211>  121
<212>  PRT
<213>  Artificial Sequence

<220>
<223>  artificial antibody-based sequence

<400>  60

Gln Val Gln Leu Gln Glu Ser Gly Pro Gly Leu Val Lys Pro Ser Glu
1               5                   10                  15


Thr Leu Ser Leu Thr Cys Lys Val Ser Gly Phe Ser Phe Ser Ser Gly
            20                  25                  30


Tyr Asp Met Cys Trp Ile Arg Gln Pro Pro Gly Lys Gly Leu Glu Trp
        35                  40                  45


Ile Gly Cys Val Val Ala Gly Ser Val Asp Ile Thr Tyr Tyr Ala Ser
    50                  55                  60


Trp Ala Lys Gly Arg Val Thr Ile Ser Val Asp Ser Ser Lys Asn Gln
65                  70                  75                  80


Phe Ser Leu Lys Leu Ser Ser Val Thr Ala Ala Asp Thr Ala Val Tyr
                85                  90                  95


Tyr Cys Ala Arg Lys Asp Ala Tyr Ser Asp Ala Phe Asn Leu Trp Gly
            100                 105                 110


Gln Gly Thr Leu Val Thr Val Ser Ser
        115                 120


<210>  61
<211>  121
<212>  PRT
<213>  Artificial Sequence

<220>
<223>  artificial antibody-based sequence

<400>  61

Gln Ser Gln Leu Gln Glu Ser Gly Pro Gly Leu Val Lys Pro Ser Glu
1               5                   10                  15


Thr Leu Ser Leu Thr Cys Lys Ala Ser Gly Phe Ser Phe Ser Ser Gly
            20                  25                  30

Tyr Asp Met Cys Trp Val Arg Gln Pro Pro Gly Lys Gly Leu Glu Trp
        35                  40                  45

Ile Ala Cys Val Val Ala Gly Ser Val Asp Ile Thr Tyr Tyr Ala Ser
    50                  55                  60

Trp Ala Lys Gly Arg Val Thr Ile Ser Lys Asp Ser Ser Lys Asn Gln
65                  70                  75                  80

Val Ser Leu Lys Leu Ser Ser Val Thr Ala Ala Asp Thr Ala Val Tyr
                85                  90                  95

Phe Cys Ala Arg Lys Asp Ala Tyr Ser Asp Ala Phe Asn Leu Trp Gly
            100                 105                 110

Gln Gly Thr Leu Val Thr Val Ser Ser
        115                 120

<210>  62
<211>  121
<212>  PRT
<213>  Artificial Sequence

<220>
<223>  artificial antibody-based sequence

<400>  62

Gln Val Gln Leu Gln Glu Ser Gly Pro Gly Leu Val Lys Pro Ser Glu
1               5                   10                  15

Thr Leu Ser Leu Thr Cys Lys Ala Ser Gly Phe Ser Phe Ser Ser Gly
            20                  25                  30

Tyr Asp Met Cys Trp Val Arg Gln Pro Pro Gly Lys Gly Leu Glu Trp
        35                  40                  45

Ile Ala Cys Val Val Ala Gly Ser Val Asp Ile Thr Tyr Tyr Ala Ser
    50                  55                  60

Trp Ala Lys Gly Arg Val Thr Ile Ser Lys Asp Ser Ser Lys Asn Gln
65                  70                  75                  80

Val Ser Leu Lys Leu Ser Ser Val Thr Ala Ala Asp Thr Ala Val Tyr
                85                  90                  95

Tyr Cys Ala Arg Lys Asp Ala Tyr Ser Asp Ala Phe Asn Leu Trp Gly
            100                 105                 110

Gln Gly Thr Leu Val Thr Val Ser Ser

115                 120

<210>  63
<211>  11
<212>  PRT
<213>  Artificial Sequence

<220>
<223>  artificial antibody-based sequence

<400>  63

Gln Ala Ser Gln Ser Ile Asn Asp Tyr Leu Ala
1               5                   10

<210>  64
<211>  7
<212>  PRT
<213>  Artificial Sequence

<220>
<223>  artificial antibody-based sequence

<400>  64

Lys Ala Ser Thr Leu Ala Ser
1               5

<210>  65
<211>  12
<212>  PRT
<213>  Artificial Sequence

<220>
<223>  artificial antibody-based sequence

<400>  65

Gln Gln Gly Tyr Ile Ile Thr Asp Ile Asp Asn Val
1               5                   10

<210>  66
<211>  111
<212>  PRT
<213>  Artificial Sequence

<220>
<223>  artificial antibody-based sequence

<400>  66

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly
            100                 105                 110

<210>  67
<211>  111
<212>  PRT
<213>  Artificial Sequence

<220>
<223>  artificial antibody-based sequence

<400>  67

Asp Phe Gln Leu Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ser Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly
            100                 105                 110

<210>  68
<211>  252
<212>  PRT
<213>  Artificial Sequence

<220>
<223>  artificial antibody-based sequence

<400>  68

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gly Gly Gly Gly Ser Gly Gly Gly Gly Ser Gly Gly
        115                 120                 125

Gly Gly Ser Gln Val Gln Leu Gln Glu Ser Gly Pro Gly Leu Val Lys
    130                 135                 140

Pro Ser Glu Thr Leu Ser Leu Thr Cys Lys Val Ser Gly Phe Ser Phe
145                 150                 155                 160

Ser Ser Gly Tyr Asp Met Cys Trp Ile Arg Gln Pro Pro Gly Lys Gly
                165                 170                 175

Leu Glu Trp Ile Gly Cys Val Val Ala Gly Ser Val Asp Ile Thr Tyr
            180                 185                 190

Tyr Ala Ser Trp Ala Lys Gly Arg Val Thr Ile Ser Val Asp Ser Ser
        195                 200                 205

Lys Asn Gln Phe Ser Leu Lys Leu Ser Ser Val Thr Ala Ala Asp Thr
    210                 215                 220

Ala Val Tyr Tyr Cys Ala Arg Lys Asp Ala Tyr Ser Asp Ala Phe Asn
225                 230                 235                 240

Leu Trp Gly Gln Gly Thr Leu Val Thr Val Ser Ser
                245                 250

<210>  69
<211>  252
<212>  PRT
<213>  Artificial Sequence

<220>
<223>  artificial antibody-based sequence

<400>  69

Asp Phe Gln Leu Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ser Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gly Gly Gly Gly Ser Gly Gly Gly Gly Ser Gly Gly
        115                 120                 125

Gly Gly Ser Gln Ser Gln Leu Gln Glu Ser Gly Pro Gly Leu Val Lys
    130                 135                 140

Pro Ser Glu Thr Leu Ser Leu Thr Cys Lys Ala Ser Gly Phe Ser Phe
145                 150                 155                 160

Ser Ser Gly Tyr Asp Met Cys Trp Val Arg Gln Pro Pro Gly Lys Gly
                165                 170                 175

Leu Glu Trp Ile Ala Cys Val Val Ala Gly Ser Val Asp Ile Thr Tyr
            180                 185                 190

Tyr Ala Ser Trp Ala Lys Gly Arg Val Thr Ile Ser Lys Asp Ser Ser
        195                 200                 205

Lys Asn Gln Val Ser Leu Lys Leu Ser Ser Val Thr Ala Ala Asp Thr
    210                 215                 220

Ala Val Tyr Phe Cys Ala Arg Lys Asp Ala Tyr Ser Asp Ala Phe Asn
225                 230                 235                 240

Leu Trp Gly Gln Gly Thr Leu Val Thr Val Ser Ser
                245                 250

<210>  70
<211>  252
<212>  PRT
<213>  Artificial Sequence

<220>
<223>  artificial antibody-based sequence

<400>  70

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gly Gly Gly Gly Ser Gly Gly Gly Gly Ser Gly Gly
        115                 120                 125

Gly Gly Ser Gln Val Gln Leu Gln Glu Ser Gly Pro Gly Leu Val Lys
    130                 135                 140

Pro Ser Glu Thr Leu Ser Leu Thr Cys Lys Ala Ser Gly Phe Ser Phe
145                 150                 155                 160

Ser Ser Gly Tyr Asp Met Cys Trp Val Arg Gln Pro Pro Gly Lys Gly
                165                 170                 175

Leu Glu Trp Ile Ala Cys Val Val Ala Gly Ser Val Asp Ile Thr Tyr
            180                 185                 190

Tyr Ala Ser Trp Ala Lys Gly Arg Val Thr Ile Ser Lys Asp Ser Ser
        195                 200                 205

Lys Asn Gln Val Ser Leu Lys Leu Ser Ser Val Thr Ala Ala Asp Thr
    210                 215                 220

Ala Val Tyr Tyr Cys Ala Arg Lys Asp Ala Tyr Ser Asp Ala Phe Asn
225                 230                 235                 240

Leu Trp Gly Gln Gly Thr Leu Val Thr Val Ser Ser
                245                 250

<210>  71
<211>  10
<212>  PRT
<213>  Artificial Sequence

<220>
<223>  artificial antibody-based sequence

<400>  71

Gly Phe Ser Leu Ser Ser Asn Ala Met Gly
1               5                   10

<210>  72
<211>  16
<212>  PRT
<213>  Artificial Sequence

<220>
<223>  artificial antibody-based sequence

<400>  72

Ile Ile Ser Val Gly Gly Phe Thr Tyr Tyr Ala Ser Trp Ala Lys Gly
1               5                   10                  15

<210>  73
<211>  14
<212>  PRT
<213>  Artificial Sequence

<220>
<223>  artificial antibody-based sequence

<400>  73

Arg Asp Arg His Gly Gly Asp Ser Ser Gly Ala Phe Tyr Leu
1               5                   10


<210>  74
<211>  121
<212>  PRT
<213>  Artificial Sequence

<220>
<223>  artificial antibody-based sequence

<400>  74

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ser Leu Ser Ser Asn
            20                  25                  30


Ala Met Gly Trp Val Arg Gln Ala Pro Gly Lys Gly Leu Glu Tyr Ile
        35                  40                  45


Gly Ile Ile Ser Val Gly Gly Phe Thr Tyr Tyr Ala Ser Trp Ala Lys
    50                  55                  60


Gly Arg Phe Thr Ile Ser Arg Asp Asn Ser Lys Asn Thr Val Tyr Leu
65                  70                  75                  80


Gln Met Asn Ser Leu Arg Ala Glu Asp Thr Ala Thr Tyr Phe Cys Ala
                85                  90                  95


Arg Asp Arg His Gly Gly Asp Ser Ser Gly Ala Phe Tyr Leu Trp Gly
            100                 105                 110


Gln Gly Thr Leu Val Thr Val Ser Ser
        115                 120


<210>  75
<211>  13
<212>  PRT
<213>  Artificial Sequence

<220>
<223>  artificial antibody-based sequence

<400>  75

Gln Ser Ser Glu Ser Val Tyr Ser Asn Asn Gln Leu Ser
1               5                   10

<210>  76
<211>  7
<212>  PRT
<213>  Artificial Sequence

<220>
<223>  artificial antibody-based sequence

<400>  76

Asp Ala Ser Asp Leu Ala Ser
1               5


<210>  77
<211>  11
<212>  PRT
<213>  Artificial Sequence

<220>
<223>  artificial antibody-based sequence

<400>  77

Ala Gly Gly Phe Ser Ser Ser Ser Asp Thr Ala
1               5                   10


<210>  78
<211>  112
<212>  PRT
<213>  Artificial Sequence

<220>
<223>  artificial antibody-based sequence

<400>  78

Asp Ile Gln Met Thr Gln Ser Pro Ser Ser Leu Ser Ala Ser Val Gly
1               5                   10                  15


Asp Arg Val Thr Ile Thr Cys Gln Ser Ser Glu Ser Val Tyr Ser Asn
            20                  25                  30


Asn Gln Leu Ser Trp Tyr Gln Gln Lys Pro Gly Gln Pro Pro Lys Leu
        35                  40                  45


Leu Ile Tyr Asp Ala Ser Asp Leu Ala Ser Gly Val Pro Ser Arg Phe
    50                  55                  60


Ser Gly Ser Gly Ser Gly Thr Asp Phe Thr Leu Thr Ile Ser Ser Leu
65                  70                  75                  80


Gln Pro Glu Asp Phe Ala Thr Tyr Tyr Cys Ala Gly Gly Phe Ser Ser
                85                  90                  95


Ser Ser Asp Thr Ala Phe Gly Gly Gly Thr Lys Leu Thr Val Leu Gly

            100                 105                 110

<210> 79
<211> 253
<212> PRT
<213> Artificial Sequence

<220>
<223> artificial antibody-based sequence

<400> 79

Asp Ile Gln Met Thr Gln Ser Pro Ser Ser Leu Ser Ala Ser Val Gly
1               5                   10                  15

Asp Arg Val Thr Ile Thr Cys Gln Ser Ser Glu Ser Val Tyr Ser Asn
            20                  25                  30

Asn Gln Leu Ser Trp Tyr Gln Gln Lys Pro Gly Gln Pro Pro Lys Leu
        35                  40                  45

Leu Ile Tyr Asp Ala Ser Asp Leu Ala Ser Gly Val Pro Ser Arg Phe
    50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Ala Gly Gly Phe Ser Ser
                85                  90                  95

Ser Ser Asp Thr Ala Phe Gly Gly Gly Thr Lys Leu Thr Val Leu Gly
            100                 105                 110

Gly Gly Gly Gly Ser Gly Gly Gly Gly Ser Gly Gly Gly Gly Ser Gly
        115                 120                 125

Gly Gly Gly Ser Glu Val Gln Leu Val Glu Ser Gly Gly Gly Leu Val
    130                 135                 140

Gln Pro Gly Gly Ser Leu Arg Leu Ser Cys Ala Ala Ser Gly Phe Ser
145                 150                 155                 160

Leu Ser Ser Asn Ala Met Gly Trp Val Arg Gln Ala Pro Gly Lys Gly
                165                 170                 175

Leu Glu Tyr Ile Gly Ile Ile Ser Val Gly Gly Phe Thr Tyr Tyr Ala
            180                 185                 190

Ser Trp Ala Lys Gly Arg Phe Thr Ile Ser Arg Asp Asn Ser Lys Asn
        195                 200                 205

Thr Val Tyr Leu Gln Met Asn Ser Leu Arg Ala Glu Asp Thr Ala Thr
    210                 215                 220

Tyr Phe Cys Ala Arg Asp Arg His Gly Gly Asp Ser Ser Gly Ala Phe
225                 230                 235                 240

Tyr Leu Trp Gly Gln Gly Thr Leu Val Thr Val Ser Ser
                245                 250

<210>  80
<211>  11
<212>  PRT
<213>  Artificial Sequence

<220>
<223>  artificial antibody-based sequence

<400>  80

Gly Phe Ser Phe Ser Ser Ser Tyr Trp Ile Cys
1               5                   10

<210>  81
<211>  17
<212>  PRT
<213>  Artificial Sequence

<220>
<223>  artificial antibody-based sequence

<400>  81

Cys Val Phe Thr Gly Asp Gly Thr Thr Tyr Tyr Ala Ser Trp Ala Lys
1               5                   10                  15

Gly

<210>  82
<211>  12
<212>  PRT
<213>  Artificial Sequence

<220>
<223>  artificial antibody-based sequence

<400>  82

Arg Pro Val Ser Val Tyr Tyr Tyr Gly Met Asp Leu
1               5                   10

<210>  83
<211>  121
<212>  PRT

<213>  Artificial Sequence

<220>
<223>  artificial antibody-based sequence

<400>  83

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Ser Ser Ser
            20                  25                  30

Tyr Trp Ile Cys Trp Val Arg Gln Ala Pro Gly Lys Gly Leu Glu Trp
        35                  40                  45

Val Gly Cys Val Phe Thr Gly Asp Gly Thr Thr Tyr Tyr Ala Ser Trp
    50                  55                  60

Ala Lys Gly Arg Phe Thr Ile Ser Arg Asp Asn Ser Lys Asn Thr Val
65                  70                  75                  80

Tyr Leu Gln Met Asn Ser Leu Arg Ala Glu Asp Thr Ala Thr Tyr Phe
                85                  90                  95

Cys Ala Arg Pro Val Ser Val Tyr Tyr Tyr Gly Met Asp Leu Trp Gly
            100                 105                 110

Gln Gly Thr Leu Val Thr Val Ser Ser
        115                 120


<210>  84
<211>  11
<212>  PRT
<213>  Artificial Sequence

<220>
<223>  artificial antibody-based sequence

<400>  84

Gln Ala Ser Gln Ile Ile Ser Ser Arg Ser Ala
1               5                   10


<210>  85
<211>  7
<212>  PRT
<213>  Artificial Sequence

<220>
<223>  artificial antibody-based sequence

<400>  85

Gln Ala Ser Lys Leu Ala Ser
1               5

<210>  86
<211>  11
<212>  PRT
<213>  Artificial Sequence

<220>
<223>  artificial antibody-based sequence

<400>  86

Gln Cys Thr Tyr Ile Asp Ser Asn Phe Gly Ala
1               5                   10

<210>  87
<211>  110
<212>  PRT
<213>  Artificial Sequence

<220>
<223>  artificial antibody-based sequence

<400>  87

Asp Val Val Met Thr Gln Ser Pro Ser Ser Leu Ser Ala Ser Val Gly
1               5                   10                  15

Asp Arg Val Thr Ile Thr Cys Gln Ala Ser Gln Ile Ile Ser Ser Arg
            20                  25                  30

Ser Ala Trp Tyr Gln Gln Lys Pro Gly Gln Pro Pro Lys Leu Leu Ile
        35                  40                  45

Tyr Gln Ala Ser Lys Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Cys Thr Tyr Ile Asp Ser Asn
                85                  90                  95

Phe Gly Ala Phe Gly Gly Gly Thr Lys Leu Thr Val Leu Gly
            100                 105                 110

<210>  88
<211>  251
<212>  PRT
<213>  Artificial Sequence

<220>
<223>  artificial antibody-based sequence

<400>  88

Asp Val Val Met Thr Gln Ser Pro Ser Ser Leu Ser Ala Ser Val Gly
1               5                   10                  15

Asp Arg Val Thr Ile Thr Cys Gln Ala Ser Gln Ile Ile Ser Ser Arg
            20                  25                  30

Ser Ala Trp Tyr Gln Gln Lys Pro Gly Gln Pro Pro Lys Leu Leu Ile
        35                  40                  45

Tyr Gln Ala Ser Lys Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Cys Thr Tyr Ile Asp Ser Asn
                85                  90                  95

Phe Gly Ala Phe Gly Gly Gly Thr Lys Leu Thr Val Leu Gly Gly Gly
            100                 105                 110

Gly Gly Ser Gly Gly Gly Gly Ser Gly Gly Gly Gly Ser Gly Gly Gly
        115                 120                 125

Gly Ser Glu Val Gln Leu Val Glu Ser Gly Gly Gly Leu Val Gln Pro
    130                 135                 140

Gly Gly Ser Leu Arg Leu Ser Cys Ala Ala Ser Gly Phe Ser Phe Ser
145                 150                 155                 160

Ser Ser Tyr Trp Ile Cys Trp Val Arg Gln Ala Pro Gly Lys Gly Leu
                165                 170                 175

Glu Trp Val Gly Cys Val Phe Thr Gly Asp Gly Thr Thr Tyr Tyr Ala
            180                 185                 190

Ser Trp Ala Lys Gly Arg Phe Thr Ile Ser Arg Asp Asn Ser Lys Asn
        195                 200                 205

Thr Val Tyr Leu Gln Met Asn Ser Leu Arg Ala Glu Asp Thr Ala Thr
    210                 215                 220

Tyr Phe Cys Ala Arg Pro Val Ser Val Tyr Tyr Tyr Gly Met Asp Leu
225                 230                 235                 240

Trp Gly Gln Gly Thr Leu Val Thr Val Ser Ser
                245                 250

<210>  89
<211>  255
<212>  PRT
<213>  Homo sapiens

<400>  89

Met Gly Asn Ser Cys Tyr Asn Ile Val Ala Thr Leu Leu Leu Val Leu
1               5                   10                  15

Asn Phe Glu Arg Thr Arg Ser Leu Gln Asp Pro Cys Ser Asn Cys Pro
            20                  25                  30

Ala Gly Thr Phe Cys Asp Asn Asn Arg Asn Gln Ile Cys Ser Pro Cys
        35                  40                  45

Pro Pro Asn Ser Phe Ser Ser Ala Gly Gly Gln Arg Thr Cys Asp Ile
    50                  55                  60

Cys Arg Gln Cys Lys Gly Val Phe Arg Thr Arg Lys Glu Cys Ser Ser
65                  70                  75                  80

Thr Ser Asn Ala Glu Cys Asp Cys Thr Pro Gly Phe His Cys Leu Gly
                85                  90                  95

Ala Gly Cys Ser Met Cys Glu Gln Asp Cys Lys Gln Gly Gln Glu Leu
            100                 105                 110

Thr Lys Lys Gly Cys Lys Asp Cys Cys Phe Gly Thr Phe Asn Asp Gln
        115                 120                 125

Lys Arg Gly Ile Cys Arg Pro Trp Thr Asn Cys Ser Leu Asp Gly Lys
    130                 135                 140

Ser Val Leu Val Asn Gly Thr Lys Glu Arg Asp Val Val Cys Gly Pro
145                 150                 155                 160

Ser Pro Ala Asp Leu Ser Pro Gly Ala Ser Ser Val Thr Pro Pro Ala
                165                 170                 175

Pro Ala Arg Glu Pro Gly His Ser Pro Gln Ile Ile Ser Phe Phe Leu
            180                 185                 190

Ala Leu Thr Ser Thr Ala Leu Leu Phe Leu Leu Phe Phe Leu Thr Leu
        195                 200                 205

Arg Phe Ser Val Val Lys Arg Gly Arg Lys Lys Leu Leu Tyr Ile Phe
    210                 215                 220

Lys Gln Pro Phe Met Arg Pro Val Gln Thr Thr Gln Glu Glu Asp Gly
225                 230                 235                 240

Cys Ser Cys Arg Phe Pro Glu Glu Glu Glu Gly Gly Cys Glu Leu
                245                 250                 255

<210>  90
<211>  290
<212>  PRT
<213>  Homo sapiens

<400>  90

Met Arg Ile Phe Ala Val Phe Ile Phe Met Thr Tyr Trp His Leu Leu
1               5                   10                  15

Asn Ala Phe Thr Val Thr Val Pro Lys Asp Leu Tyr Val Val Glu Tyr
            20                  25                  30

Gly Ser Asn Met Thr Ile Glu Cys Lys Phe Pro Val Glu Lys Gln Leu
        35                  40                  45

Asp Leu Ala Ala Leu Ile Val Tyr Trp Glu Met Glu Asp Lys Asn Ile
    50                  55                  60

Ile Gln Phe Val His Gly Glu Glu Asp Leu Lys Val Gln His Ser Ser
65                  70                  75                  80

Tyr Arg Gln Arg Ala Arg Leu Leu Lys Asp Gln Leu Ser Leu Gly Asn
                85                  90                  95

Ala Ala Leu Gln Ile Thr Asp Val Lys Leu Gln Asp Ala Gly Val Tyr
            100                 105                 110

Arg Cys Met Ile Ser Tyr Gly Gly Ala Asp Tyr Lys Arg Ile Thr Val
        115                 120                 125

Lys Val Asn Ala Pro Tyr Asn Lys Ile Asn Gln Arg Ile Leu Val Val
    130                 135                 140

Asp Pro Val Thr Ser Glu His Glu Leu Thr Cys Gln Ala Glu Gly Tyr
145                 150                 155                 160

Pro Lys Ala Glu Val Ile Trp Thr Ser Ser Asp His Gln Val Leu Ser
                165                 170                 175

Gly Lys Thr Thr Thr Thr Asn Ser Lys Arg Glu Glu Lys Leu Phe Asn
            180                 185                 190

Val Thr Ser Thr Leu Arg Ile Asn Thr Thr Thr Asn Glu Ile Phe Tyr
        195                 200                 205

Cys Thr Phe Arg Arg Leu Asp Pro Glu Glu Asn His Thr Ala Glu Leu
    210                 215                 220

Val Ile Pro Glu Leu Pro Leu Ala His Pro Pro Asn Glu Arg Thr His
225                 230                 235                 240

Leu Val Ile Leu Gly Ala Ile Leu Leu Cys Leu Gly Val Ala Leu Thr
                245                 250                 255

Phe Ile Phe Arg Leu Arg Lys Gly Arg Met Met Asp Val Lys Lys Cys
            260                 265                 270

Gly Ile Gln Asp Thr Asn Ser Lys Lys Gln Ser Asp Thr His Leu Glu
        275                 280                 285

Glu Thr
    290

<210>  91
<211>  122
<212>  PRT
<213>  Artificial Sequence

<220>
<223>  artificial antibody-based sequence

<400>  91

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Ser Ala Asn
            20                  25                  30

Tyr Tyr Pro Cys Trp Val Arg Gln Ala Pro Gly Lys Gly Leu Glu Trp
        35                  40                  45

Ile Gly Cys Ile Tyr Gly Gly Ser Ser Asp Ile Thr Tyr Asp Ala Asn
    50                  55                  60

Trp Thr Lys Gly Arg Phe Thr Ile Ser Arg Asp Asn Ser Lys Asn Thr
65                  70                  75                  80

Val Tyr Leu Gln Met Asn Ser Leu Arg Ala Glu Asp Thr Ala Val Tyr

85                  90                  95

Tyr Cys Ala Arg Ser Ala Trp Tyr Ser Gly Trp Gly Gly Asp Leu Trp
            100                 105                 110

Gly Gln Gly Thr Leu Val Thr Val Ser Ser
        115                 120

<210>  92
<211>  124
<212>  PRT
<213>  Artificial Sequence

<220>
<223>  artificial antibody-based sequence

<400>  92

Gln Val Gln Leu Gln Glu Ser Gly Pro Gly Leu Val Lys Pro Ser Glu
1               5                   10                  15

Thr Leu Ser Leu Thr Cys Lys Val Ser Gly Phe Ser Phe Ser Asn Ser
            20                  25                  30

Tyr Trp Ile Cys Trp Ile Arg Gln Pro Pro Gly Lys Gly Leu Glu Trp
        35                  40                  45

Ile Gly Cys Thr Phe Val Gly Ser Ser Asp Ser Thr Tyr Tyr Ala Asn
    50                  55                  60

Trp Ala Lys Gly Arg Val Thr Ile Ser Val Asp Ser Ser Lys Asn Gln
65                  70                  75                  80

Phe Ser Leu Lys Leu Ser Ser Val Thr Ala Ala Asp Thr Ala Val Tyr
                85                  90                  95

Tyr Cys Ala Arg His Pro Ser Asp Ala Val Tyr Gly Tyr Ala Asn Asn
            100                 105                 110

Leu Trp Gly Gln Gly Thr Leu Val Thr Val Ser Ser
        115                 120

<210>  93
<211>  108
<212>  PRT
<213>  Artificial Sequence

<220>
<223>  artificial antibody-based sequence

<400>  93

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Thr Gly Thr Lys Val Thr Val Leu Gly
            100                 105

<210>  94
<211>  11
<212>  PRT
<213>  Artificial Sequence

<220>
<223>  artificial antibody-based sequence

<400>  94

Phe Gly Thr Gly Thr Lys Val Thr Val Leu Gly
1               5                   10

<210>  95
<211>  11
<212>  PRT
<213>  Artificial Sequence

<220>
<223>  artificial antibody-based sequence

<400>  95

Phe Gly Gly Gly Thr Lys Leu Thr Val Leu Gly
1               5                   10

<210>  96
<211>  11
<212>  PRT
<213>  Artificial Sequence

<220>
<223>  artificial antibody-based sequence

<400>  96

Phe Gly Gly Gly Thr Gln Leu Ile Ile Leu Gly
1               5                   10


<210>  97
<211>  11
<212>  PRT
<213>  Artificial Sequence

<220>
<223>  artificial antibody-based sequence

<400>  97

Phe Gly Glu Gly Thr Glu Leu Thr Val Leu Gly
1               5                   10


<210>  98
<211>  11
<212>  PRT
<213>  Artificial Sequence

<220>
<223>  artificial antibody-based sequence

<400>  98

Phe Gly Ser Gly Thr Lys Val Thr Val Leu Gly
1               5                   10


<210>  99
<211>  11
<212>  PRT
<213>  Artificial Sequence

<220>
<223>  artificial antibody-based sequence

<400>  99

Phe Gly Gly Gly Thr Gln Leu Thr Val Leu Gly
1               5                   10


<210>  100
<211>  11
<212>  PRT
<213>  Artificial Sequence

<220>
<223>  artificial antibody-based sequence

<400>  100

Phe Gly Gly Gly Thr Gln Leu Thr Ala Leu Gly
1               5                   10

<210>  101
<211>  11
<212>  PRT
<213>  Artificial Sequence

<220>
<223>  artificial antibody-based sequence

<400>  101

Phe Gly Cys Gly Thr Lys Val Thr Val Leu Gly
1               5                   10


<210>  102
<211>  20
<212>  PRT
<213>  Artificial Sequence

<220>
<223>  artificial antibody-based sequence

<400>  102

Gly Gly Gly Gly Ser Gly Gly Gly Gly Ser Gly Gly Gly Gly Ser Gly
1               5                   10                  15


Gly Gly Gly Ser
            20


<210>  103
<211>  5
<212>  PRT
<213>  Artificial Sequence

<220>
<223>  artificial antibody-based sequence

<400>  103

Gly Gly Gly Gly Ser
1               5


<210>  104
<211>  10
<212>  PRT
<213>  Artificial Sequence

<220>
<223>  artificial antibody-based sequence

<400>  104

Gly Gly Gly Gly Ser Gly Gly Gly Gly Ser
1               5                   10


<210>  105
<211>  493
<212>  PRT

<213>  Artificial Sequence

<220>
<223>  artificial antibody-based sequence

<400>  105

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Val Ser Gly Phe Ser
    130                 135                 140

Phe Ser Asn Ser Tyr Trp Ile Cys Trp Ile Arg Gln Pro Pro Gly Lys
145                 150                 155                 160

Gly Leu Glu Trp Ile Gly Cys Thr Phe Val Gly Ser Ser Asp Ser Thr
                165                 170                 175

Tyr Tyr Ala Asn Trp Ala Lys Gly Arg Val Thr Ile Ser Val Asp Ser
            180                 185                 190

Ser Lys Asn Gln Phe Ser Leu Lys Leu Ser Ser Val Thr Ala Ala Asp
        195                 200                 205

Thr Ala Val Tyr Tyr Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Ala Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Thr Gly Thr Lys Val Thr Val Leu Gly Gly
        355                 360                 365

Gly Gly Gly Ser Gln Val Gln Leu Gln Glu Ser Gly Pro Gly Leu Val
    370                 375                 380

Lys Pro Ser Glu Thr Leu Ser Leu Thr Cys Lys Val Ser Gly Phe Ser
385                 390                 395                 400

Phe Ser Ser Gly Tyr Asp Met Cys Trp Ile Arg Gln Pro Pro Gly Lys
                405                 410                 415

Gly Leu Glu Trp Ile Gly Cys Val Val Ala Gly Ser Val Asp Ile Thr
            420                 425                 430

Tyr Tyr Ala Ser Trp Ala Lys Gly Arg Val Thr Ile Ser Val Asp Ser
        435                 440                 445

Ser Lys Asn Gln Phe Ser Leu Lys Leu Ser Ser Val Thr Ala Ala Asp
    450                 455                 460

Thr Ala Val Tyr Tyr Cys Ala Arg Lys Asp Ala Tyr Ser Asp Ala Phe
465                 470                 475                 480

Asn Leu Trp Gly Gln Gly Thr Leu Val Thr Val Ser Ser
                485                 490

<210>  106
<211>  499
<212>  PRT
<213>  Artificial Sequence

<220>
<223>  artificial antibody-based sequence

<400>  106

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Glu Val Gln Leu Val Glu Ser Gly Gly Gly Leu Val
        115                 120                 125

Gln Pro Gly Gly Ser Leu Arg Leu Ser Cys Ala Ala Ser Gly Phe Ser
    130                 135                 140

Phe Asn Asn Asp Tyr Asp Met Cys Trp Val Arg Gln Ala Pro Gly Lys
145                 150                 155                 160

Gly Leu Glu Trp Ile Gly Cys Ile Asp Thr Gly Asp Gly Ser Thr Tyr
                165                 170                 175

Tyr Ala Ser Trp Ala Lys Gly Arg Phe Thr Ile Ser Arg Asp Asn Ser
            180                 185                 190

Lys Asn Thr Val Tyr Leu Gln Met Asn Ser Leu Arg Ala Glu Asp Thr
        195                 200                 205

Ala Val Tyr Tyr Cys Ala Arg Glu Ala Ala Ser Ser Ser Gly Tyr Gly
    210                 215                 220

Met Gly Tyr Phe Asp Leu Trp Gly Gln Gly Thr Leu Val Thr Val Ser
225                 230                 235                 240

Ser Gly Gly Gly Gly Ser Gly Gly Gly Gly Ser Gly Gly Gly Gly Ser
                245                 250                 255

Gly Gly Gly Gly Ser Ile Gln Met Thr Gln Ser Pro Ser Ser Leu Ser
            260                 265                 270

Ala Ser Val Gly Asp Arg Val Thr Ile Thr Cys Gln Ser Ser Gln Ser
        275                 280                 285

Val Tyr Asp Asn Asn Trp Leu Ala Trp Tyr Gln Gln Lys Pro Gly Lys
    290                 295                 300

Ala Pro Lys Leu Leu Ile Tyr Arg Ala Ser Asn Leu Ala Ser Gly Val
305                 310                 315                 320

Pro Ser Arg Phe Ser Gly Ser Gly Ser Gly Thr Asp Phe Thr Leu Thr
                325                 330                 335

Ile Ser Ser Leu Gln Pro Glu Asp Phe Ala Thr Tyr Tyr Cys Gln Gly
            340                 345                 350

Thr Tyr Leu Ser Ser Asn Trp Tyr Trp Ala Phe Gly Thr Gly Thr Lys
        355                 360                 365

Val Thr Val Leu Gly Gly Gly Gly Gly Ser Gln Val Gln Leu Gln Glu
    370                 375                 380

Ser Gly Pro Gly Leu Val Lys Pro Ser Glu Thr Leu Ser Leu Thr Cys
385                 390                 395                 400

Lys Val Ser Gly Phe Ser Phe Ser Ser Gly Tyr Asp Met Cys Trp Ile
                405                 410                 415

Arg Gln Pro Pro Gly Lys Gly Leu Glu Trp Ile Gly Cys Val Val Ala
            420                 425                 430

Gly Ser Val Asp Ile Thr Tyr Tyr Ala Ser Trp Ala Lys Gly Arg Val
        435                 440                 445

Thr Ile Ser Val Asp Ser Ser Lys Asn Gln Phe Ser Leu Lys Leu Ser
    450                 455                 460

Ser Val Thr Ala Ala Asp Thr Ala Val Tyr Tyr Cys Ala Arg Lys Asp
465                 470                 475                 480

Ala Tyr Ser Asp Ala Phe Asn Leu Trp Gly Gln Gly Thr Leu Val Thr
                485                 490                 495

Val Ser Ser

<210>  107
<211>  493
<212>  PRT
<213>  Artificial Sequence

<220>
<223>  artificial antibody-based sequence

<400>  107

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Val Ser Gly Phe Ser
    130                 135                 140

Phe Ser Asn Ser Tyr Trp Ile Cys Trp Val Arg Gln Pro Pro Gly Lys
145                 150                 155                 160

Gly Leu Glu Trp Ile Gly Cys Thr Phe Val Gly Ser Ser Asp Ser Thr
                165                 170                 175

Tyr Tyr Ala Asn Trp Ala Lys Gly Arg Val Thr Ile Ser Val Asp Ser
            180                 185                 190

Ser Lys Asn Gln Val Ser Leu Lys Leu Ser Ser Val Thr Ala Ala Asp
        195                 200                 205

Thr Ala Val Tyr Phe Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Pro Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Thr Gly Thr Lys Val Thr Val Leu Gly Gly
        355                 360                 365

Gly Gly Gly Ser Gln Val Gln Leu Gln Glu Ser Gly Pro Gly Leu Val
    370                 375                 380

Lys Pro Ser Glu Thr Leu Ser Leu Thr Cys Lys Val Ser Gly Phe Ser
385                 390                 395                 400

Phe Ser Ser Gly Tyr Asp Met Cys Trp Ile Arg Gln Pro Pro Gly Lys
                405                 410                 415

Gly Leu Glu Trp Ile Gly Cys Val Val Ala Gly Ser Val Asp Ile Thr
            420                 425                 430

Tyr Tyr Ala Ser Trp Ala Lys Gly Arg Val Thr Ile Ser Val Asp Ser
        435                 440                 445

Ser Lys Asn Gln Phe Ser Leu Lys Leu Ser Ser Val Thr Ala Ala Asp
    450                 455                 460

Thr Ala Val Tyr Tyr Cys Ala Arg Lys Asp Ala Tyr Ser Asp Ala Phe
465                 470                 475                 480

Asn Leu Trp Gly Gln Gly Thr Leu Val Thr Val Ser Ser
                485                 490

<210> 108
<211> 493
<212> PRT
<213> Artificial Sequence

<220>
<223> artificial antibody-based sequence

<400> 108

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Ala Ser Gly Phe Ser
    130                 135                 140

Phe Ser Asn Ser Tyr Trp Ile Cys Trp Val Arg Gln Pro Pro Gly Lys
145                 150                 155                 160

Gly Leu Glu Trp Ile Gly Cys Thr Phe Val Gly Ser Ser Asp Ser Thr
                165                 170                 175

Tyr Tyr Ala Asn Trp Ala Lys Gly Arg Val Thr Ile Ser Lys Asp Ser
            180                 185                 190

Ser Lys Asn Gln Val Ser Leu Lys Leu Ser Ser Val Thr Ala Ala Asp
        195                 200                 205

Thr Ala Val Tyr Phe Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Leu Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Pro Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Thr Gly Thr Lys Val Thr Val Leu Gly Gly
        355                 360                 365

Gly Gly Gly Ser Gln Val Gln Leu Gln Glu Ser Gly Pro Gly Leu Val
    370                 375                 380

Lys Pro Ser Glu Thr Leu Ser Leu Thr Cys Lys Val Ser Gly Phe Ser
385                 390                 395                 400

Phe Ser Ser Gly Tyr Asp Met Cys Trp Ile Arg Gln Pro Pro Gly Lys
                405                 410                 415

Gly Leu Glu Trp Ile Gly Cys Val Val Ala Gly Ser Val Asp Ile Thr
            420                 425                 430

Tyr Tyr Ala Ser Trp Ala Lys Gly Arg Val Thr Ile Ser Val Asp Ser
        435                 440                 445

Ser Lys Asn Gln Phe Ser Leu Lys Leu Ser Ser Val Thr Ala Ala Asp
    450                 455                 460

Thr Ala Val Tyr Tyr Cys Ala Arg Lys Asp Ala Tyr Ser Asp Ala Phe
465                 470                 475                 480

Asn Leu Trp Gly Gln Gly Thr Leu Val Thr Val Ser Ser
                485                 490

<210>  109
<211>  493
<212>  PRT
<213>  Artificial Sequence

<220>
<223>  artificial antibody-based sequence

<400>  109

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ser Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Val Ser Gly Phe Ser
    130                 135                 140

Phe Ser Asn Ser Tyr Trp Ile Cys Trp Ile Arg Gln Pro Pro Gly Lys
145                 150                 155                 160

Gly Leu Glu Trp Ile Gly Cys Thr Phe Val Gly Ser Ser Asp Ser Thr
                165                 170                 175

Tyr Tyr Ala Asn Trp Ala Lys Gly Arg Val Thr Ile Ser Val Asp Ser
            180                 185                 190

Ser Lys Asn Gln Phe Ser Leu Lys Leu Ser Ser Val Thr Ala Ala Asp
        195                 200                 205

Thr Ala Val Tyr Tyr Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Ala Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
                340                 345                 350

Asn Tyr Gly Asp Phe Gly Thr Gly Thr Lys Val Thr Val Leu Gly Gly
            355                 360                 365

Gly Gly Gly Ser Gln Val Gln Leu Gln Glu Ser Gly Pro Gly Leu Val
    370                 375                 380

Lys Pro Ser Glu Thr Leu Ser Leu Thr Cys Lys Val Ser Gly Phe Ser
385                 390                 395                 400

Phe Ser Ser Gly Tyr Asp Met Cys Trp Val Arg Gln Pro Pro Gly Lys
                405                 410                 415

Gly Leu Glu Trp Ile Ala Cys Val Val Ala Gly Ser Val Asp Ile Thr
                420                 425                 430

Tyr Tyr Ala Ser Trp Ala Lys Gly Arg Val Thr Ile Ser Val Asp Ser
            435                 440                 445

Ser Lys Asn Gln Phe Ser Leu Lys Leu Ser Ser Val Thr Ala Ala Asp
    450                 455                 460

Thr Ala Val Tyr Phe Cys Ala Arg Lys Asp Ala Tyr Ser Asp Ala Phe
465                 470                 475                 480

Asn Leu Trp Gly Gln Gly Thr Leu Val Thr Val Ser Ser
                485                 490

<210>  110
<211>  493
<212>  PRT
<213>  Artificial Sequence

<220>
<223>  artificial antibody-based sequence

<400>  110

Asp Phe Gln Leu Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ser Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Val Ser Gly Phe Ser
    130                 135                 140

Phe Ser Asn Ser Tyr Trp Ile Cys Trp Ile Arg Gln Pro Pro Gly Lys
145                 150                 155                 160

Gly Leu Glu Trp Ile Gly Cys Thr Phe Val Gly Ser Ser Asp Ser Thr
                165                 170                 175

Tyr Tyr Ala Asn Trp Ala Lys Gly Arg Val Thr Ile Ser Val Asp Ser
            180                 185                 190

Ser Lys Asn Gln Phe Ser Leu Lys Leu Ser Ser Val Thr Ala Ala Asp
        195                 200                 205

Thr Ala Val Tyr Tyr Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Ala Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Thr Gly Thr Lys Val Thr Val Leu Gly Gly
        355                 360                 365

Gly Gly Gly Ser Gln Ser Gln Leu Gln Glu Ser Gly Pro Gly Leu Val
    370                 375                 380

Lys Pro Ser Glu Thr Leu Ser Leu Thr Cys Lys Ala Ser Gly Phe Ser
385                 390                 395                 400

Phe Ser Ser Gly Tyr Asp Met Cys Trp Val Arg Gln Pro Pro Gly Lys
                405                 410                 415

Gly Leu Glu Trp Ile Ala Cys Val Val Ala Gly Ser Val Asp Ile Thr
            420                 425                 430

Tyr Tyr Ala Ser Trp Ala Lys Gly Arg Val Thr Ile Ser Lys Asp Ser
        435                 440                 445

Ser Lys Asn Gln Val Ser Leu Lys Leu Ser Ser Val Thr Ala Ala Asp
    450                 455                 460

Thr Ala Val Tyr Phe Cys Ala Arg Lys Asp Ala Tyr Ser Asp Ala Phe
465                 470                 475                 480

Asn Leu Trp Gly Gln Gly Thr Leu Val Thr Val Ser Ser
                485                 490

<210>  111
<211>  493
<212>  PRT
<213>  Artificial Sequence

<220>
<223>  artificial antibody-based sequence

<400>  111

Asp Ile Gln Met Thr Gln Ser Pro Ser Ser Leu Ser Ala Ser Val Gly
1               5                   10                  15

Asp Ala Val Thr Ile Thr Cys Gln Ala Ser Gln Ser Ile Asn Asp Tyr
            20                  25                  30

Leu Ala Trp Tyr Gln Gln Lys Pro Gly Lys Ser Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Val Ser Gly Phe Ser
    130                 135                 140

Phe Ser Asn Ser Tyr Trp Ile Cys Trp Ile Arg Gln Pro Pro Gly Lys
145                 150                 155                 160

Gly Leu Glu Trp Ile Gly Cys Thr Phe Val Gly Ser Ser Asp Ser Thr
                165                 170                 175

Tyr Tyr Ala Asn Trp Ala Lys Gly Arg Val Thr Ile Ser Val Asp Ser
            180                 185                 190

Ser Lys Asn Gln Phe Ser Leu Lys Leu Ser Ser Val Thr Ala Ala Asp
        195                 200                 205

Thr Ala Val Tyr Tyr Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Ala Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Thr Gly Thr Lys Val Thr Val Leu Gly Gly
        355                 360                 365

Gly Gly Gly Ser Glu Val Gln Leu Val Glu Ser Gly Gly Gly Leu Val
    370                 375                 380

Gln Pro Gly Gly Ser Leu Arg Leu Ser Cys Ala Ala Ser Gly Phe Ser
385                 390                 395                 400

Phe Ser Ser Gly Tyr Asp Met Cys Trp Val Arg Gln Ala Pro Gly Lys
                405                 410                 415

Gly Leu Glu Trp Val Gly Cys Val Val Ala Gly Ser Val Asp Ile Thr
            420                 425                 430

Tyr Tyr Ala Ser Trp Ala Lys Gly Arg Phe Thr Ile Ser Arg Asp Asn
        435                 440                 445

Ser Lys Asn Thr Val Tyr Leu Gln Met Asn Ser Leu Arg Ala Glu Asp
    450                 455                 460

Thr Ala Thr Tyr Tyr Cys Ala Arg Lys Asp Ala Tyr Ser Asp Ala Phe
465                 470                 475                 480

Asn Leu Trp Gly Pro Gly Thr Leu Val Thr Val Ser Ser
                485                 490

<210>  112
<211>  755
<212>  PRT
<213>  Artificial Sequence

<220>

<223>  artificial antibody-based sequence

<400>  112

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Val Ser Gly Phe Ser
    130                 135                 140

Phe Ser Asn Ser Tyr Trp Ile Cys Trp Ile Arg Gln Pro Pro Gly Lys
145                 150                 155                 160

Gly Leu Glu Trp Ile Gly Cys Thr Phe Val Gly Ser Ser Asp Ser Thr
                165                 170                 175

Tyr Tyr Ala Asn Trp Ala Lys Gly Arg Val Thr Ile Ser Val Asp Ser
            180                 185                 190

Ser Lys Asn Gln Phe Ser Leu Lys Leu Ser Ser Val Thr Ala Ala Asp
        195                 200                 205

Thr Ala Val Tyr Tyr Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Ala Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Thr Gly Thr Lys Val Thr Val Leu Gly Gly
        355                 360                 365

Gly Gly Gly Ser Gln Val Gln Leu Gln Glu Ser Gly Pro Gly Leu Val
    370                 375                 380

Lys Pro Ser Glu Thr Leu Ser Leu Thr Cys Lys Val Ser Gly Phe Ser
385                 390                 395                 400

Phe Ser Ser Gly Tyr Asp Met Cys Trp Ile Arg Gln Pro Pro Gly Lys
                405                 410                 415

Gly Leu Glu Trp Ile Gly Cys Val Val Ala Gly Ser Val Asp Ile Thr
            420                 425                 430

Tyr Tyr Ala Ser Trp Ala Lys Gly Arg Val Thr Ile Ser Val Asp Ser
        435                 440                 445

Ser Lys Asn Gln Phe Ser Leu Lys Leu Ser Ser Val Thr Ala Ala Asp
    450                 455                 460

Thr Ala Val Tyr Tyr Cys Ala Arg Lys Asp Ala Tyr Ser Asp Ala Phe
465                 470                 475                 480

Asn Leu Trp Gly Gln Gly Thr Leu Val Thr Val Ser Ser Gly Gly Gly
                485                 490                 495

Gly Ser Gly Gly Gly Gly Ser Ile Gln Met Thr Gln Ser Pro Ser Ser
            500                 505                 510
Leu Ser Ala Ser Val Gly Asp Arg Val Thr Ile Thr Cys Gln Ser Ser
        515                 520                 525
Glu Ser Val Tyr Ser Asn Asn Gln Leu Ser Trp Tyr Gln Gln Lys Pro
    530                 535                 540
Gly Gln Pro Pro Lys Leu Leu Ile Tyr Asp Ala Ser Asp Leu Ala Ser
545                 550                 555                 560
Gly Val Pro Ser Arg Phe Ser Gly Ser Gly Ser Gly Thr Asp Phe Thr
                565                 570                 575
Leu Thr Ile Ser Ser Leu Gln Pro Glu Asp Phe Ala Thr Tyr Tyr Cys
            580                 585                 590
Ala Gly Gly Phe Ser Ser Ser Ser Asp Thr Ala Phe Gly Gly Gly Thr
        595                 600                 605
Lys Leu Thr Val Leu Gly Gly Gly Gly Gly Ser Gly Gly Gly Gly Ser
    610                 615                 620
Gly Gly Gly Gly Ser Gly Gly Gly Gly Ser Glu Val Gln Leu Val Glu
625                 630                 635                 640
Ser Gly Gly Gly Leu Val Gln Pro Gly Gly Ser Leu Arg Leu Ser Cys
                645                 650                 655
Ala Ala Ser Gly Phe Ser Leu Ser Ser Asn Ala Met Gly Trp Val Arg
            660                 665                 670
Gln Ala Pro Gly Lys Gly Leu Glu Tyr Ile Gly Ile Ile Ser Val Gly
        675                 680                 685
Gly Phe Thr Tyr Tyr Ala Ser Trp Ala Lys Gly Arg Phe Thr Ile Ser
    690                 695                 700
Arg Asp Asn Ser Lys Asn Thr Val Tyr Leu Gln Met Asn Ser Leu Arg
705                 710                 715                 720
Ala Glu Asp Thr Ala Thr Tyr Phe Cys Ala Arg Asp Arg His Gly Gly
                725                 730                 735
Asp Ser Ser Gly Ala Phe Tyr Leu Trp Gly Gln Gly Thr Leu Val Thr

740                 745                 750

Val Ser Ser
        755

<210>  113
<211>  761
<212>  PRT
<213>  Artificial Sequence

<220>
<223>  artificial antibody-based sequence

<400>  113

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Val Ser Gly Phe Ser
    130                 135                 140

Phe Asn Asn Asp Tyr Asp Met Cys Trp Ile Arg Gln Pro Pro Gly Lys
145                 150                 155                 160

Gly Leu Glu Trp Ile Gly Cys Ile Asp Thr Gly Asp Gly Ser Thr Tyr
                165                 170                 175

Tyr Ala Ser Trp Ala Lys Gly Arg Val Thr Ile Ser Val Asp Ser Ser
            180                 185                 190

Lys Asn Gln Phe Ser Leu Lys Leu Ser Ser Val Thr Ala Ala Asp Thr
        195                 200                 205

Ala Val Tyr Tyr Cys Ala Arg Glu Ala Ala Ser Ser Ser Gly Tyr Gly
    210                 215                 220

Met Gly Tyr Phe Asp Leu Trp Gly Gln Gly Thr Leu Val Thr Val Ser
225                 230                 235                 240

Ser Gly Gly Gly Gly Ser Gly Gly Gly Gly Ser Gly Gly Gly Gly Ser
                245                 250                 255

Gly Gly Gly Gly Ser Ile Gln Met Thr Gln Ser Pro Ser Ser Leu Ser
            260                 265                 270

Ala Ser Val Gly Asp Arg Val Thr Ile Thr Cys Gln Ser Ser Gln Ser
        275                 280                 285

Val Tyr Asp Asn Asn Trp Leu Ala Trp Tyr Gln Gln Lys Pro Gly Lys
    290                 295                 300

Ala Pro Lys Leu Leu Ile Tyr Arg Ala Ser Asn Leu Ala Ser Gly Val
305                 310                 315                 320

Pro Ser Arg Phe Ser Gly Ser Gly Ser Gly Thr Asp Phe Thr Leu Thr
                325                 330                 335

Ile Ser Ser Leu Gln Pro Glu Asp Phe Ala Thr Tyr Tyr Cys Gln Gly
            340                 345                 350

Thr Tyr Leu Ser Ser Asn Trp Tyr Trp Ala Phe Gly Thr Gly Thr Lys
        355                 360                 365

Val Thr Val Leu Gly Gly Gly Gly Gly Ser Gln Val Gln Leu Gln Glu
    370                 375                 380

Ser Gly Pro Gly Leu Val Lys Pro Ser Glu Thr Leu Ser Leu Thr Cys
385                 390                 395                 400

Lys Val Ser Gly Phe Ser Phe Ser Ser Gly Tyr Asp Met Cys Trp Ile
                405                 410                 415

Arg Gln Pro Pro Gly Lys Gly Leu Glu Trp Ile Gly Cys Val Val Ala
            420                 425                 430

Gly Ser Val Asp Ile Thr Tyr Tyr Ala Ser Trp Ala Lys Gly Arg Val

435                 440                 445

Thr Ile Ser Val Asp Ser Ser Lys Asn Gln Phe Ser Leu Lys Leu Ser
    450                 455                 460

Ser Val Thr Ala Ala Asp Thr Ala Val Tyr Tyr Cys Ala Arg Lys Asp
465                 470                 475                 480

Ala Tyr Ser Asp Ala Phe Asn Leu Trp Gly Gln Gly Thr Leu Val Thr
                485                 490                 495

Val Ser Ser Gly Gly Gly Gly Ser Gly Gly Gly Gly Ser Ile Gln Met
            500                 505                 510

Thr Gln Ser Pro Ser Ser Leu Ser Ala Ser Val Gly Asp Arg Val Thr
        515                 520                 525

Ile Thr Cys Gln Ser Ser Glu Ser Val Tyr Ser Asn Asn Gln Leu Ser
    530                 535                 540

Trp Tyr Gln Gln Lys Pro Gly Gln Pro Pro Lys Leu Leu Ile Tyr Asp
545                 550                 555                 560

Ala Ser Asp Leu Ala Ser Gly Val Pro Ser Arg Phe Ser Gly Ser Gly
                565                 570                 575

Ser Gly Thr Asp Phe Thr Leu Thr Ile Ser Ser Leu Gln Pro Glu Asp
            580                 585                 590

Phe Ala Thr Tyr Tyr Cys Ala Gly Gly Phe Ser Ser Ser Ser Asp Thr
        595                 600                 605

Ala Phe Gly Gly Gly Thr Lys Leu Thr Val Leu Gly Gly Gly Gly Gly
    610                 615                 620

Ser Gly Gly Gly Gly Ser Gly Gly Gly Gly Ser Gly Gly Gly Gly Ser
625                 630                 635                 640

Glu Val Gln Leu Val Glu Ser Gly Gly Gly Leu Val Gln Pro Gly Gly
                645                 650                 655

Ser Leu Arg Leu Ser Cys Ala Ala Ser Gly Phe Ser Leu Ser Ser Asn
            660                 665                 670

Ala Met Gly Trp Val Arg Gln Ala Pro Gly Lys Gly Leu Glu Tyr Ile
        675                 680                 685

Gly Ile Ile Ser Val Gly Gly Phe Thr Tyr Tyr Ala Ser Trp Ala Lys
    690                 695                 700

Gly Arg Phe Thr Ile Ser Arg Asp Asn Ser Lys Asn Thr Val Tyr Leu
705                 710                 715                 720

Gln Met Asn Ser Leu Arg Ala Glu Asp Thr Ala Thr Tyr Phe Cys Ala
                725                 730                 735

Arg Asp Arg His Gly Gly Asp Ser Ser Gly Ala Phe Tyr Leu Trp Gly
            740                 745                 750

Gln Gly Thr Leu Val Thr Val Ser Ser
        755                 760

<210>  114
<211>  753
<212>  PRT
<213>  Artificial Sequence

<220>
<223>  artificial antibody-based sequence

<400>  114

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Val Ser Gly Phe Ser

130                 135                 140

Phe Ser Asn Ser Tyr Trp Ile Cys Trp Ile Arg Gln Pro Pro Gly Lys
145                 150                 155                 160

Gly Leu Glu Trp Ile Gly Cys Thr Phe Val Gly Ser Ser Asp Ser Thr
                165                 170                 175

Tyr Tyr Ala Asn Trp Ala Lys Gly Arg Val Thr Ile Ser Val Asp Ser
            180                 185                 190

Ser Lys Asn Gln Phe Ser Leu Lys Leu Ser Ser Val Thr Ala Ala Asp
        195                 200                 205

Thr Ala Val Tyr Tyr Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Ala Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Thr Gly Thr Lys Val Thr Val Leu Gly Gly
        355                 360                 365

Gly Gly Gly Ser Gln Val Gln Leu Gln Glu Ser Gly Pro Gly Leu Val
    370                 375                 380

Lys Pro Ser Glu Thr Leu Ser Leu Thr Cys Lys Val Ser Gly Phe Ser
385                 390                 395                 400

Phe Ser Ser Gly Tyr Asp Met Cys Trp Ile Arg Gln Pro Pro Gly Lys
                405                 410                 415

Gly Leu Glu Trp Ile Gly Cys Val Val Ala Gly Ser Val Asp Ile Thr
            420                 425                 430

Tyr Tyr Ala Ser Trp Ala Lys Gly Arg Val Thr Ile Ser Val Asp Ser
        435                 440                 445

Ser Lys Asn Gln Phe Ser Leu Lys Leu Ser Ser Val Thr Ala Ala Asp
    450                 455                 460

Thr Ala Val Tyr Tyr Cys Ala Arg Lys Asp Ala Tyr Ser Asp Ala Phe
465                 470                 475                 480

Asn Leu Trp Gly Gln Gly Thr Leu Val Thr Val Ser Ser Gly Gly Gly
                485                 490                 495

Gly Ser Gly Gly Gly Gly Ser Val Val Met Thr Gln Ser Pro Ser Ser
            500                 505                 510

Leu Ser Ala Ser Val Gly Asp Arg Val Thr Ile Thr Cys Gln Ala Ser
        515                 520                 525

Gln Ile Ile Ser Ser Arg Ser Ala Trp Tyr Gln Gln Lys Pro Gly Gln
    530                 535                 540

Pro Pro Lys Leu Leu Ile Tyr Gln Ala Ser Lys Leu Ala Ser Gly Val
545                 550                 555                 560

Pro Ser Arg Phe Ser Gly Ser Gly Ser Gly Thr Asp Phe Thr Leu Thr
                565                 570                 575

Ile Ser Ser Leu Gln Pro Glu Asp Phe Ala Thr Tyr Tyr Cys Gln Cys
            580                 585                 590

Thr Tyr Ile Asp Ser Asn Phe Gly Ala Phe Gly Gly Gly Thr Lys Leu
        595                 600                 605

Thr Val Leu Gly Gly Gly Gly Gly Ser Gly Gly Gly Gly Ser Gly Gly
    610                 615                 620

Gly Gly Ser Gly Gly Gly Gly Ser Glu Val Gln Leu Val Glu Ser Gly
625                 630                 635                 640

Gly Gly Leu Val Gln Pro Gly Gly Ser Leu Arg Leu Ser Cys Ala Ala
                645                 650                 655

Ser Gly Phe Ser Phe Ser Ser Ser Tyr Trp Ile Cys Trp Val Arg Gln
            660                 665                 670

Ala Pro Gly Lys Gly Leu Glu Trp Val Gly Cys Val Phe Thr Gly Asp
        675                 680                 685

Gly Thr Thr Tyr Tyr Ala Ser Trp Ala Lys Gly Arg Phe Thr Ile Ser
    690                 695                 700

Arg Asp Asn Ser Lys Asn Thr Val Tyr Leu Gln Met Asn Ser Leu Arg
705                 710                 715                 720

Ala Glu Asp Thr Ala Thr Tyr Phe Cys Ala Arg Pro Val Ser Val Tyr
                725                 730                 735

Tyr Tyr Gly Met Asp Leu Trp Gly Gln Gly Thr Leu Val Thr Val Ser
            740                 745                 750

Ser

<210>  115
<211>  759
<212>  PRT
<213>  Artificial Sequence

<220>
<223>  artificial antibody-based sequence

<400>  115

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Gln Val Gln Leu Gln Glu Ser Gly Pro Gly Leu Val
        115                 120                 125

Lys Pro Ser Glu Thr Leu Ser Leu Thr Cys Lys Val Ser Gly Phe Ser
    130                 135                 140

Phe Asn Asn Asp Tyr Asp Met Cys Trp Ile Arg Gln Pro Pro Gly Lys
145                 150                 155                 160

Gly Leu Glu Trp Ile Gly Cys Ile Asp Thr Gly Asp Gly Ser Thr Tyr
                165                 170                 175

Tyr Ala Ser Trp Ala Lys Gly Arg Val Thr Ile Ser Val Asp Ser Ser
            180                 185                 190

Lys Asn Gln Phe Ser Leu Lys Leu Ser Ser Val Thr Ala Ala Asp Thr
        195                 200                 205

Ala Val Tyr Tyr Cys Ala Arg Glu Ala Ala Ser Ser Ser Gly Tyr Gly
    210                 215                 220

Met Gly Tyr Phe Asp Leu Trp Gly Gln Gly Thr Leu Val Thr Val Ser
225                 230                 235                 240

Ser Gly Gly Gly Gly Ser Gly Gly Gly Gly Ser Gly Gly Gly Gly Ser
                245                 250                 255

Gly Gly Gly Gly Ser Ile Gln Met Thr Gln Ser Pro Ser Ser Leu Ser
            260                 265                 270

Ala Ser Val Gly Asp Arg Val Thr Ile Thr Cys Gln Ser Ser Gln Ser
        275                 280                 285

Val Tyr Asp Asn Asn Trp Leu Ala Trp Tyr Gln Gln Lys Pro Gly Lys
    290                 295                 300

Ala Pro Lys Leu Leu Ile Tyr Arg Ala Ser Asn Leu Ala Ser Gly Val
305                 310                 315                 320

Pro Ser Arg Phe Ser Gly Ser Gly Ser Gly Thr Asp Phe Thr Leu Thr
                325                 330                 335

Ile Ser Ser Leu Gln Pro Glu Asp Phe Ala Thr Tyr Tyr Cys Gln Gly
            340                 345                 350

Thr Tyr Leu Ser Ser Asn Trp Tyr Trp Ala Phe Gly Thr Gly Thr Lys
        355                 360                 365

Val Thr Val Leu Gly Gly Gly Gly Gly Ser Gln Val Gln Leu Gln Glu
    370                 375                 380

Ser Gly Pro Gly Leu Val Lys Pro Ser Glu Thr Leu Ser Leu Thr Cys
385                 390                 395                 400

Lys Val Ser Gly Phe Ser Phe Ser Ser Gly Tyr Asp Met Cys Trp Ile
                405                 410                 415

Arg Gln Pro Pro Gly Lys Gly Leu Glu Trp Ile Gly Cys Val Val Ala
            420                 425                 430

Gly Ser Val Asp Ile Thr Tyr Tyr Ala Ser Trp Ala Lys Gly Arg Val
        435                 440                 445

Thr Ile Ser Val Asp Ser Ser Lys Asn Gln Phe Ser Leu Lys Leu Ser
    450                 455                 460

Ser Val Thr Ala Ala Asp Thr Ala Val Tyr Tyr Cys Ala Arg Lys Asp
465                 470                 475                 480

Ala Tyr Ser Asp Ala Phe Asn Leu Trp Gly Gln Gly Thr Leu Val Thr
                485                 490                 495

Val Ser Ser Gly Gly Gly Gly Ser Gly Gly Gly Gly Ser Val Val Met
            500                 505                 510

Thr Gln Ser Pro Ser Ser Leu Ser Ala Ser Val Gly Asp Arg Val Thr
        515                 520                 525

Ile Thr Cys Gln Ala Ser Gln Ile Ile Ser Ser Arg Ser Ala Trp Tyr
    530                 535                 540

Gln Gln Lys Pro Gly Gln Pro Pro Lys Leu Leu Ile Tyr Gln Ala Ser
545                 550                 555                 560

Lys Leu Ala Ser Gly Val Pro Ser Arg Phe Ser Gly Ser Gly Ser Gly
                565                 570                 575

Thr Asp Phe Thr Leu Thr Ile Ser Ser Leu Gln Pro Glu Asp Phe Ala
            580                 585                 590

Thr Tyr Tyr Cys Gln Cys Thr Tyr Ile Asp Ser Asn Phe Gly Ala Phe
        595                 600                 605

Gly Gly Gly Thr Lys Leu Thr Val Leu Gly Gly Gly Gly Gly Ser Gly
    610                 615                 620

Gly Gly Gly Ser Gly Gly Gly Gly Ser Gly Gly Gly Gly Ser Glu Val
625                 630                 635                 640

Gln Leu Val Glu Ser Gly Gly Gly Leu Val Gln Pro Gly Gly Ser Leu
                645                 650                 655

Arg Leu Ser Cys Ala Ala Ser Gly Phe Ser Phe Ser Ser Ser Tyr Trp
            660                 665                 670

Ile Cys Trp Val Arg Gln Ala Pro Gly Lys Gly Leu Glu Trp Val Gly
        675                 680                 685

Cys Val Phe Thr Gly Asp Gly Thr Thr Tyr Tyr Ala Ser Trp Ala Lys
    690                 695                 700

Gly Arg Phe Thr Ile Ser Arg Asp Asn Ser Lys Asn Thr Val Tyr Leu
705                 710                 715                 720

Gln Met Asn Ser Leu Arg Ala Glu Asp Thr Ala Thr Tyr Phe Cys Ala
                725                 730                 735

Arg Pro Val Ser Val Tyr Tyr Tyr Gly Met Asp Leu Trp Gly Gln Gly
            740                 745                 750

Thr Leu Val Thr Val Ser Ser
        755

<210>  116
<211>  759
<212>  PRT
<213>  Artificial Sequence

<220>
<223>  artificial antibody-based sequence

<400>  116

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Ala Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Gln Val Gln Leu Gln Glu Ser Gly Pro
        115                 120                 125

Gly Leu Val Lys Pro Ser Glu Thr Leu Ser Leu Thr Cys Lys Val Ser
    130                 135                 140

Gly Phe Ser Phe Ser Asn Ser Tyr Trp Ile Cys Trp Ile Arg Gln Pro
145                 150                 155                 160

Pro Gly Lys Gly Leu Glu Trp Ile Gly Cys Thr Phe Val Gly Ser Ser
                165                 170                 175

Asp Ser Thr Tyr Tyr Ala Asn Trp Ala Lys Gly Arg Val Thr Ile Ser
            180                 185                 190

Val Asp Ser Ser Lys Asn Gln Phe Ser Leu Lys Leu Ser Ser Val Thr
        195                 200                 205

Ala Ala Asp Thr Ala Val Tyr Tyr Cys Ala Arg His Pro Ser Asp Ala
    210                 215                 220

Val Tyr Gly Tyr Ala Asn Asn Leu Trp Gly Gln Gly Thr Leu Val Thr
225                 230                 235                 240

Val Ser Ser Gly Gly Gly Gly Ser Gly Gly Gly Gly Ser Gly Gly Gly
                245                 250                 255

Gly Ser Gly Gly Gly Gly Ser Ile Gln Met Thr Gln Ser Pro Ser Ser
            260                 265                 270

Leu Ser Ala Ser Val Gly Asp Arg Val Thr Ile Thr Cys Gln Ala Ser
        275                 280                 285

Gln Ser Ile Asn Asn Val Leu Ala Trp Tyr Gln Gln Lys Pro Gly Lys
    290                 295                 300

Ala Pro Lys Leu Leu Ile Tyr Arg Ala Ser Thr Leu Ala Ser Gly Val
305                 310                 315                 320

Pro Ser Arg Phe Ser Gly Ser Gly Ser Gly Thr Asp Phe Thr Leu Thr
                325                 330                 335

Ile Ser Ser Leu Gln Pro Glu Asp Phe Ala Thr Tyr Tyr Cys Gln Ser
            340                 345                 350

Ser Tyr Gly Asn Tyr Gly Asp Phe Gly Thr Gly Thr Lys Val Thr Val
        355                 360                 365

Leu Gly Gly Gly Gly Gly Ser Gln Val Gln Leu Gln Glu Ser Gly Pro
    370                 375                 380

Gly Leu Val Lys Pro Ser Glu Thr Leu Ser Leu Thr Cys Lys Val Ser
385                 390                 395                 400

Gly Phe Ser Phe Asn Ser Asp Tyr Trp Ile Tyr Trp Ile Arg Gln Pro
                405                 410                 415

Pro Gly Lys Gly Leu Glu Trp Ile Gly Ser Ile Tyr Gly Gly Ser Ser
            420                 425                 430

Gly Asn Thr Gln Tyr Ala Ser Trp Ala Gln Gly Arg Val Thr Ile Ser
        435                 440                 445

Val Asp Ser Ser Lys Asn Gln Phe Ser Leu Lys Leu Ser Ser Val Thr
    450                 455                 460

Ala Ala Asp Thr Ala Val Tyr Tyr Cys Ala Arg Gly Tyr Val Asp Tyr
465                 470                 475                 480

Gly Gly Ala Thr Asp Leu Trp Gly Gln Gly Thr Leu Val Thr Val Ser
                485                 490                 495

Ser Gly Gly Gly Gly Ser Gly Gly Gly Gly Ser Ile Gln Met Thr Gln
            500                 505                 510

Ser Pro Ser Ser Leu Ser Ala Ser Val Gly Asp Arg Val Thr Ile Thr
        515                 520                 525

Cys Gln Ser Ser Glu Ser Val Tyr Ser Asn Asn Gln Leu Ser Trp Tyr

530                 535                 540

Gln Gln Lys Pro Gly Gln Pro Pro Lys Leu Leu Ile Tyr Asp Ala Ser
545                 550                 555                 560

Asp Leu Ala Ser Gly Val Pro Ser Arg Phe Ser Gly Ser Gly Ser Gly
                565                 570                 575

Thr Asp Phe Thr Leu Thr Ile Ser Ser Leu Gln Pro Glu Asp Phe Ala
            580                 585                 590

Thr Tyr Tyr Cys Ala Gly Gly Phe Ser Ser Ser Ser Asp Thr Ala Phe
        595                 600                 605

Gly Gly Gly Thr Lys Leu Thr Val Leu Gly Gly Gly Gly Gly Ser Gly
    610                 615                 620

Gly Gly Gly Ser Gly Gly Gly Gly Ser Gly Gly Gly Gly Ser Glu Val
625                 630                 635                 640

Gln Leu Val Glu Ser Gly Gly Gly Leu Val Gln Pro Gly Gly Ser Leu
                645                 650                 655

Arg Leu Ser Cys Ala Ala Ser Gly Phe Ser Leu Ser Ser Asn Ala Met
            660                 665                 670

Gly Trp Val Arg Gln Ala Pro Gly Lys Gly Leu Glu Tyr Ile Gly Ile
        675                 680                 685

Ile Ser Val Gly Gly Phe Thr Tyr Tyr Ala Ser Trp Ala Lys Gly Arg
    690                 695                 700

Phe Thr Ile Ser Arg Asp Asn Ser Lys Asn Thr Val Tyr Leu Gln Met
705                 710                 715                 720

Asn Ser Leu Arg Ala Glu Asp Thr Ala Thr Tyr Phe Cys Ala Arg Asp
                725                 730                 735

Arg His Gly Gly Asp Ser Ser Gly Ala Phe Tyr Leu Trp Gly Gln Gly
            740                 745                 750

Thr Leu Val Thr Val Ser Ser
        755

<210>  117
<211>  758
<212>  PRT
<213>  Artificial Sequence

<220>
<223>  artificial antibody-based sequence

<400>  117

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Ala Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Gln Val Gln Leu Gln Glu Ser Gly Pro
        115                 120                 125

Gly Leu Val Lys Pro Ser Glu Thr Leu Ser Leu Thr Cys Lys Val Ser
    130                 135                 140

Gly Phe Ser Phe Ser Asn Ser Tyr Trp Ile Cys Trp Ile Arg Gln Pro
145                 150                 155                 160

Pro Gly Lys Gly Leu Glu Trp Ile Gly Cys Thr Phe Val Gly Ser Ser
                165                 170                 175

Asp Ser Thr Tyr Tyr Ala Asn Trp Ala Lys Gly Arg Val Thr Ile Ser
            180                 185                 190

Val Asp Ser Ser Lys Asn Gln Phe Ser Leu Lys Leu Ser Ser Val Thr
        195                 200                 205

Ala Ala Asp Thr Ala Val Tyr Tyr Cys Ala Arg His Pro Ser Asp Ala
    210                 215                 220

Val Tyr Gly Tyr Ala Asn Asn Leu Trp Gly Gln Gly Thr Leu Val Thr

225                 230                 235                 240

Val Ser Ser Gly Gly Gly Gly Ser Gly Gly Gly Gly Ser Gly Gly Gly
            245                 250                 255

Gly Ser Gly Gly Gly Gly Ser Asp Ile Gln Met Thr Gln Ser Pro Ser
        260                 265                 270

Ser Leu Ser Ala Ser Val Gly Asp Arg Val Thr Ile Thr Cys Gln Ala
        275                 280                 285

Ser Gln Ser Ile Asn Asn Val Leu Ala Trp Tyr Gln Gln Lys Pro Gly
    290                 295                 300

Lys Ala Pro Lys Leu Leu Ile Tyr Arg Ala Ser Thr Leu Ala Ser Gly
305                 310                 315                 320

Val Pro Ser Arg Phe Ser Gly Ser Gly Ser Gly Thr Asp Phe Thr Leu
            325                 330                 335

Thr Ile Ser Ser Leu Gln Pro Glu Asp Phe Ala Thr Tyr Tyr Cys Gln
        340                 345                 350

Ser Ser Tyr Gly Asn Tyr Gly Asp Phe Gly Thr Gly Thr Lys Val Thr
        355                 360                 365

Val Leu Gly Gly Gly Gly Gly Ser Gln Val Gln Leu Gln Glu Ser Gly
    370                 375                 380

Pro Gly Leu Val Lys Pro Ser Glu Thr Leu Ser Leu Thr Cys Lys Val
385                 390                 395                 400

Ser Gly Phe Ser Phe Asn Ser Asp Tyr Trp Ile Tyr Trp Ile Arg Gln
            405                 410                 415

Pro Pro Gly Lys Gly Leu Glu Trp Ile Gly Ser Ile Tyr Gly Gly Ser
        420                 425                 430

Ser Gly Asn Thr Gln Tyr Ala Ser Trp Ala Gln Gly Arg Val Thr Ile
        435                 440                 445

Ser Val Asp Ser Ser Lys Asn Gln Phe Ser Leu Lys Leu Ser Ser Val
    450                 455                 460

Thr Ala Ala Asp Thr Ala Val Tyr Tyr Cys Ala Arg Gly Tyr Val Asp
465                 470                 475                 480

Tyr Gly Gly Ala Thr Asp Leu Trp Gly Gln Gly Thr Leu Val Thr Val
                485                 490                 495

Ser Ser Gly Gly Gly Gly Ser Gly Gly Gly Gly Ser Val Val Met Thr
            500                 505                 510

Gln Ser Pro Ser Ser Leu Ser Ala Ser Val Gly Asp Arg Val Thr Ile
        515                 520                 525

Thr Cys Gln Ala Ser Gln Ile Ile Ser Ser Arg Ser Ala Trp Tyr Gln
    530                 535                 540

Gln Lys Pro Gly Gln Pro Pro Lys Leu Leu Ile Tyr Gln Ala Ser Lys
545                 550                 555                 560

Leu Ala Ser Gly Val Pro Ser Arg Phe Ser Gly Ser Gly Ser Gly Thr
                565                 570                 575

Asp Phe Thr Leu Thr Ile Ser Ser Leu Gln Pro Glu Asp Phe Ala Thr
            580                 585                 590

Tyr Tyr Cys Gln Cys Thr Tyr Ile Asp Ser Asn Phe Gly Ala Phe Gly
        595                 600                 605

Gly Gly Thr Lys Leu Thr Val Leu Gly Gly Gly Gly Gly Ser Gly Gly
    610                 615                 620

Gly Gly Ser Gly Gly Gly Gly Ser Gly Gly Gly Gly Ser Glu Val Gln
625                 630                 635                 640

Leu Val Glu Ser Gly Gly Gly Leu Val Gln Pro Gly Gly Ser Leu Arg
                645                 650                 655

Leu Ser Cys Ala Ala Ser Gly Phe Ser Phe Ser Ser Ser Tyr Trp Ile
            660                 665                 670

Cys Trp Val Arg Gln Ala Pro Gly Lys Gly Leu Glu Trp Val Gly Cys
        675                 680                 685

Val Phe Thr Gly Asp Gly Thr Thr Tyr Tyr Ala Ser Trp Ala Lys Gly
    690                 695                 700

Arg Phe Thr Ile Ser Arg Asp Asn Ser Lys Asn Thr Val Tyr Leu Gln
705                 710                 715                 720

Met Asn Ser Leu Arg Ala Glu Asp Thr Ala Thr Tyr Phe Cys Ala Arg
                725                 730                 735

Pro Val Ser Val Tyr Tyr Tyr Gly Met Asp Leu Trp Gly Gln Gly Thr
            740                 745                 750

Leu Val Thr Val Ser Ser
        755

<210>  118
<211>  759
<212>  PRT
<213>  Artificial Sequence

<220>
<223>  artificial antibody-based sequence

<400>  118

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Ala Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Glu Val Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Ser Phe Ser Asn Ser Tyr Trp Ile Cys Trp Val Arg Gln Ala
145                 150                 155                 160

Pro Gly Lys Cys Leu Glu Trp Ile Gly Cys Thr Phe Val Gly Ser Ser
                165                 170                 175

Asp Ser Thr Tyr Tyr Ala Asn Trp Ala Lys Gly Arg Phe Thr Ile Ser
        180                 185                 190

Arg Asp Asn Ser Lys Asn Thr Val Tyr Leu Gln Met Asn Ser Leu Arg
    195                 200                 205

Ala Glu Asp Thr Ala Val Tyr Tyr Cys Ala Arg His Pro Ser Asp Ala
    210                 215                 220

Val Tyr Gly Tyr Ala Asn Asn Leu Trp Gly Gln Gly Thr Leu Val Thr
225                 230                 235                 240

Val Ser Ser Gly Gly Gly Gly Ser Gly Gly Gly Gly Ser Gly Gly Gly
                245                 250                 255

Gly Ser Gly Gly Gly Gly Ser Ile Gln Met Thr Gln Ser Pro Ser Ser
        260                 265                 270

Leu Ser Ala Ser Val Gly Asp Arg Val Thr Ile Thr Cys Gln Ala Ser
    275                 280                 285

Gln Ser Ile Asn Asn Val Leu Ala Trp Tyr Gln Gln Lys Pro Gly Lys
    290                 295                 300

Ala Pro Lys Leu Leu Ile Tyr Arg Ala Ser Thr Leu Ala Ser Gly Val
305                 310                 315                 320

Pro Ser Arg Phe Ser Gly Ser Gly Ser Gly Thr Asp Phe Thr Leu Thr
                325                 330                 335

Ile Ser Ser Leu Gln Pro Glu Asp Phe Ala Thr Tyr Tyr Cys Gln Ser
        340                 345                 350

Ser Tyr Gly Asn Tyr Gly Asp Phe Gly Cys Gly Thr Lys Val Thr Val
    355                 360                 365

Leu Gly Gly Gly Gly Gly Ser Gln Val Gln Leu Gln Glu Ser Gly Pro
    370                 375                 380

Gly Leu Val Lys Pro Ser Glu Thr Leu Ser Leu Thr Cys Lys Val Ser
385                 390                 395                 400

Gly Phe Ser Phe Asn Ser Asp Tyr Trp Ile Tyr Trp Ile Arg Gln Pro
                405                 410                 415

Pro Gly Lys Gly Leu Glu Trp Ile Gly Ser Ile Tyr Gly Gly Ser Ser
        420                 425                 430

Gly Asn Thr Gln Tyr Ala Ser Trp Ala Gln Gly Arg Val Thr Ile Ser
        435                 440                 445

Val Asp Ser Ser Lys Asn Gln Phe Ser Leu Lys Leu Ser Ser Val Thr
    450                 455                 460

Ala Ala Asp Thr Ala Val Tyr Tyr Cys Ala Arg Gly Tyr Val Asp Tyr
465                 470                 475                 480

Gly Gly Ala Thr Asp Leu Trp Gly Gln Gly Thr Leu Val Thr Val Ser
                485                 490                 495

Ser Gly Gly Gly Gly Ser Gly Gly Gly Gly Ser Ile Gln Met Thr Gln
            500                 505                 510

Ser Pro Ser Ser Leu Ser Ala Ser Val Gly Asp Arg Val Thr Ile Thr
        515                 520                 525

Cys Gln Ser Ser Glu Ser Val Tyr Ser Asn Asn Gln Leu Ser Trp Tyr
    530                 535                 540

Gln Gln Lys Pro Gly Gln Pro Pro Lys Leu Leu Ile Tyr Asp Ala Ser
545                 550                 555                 560

Asp Leu Ala Ser Gly Val Pro Ser Arg Phe Ser Gly Ser Gly Ser Gly
                565                 570                 575

Thr Asp Phe Thr Leu Thr Ile Ser Ser Leu Gln Pro Glu Asp Phe Ala
            580                 585                 590

Thr Tyr Tyr Cys Ala Gly Gly Phe Ser Ser Ser Ser Asp Thr Ala Phe
        595                 600                 605

Gly Gly Gly Thr Lys Leu Thr Val Leu Gly Gly Gly Gly Gly Ser Gly
    610                 615                 620

Gly Gly Gly Ser Gly Gly Gly Gly Ser Gly Gly Gly Gly Ser Glu Val
625                 630                 635                 640

Gln Leu Val Glu Ser Gly Gly Gly Leu Val Gln Pro Gly Gly Ser Leu
                645                 650                 655

Arg Leu Ser Cys Ala Ala Ser Gly Phe Ser Leu Ser Ser Asn Ala Met
            660                 665                 670

Gly Trp Val Arg Gln Ala Pro Gly Lys Gly Leu Glu Tyr Ile Gly Ile
        675                 680                 685

Ile Ser Val Gly Gly Phe Thr Tyr Tyr Ala Ser Trp Ala Lys Gly Arg
    690                 695                 700

Phe Thr Ile Ser Arg Asp Asn Ser Lys Asn Thr Val Tyr Leu Gln Met
705                 710                 715                 720

Asn Ser Leu Arg Ala Glu Asp Thr Ala Thr Tyr Phe Cys Ala Arg Asp
                725                 730                 735

Arg His Gly Gly Asp Ser Ser Gly Ala Phe Tyr Leu Trp Gly Gln Gly
            740                 745                 750

Thr Leu Val Thr Val Ser Ser
        755

<210>  119
<211>  759
<212>  PRT
<213>  Artificial Sequence

<220>
<223>  artificial antibody-based sequence

<400>  119

Asp Ile Gln Met Thr Gln Ser Pro Ala Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Pro Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Glu Val Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Ser Phe Ser Asn Ser Tyr Trp Ile Cys Trp Val Arg Gln Ala
145                 150                 155                 160

Pro Gly Lys Cys Leu Glu Trp Ile Gly Cys Thr Phe Val Gly Ser Ser
                165                 170                 175

Asp Ser Thr Tyr Tyr Ala Asn Trp Ala Lys Gly Arg Phe Thr Ile Ser
            180                 185                 190

Arg Asp Asn Ser Lys Asn Thr Val Tyr Leu Gln Met Asn Ser Leu Arg
        195                 200                 205

Ala Glu Asp Thr Ala Val Tyr Tyr Cys Ala Arg His Pro Ser Asp Ala
    210                 215                 220

Val Tyr Gly Tyr Ala Asn Asn Leu Trp Gly Gln Gly Thr Leu Val Thr
225                 230                 235                 240

Val Ser Ser Gly Gly Gly Gly Ser Gly Gly Gly Gly Ser Gly Gly Gly
                245                 250                 255

Gly Ser Gly Gly Gly Gly Ser Ile Gln Met Thr Gln Ser Pro Ser Ser
            260                 265                 270

Leu Ser Ala Ser Val Gly Asp Arg Val Thr Ile Thr Cys Gln Ala Ser
        275                 280                 285

Gln Ser Ile Asn Asn Val Leu Ala Trp Tyr Gln Gln Lys Pro Gly Lys
    290                 295                 300

Ala Pro Lys Leu Leu Ile Tyr Arg Ala Ser Thr Leu Ala Ser Gly Val
305                 310                 315                 320

Pro Ser Arg Phe Ser Gly Ser Gly Ser Gly Thr Asp Phe Thr Leu Thr
                325                 330                 335

Ile Ser Ser Leu Gln Pro Glu Asp Phe Ala Thr Tyr Tyr Cys Gln Ser
            340                 345                 350

Ser Tyr Gly Asn Tyr Gly Asp Phe Gly Cys Gly Thr Lys Val Thr Val
        355                 360                 365

Leu Gly Gly Gly Gly Gly Ser Glu Val Gln Leu Val Glu Ser Gly Gly
    370                 375                 380

Gly Leu Val Gln Pro Gly Gly Ser Leu Arg Leu Ser Cys Ala Ala Ser
385                 390                 395                 400

Gly Phe Ser Phe Asn Ser Asp Tyr Trp Ile Tyr Trp Val Arg Gln Ala
                405                 410                 415

Pro Gly Lys Gly Leu Glu Trp Ile Ala Ser Ile Tyr Gly Gly Ser Ser
            420                 425                 430

Gly Asn Thr Gln Tyr Ala Ser Trp Ala Gln Gly Arg Phe Thr Ile Ser
        435                 440                 445

Arg Asp Asn Ser Lys Asn Thr Val Tyr Leu Gln Met Asn Ser Leu Arg
    450                 455                 460

Ala Glu Asp Thr Ala Val Tyr Phe Cys Ala Arg Gly Tyr Val Asp Tyr
465                 470                 475                 480

Gly Gly Ala Thr Asp Leu Trp Gly Gln Gly Thr Leu Val Thr Val Ser
                485                 490                 495

Ser Gly Gly Gly Gly Ser Gly Gly Gly Gly Ser Ile Gln Met Thr Gln
            500                 505                 510

Ser Pro Ser Ser Leu Ser Ala Ser Val Gly Asp Arg Val Thr Ile Thr
        515                 520                 525

Cys Gln Ser Ser Glu Ser Val Tyr Ser Asn Asn Gln Leu Ser Trp Tyr
    530                 535                 540

Gln Gln Lys Pro Gly Gln Pro Pro Lys Leu Leu Ile Tyr Asp Ala Ser
545                 550                 555                 560

Asp Leu Ala Ser Gly Val Pro Ser Arg Phe Ser Gly Ser Gly Ser Gly
                565                 570                 575

Thr Asp Phe Thr Leu Thr Ile Ser Ser Leu Gln Pro Glu Asp Phe Ala
            580                 585                 590

Thr Tyr Tyr Cys Ala Gly Gly Phe Ser Ser Ser Ser Asp Thr Ala Phe
        595                 600                 605

Gly Gly Gly Thr Lys Leu Thr Val Leu Gly Gly Gly Gly Gly Ser Gly
    610                 615                 620

Gly Gly Gly Ser Gly Gly Gly Gly Ser Gly Gly Gly Gly Ser Glu Val

625                 630                 635                 640

Gln Leu Val Glu Ser Gly Gly Gly Leu Val Gln Pro Gly Gly Ser Leu
                645                 650                 655

Arg Leu Ser Cys Ala Ala Ser Gly Phe Ser Leu Ser Ser Asn Ala Met
            660                 665                 670

Gly Trp Val Arg Gln Ala Pro Gly Lys Gly Leu Glu Tyr Ile Gly Ile
        675                 680                 685

Ile Ser Val Gly Gly Phe Thr Tyr Tyr Ala Ser Trp Ala Lys Gly Arg
    690                 695                 700

Phe Thr Ile Ser Arg Asp Asn Ser Lys Asn Thr Val Tyr Leu Gln Met
705                 710                 715                 720

Asn Ser Leu Arg Ala Glu Asp Thr Ala Thr Tyr Phe Cys Ala Arg Asp
                725                 730                 735

Arg His Gly Gly Asp Ser Ser Gly Ala Phe Tyr Leu Trp Gly Gln Gly
            740                 745                 750

Thr Leu Val Thr Val Ser Ser
        755

<210>  120
<211>  759
<212>  PRT
<213>  Artificial Sequence

<220>
<223>  artificial antibody-based sequence

<400>  120

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Cys Gly Thr Lys Val Thr Val Leu Gly Gly Gly Gly Gly
            100                 105                 110

Ser Glu Val Gln Leu Val Glu Ser Gly Gly Gly Leu Val Gln Pro Gly
        115                 120                 125

Gly Ser Leu Arg Leu Ser Cys Ala Ala Ser Gly Phe Ser Phe Asn Ser
    130                 135                 140

Asp Tyr Trp Ile Tyr Trp Val Arg Gln Ala Pro Gly Lys Gly Leu Glu
145                 150                 155                 160

Trp Ile Ala Ser Ile Tyr Gly Gly Ser Ser Gly Asn Thr Gln Tyr Ala
                165                 170                 175

Ser Trp Ala Gln Gly Arg Phe Thr Ile Ser Arg Asp Asn Ser Lys Asn
            180                 185                 190

Thr Val Tyr Leu Gln Met Asn Ser Leu Arg Ala Glu Asp Thr Ala Val
        195                 200                 205

Tyr Phe Cys Ala Arg Gly Tyr Val Asp Tyr Gly Gly Ala Thr Asp Leu
    210                 215                 220

Trp Gly Gln Gly Thr Leu Val Thr Val Ser Ser Gly Gly Gly Gly Ser
225                 230                 235                 240

Gly Gly Gly Gly Ser Gly Gly Gly Gly Ser Gly Gly Gly Gly Ser Ile
                245                 250                 255

Gln Met Thr Gln Ser Pro Ala Ser Leu Ser Ala Ser Val Gly Asp Arg
            260                 265                 270

Val Thr Ile Thr Cys Gln Ala Ser Gln Ser Ile Gly Thr Tyr Leu Ala
        275                 280                 285

Trp Tyr Gln Gln Lys Pro Gly Lys Pro Pro Lys Leu Leu Ile Tyr Arg
    290                 295                 300

Ala Phe Ile Leu Ala Ser Gly Val Pro Ser Arg Phe Ser Gly Ser Gly
305                 310                 315                 320

Ser Gly Thr Asp Phe Thr Leu Thr Ile Ser Ser Leu Gln Pro Glu Asp

                325                 330                 335

Phe Ala Thr Tyr Tyr Cys Gln Ser Asn Phe Tyr Ser Asp Ser Thr Thr
            340                 345                 350

Ile Gly Pro Asn Ala Phe Gly Thr Gly Thr Lys Val Thr Val Leu Gly
        355                 360                 365

Gly Gly Gly Gly Ser Glu Val Gln Leu Val Glu Ser Gly Gly Gly Leu
    370                 375                 380

Val Gln Pro Gly Gly Ser Leu Arg Leu Ser Cys Ala Ala Ser Gly Phe
385                 390                 395                 400

Ser Phe Ser Asn Ser Tyr Trp Ile Cys Trp Val Arg Gln Ala Pro Gly
                405                 410                 415

Lys Cys Leu Glu Trp Ile Gly Cys Thr Phe Val Gly Ser Ser Asp Ser
            420                 425                 430

Thr Tyr Tyr Ala Asn Trp Ala Lys Gly Arg Phe Thr Ile Ser Arg Asp
        435                 440                 445

Asn Ser Lys Asn Thr Val Tyr Leu Gln Met Asn Ser Leu Arg Ala Glu
    450                 455                 460

Asp Thr Ala Val Tyr Tyr Cys Ala Arg His Pro Ser Asp Ala Val Tyr
465                 470                 475                 480

Gly Tyr Ala Asn Asn Leu Trp Gly Gln Gly Thr Leu Val Thr Val Ser
                485                 490                 495

Ser Gly Gly Gly Gly Ser Gly Gly Gly Gly Ser Ile Gln Met Thr Gln
            500                 505                 510

Ser Pro Ser Ser Leu Ser Ala Ser Val Gly Asp Arg Val Thr Ile Thr
        515                 520                 525

Cys Gln Ser Ser Glu Ser Val Tyr Ser Asn Asn Gln Leu Ser Trp Tyr
    530                 535                 540

Gln Gln Lys Pro Gly Gln Pro Pro Lys Leu Leu Ile Tyr Asp Ala Ser
545                 550                 555                 560

Asp Leu Ala Ser Gly Val Pro Ser Arg Phe Ser Gly Ser Gly Ser Gly
                565                 570                 575

Thr Asp Phe Thr Leu Thr Ile Ser Ser Leu Gln Pro Glu Asp Phe Ala
            580                 585                 590

Thr Tyr Tyr Cys Ala Gly Gly Phe Ser Ser Ser Ser Asp Thr Ala Phe
        595                 600                 605

Gly Gly Gly Thr Lys Leu Thr Val Leu Gly Gly Gly Gly Gly Ser Gly
    610                 615                 620

Gly Gly Gly Ser Gly Gly Gly Gly Ser Gly Gly Gly Gly Ser Glu Val
625                 630                 635                 640

Gln Leu Val Glu Ser Gly Gly Gly Leu Val Gln Pro Gly Gly Ser Leu
                645                 650                 655

Arg Leu Ser Cys Ala Ala Ser Gly Phe Ser Leu Ser Ser Asn Ala Met
            660                 665                 670

Gly Trp Val Arg Gln Ala Pro Gly Lys Gly Leu Glu Tyr Ile Gly Ile
        675                 680                 685

Ile Ser Val Gly Gly Phe Thr Tyr Tyr Ala Ser Trp Ala Lys Gly Arg
    690                 695                 700

Phe Thr Ile Ser Arg Asp Asn Ser Lys Asn Thr Val Tyr Leu Gln Met
705                 710                 715                 720

Asn Ser Leu Arg Ala Glu Asp Thr Ala Thr Tyr Phe Cys Ala Arg Asp
                725                 730                 735

Arg His Gly Gly Asp Ser Ser Gly Ala Phe Tyr Leu Trp Gly Gln Gly
            740                 745                 750

Thr Leu Val Thr Val Ser Ser
        755

<210> 121
<211> 755
<212> PRT
<213> Artificial Sequence

<220>
<223> artificial antibody-based sequence

<400> 121

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr

            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Glu Val Gln Leu Val Glu Ser Gly Gly Gly Leu Val
        115                 120                 125

Gln Pro Gly Gly Ser Leu Arg Leu Ser Cys Ala Ala Ser Gly Phe Ser
    130                 135                 140

Phe Ser Asn Ser Tyr Trp Ile Cys Trp Val Arg Gln Ala Pro Gly Lys
145                 150                 155                 160

Cys Leu Glu Trp Ile Gly Cys Thr Phe Val Gly Ser Ser Asp Ser Thr
                165                 170                 175

Tyr Tyr Ala Asn Trp Ala Lys Gly Arg Phe Thr Ile Ser Arg Asp Asn
            180                 185                 190

Ser Lys Asn Thr Val Tyr Leu Gln Met Asn Ser Leu Arg Ala Glu Asp
        195                 200                 205

Thr Ala Val Tyr Tyr Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Ala Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Cys Gly Thr Lys Val Thr Val Leu Gly Gly
        355                 360                 365

Gly Gly Gly Ser Gln Val Gln Leu Gln Glu Ser Gly Pro Gly Leu Val
    370                 375                 380

Lys Pro Ser Glu Thr Leu Ser Leu Thr Cys Lys Ala Ser Gly Phe Ser
385                 390                 395                 400

Phe Ser Ser Gly Tyr Asp Met Cys Trp Val Arg Gln Pro Pro Gly Lys
                405                 410                 415

Gly Leu Glu Trp Ile Ala Cys Val Val Ala Gly Ser Val Asp Ile Thr
            420                 425                 430

Tyr Tyr Ala Ser Trp Ala Lys Gly Arg Val Thr Ile Ser Lys Asp Ser
        435                 440                 445

Ser Lys Asn Gln Val Ser Leu Lys Leu Ser Ser Val Thr Ala Ala Asp
    450                 455                 460

Thr Ala Val Tyr Tyr Cys Ala Arg Lys Asp Ala Tyr Ser Asp Ala Phe
465                 470                 475                 480

Asn Leu Trp Gly Gln Gly Thr Leu Val Thr Val Ser Ser Gly Gly Gly
                485                 490                 495

Gly Ser Gly Gly Gly Gly Ser Ile Gln Met Thr Gln Ser Pro Ser Ser
            500                 505                 510

Leu Ser Ala Ser Val Gly Asp Arg Val Thr Ile Thr Cys Gln Ser Ser
        515                 520                 525

Glu Ser Val Tyr Ser Asn Asn Gln Leu Ser Trp Tyr Gln Gln Lys Pro
    530                 535                 540

Gly Gln Pro Pro Lys Leu Leu Ile Tyr Asp Ala Ser Asp Leu Ala Ser
545                 550                 555                 560

Gly Val Pro Ser Arg Phe Ser Gly Ser Gly Ser Gly Thr Asp Phe Thr
                565                 570                 575

Leu Thr Ile Ser Ser Leu Gln Pro Glu Asp Phe Ala Thr Tyr Tyr Cys
            580                 585                 590

Ala Gly Gly Phe Ser Ser Ser Ser Asp Thr Ala Phe Gly Gly Gly Thr
        595                 600                 605

Lys Leu Thr Val Leu Gly Gly Gly Gly Gly Ser Gly Gly Gly Gly Ser
    610                 615                 620

Gly Gly Gly Gly Ser Gly Gly Gly Gly Ser Glu Val Gln Leu Val Glu
625                 630                 635                 640

Ser Gly Gly Gly Leu Val Gln Pro Gly Gly Ser Leu Arg Leu Ser Cys
                645                 650                 655

Ala Ala Ser Gly Phe Ser Leu Ser Ser Asn Ala Met Gly Trp Val Arg
            660                 665                 670

Gln Ala Pro Gly Lys Gly Leu Glu Tyr Ile Gly Ile Ile Ser Val Gly
        675                 680                 685

Gly Phe Thr Tyr Tyr Ala Ser Trp Ala Lys Gly Arg Phe Thr Ile Ser
    690                 695                 700

Arg Asp Asn Ser Lys Asn Thr Val Tyr Leu Gln Met Asn Ser Leu Arg
705                 710                 715                 720

Ala Glu Asp Thr Ala Thr Tyr Phe Cys Ala Arg Asp Arg His Gly Gly
                725                 730                 735

Asp Ser Ser Gly Ala Phe Tyr Leu Trp Gly Gln Gly Thr Leu Val Thr
            740                 745                 750

Val Ser Ser
        755

<210>  122
<211>  755

<212>  PRT
<213>  Artificial Sequence

<220>
<223>  artificial antibody-based sequence

<400>  122

Asp Phe Gln Leu Thr Gln Ser Pro Ser Ser Leu Ser Ala Ser Val Gly
1               5                   10                  15

Asp Arg Val Thr Ile Thr Cys Gln Ala Ser Gln Ser Ile Asn Asp Tyr
            20                  25                  30

Leu Ala Trp Tyr Gln Gln Lys Pro Gly Lys Ser Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Tyr Ile Ile Thr Asp
                85                  90                  95

Ile Asp Asn Val Phe Gly Thr Gly Thr Lys Val Thr Val Leu Gly Gly
            100                 105                 110

Gly Gly Gly Ser Glu Val Gln Leu Val Glu Ser Gly Gly Gly Leu Val
        115                 120                 125

Gln Pro Gly Gly Ser Leu Arg Leu Ser Cys Ala Ala Ser Gly Phe Ser
    130                 135                 140

Phe Ser Asn Ser Tyr Trp Ile Cys Trp Val Arg Gln Ala Pro Gly Lys
145                 150                 155                 160

Cys Leu Glu Trp Ile Gly Cys Thr Phe Val Gly Ser Ser Asp Ser Thr
                165                 170                 175

Tyr Tyr Ala Asn Trp Ala Lys Gly Arg Phe Thr Ile Ser Arg Asp Asn
            180                 185                 190

Ser Lys Asn Thr Val Tyr Leu Gln Met Asn Ser Leu Arg Ala Glu Asp
        195                 200                 205

Thr Ala Val Tyr Tyr Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Ala Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Cys Gly Thr Lys Val Thr Val Leu Gly Gly
        355                 360                 365

Gly Gly Gly Ser Gln Ser Gln Leu Gln Glu Ser Gly Pro Gly Leu Val
    370                 375                 380

Lys Pro Ser Glu Thr Leu Ser Leu Thr Cys Lys Ala Ser Gly Phe Ser
385                 390                 395                 400

Phe Ser Ser Gly Tyr Asp Met Cys Trp Val Arg Gln Pro Pro Gly Lys
                405                 410                 415

Gly Leu Glu Trp Ile Ala Cys Val Val Ala Gly Ser Val Asp Ile Thr
            420                 425                 430

Tyr Tyr Ala Ser Trp Ala Lys Gly Arg Val Thr Ile Ser Lys Asp Ser
        435                 440                 445

Ser Lys Asn Gln Val Ser Leu Lys Leu Ser Ser Val Thr Ala Ala Asp
    450                 455                 460

Thr Ala Val Tyr Phe Cys Ala Arg Lys Asp Ala Tyr Ser Asp Ala Phe
465                 470                 475                 480

Asn Leu Trp Gly Gln Gly Thr Leu Val Thr Val Ser Ser Gly Gly Gly
                485                 490                 495

Gly Ser Gly Gly Gly Gly Ser Ile Gln Met Thr Gln Ser Pro Ser Ser
            500                 505                 510

Leu Ser Ala Ser Val Gly Asp Arg Val Thr Ile Thr Cys Gln Ser Ser
        515                 520                 525

Glu Ser Val Tyr Ser Asn Asn Gln Leu Ser Trp Tyr Gln Gln Lys Pro
    530                 535                 540

Gly Gln Pro Pro Lys Leu Leu Ile Tyr Asp Ala Ser Asp Leu Ala Ser
545                 550                 555                 560

Gly Val Pro Ser Arg Phe Ser Gly Ser Gly Ser Gly Thr Asp Phe Thr
                565                 570                 575

Leu Thr Ile Ser Ser Leu Gln Pro Glu Asp Phe Ala Thr Tyr Tyr Cys
            580                 585                 590

Ala Gly Gly Phe Ser Ser Ser Ser Asp Thr Ala Phe Gly Gly Gly Thr
        595                 600                 605

Lys Leu Thr Val Leu Gly Gly Gly Gly Gly Ser Gly Gly Gly Gly Ser
    610                 615                 620

Gly Gly Gly Gly Ser Gly Gly Gly Gly Ser Glu Val Gln Leu Val Glu
625                 630                 635                 640

Ser Gly Gly Gly Leu Val Gln Pro Gly Gly Ser Leu Arg Leu Ser Cys
                645                 650                 655

Ala Ala Ser Gly Phe Ser Leu Ser Ser Asn Ala Met Gly Trp Val Arg
            660                 665                 670

Gln Ala Pro Gly Lys Gly Leu Glu Tyr Ile Gly Ile Ile Ser Val Gly
        675                 680                 685

Gly Phe Thr Tyr Tyr Ala Ser Trp Ala Lys Gly Arg Phe Thr Ile Ser
    690                 695                 700

Arg Asp Asn Ser Lys Asn Thr Val Tyr Leu Gln Met Asn Ser Leu Arg
705                 710                 715                 720

Ala Glu Asp Thr Ala Thr Tyr Phe Cys Ala Arg Asp Arg His Gly Gly

725                 730                 735

Asp Ser Ser Gly Ala Phe Tyr Leu Trp Gly Gln Gly Thr Leu Val Thr
            740                 745                 750

Val Ser Ser
        755

<210>  123
<211>  755
<212>  PRT
<213>  Artificial Sequence

<220>
<223>  artificial antibody-based sequence

<400>  123
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Ser Ile Asn Asn Val
               20                  25                  30
   Leu Ala Trp Tyr Gln Gln Lys Pro Gly Lys Ala Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Ser Ser Tyr Gly Asn Tyr Gly
                   85                  90                  95
   Asp Phe Gly Cys Gly Thr Lys Val Thr Val Leu Gly Gly Gly Gly Gly
               100                 105                 110
   Ser Gln Ser Gln Leu Gln Glu Ser Gly Pro Gly Leu Val Lys Pro Ser
           115                 120                 125
   Glu Thr Leu Ser Leu Thr Cys Lys Ala Ser Gly Phe Ser Phe Ser Ser
       130                 135                 140
   Gly Tyr Asp Met Cys Trp Val Arg Gln Pro Pro Gly Lys Gly Leu Glu
   145                 150                 155                 160
   Trp Ile Ala Cys Val Val Ala Gly Ser Val Asp Ile Thr Tyr Tyr Ala
                   165                 170                 175
   Ser Trp Ala Lys Gly Arg Val Thr Ile Ser Lys Asp Ser Ser Lys Asn
           180                 185                 190
   Gln Val Ser Leu Lys Leu Ser Ser Val Thr Ala Ala Asp Thr Ala Val
       195                 200                 205
   Tyr Phe Cys Ala Arg Lys Asp Ala Tyr Ser Asp Ala Phe Asn Leu Trp
       210                 215                 220
   Gly Gln Gly Thr Leu Val Thr Val Ser Ser Gly Gly Gly Gly Ser Gly
   225                 230                 235                 240
   Gly Gly Gly Ser Gly Gly Gly Gly Ser Gly Gly Gly Gly Ser Phe Gln
                   245                 250                 255
   Leu Thr Gln Ser Pro Ser Ser Leu Ser Ala Ser Val Gly Asp Arg Val
           260                 265                 270
   Thr Ile Thr Cys Gln Ala Ser Gln Ser Ile Asn Asp Tyr Leu Ala Trp
       275                 280                 285
   Tyr Gln Gln Lys Pro Gly Lys Ser Pro Lys Leu Leu Ile Tyr Lys Ala
       290                 295                 300
   Ser Thr Leu Ala Ser Gly Val Pro Ser Arg Phe Ser Gly Ser Gly Ser
   305                 310                 315                 320
   Gly Thr Asp Phe Thr Leu Thr Ile Ser Ser Leu Gln Pro Glu Asp Phe
                   325                 330                 335
   Ala Thr Tyr Tyr Cys Gln Gln Gly Tyr Ile Ile Thr Asp Ile Asp Asn
           340                 345                 350
   Val Phe Gly Thr Gly Thr Lys Val Thr Val Leu Gly Gly Gly Gly Gly
       355                 360                 365
   Ser Glu Val Gln Leu Val Glu Ser Gly Gly Gly Leu Val Gln Pro Gly
       370                 375                 380
   Gly Ser Leu Arg Leu Ser Cys Ala Ala Ser Gly Phe Ser Phe Ser Asn
   385                 390                 395                 400
   Ser Tyr Trp Ile Cys Trp Val Arg Gln Ala Pro Gly Lys Cys Leu Glu
                   405                 410                 415
   Trp Ile Gly Cys Thr Phe Val Gly Ser Ser Asp Ser Thr Tyr Tyr Ala
   420 425 430
   Asn Trp Ala Lys Gly Arg Phe Thr Ile Ser Arg Asp Asn Ser Lys Asn
   435 440 445
   Thr Val Tyr Leu Gln Met Asn Ser Leu Arg Ala Glu Asp Thr Ala Val
   450 455 460
   Tyr Tyr Cys Ala Arg His Pro Ser Asp Ala Val Tyr Gly Tyr Ala Asn
   465 470 475 480
   Asn Leu Trp Gly Gln Gly Thr Leu Val Thr Val Ser Ser Gly Gly Gly
   485 490 495
   Gly Ser Gly Gly Gly Gly Ser Ile Gln Met Thr Gln Ser Pro Ser Ser
   500 505 510
   Leu Ser Ala Ser Val Gly Asp Arg Val Thr Ile Thr Cys Gln Ser Ser
   515 520 525
   Glu Ser Val Tyr Ser Asn Asn Gln Leu Ser Trp Tyr Gln Gln Lys Pro
   530 535 540
   Gly Gln Pro Pro Lys Leu Leu Ile Tyr Asp Ala Ser Asp Leu Ala Ser
   545 550 555 560
   Gly Val Pro Ser Arg Phe Ser Gly Ser Gly Ser Gly Thr Asp Phe Thr
   565 570 575
   Leu Thr Ile Ser Ser Leu Gln Pro Glu Asp Phe Ala Thr Tyr Tyr Cys
   580 585 590
   Ala Gly Gly Phe Ser Ser Ser Ser Asp Thr Ala Phe Gly Gly Gly Thr
   595 600 605
   Lys Leu Thr Val Leu Gly Gly Gly Gly Gly Ser Gly Gly Gly Gly Ser
   610 615 620
   Gly Gly Gly Gly Ser Gly Gly Gly Gly Ser Glu Val Gln Leu Val Glu
   625 630 635 640
   Ser Gly Gly Gly Leu Val Gln Pro Gly Gly Ser Leu Arg Leu Ser Cys
   645 650 655
   Ala Ala Ser Gly Phe Ser Leu Ser Ser Asn Ala Met Gly Trp Val Arg
   660 665 670
   Gln Ala Pro Gly Lys Gly Leu Glu Tyr Ile Gly Ile Ile Ser Val Gly
           675                 680                 685
   Gly Phe Thr Tyr Tyr Ala Ser Trp Ala Lys Gly Arg Phe Thr Ile Ser
       690                 695                 700
   Arg Asp Asn Ser Lys Asn Thr Val Tyr Leu Gln Met Asn Ser Leu Arg
   705                 710                 715                 720
   Ala Glu Asp Thr Ala Thr Tyr Phe Cys Ala Arg Asp Arg His Gly Gly
                   725                 730                 735
   Asp Ser Ser Gly Ala Phe Tyr Leu Trp Gly Gln Gly Thr Leu Val Thr
               740                 745                 750
   Val Ser Ser
           755

<210>  124
<211>  755
<212>  PRT
<213>  Artificial Sequence

<220>
<223>  artificial antibody-based sequence

<400>  124

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Asn Asn Val
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Ser Tyr Gly Asn Tyr Gly
                85                  90                  95

Asp Phe Gly Cys Gly Thr Lys Val Thr Val Leu Gly Gly Gly Gly Gly
            100                 105                 110

Ser Gln Val Gln Leu Gln Glu Ser Gly Pro Gly Leu Val Lys Pro Ser

115                 120                 125

Glu Thr Leu Ser Leu Thr Cys Lys Ala Ser Gly Phe Ser Phe Ser Ser
    130                 135                 140

Gly Tyr Asp Met Cys Trp Val Arg Gln Pro Pro Gly Lys Gly Leu Glu
145                 150                 155                 160

Trp Ile Ala Cys Val Val Ala Gly Ser Val Asp Ile Thr Tyr Tyr Ala
                165                 170                 175

Ser Trp Ala Lys Gly Arg Val Thr Ile Ser Lys Asp Ser Ser Lys Asn
            180                 185                 190

Gln Val Ser Leu Lys Leu Ser Ser Val Thr Ala Ala Asp Thr Ala Val
        195                 200                 205

Tyr Tyr Cys Ala Arg Lys Asp Ala Tyr Ser Asp Ala Phe Asn Leu Trp
    210                 215                 220

Gly Gln Gly Thr Leu Val Thr Val Ser Ser Gly Gly Gly Gly Ser Gly
225                 230                 235                 240

Gly Gly Gly Ser Gly Gly Gly Gly Ser Gly Gly Gly Gly Ser Ile Gln
                245                 250                 255

Met Thr Gln Ser Pro Ser Ser Leu Ser Ala Ser Val Gly Asp Arg Val
            260                 265                 270

Thr Ile Thr Cys Gln Ala Ser Gln Ser Ile Asn Asp Tyr Leu Ala Trp
        275                 280                 285

Tyr Gln Gln Lys Pro Gly Lys Ala Pro Lys Leu Leu Ile Tyr Lys Ala
    290                 295                 300

Ser Thr Leu Ala Ser Gly Val Pro Ser Arg Phe Ser Gly Ser Gly Ser
305                 310                 315                 320

Gly Thr Asp Phe Thr Leu Thr Ile Ser Ser Leu Gln Pro Glu Asp Phe
                325                 330                 335

Ala Thr Tyr Tyr Cys Gln Gln Gly Tyr Ile Ile Thr Asp Ile Asp Asn
            340                 345                 350

Val Phe Gly Thr Gly Thr Lys Val Thr Val Leu Gly Gly Gly Gly Gly
        355                 360                 365

Ser Glu Val Gln Leu Val Glu Ser Gly Gly Gly Leu Val Gln Pro Gly
    370                 375                 380

Gly Ser Leu Arg Leu Ser Cys Ala Ala Ser Gly Phe Ser Phe Ser Asn
385                 390                 395                 400

Ser Tyr Trp Ile Cys Trp Val Arg Gln Ala Pro Gly Lys Cys Leu Glu
                405                 410                 415

Trp Ile Gly Cys Thr Phe Val Gly Ser Ser Asp Ser Thr Tyr Tyr Ala
            420                 425                 430

Asn Trp Ala Lys Gly Arg Phe Thr Ile Ser Arg Asp Asn Ser Lys Asn
        435                 440                 445

Thr Val Tyr Leu Gln Met Asn Ser Leu Arg Ala Glu Asp Thr Ala Val
    450                 455                 460

Tyr Tyr Cys Ala Arg His Pro Ser Asp Ala Val Tyr Gly Tyr Ala Asn
465                 470                 475                 480

Asn Leu Trp Gly Gln Gly Thr Leu Val Thr Val Ser Ser Gly Gly Gly
                485                 490                 495

Gly Ser Gly Gly Gly Gly Ser Ile Gln Met Thr Gln Ser Pro Ser Ser
            500                 505                 510

Leu Ser Ala Ser Val Gly Asp Arg Val Thr Ile Thr Cys Gln Ser Ser
        515                 520                 525

Glu Ser Val Tyr Ser Asn Asn Gln Leu Ser Trp Tyr Gln Gln Lys Pro
    530                 535                 540

Gly Gln Pro Pro Lys Leu Leu Ile Tyr Asp Ala Ser Asp Leu Ala Ser
545                 550                 555                 560

Gly Val Pro Ser Arg Phe Ser Gly Ser Gly Ser Gly Thr Asp Phe Thr
                565                 570                 575

Leu Thr Ile Ser Ser Leu Gln Pro Glu Asp Phe Ala Thr Tyr Tyr Cys
            580                 585                 590

Ala Gly Gly Phe Ser Ser Ser Ser Asp Thr Ala Phe Gly Gly Gly Thr
        595                 600                 605

Lys Leu Thr Val Leu Gly Gly Gly Gly Gly Ser Gly Gly Gly Gly Ser
    610                 615                 620

Gly Gly Gly Gly Ser Gly Gly Gly Gly Ser Glu Val Gln Leu Val Glu
625                 630                 635                 640

Ser Gly Gly Gly Leu Val Gln Pro Gly Gly Ser Leu Arg Leu Ser Cys
                645                 650                 655

Ala Ala Ser Gly Phe Ser Leu Ser Ser Asn Ala Met Gly Trp Val Arg
            660                 665                 670

Gln Ala Pro Gly Lys Gly Leu Glu Tyr Ile Gly Ile Ile Ser Val Gly
        675                 680                 685

Gly Phe Thr Tyr Tyr Ala Ser Trp Ala Lys Gly Arg Phe Thr Ile Ser
    690                 695                 700

Arg Asp Asn Ser Lys Asn Thr Val Tyr Leu Gln Met Asn Ser Leu Arg
705                 710                 715                 720

Ala Glu Asp Thr Ala Thr Tyr Phe Cys Ala Arg Asp Arg His Gly Gly
                725                 730                 735

Asp Ser Ser Gly Ala Phe Tyr Leu Trp Gly Gln Gly Thr Leu Val Thr
            740                 745                 750

Val Ser Ser
        755

<210>  125
<211>  759
<212>  PRT
<213>  Artificial Sequence

<220>
<223>  artificial antibody-based sequence

<400>  125

Asp Ile Gln Met Thr Gln Ser Pro Ala Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Pro Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Glu Val Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Ser Phe Ser Ala Asn Tyr Tyr Pro Cys Trp Val Arg Gln Ala
145                 150                 155                 160

Pro Gly Lys Gly Leu Glu Trp Ile Gly Cys Ile Tyr Gly Gly Ser Ser
                165                 170                 175

Asp Ile Thr Tyr Asp Ala Asn Trp Thr Lys Gly Arg Phe Thr Ile Ser
            180                 185                 190

Arg Asp Asn Ser Lys Asn Thr Val Tyr Leu Gln Met Asn Ser Leu Arg
        195                 200                 205

Ala Glu Asp Thr Ala Val Tyr Tyr Cys Ala Arg Ser Ala Trp Tyr Ser
    210                 215                 220

Gly Trp Gly Gly Asp Leu Trp Gly Gln Gly Thr Leu Val Thr Val Ser
225                 230                 235                 240

Ser Gly Gly Gly Gly Ser Gly Gly Gly Gly Ser Gly Gly Gly Gly Ser
                245                 250                 255

Gly Gly Gly Gly Ser Ile Gln Met Thr Gln Ser Pro Ser Ser Leu Ser
            260                 265                 270

Ala Ser Val Gly Asp Arg Val Thr Ile Thr Cys Gln Ala Ser Gln Ser
        275                 280                 285

Ile Ser Asn Arg Leu Ala Trp Tyr Gln Gln Lys Pro Gly Lys Ala Pro
    290                 295                 300

Lys Leu Leu Ile Tyr Ser Ala Ser Thr Leu Ala Ser Gly Val Pro Ser
305                 310                 315                 320

Arg Phe Ser Gly Ser Gly Ser Gly Thr Asp Phe Thr Leu Thr Ile Ser
                325                 330                 335

Ser Leu Gln Pro Glu Asp Phe Ala Thr Tyr Tyr Cys Gln Ser Thr Tyr
            340                 345                 350

Tyr Gly Asn Asp Gly Asn Ala Phe Gly Thr Gly Thr Lys Val Thr Val
        355                 360                 365

Leu Gly Gly Gly Gly Gly Ser Glu Val Gln Leu Val Glu Ser Gly Gly
    370                 375                 380

Gly Leu Val Gln Pro Gly Gly Ser Leu Arg Leu Ser Cys Ala Ala Ser
385                 390                 395                 400

Gly Phe Ser Phe Asn Ser Asp Tyr Trp Ile Tyr Trp Val Arg Gln Ala
                405                 410                 415

Pro Gly Lys Gly Leu Glu Trp Ile Ala Ser Ile Tyr Gly Gly Ser Ser
            420                 425                 430

Gly Asn Thr Gln Tyr Ala Ser Trp Ala Gln Gly Arg Phe Thr Ile Ser
        435                 440                 445

Arg Asp Asn Ser Lys Asn Thr Val Tyr Leu Gln Met Asn Ser Leu Arg
    450                 455                 460

Ala Glu Asp Thr Ala Val Tyr Phe Cys Ala Arg Gly Tyr Val Asp Tyr
465                 470                 475                 480

Gly Gly Ala Thr Asp Leu Trp Gly Gln Gly Thr Leu Val Thr Val Ser
                485                 490                 495

Ser Gly Gly Gly Gly Ser Gly Gly Gly Gly Ser Ile Gln Met Thr Gln
            500                 505                 510

Ser Pro Ser Ser Leu Ser Ala Ser Val Gly Asp Arg Val Thr Ile Thr
        515                 520                 525

Cys Gln Ser Ser Glu Ser Val Tyr Ser Asn Asn Gln Leu Ser Trp Tyr
    530                 535                 540

Gln Gln Lys Pro Gly Gln Pro Pro Lys Leu Leu Ile Tyr Asp Ala Ser
545                 550                 555                 560

Asp Leu Ala Ser Gly Val Pro Ser Arg Phe Ser Gly Ser Gly Ser Gly
                565                 570                 575

Thr Asp Phe Thr Leu Thr Ile Ser Ser Leu Gln Pro Glu Asp Phe Ala
            580                 585                 590

Thr Tyr Tyr Cys Ala Gly Gly Phe Ser Ser Ser Ser Asp Thr Ala Phe
        595                 600                 605

Gly Gly Gly Thr Lys Leu Thr Val Leu Gly Gly Gly Gly Gly Ser Gly
    610                 615                 620

Gly Gly Gly Ser Gly Gly Gly Gly Ser Gly Gly Gly Gly Ser Glu Val
625                 630                 635                 640

Gln Leu Val Glu Ser Gly Gly Gly Leu Val Gln Pro Gly Gly Ser Leu
                645                 650                 655

Arg Leu Ser Cys Ala Ala Ser Gly Phe Ser Leu Ser Ser Asn Ala Met
            660                 665                 670

Gly Trp Val Arg Gln Ala Pro Gly Lys Gly Leu Glu Tyr Ile Gly Ile
        675                 680                 685

Ile Ser Val Gly Gly Phe Thr Tyr Tyr Ala Ser Trp Ala Lys Gly Arg
    690                 695                 700

Phe Thr Ile Ser Arg Asp Asn Ser Lys Asn Thr Val Tyr Leu Gln Met
705                 710                 715                 720

Asn Ser Leu Arg Ala Glu Asp Thr Ala Thr Tyr Phe Cys Ala Arg Asp
                725                 730                 735

Arg His Gly Gly Asp Ser Ser Gly Ala Phe Tyr Leu Trp Gly Gln Gly
            740                 745                 750

Thr Leu Val Thr Val Ser Ser
        755

<210>  126
<211>  759
<212>  PRT
<213>  Artificial Sequence

<220>
<223>  artificial antibody-based sequence

<400>  126

Asp Ile Gln Met Thr Gln Ser Pro Ala Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Pro Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Glu Ser Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Ser Phe Ser Ala Asn Tyr Tyr Pro Cys Trp Val Arg Gln Ala
145                 150                 155                 160

Pro Gly Lys Gly Leu Glu Trp Ile Gly Cys Ile Tyr Gly Gly Ser Ser
                165                 170                 175

Asp Ile Thr Tyr Asp Ala Asn Trp Thr Lys Gly Arg Phe Thr Ile Ser
            180                 185                 190

Arg Asp Asn Ser Lys Asn Thr Val Tyr Leu Gln Met Asn Ser Leu Arg
        195                 200                 205

Ala Glu Asp Thr Ala Val Tyr Phe Cys Ala Arg Ser Ala Trp Tyr Ser
    210                 215                 220

Gly Trp Gly Gly Asp Leu Trp Gly Pro Gly Thr Leu Val Thr Val Ser
225                 230                 235                 240

Ser Gly Gly Gly Gly Ser Gly Gly Gly Gly Ser Gly Gly Gly Gly Ser
                245                 250                 255

Gly Gly Gly Gly Ser Phe Gln Leu Thr Gln Ser Pro Ser Ser Leu Ser
            260                 265                 270

Ala Ser Val Gly Asp Arg Val Thr Ile Thr Cys Gln Ala Ser Gln Ser
        275                 280                 285

Ile Ser Asn Arg Leu Ala Trp Tyr Gln Gln Lys Pro Gly Lys Pro Pro
    290                 295                 300

Lys Leu Leu Ile Tyr Ser Ala Ser Thr Leu Ala Ser Gly Val Pro Ser
305                 310                 315                 320

Arg Phe Ser Gly Ser Gly Ser Gly Thr Asp Phe Thr Leu Thr Ile Ser
                325                 330                 335

Ser Leu Gln Pro Glu Asp Phe Ala Thr Tyr Tyr Cys Gln Ser Thr Tyr
            340                 345                 350

Tyr Gly Asn Asp Gly Asn Ala Phe Gly Thr Gly Thr Lys Val Thr Val
        355                 360                 365

Leu Gly Gly Gly Gly Gly Ser Glu Val Gln Leu Val Glu Ser Gly Gly
    370                 375                 380

Gly Leu Val Gln Pro Gly Gly Ser Leu Arg Leu Ser Cys Ala Ala Ser
385                 390                 395                 400

Gly Phe Ser Phe Asn Ser Asp Tyr Trp Ile Tyr Trp Val Arg Gln Ala
                405                 410                 415

Pro Gly Lys Gly Leu Glu Trp Ile Ala Ser Ile Tyr Gly Gly Ser Ser
            420                 425                 430

Gly Asn Thr Gln Tyr Ala Ser Trp Ala Gln Gly Arg Phe Thr Ile Ser
        435                 440                 445

Arg Asp Asn Ser Lys Asn Thr Val Tyr Leu Gln Met Asn Ser Leu Arg
    450                 455                 460

Ala Glu Asp Thr Ala Val Tyr Phe Cys Ala Arg Gly Tyr Val Asp Tyr
465                 470                 475                 480

Gly Gly Ala Thr Asp Leu Trp Gly Gln Gly Thr Leu Val Thr Val Ser
                485                 490                 495

Ser Gly Gly Gly Gly Ser Gly Gly Gly Gly Ser Ile Gln Met Thr Gln
            500                 505                 510

Ser Pro Ser Ser Leu Ser Ala Ser Val Gly Asp Arg Val Thr Ile Thr

515                 520                 525

Cys Gln Ser Ser Glu Ser Val Tyr Ser Asn Asn Gln Leu Ser Trp Tyr
    530                 535                 540

Gln Gln Lys Pro Gly Gln Pro Pro Lys Leu Leu Ile Tyr Asp Ala Ser
545                 550                 555                 560

Asp Leu Ala Ser Gly Val Pro Ser Arg Phe Ser Gly Ser Gly Ser Gly
                565                 570                 575

Thr Asp Phe Thr Leu Thr Ile Ser Ser Leu Gln Pro Glu Asp Phe Ala
            580                 585                 590

Thr Tyr Tyr Cys Ala Gly Gly Phe Ser Ser Ser Ser Asp Thr Ala Phe
        595                 600                 605

Gly Gly Gly Thr Lys Leu Thr Val Leu Gly Gly Gly Gly Gly Ser Gly
    610                 615                 620

Gly Gly Gly Ser Gly Gly Gly Gly Ser Gly Gly Gly Gly Ser Glu Val
625                 630                 635                 640

Gln Leu Val Glu Ser Gly Gly Gly Leu Val Gln Pro Gly Gly Ser Leu
                645                 650                 655

Arg Leu Ser Cys Ala Ala Ser Gly Phe Ser Leu Ser Ser Asn Ala Met
            660                 665                 670

Gly Trp Val Arg Gln Ala Pro Gly Lys Gly Leu Glu Tyr Ile Gly Ile
        675                 680                 685

Ile Ser Val Gly Gly Phe Thr Tyr Tyr Ala Ser Trp Ala Lys Gly Arg
    690                 695                 700

Phe Thr Ile Ser Arg Asp Asn Ser Lys Asn Thr Val Tyr Leu Gln Met
705                 710                 715                 720

Asn Ser Leu Arg Ala Glu Asp Thr Ala Thr Tyr Phe Cys Ala Arg Asp
                725                 730                 735

Arg His Gly Gly Asp Ser Ser Gly Ala Phe Tyr Leu Trp Gly Gln Gly
            740                 745                 750

Thr Leu Val Thr Val Ser Ser
        755

<210>  127
<211>  759
<212>  PRT
<213>  Artificial Sequence

<220>
<223>  artificial antibody-based sequence

<400>  127

Asp Ile Gln Met Thr Gln Ser Pro Ala Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Pro Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Glu Val Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Ser Phe Ser Ala Asn Tyr Tyr Pro Cys Trp Val Arg Gln Ala
145                 150                 155                 160

Pro Gly Lys Cys Leu Glu Trp Ile Gly Cys Ile Tyr Gly Gly Ser Ser
                165                 170                 175

Asp Ile Thr Tyr Asp Ala Asn Trp Thr Lys Gly Arg Phe Thr Ile Ser
            180                 185                 190

Arg Asp Asn Ser Lys Asn Thr Val Tyr Leu Gln Met Asn Ser Leu Arg
        195                 200                 205

Ala Glu Asp Thr Ala Val Tyr Tyr Cys Ala Arg Ser Ala Trp Tyr Ser

210                 215                 220

Gly Trp Gly Gly Asp Leu Trp Gly Gln Gly Thr Leu Val Thr Val Ser
225                 230                 235                 240

Ser Gly Gly Gly Gly Ser Gly Gly Gly Gly Ser Gly Gly Gly Gly Ser
                245                 250                 255

Gly Gly Gly Gly Ser Ile Gln Met Thr Gln Ser Pro Ser Ser Leu Ser
            260                 265                 270

Ala Ser Val Gly Asp Arg Val Thr Ile Thr Cys Gln Ala Ser Gln Ser
        275                 280                 285

Ile Ser Asn Arg Leu Ala Trp Tyr Gln Gln Lys Pro Gly Lys Ala Pro
    290                 295                 300

Lys Leu Leu Ile Tyr Ser Ala Ser Thr Leu Ala Ser Gly Val Pro Ser
305                 310                 315                 320

Arg Phe Ser Gly Ser Gly Ser Gly Thr Asp Phe Thr Leu Thr Ile Ser
                325                 330                 335

Ser Leu Gln Pro Glu Asp Phe Ala Thr Tyr Tyr Cys Gln Ser Thr Tyr
            340                 345                 350

Tyr Gly Asn Asp Gly Asn Ala Phe Gly Cys Gly Thr Lys Val Thr Val
        355                 360                 365

Leu Gly Gly Gly Gly Gly Ser Glu Val Gln Leu Val Glu Ser Gly Gly
    370                 375                 380

Gly Leu Val Gln Pro Gly Gly Ser Leu Arg Leu Ser Cys Ala Ala Ser
385                 390                 395                 400

Gly Phe Ser Phe Asn Ser Asp Tyr Trp Ile Tyr Trp Val Arg Gln Ala
                405                 410                 415

Pro Gly Lys Gly Leu Glu Trp Ile Ala Ser Ile Tyr Gly Gly Ser Ser
            420                 425                 430

Gly Asn Thr Gln Tyr Ala Ser Trp Ala Gln Gly Arg Phe Thr Ile Ser
        435                 440                 445

Arg Asp Asn Ser Lys Asn Thr Val Tyr Leu Gln Met Asn Ser Leu Arg
    450                 455                 460

Ala Glu Asp Thr Ala Val Tyr Phe Cys Ala Arg Gly Tyr Val Asp Tyr
465                 470                 475                 480

Gly Gly Ala Thr Asp Leu Trp Gly Gln Gly Thr Leu Val Thr Val Ser
                485                 490                 495

Ser Gly Gly Gly Gly Ser Gly Gly Gly Gly Ser Ile Gln Met Thr Gln
            500                 505                 510

Ser Pro Ser Ser Leu Ser Ala Ser Val Gly Asp Arg Val Thr Ile Thr
        515                 520                 525

Cys Gln Ser Ser Glu Ser Val Tyr Ser Asn Asn Gln Leu Ser Trp Tyr
    530                 535                 540

Gln Gln Lys Pro Gly Gln Pro Pro Lys Leu Leu Ile Tyr Asp Ala Ser
545                 550                 555                 560

Asp Leu Ala Ser Gly Val Pro Ser Arg Phe Ser Gly Ser Gly Ser Gly
                565                 570                 575

Thr Asp Phe Thr Leu Thr Ile Ser Ser Leu Gln Pro Glu Asp Phe Ala
            580                 585                 590

Thr Tyr Tyr Cys Ala Gly Gly Phe Ser Ser Ser Ser Asp Thr Ala Phe
        595                 600                 605

Gly Gly Gly Thr Lys Leu Thr Val Leu Gly Gly Gly Gly Gly Ser Gly
    610                 615                 620

Gly Gly Gly Ser Gly Gly Gly Gly Ser Gly Gly Gly Gly Ser Glu Val
625                 630                 635                 640

Gln Leu Val Glu Ser Gly Gly Gly Leu Val Gln Pro Gly Gly Ser Leu
                645                 650                 655

Arg Leu Ser Cys Ala Ala Ser Gly Phe Ser Leu Ser Ser Asn Ala Met
            660                 665                 670

Gly Trp Val Arg Gln Ala Pro Gly Lys Gly Leu Glu Tyr Ile Gly Ile
        675                 680                 685

Ile Ser Val Gly Gly Phe Thr Tyr Tyr Ala Ser Trp Ala Lys Gly Arg
    690                 695                 700

Phe Thr Ile Ser Arg Asp Asn Ser Lys Asn Thr Val Tyr Leu Gln Met
705                 710                 715                 720

Asn Ser Leu Arg Ala Glu Asp Thr Ala Thr Tyr Phe Cys Ala Arg Asp
                725                 730                 735

Arg His Gly Gly Asp Ser Ser Gly Ala Phe Tyr Leu Trp Gly Gln Gly
            740                 745                 750

Thr Leu Val Thr Val Ser Ser
        755

<210>  128
<211>  761
<212>  PRT
<213>  Artificial Sequence

<220>
<223>  artificial antibody-based sequence

<400>  128

Met Thr Gln Ser Pro Ser Ser Leu Ser Ala Ser Val Gly Asp Arg Val
1               5                   10                  15

Thr Ile Thr Cys Gln Ala Ser Gln Ser Ile Gly Thr Tyr Leu Ala Trp
            20                  25                  30

Tyr Gln Gln Lys Pro Gly Lys Ala Pro Lys Leu Leu Ile Tyr Arg Ala
        35                  40                  45

Phe Ile Leu Ala Ser Gly Val Pro Ser Arg Phe Ser Gly Ser Gly Ser
    50                  55                  60

Gly Thr Asp Phe Thr Leu Thr Ile Ser Ser Leu Gln Pro Glu Asp Phe
65                  70                  75                  80

Ala Thr Tyr Tyr Cys Gln Ser Asn Phe Tyr Ser Asp Ser Thr Thr Ile
                85                  90                  95

Gly Pro Asn Ala Phe Gly Thr Gly Thr Lys Val Thr Val Leu Gly Gly
            100                 105                 110

Gly Gly Gly Ser Glu Val Gln Leu Val Glu Ser Gly Gly Gly Leu Val
        115                 120                 125

Gln Pro Gly Gly Ser Leu Arg Leu Ser Cys Ala Ala Ser Gly Phe Ser
    130                 135                 140

Phe Ser Asn Ser Tyr Trp Ile Cys Trp Val Arg Gln Ala Pro Gly Lys
145                 150                 155                 160

Cys Leu Glu Trp Ile Gly Cys Thr Phe Val Gly Ser Ser Asp Ser Thr
                    165                 170                 175

Tyr Tyr Ala Asn Trp Ala Lys Gly Arg Phe Thr Ile Ser Arg Asp Asn
            180                 185                 190

Ser Lys Asn Thr Val Tyr Leu Gln Met Asn Ser Leu Arg Ala Glu Asp
        195                 200                 205

Thr Ala Val Tyr Tyr Cys Ala Arg His Pro Ser Asp Ala Val Tyr Gly
    210                 215                 220

Tyr Ala Asn Asn Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ala Ser Gln Ser Ile
        275                 280                 285

Asn Asn Val Leu Ala Trp Tyr Gln Gln Lys Pro Gly Lys Ala Pro Lys
    290                 295                 300

Leu Leu Ile Tyr Arg Ala Ser Thr Leu Ala Ser Gly Val Pro Ser Arg
305                 310                 315                 320

Phe Ser Gly Ser Gly Ser Gly Thr Asp Phe Thr Leu Thr Ile Ser Ser
                325                 330                 335

Leu Gln Pro Glu Asp Phe Ala Thr Tyr Tyr Cys Gln Ser Ser Tyr Gly
            340                 345                 350

Asn Tyr Gly Asp Phe Gly Cys Gly Thr Lys Val Thr Val Leu Gly Gly
        355                 360                 365

Gly Gly Gly Ser Gln Val Gln Leu Gln Glu Ser Gly Pro Gly Leu Val
    370                 375                 380

Lys Pro Ser Glu Thr Leu Ser Leu Thr Cys Lys Val Ser Gly Phe Ser
385                 390                 395                 400

Phe Asn Ser Asp Tyr Trp Ile Tyr Trp Ile Arg Gln Pro Pro Gly Lys
                405                 410                 415

Gly Leu Glu Trp Ile Gly Ser Ile Tyr Gly Gly Ser Ser Gly Asn Thr
        420                 425                 430

Gln Tyr Ala Ser Trp Ala Gln Gly Arg Val Thr Ile Ser Val Asp Ser
    435                 440                 445

Ser Lys Asn Gln Phe Ser Leu Lys Leu Ser Ser Val Thr Ala Ala Asp
    450                 455                 460

Thr Ala Val Tyr Tyr Cys Ala Arg Gly Tyr Val Asp Tyr Gly Gly Ala
465                 470                 475                 480

Thr Asp Leu Trp Gly Gln Gly Thr Leu Val Thr Val Ser Ser Gly Gly
                485                 490                 495

Gly Gly Ser Gly Gly Gly Gly Ser Val Val Met Thr Gln Ser Pro Ser
            500                 505                 510

Ser Leu Ser Ala Ser Val Gly Asp Arg Val Thr Ile Thr Cys Gln Ala
        515                 520                 525

Ser Gln Ile Ile Ser Ser Arg Ser Ala Trp Tyr Gln Gln Lys Pro Gly
    530                 535                 540

Gln Pro Pro Lys Leu Leu Ile Tyr Gln Ala Ser Lys Leu Ala Ser Gly
545                 550                 555                 560

Val Pro Ser Arg Phe Ser Gly Ser Gly Ser Gly Thr Asp Phe Thr Leu
                565                 570                 575

Thr Ile Ser Ser Leu Gln Pro Glu Asp Phe Ala Thr Tyr Tyr Cys Gln
            580                 585                 590

Cys Thr Tyr Ile Asp Ser Asn Phe Gly Ala Phe Gly Gly Gly Thr Lys
        595                 600                 605

Leu Thr Val Leu Gly Gly Gly Gly Gly Ser Gly Gly Gly Gly Ser Gly
    610                 615                 620

Gly Gly Gly Ser Gly Gly Gly Gly Ser Glu Val Gln Leu Val Glu Ser
625                 630                 635                 640

Gly Gly Gly Leu Val Gln Pro Gly Gly Ser Leu Arg Leu Ser Cys Ala
                645                 650                 655

Ala Ser Gly Phe Ser Phe Ser Ser Ser Tyr Trp Ile Cys Trp Val Arg
            660                 665                 670

Gln Ala Pro Gly Lys Gly Leu Glu Trp Val Gly Cys Val Phe Thr Gly
        675                 680                 685

Asp Gly Thr Thr Tyr Tyr Ala Ser Trp Ala Lys Gly Arg Phe Thr Ile
    690                 695                 700

Ser Arg Asp Asn Ser Lys Asn Thr Val Tyr Leu Gln Met Asn Ser Leu
705                 710                 715                 720

Arg Ala Glu Asp Thr Ala Thr Tyr Phe Cys Ala Arg Pro Val Ser Val
                725                 730                 735

Tyr Tyr Tyr Gly Met Asp Leu Trp Gly Gln Gly Thr Leu Val Thr Val
            740                 745                 750

Ser Ser Leu Glu Ser Arg Gly Pro Val
        755                 760

<210> 129
<211> 757
<212> PRT
<213> Artificial Sequence

<220>
<223> artificial antibody-based sequence

<400> 129

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Ala Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Ser Glu Val Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Ser Phe Ser Asn Ser Tyr Trp Ile Cys Trp Val Arg Gln Ala
145                 150                 155                 160

Pro Gly Lys Cys Leu Glu Trp Ile Gly Cys Thr Phe Val Gly Ser Ser
                165                 170                 175

Asp Ser Thr Tyr Tyr Ala Asn Trp Ala Lys Gly Arg Phe Thr Ile Ser
            180                 185                 190

Arg Asp Asn Ser Lys Asn Thr Val Tyr Leu Gln Met Asn Ser Leu Arg
        195                 200                 205

Ala Glu Asp Thr Ala Val Tyr Tyr Cys Ala Arg His Pro Ser Asp Ala
    210                 215                 220

Val Tyr Gly Tyr Ala Asn Asn Leu Trp Gly Gln Gly Thr Leu Val Thr
225                 230                 235                 240

Val Ser Ser Gly Gly Gly Gly Ser Gly Gly Gly Gly Ser Gly Gly Gly
                245                 250                 255

Gly Ser Gly Gly Gly Gly Ser Ile Gln Met Thr Gln Ser Pro Ser Ser
            260                 265                 270

Leu Ser Ala Ser Val Gly Asp Arg Val Thr Ile Thr Cys Gln Ala Ser
        275                 280                 285

Gln Ser Ile Asn Asn Val Leu Ala Trp Tyr Gln Gln Lys Pro Gly Lys
    290                 295                 300

Ala Pro Lys Leu Leu Ile Tyr Arg Ala Ser Thr Leu Ala Ser Gly Val
305                 310                 315                 320

Pro Ser Arg Phe Ser Gly Ser Gly Ser Gly Thr Asp Phe Thr Leu Thr
                325                 330                 335

Ile Ser Ser Leu Gln Pro Glu Asp Phe Ala Thr Tyr Tyr Cys Gln Ser
            340                 345                 350

Ser Tyr Gly Asn Tyr Gly Asp Phe Gly Cys Gly Thr Lys Val Thr Val
        355                 360                 365

Leu Gly Gly Gly Gly Gly Ser Gln Val Gln Leu Gln Glu Ser Gly Pro
    370                 375                 380

Gly Leu Val Lys Pro Ser Glu Thr Leu Ser Leu Thr Cys Lys Val Ser
385                 390                 395                 400

Gly Phe Ser Phe Asn Ser Asp Tyr Trp Ile Tyr Trp Ile Arg Gln Pro
                405                 410                 415

Pro Gly Lys Gly Leu Glu Trp Ile Gly Ser Ile Tyr Gly Gly Ser Ser
            420                 425                 430

Gly Asn Thr Gln Tyr Ala Ser Trp Ala Gln Gly Arg Val Thr Ile Ser
        435                 440                 445

Val Asp Ser Ser Lys Asn Gln Phe Ser Leu Lys Leu Ser Ser Val Thr
    450                 455                 460

Ala Ala Asp Thr Ala Val Tyr Tyr Cys Ala Arg Gly Tyr Val Asp Tyr
465                 470                 475                 480

Gly Gly Ala Thr Asp Leu Trp Gly Gln Gly Thr Leu Val Thr Val Ser
                485                 490                 495

Ser Gly Gly Gly Gly Ser Gly Gly Gly Gly Ser Val Val Met Thr Gln
            500                 505                 510

Ser Pro Ser Ser Leu Ser Ala Ser Val Gly Asp Arg Val Thr Ile Thr
        515                 520                 525

Cys Gln Ala Ser Gln Ile Ile Ser Ser Arg Ser Ala Trp Tyr Gln Gln
    530                 535                 540

Lys Pro Gly Gln Pro Pro Lys Leu Leu Ile Tyr Gln Ala Ser Lys Leu
545                 550                 555                 560

Ala Ser Gly Val Pro Ser Arg Phe Ser Gly Ser Gly Ser Gly Thr Asp
                565                 570                 575

Phe Thr Leu Thr Ile Ser Ser Leu Gln Pro Glu Asp Phe Ala Thr Tyr
            580                 585                 590

Tyr Cys Gln Cys Thr Tyr Ile Asp Ser Asn Phe Gly Ala Phe Gly Gly
        595                 600                 605

Gly Thr Lys Leu Thr Val Leu Gly Gly Gly Gly Gly Ser Gly Gly Gly

610                 615                 620

Gly Ser Gly Gly Gly Gly Ser Gly Gly Gly Gly Ser Glu Val Gln Leu
625                 630                 635                 640

Val Glu Ser Gly Gly Gly Leu Val Gln Pro Gly Gly Ser Leu Arg Leu
                645                 650                 655

Ser Cys Ala Ala Ser Gly Phe Ser Phe Ser Ser Ser Tyr Trp Ile Cys
            660                 665                 670

Trp Val Arg Gln Ala Pro Gly Lys Gly Leu Glu Trp Val Gly Cys Val
        675                 680                 685

Phe Thr Gly Asp Gly Thr Thr Tyr Tyr Ala Ser Trp Ala Lys Gly Arg
    690                 695                 700

Phe Thr Ile Ser Arg Asp Asn Ser Lys Asn Thr Val Tyr Leu Gln Met
705                 710                 715                 720

Asn Ser Leu Arg Ala Glu Asp Thr Ala Thr Tyr Phe Cys Ala Arg Pro
                725                 730                 735

Val Ser Val Tyr Tyr Tyr Gly Met Asp Leu Trp Gly Gln Gly Thr Leu
            740                 745                 750

Val Thr Val Ser Ser
        755

<210>  130
<211>  757
<212>  PRT
<213>  Artificial Sequence

<220>
<223>  artificial antibody-based sequence

<400>  130

Asp Ile Gln Met Thr Gln Ser Pro Ala Ser Leu Ser Ala Ser Val Gly
1               5                   10                  15

Asp Arg Val Thr Ile Thr Cys Gln Ala Ser Gln Ser Ile Gly Thr Tyr
            20                  25                  30

Leu Ala Trp Tyr Gln Gln Lys Pro Gly Lys Pro Pro Lys Leu Leu Ile
        35                  40                  45

Tyr Arg Ala Phe Ile Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Asn Phe Tyr Ser Asp Ser
                85                  90                  95

Thr Thr Ile Gly Pro Asn Ala Phe Gly Thr Gly Thr Lys Val Thr Val
            100                 105                 110

Leu Gly Gly Gly Gly Gly Ser Glu Val Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Ser Phe Ser Ala Asn Tyr Tyr Pro Cys Trp Val Arg Gln Ala
145                 150                 155                 160

Pro Gly Lys Gly Leu Glu Trp Ile Gly Cys Ile Tyr Gly Gly Ser Ser
                165                 170                 175

Asp Ile Thr Tyr Asp Ala Asn Trp Thr Lys Gly Arg Phe Thr Ile Ser
            180                 185                 190

Arg Asp Asn Ser Lys Asn Thr Val Tyr Leu Gln Met Asn Ser Leu Arg
        195                 200                 205

Ala Glu Asp Thr Ala Val Tyr Tyr Cys Ala Arg Ser Ala Trp Tyr Ser
    210                 215                 220

Gly Trp Gly Gly Asp Leu Trp Gly Gln Gly Thr Leu Val Thr Val Ser
225                 230                 235                 240

Ser Gly Gly Gly Gly Ser Gly Gly Gly Gly Ser Gly Gly Gly Gly Ser
                245                 250                 255

Gly Gly Gly Gly Ser Ile Gln Met Thr Gln Ser Pro Ser Ser Leu Ser
            260                 265                 270

Ala Ser Val Gly Asp Arg Val Thr Ile Thr Cys Gln Ala Ser Gln Ser
        275                 280                 285

Ile Ser Asn Arg Leu Ala Trp Tyr Gln Gln Lys Pro Gly Lys Ala Pro
    290                 295                 300

Lys Leu Leu Ile Tyr Ser Ala Ser Thr Leu Ala Ser Gly Val Pro Ser

305                 310                 315                 320

Arg Phe Ser Gly Ser Gly Ser Gly Thr Asp Phe Thr Leu Thr Ile Ser
                325                 330                 335

Ser Leu Gln Pro Glu Asp Phe Ala Thr Tyr Tyr Cys Gln Ser Thr Tyr
            340                 345                 350

Tyr Gly Asn Asp Gly Asn Ala Phe Gly Thr Gly Thr Lys Val Thr Val
        355                 360                 365

Leu Gly Gly Gly Gly Gly Ser Glu Val Gln Leu Val Glu Ser Gly Gly
    370                 375                 380

Gly Leu Val Gln Pro Gly Gly Ser Leu Arg Leu Ser Cys Ala Ala Ser
385                 390                 395                 400

Gly Phe Ser Phe Asn Ser Asp Tyr Trp Ile Tyr Trp Val Arg Gln Ala
                405                 410                 415

Pro Gly Lys Gly Leu Glu Trp Ile Ala Ser Ile Tyr Gly Gly Ser Ser
            420                 425                 430

Gly Asn Thr Gln Tyr Ala Ser Trp Ala Gln Gly Arg Phe Thr Ile Ser
        435                 440                 445

Arg Asp Asn Ser Lys Asn Thr Val Tyr Leu Gln Met Asn Ser Leu Arg
    450                 455                 460

Ala Glu Asp Thr Ala Val Tyr Phe Cys Ala Arg Gly Tyr Val Asp Tyr
465                 470                 475                 480

Gly Gly Ala Thr Asp Leu Trp Gly Gln Gly Thr Leu Val Thr Val Ser
                485                 490                 495

Ser Gly Gly Gly Gly Ser Gly Gly Gly Gly Ser Val Val Met Thr Gln
            500                 505                 510

Ser Pro Ser Ser Leu Ser Ala Ser Val Gly Asp Arg Val Thr Ile Thr
        515                 520                 525

Cys Gln Ala Ser Gln Ile Ile Ser Ser Arg Ser Ala Trp Tyr Gln Gln
    530                 535                 540

Lys Pro Gly Gln Pro Pro Lys Leu Leu Ile Tyr Gln Ala Ser Lys Leu
545                 550                 555                 560

Ala Ser Gly Val Pro Ser Arg Phe Ser Gly Ser Gly Ser Gly Thr Asp
                565                 570                 575

Phe Thr Leu Thr Ile Ser Ser Leu Gln Pro Glu Asp Phe Ala Thr Tyr
            580                 585                 590

Tyr Cys Gln Cys Thr Tyr Ile Asp Ser Asn Phe Gly Ala Phe Gly Gly
        595                 600                 605

Gly Thr Lys Leu Thr Val Leu Gly Gly Gly Gly Gly Ser Gly Gly Gly
    610                 615                 620

Gly Ser Gly Gly Gly Gly Ser Gly Gly Gly Gly Ser Glu Val Gln Leu
625                 630                 635                 640

Val Glu Ser Gly Gly Gly Leu Val Gln Pro Gly Gly Ser Leu Arg Leu
                645                 650                 655

Ser Cys Ala Ala Ser Gly Phe Ser Phe Ser Ser Ser Tyr Trp Ile Cys
            660                 665                 670

Trp Val Arg Gln Ala Pro Gly Lys Gly Leu Glu Trp Val Gly Cys Val
        675                 680                 685

Phe Thr Gly Asp Gly Thr Thr Tyr Tyr Ala Ser Trp Ala Lys Gly Arg
    690                 695                 700

Phe Thr Ile Ser Arg Asp Asn Ser Lys Asn Thr Val Tyr Leu Gln Met
705                 710                 715                 720

Asn Ser Leu Arg Ala Glu Asp Thr Ala Thr Tyr Phe Cys Ala Arg Pro
                725                 730                 735

Val Ser Val Tyr Tyr Tyr Gly Met Asp Leu Trp Gly Gln Gly Thr Leu
            740                 745                 750

Val Thr Val Ser Ser
        755

<210>  131
<211>  10
<212>  PRT
<213>  Artificial Sequence

<220>
<223>  artificial antibody-based sequence

<400>  131

Gly Phe Ser Leu Ser Ser Tyr Asp Met Ser

1               5                   10


<210>  132
<211>  16
<212>  PRT
<213>  Artificial Sequence

<220>
<223>  artificial antibody-based sequence

<400>  132

Ala Ser Tyr Ala Ser Gly Pro Thr Tyr Tyr Ala Ser Trp Ala Lys Gly
1               5                   10                  15


<210>  133
<211>  12
<212>  PRT
<213>  Artificial Sequence

<220>
<223>  artificial antibody-based sequence

<400>  133

Arg Gly Gly Trp Thr Gly Thr Ser His Ser Asn Ile
1               5                   10


<210>  134
<211>  119
<212>  PRT
<213>  Artificial Sequence

<220>
<223>  artificial antibody-based sequence

<400>  134

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ser Leu Ser Ser Tyr
            20                  25                  30


Asp Met Ser Trp Val Arg Gln Ala Pro Gly Lys Gly Leu Ala Trp Ile
        35                  40                  45


Gly Ala Ser Tyr Ala Ser Gly Pro Thr Tyr Tyr Ala Ser Trp Ala Lys
    50                  55                  60


Gly Arg Phe Thr Ile Ser Arg Asp Asn Ser Lys Asn Thr Val Tyr Leu
65                  70                  75                  80


Gln Met Asn Ser Leu Arg Ala Glu Asp Thr Ala Thr Tyr Phe Cys Ala
                85                  90                  95

Arg Gly Gly Trp Thr Gly Thr Ser His Ser Asn Ile Trp Gly Gln Gly
            100                 105                 110

Thr Leu Val Thr Val Ser Ser
        115

<210>  135
<211>  13
<212>  PRT
<213>  Artificial Sequence

<220>
<223>  artificial antibody-based sequence

<400>  135

Gln Ser Ser Gln Ser Val Phe Ser Asn Asn Tyr Leu Ala
1               5                   10

<210>  136
<211>  7
<212>  PRT
<213>  Artificial Sequence

<220>
<223>  artificial antibody-based sequence

<400>  136

Ser Ala Ser Thr Leu Ala Ser
1               5

<210>  137
<211>  13
<212>  PRT
<213>  Artificial Sequence

<220>
<223>  artificial antibody-based sequence

<400>  137

Leu Gly Ser Tyr Ala Cys Ser Ser Ala Asp Cys Tyr Val
1               5                   10

<210>  138
<211>  114
<212>  PRT
<213>  Artificial Sequence

<220>
<223>  artificial antibody-based sequence

<400>  138

Asp Ile Gln Met Thr Gln Ser Pro Ser Ser Leu Ser Ala Ser Val Gly

1               5                   10                  15

Asp Arg Val Thr Ile Thr Cys Gln Ser Ser Gln Ser Val Phe Ser Asn
            20                  25                  30

Asn Tyr Leu Ala Trp Phe Gln Gln Lys Pro Gly Gln Ser Pro Lys Arg
        35                  40                  45

Leu Ile Tyr Ser Ala Ser Thr Leu Ala Ser Gly Val Pro Ser Arg Phe
    50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Leu Gly Ser Tyr Ala Cys
                85                  90                  95

Ser Ser Ala Asp Cys Tyr Val Phe Gly Thr Gly Thr Lys Val Thr Val
            100                 105                 110

Leu Gly

<210>  139
<211>  11
<212>  PRT
<213>  Artificial Sequence

<220>
<223>  artificial antibody-based sequence

<400>  139

Gly Ile Ser Val Ser Asn Asp Tyr Tyr Met Cys
1               5                   10

<210>  140
<211>  17
<212>  PRT
<213>  Artificial Sequence

<220>
<223>  artificial antibody-based sequence

<400>  140

Cys Ile Ser Thr Tyr Ile Gly Asn Thr His Tyr Ala Ser Trp Ala Lys
1               5                   10                  15

Gly

<210>  141
<211>  14
<212>  PRT
<213>  Artificial Sequence

<220>
<223>  artificial antibody-based sequence

<400>  141

Lys Asn Ala Gly Tyr Pro Gly Tyr Arg Tyr Ala Ile Asp Leu
1               5                   10


<210>  142
<211>  123
<212>  PRT
<213>  Artificial Sequence

<220>
<223>  artificial antibody-based sequence

<400>  142

Glu Val Gln Leu Val Glu Ser Gly Gly Gly Leu Val Gln Pro Gly Gly
1               5                   10                  15


Ser Leu Arg Leu Ser Cys Ala Ala Ser Gly Ile Ser Val Ser Asn Asp
            20                  25                  30


Tyr Tyr Met Cys Trp Val Arg Gln Ala Pro Gly Lys Gly Leu Glu Trp
        35                  40                  45


Ile Gly Cys Ile Ser Thr Tyr Ile Gly Asn Thr His Tyr Ala Ser Trp
    50                  55                  60


Ala Lys Gly Arg Phe Thr Ile Ser Arg Asp Asn Ser Lys Asn Thr Val
65                  70                  75                  80


Tyr Leu Gln Met Asn Ser Leu Arg Ala Glu Asp Thr Ala Val Tyr Tyr
                85                  90                  95


Cys Ala Lys Asn Ala Gly Tyr Pro Gly Tyr Arg Tyr Ala Ile Asp Leu
            100                 105                 110


Trp Gly Gln Gly Thr Leu Val Thr Val Ser Ser
        115                 120


<210>  143
<211>  11
<212>  PRT
<213>  Artificial Sequence

<220>
<223>  artificial antibody-based sequence

<400>  143

Gln Ala Ser Glu Ser Ile Gly Asn Tyr Leu Ala
1               5                   10


<210>  144
<211>  7
<212>  PRT
<213>  Artificial Sequence

<220>
<223>  artificial antibody-based sequence

<400>  144

Ser Ala Ser Thr Leu Ala Ser
1               5


<210>  145
<211>  10
<212>  PRT
<213>  Artificial Sequence

<220>
<223>  artificial antibody-based sequence

<400>  145

Gln Ser Thr Asp Tyr Gly Asp Ser Tyr Ile
1               5                   10


<210>  146
<211>  109
<212>  PRT
<213>  Artificial Sequence

<220>
<223>  artificial antibody-based sequence

<400>  146

Asp Ile Gln Met Thr Gln Ser Pro Ser Ser Leu Ser Ala Ser Val Gly
1               5                   10                  15


Asp Arg Val Thr Ile Thr Cys Gln Ala Ser Glu Ser Ile Gly Asn Tyr
            20                  25                  30


Leu Ala Trp Tyr Gln Gln Lys Pro Gly Lys Ala Pro Lys Leu Leu Ile
        35                  40                  45


Tyr Ser Ala Ser Thr Leu Ala Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Thr Asp Tyr Gly Asp Ser
                85                  90                  95

Tyr Ile Phe Gly Thr Gly Thr Lys Val Thr Val Leu Gly
            100                 105

<210>  147
<211>  10
<212>  PRT
<213>  Artificial Sequence

<220>
<223>  artificial antibody-based sequence

<400>  147

Gly Phe Ser Leu Ser Ser Tyr Ala Met Gly
1               5                   10

<210>  148
<211>  16
<212>  PRT
<213>  Artificial Sequence

<220>
<223>  artificial antibody-based sequence

<400>  148

Tyr Ile Ser Thr Ile Asn Asn Thr Tyr Tyr Ala Ser Trp Ala Lys Gly
1               5                   10                  15

<210>  149
<211>  14
<212>  PRT
<213>  Artificial Sequence

<220>
<223>  artificial antibody-based sequence

<400>  149

Arg Glu Ile Arg Ser Gly Trp Val Asp Tyr Gly Phe Ser Ile
1               5                   10

<210>  150
<211>  121
<212>  PRT
<213>  Artificial Sequence

<220>
<223>  artificial antibody-based sequence

<400>  150

Gln Val Gln Leu Val Glu Ser Gly Gly Gly Leu Val Gln Pro Gly Gly

1                   5                   10                  15

Ser Leu Arg Leu Ser Cys Ala Ala Ser Gly Phe Ser Leu Ser Ser Tyr
            20                  25                  30

Ala Met Gly Trp Val Arg Gln Ala Pro Gly Lys Gly Leu Glu Trp Ile
        35                  40                  45

Gly Tyr Ile Ser Thr Ile Asn Asn Thr Tyr Tyr Ala Ser Trp Ala Lys
    50                  55                  60

Gly Arg Phe Thr Ile Ser Arg Asp Asn Ser Lys Asn Thr Val Tyr Leu
65                  70                  75                  80

Gln Met Asn Ser Leu Arg Ala Glu Asp Thr Ala Val Tyr Tyr Cys Ala
                85                  90                  95

Arg Glu Ile Arg Ser Gly Trp Val Asp Tyr Gly Phe Ser Ile Trp Gly
            100                 105                 110

Gln Gly Thr Leu Val Thr Val Ser Ser
        115                 120

<210>  151
<211>  16
<212>  PRT
<213>  Artificial Sequence

<220>
<223>  artificial antibody-based sequence

<400>  151

Tyr Ile Ser Thr Ile Ala Asn Thr Tyr Tyr Ala Ser Trp Ala Lys Gly
1               5                   10                  15

<210>  152
<211>  121
<212>  PRT
<213>  Artificial Sequence

<220>
<223>  artificial antibody-based sequence

<400>  152

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Leu Ser Ser Tyr
            20                  25                  30

Ala Met Gly Trp Val Arg Gln Ala Pro Gly Lys Gly Leu Glu Trp Ile
        35                  40                  45

Gly Tyr Ile Ser Thr Ile Ala Asn Thr Tyr Tyr Ala Ser Trp Ala Lys
    50                  55                  60

Gly Arg Phe Thr Ile Ser Arg Asp Asn Ser Lys Asn Thr Val Tyr Leu
65                  70                  75                  80

Gln Met Asn Ser Leu Arg Ala Glu Asp Thr Ala Val Tyr Tyr Cys Ala
                85                  90                  95

Arg Glu Ile Arg Ser Gly Trp Val Asp Tyr Gly Phe Ser Ile Trp Gly
            100                 105                 110

Gln Gly Thr Leu Val Thr Val Ser Ser
        115                 120

<210>  153
<211>  11
<212>  PRT
<213>  Artificial Sequence

<220>
<223>  artificial antibody-based sequence

<400>  153

Gln Ala Ser Gln Asn Ile Tyr Ser Asn Leu Ala
1               5                   10

<210>  154
<211>  7
<212>  PRT
<213>  Artificial Sequence

<220>
<223>  artificial antibody-based sequence

<400>  154

Asp Ala Ser Asp Leu Ala Ser
1               5

<210>  155
<211>  12
<212>  PRT
<213>  Artificial Sequence

<220>
<223>  artificial antibody-based sequence

<400>  155

Gln Gln Val Arg Ser Ser Ser Asp Ile Asp Asn Pro

1               5                   10


<210>  156
<211>  111
<212>  PRT
<213>  Artificial Sequence

<220>
<223>  artificial antibody-based sequence

<400>  156

Asp Ile Gln Met Thr Gln Ser Pro Ser Ser Leu Ser Ala Ser Val Gly
1               5                   10                  15


Asp Arg Val Thr Ile Thr Cys Gln Ala Ser Gln Asn Ile Tyr Ser Asn
            20                  25                  30


Leu Ala Trp Tyr Gln Gln Lys Pro Gly Lys Ala Pro Lys Leu Leu Ile
        35                  40                  45


Tyr Asp Ala Ser Asp Leu Ala Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Val Arg Ser Ser Ser Asp
                85                  90                  95


Ile Asp Asn Pro Phe Gly Thr Gly Thr Lys Val Thr Val Leu Gly
            100                 105                 110


<210>  157
<211>  10
<212>  PRT
<213>  Artificial Sequence

<220>
<223>  artificial antibody-based sequence

<400>  157

Gly Phe Ser Leu Ser Ser Tyr Ala Met Gly
1               5                   10


<210>  158
<211>  16
<212>  PRT
<213>  Artificial Sequence

<220>
<223>  artificial antibody-based sequence

<400>  158

Tyr Ile Ser Lys Ile Gly Thr Thr Tyr Tyr Ala Ser Trp Ala Lys Gly
1               5                   10                  15


<210>  159
<211>  15
<212>  PRT
<213>  Artificial Sequence

<220>
<223>  artificial antibody-based sequence

<400>  159

Arg Gly Ser Ser Ser Gly Gly Tyr Leu Asp Asp Gly Phe Asp Pro
1               5                   10                  15


<210>  160
<211>  122
<212>  PRT
<213>  Artificial Sequence

<220>
<223>  artificial antibody-based sequence

<400>  160

Gln Ser Gln Leu Val Glu Ser Gly Gly Gly Leu Val Gln Pro Gly Gly
1               5                   10                  15


Ser Leu Arg Leu Ser Cys Ala Val Ser Gly Phe Ser Leu Ser Ser Tyr
            20                  25                  30


Ala Met Gly Trp Val Arg Gln Ala Pro Gly Lys Gly Leu Glu Tyr Ile
        35                  40                  45


Gly Tyr Ile Ser Lys Ile Gly Thr Thr Tyr Tyr Ala Ser Trp Ala Lys
    50                  55                  60


Gly Arg Phe Thr Ile Ser Lys Asp Asn Ser Lys Asn Thr Val Tyr Leu
65                  70                  75                  80


Gln Met Asn Ser Leu Arg Ala Glu Asp Thr Ala Val Tyr Phe Cys Ala
                85                  90                  95


Arg Gly Ser Ser Ser Gly Gly Tyr Leu Asp Asp Gly Phe Asp Pro Trp
            100                 105                 110


Gly Gln Gly Thr Leu Val Thr Val Ser Ser
        115                 120


<210>  161

<211>  11
<212>  PRT
<213>  Artificial Sequence

<220>
<223>  artificial antibody-based sequence

<400>  161

Gln Ala Ser Gln Ser Ile Ser Asn Tyr Leu Ala
1               5                   10


<210>  162
<211>  7
<212>  PRT
<213>  Artificial Sequence

<220>
<223>  artificial antibody-based sequence

<400>  162

Asp Ala Ser Asp Leu Ala Ser
1               5


<210>  163
<211>  12
<212>  PRT
<213>  Artificial Sequence

<220>
<223>  artificial antibody-based sequence

<400>  163

Gln Gln Val Tyr Asp Ser Asn Asn Val Glu Asn Val
1               5                   10


<210>  164
<211>  111
<212>  PRT
<213>  Artificial Sequence

<220>
<223>  artificial antibody-based sequence

<400>  164

Ala Leu Gln Met Thr Gln Ser Pro Ser Ser Leu Ser Ala Ser Val Gly
1               5                   10                  15


Asp Arg Val Thr Ile Thr Cys Gln Ala Ser Gln Ser Ile Ser Asn Tyr
            20                  25                  30


Leu Ala Trp Tyr Gln Gln Lys Pro Gly Lys Pro Pro Lys Phe Leu Ile
        35                  40                  45

Tyr Asp Ala Ser Asp Leu Ala Ser Gly Val Ser Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Tyr Asp Ser Asn Asn
                85                  90                  95

Val Glu Asn Val Phe Gly Thr Gly Thr Lys Val Thr Val Leu Gly
            100                 105                 110

<210>  165
<211>  10
<212>  PRT
<213>  Artificial Sequence

<220>
<223>  artificial antibody-based sequence

<400>  165

Gly Phe Asn Ile Lys Asp Thr Tyr Ile His
1               5                   10

<210>  166
<211>  17
<212>  PRT
<213>  Artificial Sequence

<220>
<223>  artificial antibody-based sequence

<400>  166

Arg Ile Tyr Pro Thr Asn Gly Tyr Thr Arg Tyr Ala Asp Ser Val Lys
1               5                   10                  15

Gly

<210>  167
<211>  12
<212>  PRT
<213>  Artificial Sequence

<220>
<223>  artificial antibody-based sequence

<400>  167

Arg Trp Gly Gly Asp Gly Phe Tyr Ala Met Asp Tyr
1               5                   10

<210>  168

<211>  120
<212>  PRT
<213>  Artificial Sequence

<220>
<223>  artificial antibody-based sequence

<400>  168


<210>  169
<211>  120
<212>  PRT
<213>  Artificial Sequence

<220>
<223>  artificial antibody-based sequence

<400>  169

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Cys Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser
        115                 120

<210>  170
<211>  11
<212>  PRT
<213>  Artificial Sequence

<220>
<223>  artificial antibody-based sequence

<400>  170

Arg Ala Ser Gln Asp Val Asn Thr Ala Val Ala
1               5                   10

<210>  171
<211>  7
<212>  PRT
<213>  Artificial Sequence

<220>
<223>  artificial antibody-based sequence

<400>  171

Ser Ala Ser Phe Leu Tyr Ser
1               5

<210>  172
<211>  9
<212>  PRT
<213>  Artificial Sequence

<220>
<223>  artificial antibody-based sequence

<400>  172

Gln Gln His Tyr Thr Thr Pro Pro Thr
1               5

<210>  173

<211>  108
<212>  PRT
<213>  Artificial Sequence

<220>
<223>  artificial antibody-based sequence

<400>  173

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gly Gly Thr Lys Leu Thr Val Leu Gly
            100                 105


<210>  174
<211>  108
<212>  PRT
<213>  Artificial Sequence

<220>
<223>  artificial antibody-based sequence

<400>  174

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Cys Gly Thr Lys Leu Thr Val Leu Gly
            100                 105

<210>  175
<211>  756
<212>  PRT
<213>  Artificial Sequence

<220>
<223>  artificial antibody-based sequence

<400>  175

Asp Val Val Met Thr Gln Ser Pro Ser Ser Leu Ser Ala Ser Val Gly
1               5                   10                  15

Asp Arg Val Thr Ile Thr Cys Gln Ala Ser Gln Ile Ile Ser Ser Arg
            20                  25                  30

Ser Ala Trp Tyr Gln Gln Lys Pro Gly Gln Pro Pro Lys Leu Leu Ile
        35                  40                  45

Tyr Gln Ala Ser Lys Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Cys Thr Tyr Ile Asp Ser Asn
                85                  90                  95

Phe Gly Ala Phe Gly Cys Gly Thr Lys Leu Thr Val Leu Gly Gly Gly
            100                 105                 110

Gly Gly Ser Glu Val Gln Leu Val Glu Ser Gly Gly Gly Leu Val Gln
        115                 120                 125

Pro Gly Gly Ser Leu Arg Leu Ser Cys Ala Ala Ser Gly Phe Ser Leu
    130                 135                 140

Ser Ser Tyr Asp Met Ser Trp Val Arg Gln Ala Pro Gly Lys Gly Leu
145                 150                 155                 160

Ala Trp Ile Gly Ala Ser Tyr Ala Ser Gly Pro Thr Tyr Tyr Ala Ser

                165                 170                 175

Trp Ala Lys Gly Arg Phe Thr Ile Ser Arg Asp Asn Ser Lys Asn Thr
            180                 185                 190

Val Tyr Leu Gln Met Asn Ser Leu Arg Ala Glu Asp Thr Ala Thr Tyr
        195                 200                 205

Phe Cys Ala Arg Gly Gly Trp Thr Gly Thr Ser His Ser Asn Ile Trp
    210                 215                 220

Gly Gln Gly Thr Leu Val Thr Val Ser Ser Gly Gly Gly Gly Ser Gly
225                 230                 235                 240

Gly Gly Gly Ser Gly Gly Gly Gly Ser Gly Gly Gly Gly Ser Asp Ile
                245                 250                 255

Gln Met Thr Gln Ser Pro Ser Ser Leu Ser Ala Ser Val Gly Asp Arg
            260                 265                 270

Val Thr Ile Thr Cys Gln Ser Ser Gln Ser Val Phe Ser Asn Asn Tyr
        275                 280                 285

Leu Ala Trp Phe Gln Gln Lys Pro Gly Gln Ser Pro Lys Arg Leu Ile
    290                 295                 300

Tyr Ser Ala Ser Thr Leu Ala Ser Gly Val Pro Ser Arg Phe Ser Gly
305                 310                 315                 320

Ser Gly Ser Gly Thr Asp Phe Thr Leu Thr Ile Ser Ser Leu Gln Pro
                325                 330                 335

Glu Asp Phe Ala Thr Tyr Tyr Cys Leu Gly Ser Tyr Ala Cys Ser Ser
            340                 345                 350

Ala Asp Cys Tyr Val Phe Gly Thr Gly Thr Lys Val Thr Val Leu Gly
        355                 360                 365

Gly Gly Gly Gly Ser Glu Val Gln Leu Val Glu Ser Gly Gly Gly Leu
    370                 375                 380

Val Gln Pro Gly Gly Ser Leu Arg Leu Ser Cys Ala Ala Ser Gly Phe
385                 390                 395                 400

Ser Phe Ser Ser Ser Tyr Trp Ile Cys Trp Val Arg Gln Ala Pro Gly
                405                 410                 415

Lys Cys Leu Glu Trp Val Gly Cys Val Phe Thr Gly Asp Gly Thr Thr
        420                 425                 430

Tyr Tyr Ala Ser Trp Ala Lys Gly Arg Phe Thr Ile Ser Arg Asp Asn
    435                 440                 445

Ser Lys Asn Thr Val Tyr Leu Gln Met Asn Ser Leu Arg Ala Glu Asp
    450                 455                 460

Thr Ala Thr Tyr Phe Cys Ala Arg Pro Val Ser Val Tyr Tyr Tyr Gly
465                 470                 475                 480

Met Asp Leu Trp Gly Gln Gly Thr Leu Val Thr Val Ser Ser Gly Gly
                485                 490                 495

Gly Gly Ser Gly Gly Gly Gly Ser Asp Ile Gln Met Thr Gln Ser Pro
        500                 505                 510

Ser Ser Leu Ser Ala Ser Val Gly Asp Arg Val Thr Ile Thr Cys Gln
    515                 520                 525

Ala Ser Glu Ser Ile Gly Asn Tyr Leu Ala Trp Tyr Gln Gln Lys Pro
    530                 535                 540

Gly Lys Ala Pro Lys Leu Leu Ile Tyr Ser Ala Ser Thr Leu Ala Ser
545                 550                 555                 560

Gly Val Pro Ser Arg Phe Ser Gly Ser Gly Ser Gly Thr Asp Phe Thr
                565                 570                 575

Leu Thr Ile Ser Ser Leu Gln Pro Glu Asp Phe Ala Thr Tyr Tyr Cys
        580                 585                 590

Gln Ser Thr Asp Tyr Gly Asp Ser Tyr Ile Phe Gly Thr Gly Thr Lys
    595                 600                 605

Val Thr Val Leu Gly Gly Gly Gly Gly Ser Gly Gly Gly Gly Ser Gly
    610                 615                 620

Gly Gly Gly Ser Gly Gly Gly Gly Ser Glu Val Gln Leu Val Glu Ser
625                 630                 635                 640

Gly Gly Gly Leu Val Gln Pro Gly Gly Ser Leu Arg Leu Ser Cys Ala
                645                 650                 655

Ala Ser Gly Ile Ser Val Ser Asn Asp Tyr Tyr Met Cys Trp Val Arg
        660                 665                 670

Gln Ala Pro Gly Lys Gly Leu Glu Trp Ile Gly Cys Ile Ser Thr Tyr
        675                 680                 685

Ile Gly Asn Thr His Tyr Ala Ser Trp Ala Lys Gly Arg Phe Thr Ile
    690                 695                 700

Ser Arg Asp Asn Ser Lys Asn Thr Val Tyr Leu Gln Met Asn Ser Leu
705                 710                 715                 720

Arg Ala Glu Asp Thr Ala Val Tyr Tyr Cys Ala Lys Asn Ala Gly Tyr
                725                 730                 735

Pro Gly Tyr Arg Tyr Ala Ile Asp Leu Trp Gly Gln Gly Thr Leu Val
            740                 745                 750

Thr Val Ser Ser
        755

<210>  176
<211>  510
<212>  PRT
<213>  Artificial Sequence

<220>
<223>  artificial antibody-based sequence

<400>  176

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asn Ile Tyr Ser Asn
            20                  25                  30

Leu Ala Trp Tyr Gln Gln Lys Pro Gly Lys Ala Pro Lys Leu Leu Ile
        35                  40                  45

Tyr Asp Ala Ser Asp Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Arg Ser Ser Ser Asp
                85                  90                  95

Ile Asp Asn Pro Phe Gly Thr Gly Thr Lys Val Thr Val Leu Gly Gly
            100                 105                 110

Gly Gly Gly Ser Gly Gly Gly Gly Ser Gly Gly Gly Gly Ser Gly Gly
        115                 120                 125

Gly Gly Ser Gln Val Gln Leu Val Glu Ser Gly Gly Gly Leu Val Gln
    130                 135                 140

Pro Gly Gly Ser Leu Arg Leu Ser Cys Ala Ala Ser Gly Phe Ser Leu
145                 150                 155                 160

Ser Ser Tyr Ala Met Gly Trp Val Arg Gln Ala Pro Gly Lys Gly Leu
                165                 170                 175

Glu Trp Ile Gly Tyr Ile Ser Thr Ile Asn Asn Thr Tyr Tyr Ala Ser
            180                 185                 190

Trp Ala Lys Gly Arg Phe Thr Ile Ser Arg Asp Asn Ser Lys Asn Thr
        195                 200                 205

Val Tyr Leu Gln Met Asn Ser Leu Arg Ala Glu Asp Thr Ala Val Tyr
    210                 215                 220

Tyr Cys Ala Arg Glu Ile Arg Ser Gly Trp Val Asp Tyr Gly Phe Ser
225                 230                 235                 240

Ile Trp Gly Gln Gly Thr Leu Val Thr Val Ser Ser Gly Gly Gly Gly
                245                 250                 255

Ser Gly Gly Gly Gly Ser Glu Val Gln Leu Val Glu Ser Gly Gly Gly
            260                 265                 270

Leu Val Gln Pro Gly Gly Ser Leu Arg Leu Ser Cys Ala Ala Ser Gly
        275                 280                 285

Phe Ser Leu Ser Ser Tyr Asp Met Ser Trp Val Arg Gln Ala Pro Gly
    290                 295                 300

Lys Gly Leu Ala Trp Ile Gly Ala Ser Tyr Ala Ser Gly Pro Thr Tyr
305                 310                 315                 320

Tyr Ala Ser Trp Ala Lys Gly Arg Phe Thr Ile Ser Arg Asp Asn Ser
                325                 330                 335

Lys Asn Thr Val Tyr Leu Gln Met Asn Ser Leu Arg Ala Glu Asp Thr
            340                 345                 350

Ala Thr Tyr Phe Cys Ala Arg Gly Gly Trp Thr Gly Thr Ser His Ser
        355                 360                 365

Asn Ile Trp Gly Gln Gly Thr Leu Val Thr Val Ser Ser Gly Gly Gly
    370                 375                 380

Ser Gly Gly Gly Ser Glu Val Gln Leu Val Glu Ser Gly Gly Gly Leu
385                 390                 395                 400

Val Gln Pro Gly Gly Ser Leu Arg Leu Ser Cys Ala Ala Ser Gly Phe
                405                 410                 415

Ser Phe Ser Ser Ser Tyr Trp Ile Cys Trp Val Arg Gln Ala Pro Gly
            420                 425                 430

Lys Cys Leu Glu Trp Val Gly Cys Val Phe Thr Gly Asp Gly Thr Thr
        435                 440                 445

Tyr Tyr Ala Ser Trp Ala Lys Gly Arg Phe Thr Ile Ser Arg Asp Asn
    450                 455                 460

Ser Lys Asn Thr Val Tyr Leu Gln Met Asn Ser Leu Arg Ala Glu Asp
465                 470                 475                 480

Thr Ala Thr Tyr Phe Cys Ala Arg Pro Val Ser Val Tyr Tyr Tyr Gly
                485                 490                 495

Met Asp Leu Trp Gly Gln Gly Thr Leu Val Thr Val Ser Ser
            500                 505                 510

<210>  177
<211>  492
<212>  PRT
<213>  Artificial Sequence

<220>
<223>  artificial antibody-based sequence

<400>  177

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asn Ile Tyr Ser Asn
            20                  25                  30

Leu Ala Trp Tyr Gln Gln Lys Pro Gly Lys Ala Pro Lys Leu Leu Ile
        35                  40                  45

Tyr Asp Ala Ser Asp Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Arg Ser Ser Ser Asp
                85                  90                  95

Ile Asp Asn Pro Phe Gly Thr Gly Thr Lys Val Thr Val Leu Gly Gly
            100                 105                 110

Gly Gly Gly Ser Gly Gly Gly Gly Ser Gly Gly Gly Gly Ser Gly Gly
        115                 120                 125

Gly Gly Ser Gln Val Gln Leu Val Glu Ser Gly Gly Gly Leu Val Gln
    130                 135                 140

Pro Gly Gly Ser Leu Arg Leu Ser Cys Ala Ala Ser Gly Phe Ser Leu
145                 150                 155                 160

Ser Ser Tyr Ala Met Gly Trp Val Arg Gln Ala Pro Gly Lys Gly Leu
                165                 170                 175

Glu Trp Ile Gly Tyr Ile Ser Thr Ile Asn Asn Thr Tyr Tyr Ala Ser
            180                 185                 190

Trp Ala Lys Gly Arg Phe Thr Ile Ser Arg Asp Asn Ser Lys Asn Thr
        195                 200                 205

Val Tyr Leu Gln Met Asn Ser Leu Arg Ala Glu Asp Thr Ala Val Tyr
    210                 215                 220

Tyr Cys Ala Arg Glu Ile Arg Ser Gly Trp Val Asp Tyr Gly Phe Ser
225                 230                 235                 240

Ile Trp Gly Gln Gly Thr Leu Val Thr Val Ser Ser Gly Gly Gly Gly
                245                 250                 255

Ser Gly Gly Gly Gly Ser Asp Val Val Met Thr Gln Ser Pro Ser Ser
            260                 265                 270

Leu Ser Ala Ser Val Gly Asp Arg Val Thr Ile Thr Cys Gln Ala Ser
        275                 280                 285

Gln Ile Ile Ser Ser Arg Ser Ala Trp Tyr Gln Gln Lys Pro Gly Gln
    290                 295                 300

Pro Pro Lys Leu Leu Ile Tyr Gln Ala Ser Lys Leu Ala Ser Gly Val
305                 310                 315                 320

Pro Ser Arg Phe Ser Gly Ser Gly Ser Gly Thr Asp Phe Thr Leu Thr
                325                 330                 335

Ile Ser Ser Leu Gln Pro Glu Asp Phe Ala Thr Tyr Tyr Cys Gln Cys
            340                 345                 350

Thr Tyr Ile Asp Ser Asn Phe Gly Ala Phe Gly Cys Gly Thr Lys Leu
        355                 360                 365

Thr Val Leu Gly Gly Gly Ser Gly Gly Ser Asp Ile Gln Met Thr Gln
    370                 375                 380

Ser Pro Ser Ser Leu Ser Ala Ser Val Gly Asp Arg Val Thr Ile Thr
385                 390                 395                 400

Cys Gln Ser Ser Gln Ser Val Phe Ser Asn Asn Tyr Leu Ala Trp Phe
                405                 410                 415

Gln Gln Lys Pro Gly Gln Ser Pro Lys Arg Leu Ile Tyr Ser Ala Ser
            420                 425                 430

Thr Leu Ala Ser Gly Val Pro Ser Arg Phe Ser Gly Ser Gly Ser Gly
        435                 440                 445

Thr Asp Phe Thr Leu Thr Ile Ser Ser Leu Gln Pro Glu Asp Phe Ala
    450                 455                 460

Thr Tyr Tyr Cys Leu Gly Ser Tyr Ala Cys Ser Ser Ala Asp Cys Tyr
465                 470                 475                 480

Val Phe Gly Thr Gly Thr Lys Val Thr Val Leu Gly
                485                 490

<210>  178
<211>  510
<212>  PRT
<213>  Artificial Sequence

<220>
<223>  artificial antibody-based sequence

<400>  178

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asn Ile Tyr Ser Asn
            20                  25                  30

Leu Ala Trp Tyr Gln Gln Lys Pro Gly Lys Ala Pro Lys Leu Leu Ile
        35                  40                  45

Tyr Asp Ala Ser Asp Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Arg Ser Ser Ser Asp
                85                  90                  95

Ile Asp Asn Pro Phe Gly Thr Gly Thr Lys Val Thr Val Leu Gly Gly
            100                 105                 110

Gly Gly Gly Ser Gly Gly Gly Gly Ser Gly Gly Gly Gly Ser Gly Gly
        115                 120                 125

Gly Gly Ser Gln Val Gln Leu Val Glu Ser Gly Gly Gly Leu Val Gln
    130                 135                 140

Pro Gly Gly Ser Leu Arg Leu Ser Cys Ala Ala Ser Gly Phe Ser Leu
145                 150                 155                 160

Ser Ser Tyr Ala Met Gly Trp Val Arg Gln Ala Pro Gly Lys Gly Leu
                165                 170                 175

Glu Trp Ile Gly Tyr Ile Ser Thr Ile Ala Asn Thr Tyr Tyr Ala Ser
            180                 185                 190

Trp Ala Lys Gly Arg Phe Thr Ile Ser Arg Asp Asn Ser Lys Asn Thr
        195                 200                 205

Val Tyr Leu Gln Met Asn Ser Leu Arg Ala Glu Asp Thr Ala Val Tyr
    210                 215                 220

Tyr Cys Ala Arg Glu Ile Arg Ser Gly Trp Val Asp Tyr Gly Phe Ser
225                 230                 235                 240

Ile Trp Gly Gln Gly Thr Leu Val Thr Val Ser Ser Gly Gly Gly Gly
                245                 250                 255

Ser Gly Gly Gly Gly Ser Glu Val Gln Leu Val Glu Ser Gly Gly Gly
            260                 265                 270

Leu Val Gln Pro Gly Gly Ser Leu Arg Leu Ser Cys Ala Ala Ser Gly
        275                 280                 285

Phe Ser Leu Ser Ser Tyr Asp Met Ser Trp Val Arg Gln Ala Pro Gly
    290                 295                 300

Lys Gly Leu Ala Trp Ile Gly Ala Ser Tyr Ala Ser Gly Pro Thr Tyr
305                 310                 315                 320

Tyr Ala Ser Trp Ala Lys Gly Arg Phe Thr Ile Ser Arg Asp Asn Ser
                325                 330                 335

Lys Asn Thr Val Tyr Leu Gln Met Asn Ser Leu Arg Ala Glu Asp Thr
            340                 345                 350

Ala Thr Tyr Phe Cys Ala Arg Gly Gly Trp Thr Gly Thr Ser His Ser
        355                 360                 365

Asn Ile Trp Gly Gln Gly Thr Leu Val Thr Val Ser Ser Gly Gly Gly
    370                 375                 380

Ser Gly Gly Gly Ser Glu Val Gln Leu Val Glu Ser Gly Gly Gly Leu
385                 390                 395                 400

Val Gln Pro Gly Gly Ser Leu Arg Leu Ser Cys Ala Ala Ser Gly Phe
                405                 410                 415

Ser Phe Ser Ser Ser Tyr Trp Ile Cys Trp Val Arg Gln Ala Pro Gly
            420                 425                 430

Lys Cys Leu Glu Trp Val Gly Cys Val Phe Thr Gly Asp Gly Thr Thr
        435                 440                 445

Tyr Tyr Ala Ser Trp Ala Lys Gly Arg Phe Thr Ile Ser Arg Asp Asn
    450                 455                 460

Ser Lys Asn Thr Val Tyr Leu Gln Met Asn Ser Leu Arg Ala Glu Asp
465                 470                 475                 480

Thr Ala Thr Tyr Phe Cys Ala Arg Pro Val Ser Val Tyr Tyr Tyr Gly
                485                 490                 495

Met Asp Leu Trp Gly Gln Gly Thr Leu Val Thr Val Ser Ser
            500                 505                 510

<210>  179
<211>  492
<212>  PRT
<213>  Artificial Sequence

<220>

<223>  artificial antibody-based sequence

<400>  179

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asn Ile Tyr Ser Asn
            20                  25                  30

Leu Ala Trp Tyr Gln Gln Lys Pro Gly Lys Ala Pro Lys Leu Leu Ile
        35                  40                  45

Tyr Asp Ala Ser Asp Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Arg Ser Ser Ser Asp
                85                  90                  95

Ile Asp Asn Pro Phe Gly Thr Gly Thr Lys Val Thr Val Leu Gly Gly
            100                 105                 110

Gly Gly Gly Ser Gly Gly Gly Gly Ser Gly Gly Gly Gly Ser Gly Gly
        115                 120                 125

Gly Gly Ser Gln Val Gln Leu Val Glu Ser Gly Gly Gly Leu Val Gln
    130                 135                 140

Pro Gly Gly Ser Leu Arg Leu Ser Cys Ala Ala Ser Gly Phe Ser Leu
145                 150                 155                 160

Ser Ser Tyr Ala Met Gly Trp Val Arg Gln Ala Pro Gly Lys Gly Leu
                165                 170                 175

Glu Trp Ile Gly Tyr Ile Ser Thr Ile Ala Asn Thr Tyr Tyr Ala Ser
            180                 185                 190

Trp Ala Lys Gly Arg Phe Thr Ile Ser Arg Asp Asn Ser Lys Asn Thr
        195                 200                 205

Val Tyr Leu Gln Met Asn Ser Leu Arg Ala Glu Asp Thr Ala Val Tyr
    210                 215                 220

Tyr Cys Ala Arg Glu Ile Arg Ser Gly Trp Val Asp Tyr Gly Phe Ser
225                 230                 235                 240

Ile Trp Gly Gln Gly Thr Leu Val Thr Val Ser Ser Gly Gly Gly Gly
                245                 250                 255

Ser Gly Gly Gly Gly Ser Asp Val Val Met Thr Gln Ser Pro Ser Ser
            260                 265                 270

Leu Ser Ala Ser Val Gly Asp Arg Val Thr Ile Thr Cys Gln Ala Ser
        275                 280                 285

Gln Ile Ile Ser Ser Arg Ser Ala Trp Tyr Gln Gln Lys Pro Gly Gln
    290                 295                 300

Pro Pro Lys Leu Leu Ile Tyr Gln Ala Ser Lys Leu Ala Ser Gly Val
305                 310                 315                 320

Pro Ser Arg Phe Ser Gly Ser Gly Ser Gly Thr Asp Phe Thr Leu Thr
                325                 330                 335

Ile Ser Ser Leu Gln Pro Glu Asp Phe Ala Thr Tyr Tyr Cys Gln Cys
            340                 345                 350

Thr Tyr Ile Asp Ser Asn Phe Gly Ala Phe Gly Cys Gly Thr Lys Leu
        355                 360                 365

Thr Val Leu Gly Gly Gly Ser Gly Gly Ser Asp Ile Gln Met Thr Gln
    370                 375                 380

Ser Pro Ser Ser Leu Ser Ala Ser Val Gly Asp Arg Val Thr Ile Thr
385                 390                 395                 400

Cys Gln Ser Ser Gln Ser Val Phe Ser Asn Asn Tyr Leu Ala Trp Phe
                405                 410                 415

Gln Gln Lys Pro Gly Gln Ser Pro Lys Arg Leu Ile Tyr Ser Ala Ser
            420                 425                 430

Thr Leu Ala Ser Gly Val Pro Ser Arg Phe Ser Gly Ser Gly Ser Gly
        435                 440                 445

Thr Asp Phe Thr Leu Thr Ile Ser Ser Leu Gln Pro Glu Asp Phe Ala
    450                 455                 460

Thr Tyr Tyr Cys Leu Gly Ser Tyr Ala Cys Ser Ser Ala Asp Cys Tyr
465                 470                 475                 480

Val Phe Gly Thr Gly Thr Lys Val Thr Val Leu Gly
                485                 490

<210>  180
<211>  752
<212>  PRT
<213>  Artificial Sequence

<220>
<223>  artificial antibody-based sequence

<400>  180
   Asp Ile Gln Met Thr Gln Ser Pro Ser Ser Leu Ser Ala Ser Val Gly
   1               5                   10                  15
   Asp Arg Val Thr Ile Thr Cys Gln Ser Ser Gln Ser Val Phe Ser Asn
               20                  25                  30
   Asn Tyr Leu Ala Trp Phe Gln Gln Lys Pro Gly Gln Ser Pro Lys Arg
           35                  40                  45
   Leu Ile Tyr Ser Ala Ser Thr Leu Ala Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Leu Gly Ser Tyr Ala Cys
                   85                  90                  95
   Ser Ser Ala Asp Cys Tyr Val Phe Gly Thr Gly Thr Lys Val Thr Val
               100                 105                 110
   Leu Gly Gly Gly Gly Gly Ser Glu Val Gln Leu Val Glu Ser Gly Gly
           115                 120                 125
   Gly Leu Val Gln Pro Gly Gly Ser Leu Arg Leu Ser Cys Ala Ala Ser
       130                 135                 140
   Gly Phe Ser Leu Ser Ser Asn Ala Met Gly Trp Val Arg Gln Ala Pro
   145                 150                 155                 160
   Gly Lys Gly Leu Glu Tyr Ile Gly Ile Ile Ser Val Gly Gly Phe Thr
                   165                 170                 175
   Tyr Tyr Ala Ser Trp Ala Lys Gly Arg Phe Thr Ile Ser Arg Asp Asn
               180                 185                 190
   Ser Lys Asn Thr Val Tyr Leu Gln Met Asn Ser Leu Arg Ala Glu Asp
           195                 200                 205
   Thr Ala Thr Tyr Phe Cys Ala Arg Asp Arg His Gly Gly Asp Ser Ser
       210                 215                 220
   Gly Ala Phe Tyr Leu Trp Gly Gln Gly Thr Leu Val Thr Val Ser Ser
   225                 230                 235                 240
   Gly Gly Gly Gly Ser Gly Gly Gly Gly Ser Gly Gly Gly Gly Ser Gly
                   245                 250                 255
   Gly Gly Gly Ser Ile Gln Met Thr Gln Ser Pro Ser Ser Leu Ser Ala
               260                 265                 270
   Ser Val Gly Asp Arg Val Thr Ile Thr Cys Gln Ser Ser Glu Ser Val
           275                 280                 285
   Tyr Ser Asn Asn Gln Leu Ser Trp Tyr Gln Gln Lys Pro Gly Gln Pro
       290                 295                 300
   Pro Lys Leu Leu Ile Tyr Asp Ala Ser Asp Leu Ala Ser Gly Val Pro
   305                 310                 315                 320
   Ser Arg Phe Ser Gly Ser Gly Ser Gly Thr Asp Phe Thr Leu Thr Ile
                   325                 330                 335
   Ser Ser Leu Gln Pro Glu Asp Phe Ala Thr Tyr Tyr Cys Ala Gly Gly
               340                 345                 350
   Phe Ser Ser Ser Ser Asp Thr Ala Phe Gly Gly Gly Thr Lys Leu Thr
           355                 360                 365
   Val Leu Gly Gly Gly Gly Gly Ser Glu Val Gln Leu Val Glu Ser Gly
       370                 375                 380
   Gly Gly Leu Val Gln Pro Gly Gly Ser Leu Arg Leu Ser Cys Ala Ala
   385                 390                 395                 400
   Ser Gly Phe Ser Leu Ser Ser Tyr Asp Met Ser Trp Val Arg Gln Ala
                   405                 410                 415
   Pro Gly Lys Gly Leu Ala Trp Ile Gly Ala Ser Tyr Ala Ser Gly Pro
               420                 425                 430
   Thr Tyr Tyr Ala Ser Trp Ala Lys Gly Arg Phe Thr Ile Ser Arg Asp
           435                 440                 445
   Asn Ser Lys Asn Thr Val Tyr Leu Gln Met Asn Ser Leu Arg Ala Glu
       450                 455                 460
   Asp Thr Ala Thr Tyr Phe Cys Ala Arg Gly Gly Trp Thr Gly Thr Ser
   465                 470                 475                 480
   His Ser Asn Ile Trp Gly Gln Gly Thr Leu Val Thr Val Ser Ser Gly
                   485                 490                 495
   Gly Gly Gly Ser Gly Gly Gly Gly Ser Ile Gln Met Thr Gln Ser Pro
               500                 505                 510
   Ser Ser Leu Ser Ala Ser Val Gly Asp Arg Val Thr Ile Thr Cys Arg
           515                 520                 525
   Ala Ser Gln Asp Val Asn Thr Ala Val Ala Trp Tyr Gln Gln Lys Pro
       530                 535                 540
   Gly Lys Ala Pro Lys Leu Leu Ile Tyr Ser Ala Ser Phe Leu Tyr Ser
   545                 550                 555                 560
   Gly Val Pro Ser Arg Phe Ser Gly Ser Arg Ser Gly Thr Asp Phe Thr
                   565                 570                 575
   Leu Thr Ile Ser Ser Leu Gln Pro Glu Asp Phe Ala Thr Tyr Tyr Cys
               580                 585                 590
   Gln Gln His Tyr Thr Thr Pro Pro Thr Phe Gly Cys Gly Thr Lys Leu
           595                 600                 605
   Thr Val Leu Gly Gly Gly Gly Gly Ser Gly Gly Gly Gly Ser Gly Gly
       610                 615                 620
   Gly Gly Ser Gly Gly Gly Gly Ser Glu Val Gln Leu Val Glu Ser Gly
   625                 630                 635                 640
   Gly Gly Leu Val Gln Pro Gly Gly Ser Leu Arg Leu Ser Cys Ala Ala
                   645                 650                 655
   Ser Gly Phe Asn Ile Lys Asp Thr Tyr Ile His Trp Val Arg Gln Ala
               660                 665                 670
   Pro Gly Lys Cys Leu Glu Trp Val Ala Arg Ile Tyr Pro Thr Asn Gly
           675                 680                 685
   Tyr Thr Arg Tyr Ala Asp Ser Val Lys Gly Arg Phe Thr Ile Ser Ala
       690                 695                 700
   Asp Thr Ser Lys Asn Thr Ala Tyr Leu Gln Met Asn Ser Leu Arg Ala
   705                 710                 715                 720
   Glu Asp Thr Ala Val Tyr Tyr Cys Ser Arg Trp Gly Gly Asp Gly Phe
                   725                 730                 735
   Tyr Ala Met Asp Tyr Trp Gly Gln Gly Thr Leu Val Thr Val Ser Ser
               740                 745                 750

<210>  181
<211>  752
<212>  PRT
<213>  Artificial Sequence

<220>
<223>  artificial antibody-based sequence

<400>  181

Asp Ile Gln Met Thr Gln Ser Pro Ser Ser Leu Ser Ala Ser Val Gly
1               5                   10                  15

Asp Arg Val Thr Ile Thr Cys Gln Ser Ser Gln Ser Val Phe Ser Asn
            20                  25                  30

Asn Tyr Leu Ala Trp Phe Gln Gln Lys Pro Gly Gln Ser Pro Lys Arg
        35                  40                  45

Leu Ile Tyr Ser Ala Ser Thr Leu Ala Ser Gly Val Pro Ser Arg Phe
    50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Leu Gly Ser Tyr Ala Cys
                85                  90                  95

Ser Ser Ala Asp Cys Tyr Val Phe Gly Cys Gly Thr Lys Val Thr Val
            100                 105                 110

Leu Gly Gly Gly Gly Gly Ser Glu Val Gln Leu Val Glu Ser Gly Gly
        115                 120                 125

Gly Leu Val Gln Pro Gly Gly Ser Leu Arg Leu Ser Cys Ala Ala Ser
    130                 135                 140

Gly Phe Ser Leu Ser Ser Asn Ala Met Gly Trp Val Arg Gln Ala Pro
145                 150                 155                 160

Gly Lys Gly Leu Glu Tyr Ile Gly Ile Ile Ser Val Gly Gly Phe Thr
                165                 170                 175

Tyr Tyr Ala Ser Trp Ala Lys Gly Arg Phe Thr Ile Ser Arg Asp Asn
            180                 185                 190

Ser Lys Asn Thr Val Tyr Leu Gln Met Asn Ser Leu Arg Ala Glu Asp
        195                 200                 205

Thr Ala Thr Tyr Phe Cys Ala Arg Asp Arg His Gly Gly Asp Ser Ser
    210                 215                 220

Gly Ala Phe Tyr Leu Trp Gly Gln Gly Thr Leu Val Thr Val Ser Ser
225                 230                 235                 240

Gly Gly Gly Gly Ser Gly Gly Gly Gly Ser Gly Gly Gly Gly Ser Gly
                245                 250                 255

Gly Gly Gly Ser Ile Gln Met Thr Gln Ser Pro Ser Ser Leu Ser Ala
            260                 265                 270

Ser Val Gly Asp Arg Val Thr Ile Thr Cys Gln Ser Ser Glu Ser Val
        275                 280                 285

Tyr Ser Asn Asn Gln Leu Ser Trp Tyr Gln Gln Lys Pro Gly Gln Pro
    290                 295                 300

Pro Lys Leu Leu Ile Tyr Asp Ala Ser Asp Leu Ala Ser Gly Val Pro
305                 310                 315                 320

Ser Arg Phe Ser Gly Ser Gly Ser Gly Thr Asp Phe Thr Leu Thr Ile
                325                 330                 335

Ser Ser Leu Gln Pro Glu Asp Phe Ala Thr Tyr Tyr Cys Ala Gly Gly
            340                 345                 350

Phe Ser Ser Ser Ser Asp Thr Ala Phe Gly Gly Gly Thr Lys Leu Thr
        355                 360                 365

Val Leu Gly Gly Gly Gly Gly Ser Glu Val Gln Leu Val Glu Ser Gly
    370                 375                 380

Gly Gly Leu Val Gln Pro Gly Gly Ser Leu Arg Leu Ser Cys Ala Ala
385                 390                 395                 400

Ser Gly Phe Ser Leu Ser Ser Tyr Asp Met Ser Trp Val Arg Gln Ala
                405                 410                 415

Pro Gly Lys Cys Leu Ala Trp Ile Gly Ala Ser Tyr Ala Ser Gly Pro

            420                 425                 430

Thr Tyr Tyr Ala Ser Trp Ala Lys Gly Arg Phe Thr Ile Ser Arg Asp
        435                 440                 445

Asn Ser Lys Asn Thr Val Tyr Leu Gln Met Asn Ser Leu Arg Ala Glu
    450                 455                 460

Asp Thr Ala Thr Tyr Phe Cys Ala Arg Gly Gly Trp Thr Gly Thr Ser
465                 470                 475                 480

His Ser Asn Ile Trp Gly Gln Gly Thr Leu Val Thr Val Ser Ser Gly
                485                 490                 495

Gly Gly Gly Ser Gly Gly Gly Gly Ser Ile Gln Met Thr Gln Ser Pro
            500                 505                 510

Ser Ser Leu Ser Ala Ser Val Gly Asp Arg Val Thr Ile Thr Cys Arg
        515                 520                 525

Ala Ser Gln Asp Val Asn Thr Ala Val Ala Trp Tyr Gln Gln Lys Pro
    530                 535                 540

Gly Lys Ala Pro Lys Leu Leu Ile Tyr Ser Ala Ser Phe Leu Tyr Ser
545                 550                 555                 560

Gly Val Pro Ser Arg Phe Ser Gly Ser Arg Ser Gly Thr Asp Phe Thr
                565                 570                 575

Leu Thr Ile Ser Ser Leu Gln Pro Glu Asp Phe Ala Thr Tyr Tyr Cys
            580                 585                 590

Gln Gln His Tyr Thr Thr Pro Pro Thr Phe Gly Cys Gly Thr Lys Leu
        595                 600                 605

Thr Val Leu Gly Gly Gly Gly Gly Ser Gly Gly Gly Gly Ser Gly Gly
    610                 615                 620

Gly Gly Ser Gly Gly Gly Gly Ser Glu Val Gln Leu Val Glu Ser Gly
625                 630                 635                 640

Gly Gly Leu Val Gln Pro Gly Gly Ser Leu Arg Leu Ser Cys Ala Ala
                645                 650                 655

Ser Gly Phe Asn Ile Lys Asp Thr Tyr Ile His Trp Val Arg Gln Ala
            660                 665                 670

Pro Gly Lys Cys Leu Glu Trp Val Ala Arg Ile Tyr Pro Thr Asn Gly
        675                 680                 685

Tyr Thr Arg Tyr Ala Asp Ser Val Lys Gly Arg Phe Thr Ile Ser Ala
    690                 695                 700

Asp Thr Ser Lys Asn Thr Ala Tyr Leu Gln Met Asn Ser Leu Arg Ala
705                 710                 715                 720

Glu Asp Thr Ala Val Tyr Tyr Cys Ser Arg Trp Gly Gly Asp Gly Phe
                725                 730                 735

Tyr Ala Met Asp Tyr Trp Gly Gln Gly Thr Leu Val Thr Val Ser Ser
            740                 745                 750

## Description

### FIELD OF THE INVENTION

The present invention relates to a pharmaceutical composition comprising a first multispecific antibody (MA1) comprising one binding domain, which specifically binds to CD137 (CD137-BD), and one binding domain, which specifically binds to PDL1 (PDL1-BD); a second multispecific antibody (MA2) comprising at least one binding domain, which specifically binds to a tumor cell associated antigen (TAA-BD), and one binding domain, which specifically binds to CD3 (CD3-BD); and a pharmaceutically acceptable carrier. The present invention further relates to a kit comprising said first multispecific antibody MA1 and said second multispecific antibody MA2. The present invention further relates to the use as well as to methods of use of said pharmaceutical composition or said kit. Finally, the present invention relates to a method for treating a patient suffering from cancer, comprising the step of administering a first multispecific antibody (MA1) comprising one binding domain, which specifically binds to CD137 (CD137-BD), and one binding domain, which specifically binds to PDL1 (PDL1-BD), and a second multispecific antibody (MA2) comprising at least one binding domain, which specifically binds to a tumor cell associated antigen (TAA-BD), and one binding domain, which specifically binds to CD3 (CD3-BD).

### BACKGROUND OF THE INVENTION

Cancer continues to pose a major unmet medical need, despite the considerable progress made in its treatment. Some of the most substantial progress made in cancer treatment in recent years has come with the advent of immunotherapies of various molecular classes, including, but not limited to: monoclonal antibodies (mAbs), bispecific antibodies (bsAbs), recombinant proteins, and chimeric antigen receptor-T cell (CAR-T cell) therapies. Such therapies induce anti-tumor immunity by: a) actively directing immune-effector cells to tumor-resident cells and/or b) stimulating immune-effector cells and/or c) relieving tumor-mediated immune-suppression. These immunotherapies commonly exploit the overexpression of specific antigens by tumor-resident cells (e. g., malignant cells, cells of the tumor vasculature, stromal cells, immune cells, etc.) - as compared to extratumoral loci - to target their pharmacological activity to tumors. Among these antigens, tumor-associated antigens (TAAs) comprise cell-surface proteins selectively overexpressed by malignant cells. By binding to TAAs with high affinity, immunotherapies can restrict their immunomodulatory activity to immunological synapses between tumor cells and immune effector cells to a degree.

A common class of TAA-binding immunotherapeutics are mAbs that elicit anti-tumor immunity by opsonizing tumor-cells and by triggering antibody-dependent cell-mediated cytotoxicity (ADCC) by Fcγ receptor (FcγR)-expressing cells, primarily natural killer (NK) cells. Other TAA-binding immunotherapies leverage cytotoxic T lymphocytes (CTLs) to induce targeted depletion of malignant cells, such as CAR-T cells as well as bsAbs that simultaneously engage the T cell antigen CD3 (TAA/CD3 bsAbs).

While the therapeutic utility of TAA-(re)directed CTLs and conventional TAA/CD3 bsAbs have been clinically validated, dose-limiting toxicities (DLTs) often preclude administration at maximally effective doses (MEDs) or lead to discontinuation of treatment, resulting in limited efficacy.

One reason for the DLTs is that conventional TAA/CD3 bsAbs are also commonly associated with cytokine release syndrome (CRS), putatively due to excessive activity of anti-CD3 domains. Extratumoral activity of immunotherapies results in the secretion of pro-inflammatory cytokines in healthy tissues, which can result in undesirable safety profiles. Furthermore, while TAA/CD3 bsAbs potently deplete TAA-overexpressing cells, they do so by recruiting and stimulating CTLs regardless of whether such cells express a T cell receptor (TCR) that recognizes a tumor-antigen(s) (*i*.*e*., tumor-reactive T cell). Therefore, rather than stimulating or reactivating the host's native anti-tumor immunity, TAA/CD3 bsAbs somewhat indiscriminately stimulate CTLs, potentially posing safety risks.

Although the exact pathways by which such DLTs arise can vary, the risk of immunotherapy-related toxicities can typically be minimized or eliminated by enhancing the tumor-localization of pharmacological activity.

TAAs that are almost exclusively expressed on cancer cells, such as oncofetal tumor antigens, are referred to as clean TAAs. TAA that are also expressed on normal, non-cancer cells - typically at lower levels compared to cancer cells - are considered non-clean TAAs. Due to the very high potency of TAA/CD3 bsAbs approaches, non-clean TAAs are a challenge as they damage non-tumor cells that also express the TAA. Mesothelin (MSLN), EGFR, EpCAM and HER2 are examples of non-clean TAAs; they are not only expressed on tumor cells but also in various other tissues, albeit at a lower level. Therefore, when targeting non-clean TAAs, novel therapies that improve the selectivity of TAA/CD3 bsAb approaches for tumor tissues and minimize off-tumor/on-target effects are needed. This particularly applies to MSLN/CD3 bsAb and HER2/CD3 bsAb approaches.

Different strategies are pursued to increase the efficiency and selectivity of TAA/CD3 bsAb approaches.

One strategy involves the use of avidity effects by providing multispecific T-cell engaging antibodies that are bivalent for the desired TAA, i.e. T-cell engaging multispecific antibodies having two or more binding domains that are specific for the same TAA (TAA-BDs), e.g. two MSLN-BDs or two HER2-BDs, and one binding domain that specifically binds to CD3 (CD3-BD), wherein the binding affinity of the two or more TAA-BDs are in a well-balanced range. This bivalent binding strategy allows the efficient avidity driven targeting of tumor cells with high TAA expression levels, while healthy, i.e. low TAA expressing cells, are much less affected. Such multispecific antibodies are theoretically capable of eliciting a high tumor localization and improved selectivity, which could provide safer and more effective therapies for a variety of cancers. However, implementation of multispecific antibodies for therapeutic use has been complicated due to issues with their molecular architecture, the properties of their component antigen-binding domains, their producibility and/or poor biophysical properties. Thus, only a limited number of such multispecific molecules have been developed so far.

WO 2019/157308 for example describes bispecific 1Fab-IgG-based antibodies having two low-affinity anti-HER2 binding domains and one anti-CD3ε binding domain, which exhibit improved in vitro selectivity for high HER2-expressing cell lines.

Yoon et al. (Biomolecules, 2020,10(3), 399) describe a bispecific antibody having two low-affinity anti-MSLN binding domains and one anti-CD3ε binding domain. This bispecific antibody is IgG-based having two scFab arms, with specificity for MSLN and CD3ε, and one additional MSLN-specific scFab fragment fused to the N-terminus of the CD3ε-specific scFab.

Another strategy involves co-administration of additional immunotherapeutic antibodies with the aim to enhance patient response to the TAA-(re)directed immunotherapy, e.g. by relieving tumor-mediated immune-suppression and/or by increasing the immune response by further activating co-stimulating receptors, but without increasing side effects. Ideally, this approach should allow a reduction of the effective dose level of the DLT-critical TAA/CD3 bispecific antibody component, resulting in broadening the therapeutic window.

Tumor-mediated immune-suppression is often induced by the expression of immune-checkpoint ligands/receptors (e. g., PD-1, PDL1, CTLA-4). Immune checkpoints are regulators of the immune system and are involved in processes such as self-tolerance or immune suppression in cancer. Monoclonal antibodies that block immune-suppressive antigens, such as CTLA-4 (e. g., ipilimumab), PD-1 (e. *g.,* nivolumab, pembrolizumab) and PDL1 (e. g., avelumab, atezolizumab), have elicited impressive response rates in patients exhibiting a variety of tumor histology phenotypes.

PDL1 (CD274, B7-H1) is a 40 kDa type I transmembrane protein. PDL1 is a surface glycoprotein ligand for PD-1, a key immune checkpoint receptor expressed by activated T and B cells, and mediates immunosuppression. PDL1 is implicated in the suppression of immune system responses during chronic infections, pregnancy, tissue allografts, autoimmune diseases, and cancer. PDL1 is found on both antigen-presenting cells and human cancer cells, such as squamous cell carcinoma of the head and neck, melanoma, and brain, thyroid, thymus, esophagus, lung, breast, gastrointestinal tract, colorectum, liver, pancreas, kidney, adrenal cortex, bladder, urothelium, ovary, and skin tumors (Katsuya Y, et al., Lung Cancer.88(2):154-159 (2015); Nakanishi J, et al., Cancer Immunol Immunother. 56(8):1173-1182 (2007); Nomi T, et al., Clin Cancer Res. 13(7):2151-2157 (2007); Fay AP, et al., J Immunother Cancer. 3:3 (2015); Strome SE, et al., Cancer Res. 63(19):6501-6505 (2003); Jacobs JF, et al. Neuro Oncol.11(4):394-402 (2009); Wilmotte R, et al. Neuroreport. 16(10):1081-1085 (2005)). PDL1 is rarely expressed on normal tissues but inducibly expressed on tumor sites (Dong H, et al., Nat Med. 8(8):793-800 (2002); Wang et al., Onco Targets Ther. 9: 5023-5039 (2016)). PDL1 downregulates T cell activation and cytokine secretion by binding to PD-1 (Freeman et al., 2000; Latchman et al, 2001). PD-1, activated by PDL1, potentially provides an immune-tolerant environment for tumor development and growth. PDL1 also negatively regulates T cell function through interaction with another receptor, B7.1 (B7-1, CD80).

A number of antibodies that disrupt PD-1 signaling have entered clinical development. These antibodies belong to the following two main categories: those that target PD-1 (nivolumab, Bristol-Myers Squibb; pembrolizumab, Merck, Whitehouse Station, NJ; pidilizumab, CureTech, Yavne, Israel) and those that target PDL1 (MPDL3280A, Genentech, South San Francisco, CA; MEDI4736, Medlmmune/AstraZeneca; BMS-936559, Bristol-Myers Squibb; MSB0010718C, EMD Serono, Rockland, MA) (for review see Postow MA et al., J Clin Oncol. Jun 10;33(17):1974-82 (2015)). Targeting PDL1 versus targeting PD-1 may result in different biologic effects. PD-1 antibodies prevent interaction of PD-1 with both its ligands, PDL1 and PDL2. PDL1 antibodies do not prevent PD-1 from interacting with PDL2, although the effect of this interaction remains unknown. PDL1 antibodies however prevent interaction of PDL1 with not only PD-1, but also B7-1 (Butte MJ, et al., Immunity 27:111-122, (2007)), which is believed to exert negative signals on T cells. Blocking PDL1 has demonstrated promising early data, and currently, four clinical anti-PDL1 mAbs are in the testing: atezolizumab and MEDI4736 (both are Fc null variants of human IgG1), MSB001078C (IgG1), and BMS-936559 (IgG4) (Chester C., et al., Cancer Immunol Immunother Oct;65(10):1243-8 (2016)).

New and emerging co-administration treatments frequently combine anti-PDL1/PD1 antibodies with TAA-(re)directed CTL-based immunotherapies.

WO 2017/112775, for example, describes a method for treating or inhibiting the growth of a tumor, comprising the administering of an anti PD-1 antibody in combination with a bispecific antibody comprising a first antigen-binding arm that specifically binds CD20 and a second antigen-binding arm that specifically binds CD3.

WO 2015/095418 describes a method for treating HER2-positive cancers, comprising the administering of an anti PD-1 antibody in combination with a bispecific antibody that specifically binds HER2 and CD3.

In addition, T cell co-stimulatory receptors (e. g., CD137, OX40, ICOS, GITR) are currently being clinically evaluated as targets for therapeutic stimulation of T cells in cancer. One putative advantage of anti-tumor T cell stimulation via such targets is that they are transiently expressed upon TCR signaling. As such, their expression tends to be selectively increased in inflamed tumor microenvironments (TMEs), particularly on tumor-reactive T cells, whose TCRs are receiving consistent stimulation through interaction with major histocompatibility complexes (MHCs) expressed by malignant cells and antigen-presenting cells (APCs). Therefore, targeting co-stimulatory receptors with, e. g., mAbs and bsAbs, should more selectively stimulate and expand pre-existing anti-tumor T cells than e.g. CD3-targeting approaches, potentially rendering such biologics safer and their effects more durable.

Among co-stimulatory receptors, CD137 (4-1BB, TNF-receptor superfamily 9, TNFRSF9) has emerged as especially promising due to its expression profile and its role as a multipotent mediator of anti-tumor immunity (Bartkowiak and Curran, Front Oncol. 2015, 5, 117; Yonezawa et al., Clin Cancer Res. 2015, 21, 3113-20). CD137 is an inducible T cell co-stimulatory receptor. Its expression is activation-dependent and encompasses a broad subset of immune cells, including activated CD8⁺ T cells, CD4⁺ T cells, NK cells, NKT cells, Tregs, dendritic cells (DC), including follicular DC, stimulated mast cells, differentiating myeloid cells, monocytes, neutrophils, eosinophils (Wang et al, Immunol Rev. 229(1): 192-215 (2009)), and activated B cells (Zhang et al, J Immunol. 184(2):787-795 (2010)). In addition, CD137 expression has also been demonstrated on tumor vasculature (Broil K et al., Am J Clin Pathol. 115(4):543-549 (2001); Seaman et al, Cancer Cell 11(6):539-554 (2007)) and atherosclerotic endothelium (Olofsson et al, Circulation 117(10): 1292 1301 (2008)).

CD137 co-stimulates T cells to carry out effector functions such as eradication of established tumors, broadening primary CD8⁺ T cell responses, and enhancing the memory pool of antigen-specific CD8⁺ T cells. *In vivo* efficacy studies in mice have revealed that CD137-agonistic mAbs, administered both as a monotherapy and as a component of combination regimens, leads to anti-tumor protective T cell memory responses and tumor regression in multiple tumor models.

Thus, several co-administration treatments are currently under investigation that combine CD137-agonistic mAbs with TAA-(re)directed CTLs immunotherapies.

WO 2018/114754 and WO 2018/114748 for example describe methods for treating or delaying progression of cancer, wherein a CD137-agonistic antibody is applied in combination with a bispecific antibody that specifically binds to a TAA and CD3, particularly to CD20 and CD3.

Although utilization of CD137-agonistic mAbs is a very promising treatment strategy, clinical data collected thus far suggest that a mAb-based approach to CD137 stimulation results in a trade-off between efficacy and safety. Namely, highly active CD137-agonistic mAbs elicit dose limiting toxicities (DLTs) that attenuate treatment efficacy, whereas weakly active CD137-agonistic mAbs are well tolerated but do not seem to be highly efficacious, including at their predicted minimum effective dose (MED).

Highly active CD137-agonistic mAbs lead to alterations in the immune system and organ function, increasing risks of toxicities. High doses of such mAbs in naïve and tumor-bearing mice have been reported to induce T cell infiltration to the liver and elevations of aspartate aminotransferase and alanine aminotransferase, consistent with liver inflammation (Niu L, et al. J Immunol 178(7):4194-4213 (2007); Dubrot J, et al., Int J Cancer 128(1):105-118 (2011); Segal NH et al. Clin Canc Res: 1929-1936 (2016)), as well as FcyR induced immune cell accumulation in the liver of wild-type mice (Claus et al. Sci Transl Med (11): 1-12 (2019)). Initial clinical studies into the human therapeutic use of CD137-agonistic mAbs have also demonstrated elevation of liver enzymes and increased incidence of hepatitis (Sznol M., et al., J Clin Oncol 26(115S):3007 (2008); Ascierto PA, et al., Semin Oncol 37(5):508-516 (2010); Chester C., et al., Cancer Immunol Immunother Oct;65(10):1243-8 (2016)). Potentially fatal hepatitis was observed in a Bristol-Myers Squibb (BMS) phase II anti-CD137 study for previously treated stage III/IV melanoma, National Clinical Trial (NCT) 00612664. This study and several others (NCT00803374, NCT00309023, NCT00461110, NCT00351325) were terminated due to adverse events (Chester C., et al., Cancer Immunol Immunother Oct;65(10):1243-8 (2016)). Such adverse events are most probably due to systemic overstimulation of T cells. CD137-mediated liver toxicity is believed to be caused by on-target activation of the myeloid cells in the liver, followed by recruitment of CD4⁺/CD8⁺ T cells, which are activated and cause damage.

To gain additional cross-linking function and achieve certain levels of TNRSF, in particular CD137, activation, it has recently been suggested to use multivalent and multispecific fusion polypeptides that bind PDL1 and TNRSF members, or folate receptor alpha (FRα) and TNRSF members, wherein the binding to PDL1 or FRα is capable of providing additional crosslinking function (WO 2017/123650). Eckelman et al. have demonstrated that bivalent engagement of CD137, as in the case of INBRX-105, a multispecific and multivalent polypeptide having two PDL1 binding domains, two CD137 binding domains and an Fc region, is insufficient to effectively cluster and mediate productive CD137 signaling in absence of an exogenous clustering event, using an assay isolating the effects of the molecule on a reporter T cell-line. In contrast, engagement for a second cell surface antigen PDL1 in the presence of PDL1-positive cells enables further clustering of CD137 and productive signaling (WO 2017/123650).

Recently, the effect of multivalent and multispecific fusion polypeptides that bind PDL1 and CD137 on T cell activation and proliferation has been evaluated in *vitro.* Using an autologous *in vitro* co-culture system implementing immature DC and donor matched T cells, it has been demonstrated that INBRX-105, a multispecific and multivalent polypeptide having two PDL1 binding domains, two CD137 binding domains and an Fc region, is superior in inducing interferon-gamma (INFγ) production or mediating CD8⁺ T cell proliferation and activation, when compared to the monospecific PDL1 sd-Ab-Fc fusion protein, the CD137 sdAb-Fc fusion protein, the combination of the two, the anti-PDL1 antibody atezolizumab, the anti-CD137 antibody utomilumab (PF-05082566), or the anti-PDL1 antibody prembrolizumab, and combinations thereof (WO 2017/123650).

Such multivalent CD137-agonistic and PD-1/PDL1-antagonistic bispecific antibodies have recently been tested in combination with TAA/CD3 bsAbs.

Breznoy et al., poster PB-062 presented at the 30th EORTC/AACR/NCI Symposium, 2018, Dublin, describe the co-administration of a trivalent bispecific TRIDENT^{®} antibody, which has one PDL1 binding domain, two CD137 binding domains and an Fc region, with a bivalent bispecific anti-5T4 x CD3 dual-affinity re-targeting (DART^{®}) antibody comprising an Fc region in a RKO tumor mouse model. It has been demonstrated that the combined administration of these two bispecific antibodies results in a strong improvement of T-cell mediated cancer cell killing when compared to the administration of the individual bispecific components alone.

The data disclosed in the above mentioned studies relating to the single application and/or the co-application of CD137-agonizing and PDL1-antagonizing bispecific antibodies implies that it is highly favorable - or even required - that these bispecific antibodies comprise at least two CD137 binding domains in order to ensure efficient clustering of CD137 and productive signaling and, in turn, a reasonably high increase in the efficacy of TAA-(re)directed T-cell mediated killing of cancer cells. Also, all CD137-agonizing and PDL1-antagonizing bispecific antibodies developed so far have an immunoglobulin Fc region. However, the combination of bispecific antibodies that target CD3 with multispecific antibodies having two CD137 binding domains raises concerns about the safety of such co-treatment approaches. These concerns are further fortified by the presence of Fc regions in the applied molecules, which generally cause off-tumor effects such as systematic activation of T-cells. This holds particularly true in cases, where the TAA/CD3 bsAbs are directed to non-clean TAAs, i.e. TAAs that are also expressed in healthy cells albeit to a lower extent than in the particular tumor cells.

Apart from that, the development of such co-treatment approaches is a challenging task. The biophysical and functional characterization as well as the biological testing of the multispecific antibodies applied therein, be it alone or in combination, are typically difficult and laborious, due to their multispecific architecture and the complexity of their interaction in biological systems. Thus, such co-treatment strategies generally have significant economic and commercial drawbacks over single component therapies, due to their higher development, approval and production costs. Consequently, even for those co-treatment strategies where a gain in risk-to-benefit can be achieved, such improvement must surpass the drawbacks of higher development efforts and development costs.

In summary, there remains a clear need for novel TAA-(re)directed immunotherapies with increased tumor cell localization and effective T cell activation, which at the same time have a tolerable toxicological profile, i.e. immunotherapies with an improved risk-to-benefit ratio, in particular for novel TAA-(re)directed immunotherapies that target non-clean TAAs.

### SUMMARY OF THE INVENTION

It is an object of the present invention to provide a medicament to improve treatment of a proliferative disease, particularly a cancer. More specifically, it was an object of the present invention to provide novel TAA-(re)directed immunotherapies with increased on target efficacy and a tolerable toxicological profile.

The inventors could previously identify a series of multispecific antibodies comprising one CD137 binding domain (CD137-BD) and one PDL1 binding domain (PDL1-BD). Said multispecific antibodies are able to cluster and to agonize CD137 signaling in a targeted manner, i.e. solely in the presence of PDL1-positive cells such as in PDL1-positive tumor microenvironment, thus avoiding systemic activation of CD137. At the same time, said multispecific antibodies block the binding of PDL1 to PDL. These multispecific antibodies as well as pharmaceutical compositions and methods of use thereof are disclosed in the patent application WO 2019/072868, which is herewith incorporated by reference in its entirety.

The inventors have now surprisingly found that the combined application of such a CD137-agonizing and PDL1-blocking multispecific antibody, as defined herein, with a multispecific antibody that specifically targets TAAs and CD3 results in a significantly greater efficacy in TAA-(re)directed target cell killing *(in vitro)* and a substantially increased tumor growth inhibition *(in vivo)* relative to the application of said individual multispecific antibodies alone, despite of the fact that these CD137-agonizing and PDL1-blocking multispecific antibodies have only one CD137 binding domain, only one PDL1 binding domain and also do not comprise a immunoglobulin Fc region.

At the same time, the toxicological profile of the combined application of the PDL1-/CD137-binding multispecific antibodies, as described herein, with anti-TAAxCD3 multispecific antibodies is not worse than the toxicological profile of the application of the TAAxCD3 multispecific antibodies alone.

Accordingly, in a first aspect, the present invention relates to a pharmaceutical composition comprising:
1) a first multispecific antibody (MA1) comprising
   a) one binding domain, which specifically binds to CD137 (CD137-BD), and
   b) one binding domain, which specifically binds to PDL1 (PDL1-BD),
2) a second multispecific antibody (MA2) comprising
   a) at least one binding domain, which specifically bind(s) to a tumor cell associated antigen (TAA-BD),
   b) one binding domain, which specifically binds to CD3 (CD3-BD),
3) a pharmaceutically acceptable carrier.

In a second aspect, the present invention relates to a kit comprising:
1) a first multispecific antibody (MA1) comprising
   a) one binding domain, which specifically binds to CD137 (CD137-BD), and
   b) one binding domain, which specifically binds to PDL1 (PDL1-BD), and
2) a second multispecific antibody (MA2) comprising
   a) at least one binding domain, which specifically binds to a tumor cell associated antigen (TAA-BD), and
   b) one binding domain, which specifically binds to CD3 (CD3-BD).

In a third aspect, the present invention relates to a pharmaceutical composition or a kit of the present invention for use as a medicament.

In a fourth aspect, the present invention relates to a pharmaceutical composition or a kit of the present invention for use in the treatment of a disease, particularly a human disease, more particularly a human disease selected from cancers.

In a fifth aspect, the present invention relates to a method for the treatment of a disease, particularly a human disease, more particularly a human disease selected from cancers, comprising the step of administering the pharmaceutical composition or the kit of the present invention.

In a sixth aspect, the present invention relates to a method for treating a patient suffering from cancer, comprising the step of administering a first multispecific antibody (MA1) comprising one binding domain, which specifically binds to CD137 (CD137-BD), and one binding domain, which specifically binds to PDL1 (PDL1-BD), and a second multispecific antibody (MA2) comprising at least one binding domain, which specifically binds to a tumor cell associated antigen (TAA-BD), and one binding domain, which specifically binds to CD3 (CD3-BD).

In a seventh aspect, the present invention relates to a multispecific antibody MA1 comprising one binding domain, which specifically binds to CD137 (CD137-BD), and one binding domain, which specifically binds to PDL1 (PDL1-BD) for use in the treatment of a subject suffering from a proliferative disease, particularly a cancer, wherein said multispecific antibody MA1 is administered to said subject in combination with a multispecific antibody MA2 comprising at least one binding domain, which specifically binds to a tumor cell associated antigen (TAA-BD), and one binding domain, which specifically binds to CD3 (CD3-BD).

In an eight aspect, the present invention relates to a multispecific antibody MA2 comprising at least one binding domain, which specifically binds to a tumor cell associated antigen (TAA-BD), and one binding domain, which specifically binds to CD3 (CD3-BD) for use in the treatment of a subject suffering from a proliferative disease, particularly a cancer, wherein said multispecific antibody MA2 is administered to said subject in combination with a multispecific antibody MA1 comprising one binding domain, which specifically binds to CD137 (CD137-BD), and one binding domain, which specifically binds to PDL1 (PDL1-BD).

The aspects, advantageous features and preferred embodiments of the present invention summarized in the following items, respectively alone or in combination, further contribute to solving the object of the invention:
1. A pharmaceutical composition comprising:
   1) a first multispecific antibody (MA1) comprising
      a) one binding domain, which specifically binds to CD137 (CD137-BD), and
      b) one binding domain, which specifically binds to PDL1 (PDL1-BD),
   2) a second multispecific antibody (MA2) comprising
      a) at least one binding domain, which specifically bind(s) to a tumor cell associated antigen (TAA-BD),
      b) one binding domain, which specifically binds to CD3 (CD3-BD),
   3) a pharmaceutically acceptable carrier.
2. A kit comprising:
   1) a first multispecific antibody (MA1) comprising
      a) one binding domain, which specifically binds to CD137 (CD137-BD), and
      b) one binding domain, which specifically binds to PDL1 (PDL1-BD), and
   2) a second multispecific antibody (MA2) comprising
      a) at least one binding domain, which specifically binds to a tumor cell associated antigen (TAA-BD), and
      b) one binding domain, which specifically binds to CD3 (CD3-BD).
3. The pharmaceutical composition of item 1, or the kit of item 2, wherein said MA2 comprises one or two TAA-BD(s).
4. The pharmaceutical composition of item 1 or 3, or the kit of item 2 or 3, wherein said MA1 or said MA2, or both antibodies MA1 and MA2, do not comprise an immunoglobulin Fc region polypeptide.
5. The pharmaceutical composition of any one of the preceding items, wherein said pharmaceutical composition comprises:
   1) a first multispecific antibody (MA1) comprising
      a) one binding domain, which specifically binds to CD137 (CD137-BD), and
      b) one binding domain, which specifically binds to PDL1 (PDL1-BD),
   2) a second multispecific antibody (MA2) comprising
      a) one or two binding domain(s), which specifically bind(s) to a tumor cell associated antigen (TAA-BD),
      b) one binding domain, which specifically binds to CD3 (CD3-BD),
   3) a pharmaceutically acceptable carrier,
   wherein said MA1 does not comprise an immunoglobulin Fc region polypeptide.
6. The kit of any one of the preceding items, wherein said kit comprises:
   1) a first multispecific antibody (MA1) comprising
      a) one binding domain, which specifically binds to CD137 (CD137-BD), and
      b) one binding domain, which specifically binds to PDL1 (PDL1-BD), and
   2) a second multispecific antibody (MA2) comprising
      a) one or two binding domain(s), which specifically bind(s) to a tumor cell associated antigen (TAA-BD), and
      b) one binding domain, which specifically binds to CD3 (CD3-BD),
   wherein said MA1 does not comprise an immunoglobulin Fc region polypeptide.
7. The pharmaceutical composition or the kit of any one of the preceding items, wherein said MA1 or MA2, or both antibodies MA1 and MA2, further comprises one human serum albumin binding domain (hSA-BD).
8. The pharmaceutical composition or the kit of any one of the preceding items, wherein the binding domains of said MA1 and MA2, e.g., PDL1-BD, CD137-BD, TAA-BD, CD3-BD or hSA-BD, are independently of each other selected from the group consisting of a Fab, an Fv, an scFv, a dsFv, an sdAb, a STAB, and binding domains based on alternative scaffolds including but not limited to ankyrin-based domains, fynomers, avimers, anticalins, fibronectins, and binding sites being built into constant regions of antibodies (e.g. f-star technology), particularly wherein the binding domains of MA1 and MA2 are independently of each other selected from the group consisting of a Fab, an Fv, an scFv, a dsFv, an sdAb and a STAB, more particularly wherein the binding domains of MA1 and MA2 are independently of each other selected from the group consisting of an Fv, an scFv, a dsFv and a STAB.
9. The pharmaceutical composition or the kit of any one of the preceding items, wherein said MA1 or said MA2, or both antibodies MA1 and MA2, do not comprise CH1 and/or CL regions, in particular wherein said MA1 or said MA2, or both antibodies MA1 and MA2, exclusively comprise immunoglobulin variable domains.
10. The pharmaceutical composition or the kit of any one of the preceding items, wherein each of said binding domains comprised in MA1 and MA2 is independently selected from
   (a) a cognate pair of a VL domain and a VH domain (Fv fragment); or
   (b) a cognate pair of a VL domain and a VH domain linked by a polypeptide linker (scFv fragment) and/or a disulfide bond.
11. The pharmaceutical composition or the kit of any one of the preceding items, wherein the binding domains of said MA1 are capable of binding simultaneously to their respective antigens.
12. The pharmaceutical composition or the kit of any one of items 7 to 11, wherein said MA1 consists of one CD137-BD, one PDL1-BD and one hSA-BD.
13. The pharmaceutical composition or the kit of any one of the preceding items, wherein said MA1 acts as an agonist of CD137 in the presence of PDL1-positive cells.
14. The pharmaceutical composition or the kit of any one of the preceding items, wherein said MA1 does not inhibit the interaction between CD137 and its ligand CD137L, in particular as measured by a competition ELISA.
15. The pharmaceutical composition or the kit of any one of the preceding items, wherein said CD137-BD:
   a. binds to human CD137 with a monovalent dissociation constant (K_{D}) of less than 50nM, particularly with a monovalent K_{D} of 0.005 to 50 nM, more particularly of 0.01 to 30 nM, more particularly of 0.01 to 10 nM, more particularly of 0.1 to 5 nM, as measured by surface plasmon resonance (SPR), particularly wherein said CD137-BD is an scFv;
   b. binds to human CD137 with a K_{off} rate of 10⁻² s⁻¹ or less, more particularly of 10⁻² s⁻¹ to 10⁻⁶ s⁻¹ more particularly of 5x10⁻³ s⁻¹ to 10⁻⁵ s⁻¹, more particularly of 10⁻³ s⁻¹ to 5x10⁻⁴ s⁻¹, as measured by SPR;
   c. binds to human CD137 with a Kₒₙ rate of at least 10⁴ M⁻¹s⁻¹ or greater, more particularly of 10⁴ M⁻¹s⁻¹ to 10⁷ M⁻¹s⁻¹, more particularly of 10⁵ M⁻¹s⁻¹ to 5x10⁶ M⁻¹s⁻¹, as measured by SPR;
   d. does not cross-compete with urelumab and utomilumab; and/or
   e. is cross-reactive with Macaca fascicularis (cynomolgus) CD137, in particular binds to cynomolgus CD137 with a monovalent K_{D} of less than 50 nM, particularly with a monovalent K_{D} of 0.005 to 50 nM, more particularly of 0.01 to 30 nM, more particularly of 0.01 to 10 nM, more particularly of 0.1 to 5 nM, as measured by SPR, particularly wherein said CD137-BD is an scFv; and/or
   f. when being in scFv format, has a melting temperature (Tm), determined by differential scanning fluorimetry, of at least 50°C, preferably of at least 55°C, more preferably at least 60°C, in particular wherein said antibody or antigen binding fragment thereof is formulated in phosphate citrate buffer at pH 6.4, 150 mM NaCl, in particular wherein said antibody is formulated in 50 mM phosphate citrate buffer with 150 mM NaCl at pH 6.4;
   g. when being in scFv format, has a loss in monomer content, after storage for at least two weeks, particularly for at least four weeks, at 4°C, of less than 7 %, e.g. less than 6 %, less than 5 %, less than 4 %, less than 3 %, less than 2 %, preferably less than 1 %, when said antibody is at a starting concentration of 10 mg/ml, and in particular wherein said antibody is formulated in 50 mM phosphate citrate buffer with 150 mM NaCl at pH 6.4; and/or
   h. when being in scFv format, has a loss in monomer content, after storage for at least two weeks, particularly for at least four weeks, at 40°C, of less than 5 %, e.g. less than 4 %, less than 3 %, less than 2 %, preferably less than 1 %, when said antibody is at a starting concentration of 10 mg/ml, and in particular wherein said antibody is formulated in 50 mM phosphate citrate buffer with 150 mM NaCl at pH 6.4.
16. The pharmaceutical composition or the kit of any one of the preceding items, wherein said CD137-BD binds to human CD137 with a monovalent dissociation constant (K_{D}) which is at least 5 times, preferably at least 10 times, e.g., at least 20, at least 30, at least 50, at least 100 times, higher than the monovalent dissociation constant (K_{D}) of said PDL1-BD for binding to human PDL1, when measured by SPR.
17. The pharmaceutical composition or the kit of any one of the preceding items, wherein said CD137-BD binds to human CD137 with a monovalent dissociation constant (K_{D}) that is 20 to 1000 times, preferably 30 to 800 times, in particular 50 to 500 times higher than the monovalent dissociation constant (K_{D}) of said PDL1-BD for binding to human PDL1, when measured by SPR.
18. The pharmaceutical composition or the kit of any one of the items 13 to 17, wherein said CD137-BD comprises
   (i) the HCDR1, HCDR2, and HCDR3 sequences of SEQ ID NOs: 1, 2 and 3, respectively, and the LCDR1, LCDR2, and LCDR3 sequences of SEQ ID NOs: 8, 9 and 10, respectively; and
   (ii) VH3 or VH4 domain framework sequences FR1 to FR4; particularly VH3 domain framework sequences FR1 to FR4; and
   (iii) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
19. The pharmaceutical composition or the kit of item 18, wherein said CD137-BD comprises
   a) HCDR1, HCDR2, and HCDR3 sequences of SEQ ID NOs: 1, 2 and 3, respectively,
   b) LCDR1, LCDR2, and LCDR3 sequences of SEQ ID NOs: 8, 9 and 10, respectively,
   c) a VH sequence at least 90, 91, 92, 93, 94, 95, 96, 97, 98 or 99 percent identical to the amino acid sequences SEQ ID NO: 4, 5, 6 or 7, and
   d) a VL sequence at least 90, 91, 92, 93, 94, 95, 96, 97, 98 or 99 percent identical to the amino acid sequences SEQ ID NO: 11, 12, 13 or 14.
20. The pharmaceutical composition or the kit of item 18 or 19, wherein said VH domain comprises a cysteine at position 51 (AHo numbering) and said VL domain comprises a cysteine at position 141 (AHo numbering).
21. The pharmaceutical composition or the kit of any one of the items 18 to 20, wherein said CD137-BD comprises: a VH domain comprising an amino acid sequence selected from any of SEQ ID NOs: 4, 5, 6 and 7; and a VL domain comprising an amino acid sequence selected from any of SEQ ID NOs: 11, 12, 13 and 14.
22. The pharmaceutical composition or the kit of item 21, wherein said CD137-BD comprises
   (a) a VH sequence of SEQ ID NO: 4 and a VL sequence of SEQ ID NO: 11;
   (b) a VH sequence of SEQ ID NO: 5 and a VL sequence of SEQ ID NO: 12;
   (c) a VH sequence of SEQ ID NO: 6 and a VL sequence of SEQ ID NO: 13; or
   (d) a VH sequence of SEQ ID NO: 7 and a VL sequence of SEQ ID NO: 14.
23. The pharmaceutical composition or the kit of any one of items 18 to 20, wherein said CD137-BD comprising: a VH sequence at least 90, 91, 92, 93, 94, 95, 96, 97, 98 or 99 percent identical to the amino acid sequence SEQ ID NO: 7; and a VL sequence at least 90, 91, 92, 93, 94, 95, 96, 97, 98 or 99 percent identical to the amino acid sequence SEQ ID NO: 14, wherein said VH domain comprises a cysteine at position 51 (AHo numbering) and said VL domain comprises a cysteine at position 141 (AHo numbering.
24. The pharmaceutical composition or the kit of any one of the items 13 to 17, wherein said CD137-BD comprises
   (i) the HCDR1, HCDR2, and HCDR3 sequences of SEQ ID NOs: 19, 20 and 21, respectively, and the LCDR1, LCDR2, and LCDR3 sequences of SEQ ID NOs: 23, 24 and 25, respectively; and
   (ii) VH3 or VH4 domain framework sequences FR1 to FR4; particularly VH3 domain framework sequences FR1 to FR4; and
   (iii) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
25. The pharmaceutical composition or the kit of item 24, wherein said CD137-BD comprises: a VH sequence that is at least 90, 91, 92, 93, 94, 95, 96, 97, 98 or 99 percent identical to an amino acid sequence of SEQ ID NO: 22; and a VL sequence that is at least 90, 91, 92, 93, 94, 95, 96, 97, 98 or 99 percent identical to an amino acid sequence of SEQ ID NO: 26.
26. The pharmaceutical composition or the kit of item 24 or 25, wherein said VH domain or VH sequence comprises a cysteine at position 51 (AHo numbering) and said VL domain or VL sequence comprises a cysteine at position 141 (AHo numbering).
27. The pharmaceutical composition or the kit of any one of the items 24 to 26, wherein said CD137- BD comprises: a VH sequence of SEQ ID NO: 22 and a VL sequence of SEQ ID NO: 26.
28. The pharmaceutical composition or the kit of any one of items 13 to 17, wherein said CD137-BD comprises
   (i) the HCDR1, HCDR2, and HCDR3 sequences of SEQ ID NOs: 28, 29 and 30, respectively, and the LCDR1, LCDR2, and LCDR3 sequences of SEQ ID NOs: 34, 35 and 36, respectively; and
   (ii) VH3 or VH4 domain framework sequences FR1 to FR4; particularly VH3 domain framework sequences FR1 to FR4; and
   (iii) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
29. The pharmaceutical composition or the kit of item 28, wherein said CD137-BD comprises: a VH sequence that is at least 90, 91, 92, 93, 94, 95, 96, 97, 98 or 99 percent identical to an amino acid sequence selected from the group consisting of SEQ ID NOs: 31, 32 and 33; and a VL sequence that is at least 90, 91, 92, 93, 94, 95, 96, 97, 98 or 99 percent identical to an amino acid sequence selected from the group consisting of SEQ ID NOs: 37, 38 and 39.
30. The pharmaceutical composition or the kit of item 28 or 29, wherein said VH sequence or VH domain comprises a cysteine at position 51 (AHo numbering) and said VL domain or VL sequence comprises a cysteine at position 141 (AHo numbering).
31. The pharmaceutical composition or the kit of any one of items 28 to 30, wherein said CD137- BD comprises:
   (a) a VH sequence of SEQ ID NO: 31 and a VL sequence of SEQ ID NO: 37;
   (b) a VH sequence of SEQ ID NO: 32 and a VL sequence of SEQ ID NO: 38; or
   (c) a VH sequence of SEQ ID NO: 33 and a VL sequence of SEQ ID NO: 39.
32. The pharmaceutical composition or the kit of any one of the items 28 to 31, wherein said CD137-BD comprises: a VH sequence at least 90, 91, 92, 93, 94, 95, 96, 97, 98 or 99 percent identical to the amino acid sequence SEQ ID NO: 33; and a VL sequence at least 90, 91, 92, 93, 94, 95, 96, 97, 98 or 99 percent identical to the amino acid sequence SEQ ID NO: 39, wherein said VH sequence comprises a cysteine at position 51 (AHo numbering) and said VL sequence comprises a cysteine at position 141 (AHo numbering).
33. The pharmaceutical composition or the kit of any of the preceding items, wherein said PDL1-BD is a blocker of PDL1.
34. The pharmaceutical composition or the kit of item 33, wherein said PDL1-BD:
   a. binds to human PDL1 with a monovalent dissociation constant (K_{D}) of less than 10 nM, particularly with a monovalent K_{D} of 0.05 pM to 10 nM, more particularly of 0.1 pM to 5 nM, more particularly of 0.2 pM to 1 nM, more particularly 0.5 pM to 500 pM, more particularly of 1 pM to 200 pM, more particularly of 1 pM to 100 pM, as measured by SPR;
   b. binds to human PDL1 with a K_{off} rate of 5x10⁻³ s⁻¹ or less, more particularly of 5x10⁻³ s⁻¹ to 10⁻⁷ s⁻¹, more particularly of 10⁻³ s⁻¹ to 5x10⁻⁶ s⁻¹, more particularly of 10⁻³ s⁻¹ to 10⁻⁶ s⁻¹, as measured by SPR;
   c. binds to human PDL1 with a Kₒₙ rate of at least 10⁴ M⁻¹s⁻¹ or greater, more particularly of 10⁴ M⁻¹s⁻¹ to 10⁸ M⁻¹s⁻¹ , more particularly of 10⁵ M⁻¹s⁻¹ to 5x10⁷ M⁻¹s⁻¹, more particularly of 5x10⁵ M⁻¹s⁻¹ to 10⁷ M⁻¹s⁻¹, as measured by SPR;
   d. is cross-reactive with Macaca fascicularis (cynomolgus) PDL1;
   e. is non-cross reactive to Mus musculus PDL1; and/or
   f. when being in scFv format, has a melting temperature (Tm), determined by differential scanning fluorimetry, of at least 55°C, e.g. at least 60°C, preferably at least 65°C, more preferably at least 70°C, in particular wherein said antibody or antigen-binding fragment thereof is formulated in phosphate citrate buffer at pH 6.4, 150 mM NaCl, in particular wherein said antibody is formulated in 50 mM phosphate citrate buffer with 150 mM NaCl at pH 6.4;
   g. when being in scFv format, has a loss in monomer content, after five consecutive freeze-thaw cycles, of less than 5 %, preferably less than 3 %, more preferably less than 1 %, when said antibody is at a starting concentration of 10 mg/ml, in particular wherein said antibody is formulated in 50 mM phosphate citrate buffer with 150 mM NaCl at pH 6.4; and/or
   h. when being in scFv format, has a loss in monomer content, after storage for at least two weeks, particularly for at least four weeks, at 4°C, of less than 15 %, e.g. less than 12 %, less than 10 %, less than 7 %, less than 5 %, less than 4 %, less than 3 %, less than 2 %, preferably less than 1 %, when said antibody is at a starting concentration of 10 mg/ml, and in particular wherein said antibody is formulated in 50 mM phosphate citrate buffer with 150 mM NaCl at pH 6.4.
35. The pharmaceutical composition or the kit of item 33 or 34, wherein said PDL1-BD comprises
   (i) the HCDR1, HCDR2, and HCDR3 sequences of SEQ ID NOs: 43, 44 and 45, respectively, and the LCDR1, LCDR2, and LCDR3 sequences of SEQ ID NOs: 49, 50 and 51, respectively; and
   (ii) VH3 or VH4 domain framework sequences FR1 to FR4; particularly VH3 domain framework sequences FR1 to FR4; and
   (iii) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
36. The pharmaceutical composition or the kit of item 33 or 34, wherein said PDL1-BD comprises
   (i) the HCDR1, HCDR2, and HCDR3 sequences of SEQ ID NOs: 57, 58 and 59, respectively, and the LCDR1, LCDR2, and LCDR3 sequences of SEQ ID NOs: 63, 64 and 65, respectively; and
   (ii) VH3 or VH4 domain framework sequences FR1 to FR4; particularly VH3 domain framework sequences FR1 to FR4; and
   (iii) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
37. The pharmaceutical composition or the kit of any one of items 33 to 36, wherein said PDL1-BD comprises: a VH sequence that is at least 90, 91, 92, 93, 94, 95, 96, 97, 98 or 99 percent identical to an amino acid sequence selected from the group consisting of SEQ ID NOs: 46, 47, 48, 60, 61 and 62; and a VL sequence that is at least 90, 91, 92, 93, 94, 95, 96, 97, 98 or 99 percent identical to an amino acid sequence selected from the group consisting of SEQ ID NOs: 52, 53, 66 and 67.
38. The pharmaceutical composition or the kit of any one of items 33 to 36, wherein said PDL1-BD comprises:
   (a) a VH sequence of SEQ ID NO: 46 and a VL sequence of SEQ ID NO: 52;
   (b) a VH sequence of SEQ ID NO: 47 and a VL sequence of SEQ ID NO: 52;
   (c) a VH sequence of SEQ ID NO: 48 and a VL sequence of SEQ ID NO: 53;
   (d) a VH sequence of SEQ ID NO: 60 and a VL sequence of SEQ ID NO: 66;
   (e) a VH sequence of SEQ ID NO: 61 and a VL sequence of SEQ ID NO: 67; or
   (f) a VH sequence of SEQ ID NO: 62 and a VL sequence of SEQ ID NO: 66.
39. The pharmaceutical composition or the kit of item 38, wherein said PDL1-BD comprises:
   (a) a VH sequence of SEQ ID NO: 46 and a VL sequence of SEQ ID NO: 52; or
   (b) a VH sequence of SEQ ID NO: 48 and a VL sequence of SEQ ID NO: 53.
40. The pharmaceutical composition or the kit of item 38, wherein said PDL1-BD comprises:
   (a) a VH sequence of SEQ ID NO: 61 and a VL sequence of SEQ ID NO: 67; or
   (b) a VH sequence of SEQ ID NO: 62 and a VL sequence of SEQ ID NO: 66.
41. The pharmaceutical composition or the kit of any one of items 7 to 40, wherein said hSA-BD comprises
   (i) HCDR1, HCDR2, and HCDR3 sequences of SEQ ID NOs: 71, 72 and 73, embedded in an antibody VH framework, particularly in a human antibody VH framework, particularly in a human VH3 framework, and
   (ii) LCDR1, LCDR2, and LCDR3 sequences of SEQ ID NOs: 75, 76 and 77, embedded in an antibody VL framework, particularly in a human antibody VL framework, wherein the VL framework comprises framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101;
   or
   (i) HCDR1, HCDR2, and HCDR3 sequences of SEQ ID NOs: 80, 81 and 82, embedded in an antibody VH framework, particularly in a human antibody VH framework, particularly in a human VH3 framework, and
   (ii) LCDR1, LCDR2, and LCDR3 sequences of SEQ ID NOs: 84, 85 and 86, embedded in an antibody VH framework, particularly in a human antibody VL framework, wherein the VL framework comprises framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
42. The pharmaceutical composition or the kit of item 41, wherein said hSA-BD comprises: a VH sequence that is at least 90, 91, 92, 93, 94, 95, 96, 97, 98 or 99 percent identical to an amino acid sequence selected from the group consisting of SEQ ID NOs: 74 and 83; and a VL sequence that is at least 90, 91, 92, 93, 94, 95, 96, 97, 98 or 99 percent identical to an amino acid sequence selected from the group consisting of SEQ ID NOs: 78 and 87.
43. The pharmaceutical composition or the kit of any one of items 41 or 42, wherein said hSA-BD comprises:
   (a) a VH sequence of SEQ ID NO: 74 and a VL sequence of SEQ ID NO: 78;
   (b) a VH sequence of SEQ ID NO: 83 and a VL sequence of SEQ ID NO: 87;
44. The pharmaceutical composition or the kit of item 43, wherein said VH domain comprises a cysteine at position 51 (AHo numbering) and said VL domain comprises a cysteine at position 141 (AHo numbering).
45. The pharmaceutical composition or the kit of any one of the preceding items, wherein said multispecific antibody MA1 is in a format selected from the group consisting of: scDB; a bispecific T cell engager (BiTE; tandem di-scFv), tandem tri-scFv; Fab-(scFv); scFab-dsscFv; tribody (Fab-(scFv)₂); Fab₂; Fab-Fv₂; diabody; triabody; scDb-scFv; a scDb, a tandem tri-scFv, a Fab-(scFv), a scFab-dsscFv, a Fab-(scFv)₂, a Fab₂, a Fab-Fv₂, a diabody or a scDb-scFv fused to the N- and/or the C-terminus of a heterodimerization domain other than heterodimeric Fc domains; and a MATCH.
46. The pharmaceutical composition or the kit of any one of items 1 to 6, wherein said MA1 is an scDb comprising an amino acid sequence selected from any of SEQ ID NOs: 105, 106, 107, 108, 109, 110, and 111.
47. The pharmaceutical composition or the kit of any one of items 1 to 6, wherein said MA1 is an scDb-scFv comprising an amino acid sequence selected from any of SEQ ID NOs: 112, 113, 114, 115, 116, 117, 118, 119, 120, 121, 122, 123, 124, 125, 126, 127, 128, 129 and 130, preferably wherein said MA1 is an scDb-scFv comprising an amino acid sequence selected from SEQ ID NO: 125, 126, 127 and 130, more preferably wherein said MA1 is an scDb-scFv comprising an amino acid sequence selected from SEQ ID NO: 125, 127 and 130.
48. The pharmaceutical composition or the kit of any one of the preceding items, wherein said CD3-BD of MA2 is binding to CD3ε.
49. The pharmaceutical composition or the kit of item 48, wherein said CD3-BD binds CD3_{ε} with a monovalent K_{D} of less than 50 nM, particularly with a monovalent K_{D} of 0.5 to 50 nM, particularly of 1 to 40 nM, particularly of 2 to 35 nM, particularly of 3 to 30 nM, as measured by SPR.
50. The pharmaceutical composition or the kit of item 48 or 49, wherein said CD3-BD comprises
   (i) HCDR1, HCDR2, and HCDR3 sequences of SEQ ID NOs: 131, 132 and 133, embedded in an antibody VH framework, particularly in a human antibody VH framework, particularly in a human VH3 framework, and
   (ii) LCDR1, LCDR2, and LCDR3 sequences of SEQ ID NOs: 135, 136 and 137, embedded in an antibody VH framework, particularly in a human antibody VL framework, wherein the VL framework comprises framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
51. The pharmaceutical composition or the kit of item 50, wherein said CD3-BD comprises
   (i) a VH domain comprising the amino acid sequence of SEQ ID NO: 134, and
   (ii) a VL domain comprising the amino acid sequence of SEQ ID NO: 138.
52. The pharmaceutical composition or the kit of any one of the preceding items, wherein the tumor cell associated antigen (TAA) is selected from the group consisting of CD138, CD79b, TPBG (5T4), HER2, MSLN, MUC1, CA-125 (MUC16), PSMA, BCMA, CD19, EpCAM, CLEC12A (CLL1), CD20, CD22, CEA, CD33, EGFR, GPC3, CD123, CD38, CD33, CD276, CDH3 (cadherin 3), FGFR1, SSTR2, CD133, EPHA2, HLA-A2, IL13RA2, ROR1, CEACAM6, CD135, GD-2, GA733, CD135 (FLT3), CSPG4 and TAG-72,
   preferably, wherein the TAA is selected from the group consisting of CD138, CD79b, CD123, HER2, MSLN, PSMA, BCMA, CD19, CD20, CEA, CD38, CD33, CLEC12a and ROR1,
   in particular wherein the TAA is selected from the group consisting of HER2, MSLN and ROR1.
53. The pharmaceutical composition or the kit of any one of the items 1 to 52, wherein said MA2 comprises one TAA-BD.
54. The pharmaceutical composition or the kit of item 53, wherein said TAA-BD binds to said TAA with a monovalent dissociation constant (K_{D}) of less than 50 nM, particularly less than 20 nM, particularly less than 10 nM, particularly less than 5 nM, particularly of 0.01 to 2 nM, particularly of 0.02 to 1 nM, particularly of 0.03 to 0.5 nM as measured by SPR, particularly wherein said TAA-BD is an scFv.
55. The pharmaceutical composition or the kit of item 53 or 54, wherein said TAA-BD is a mesothelin binding domain (MSLN-BD), which specifically binds to mesothelin (MSLN).
56. The pharmaceutical composition or the kit of item 55, wherein said MSLN-BD comprises
   (i) the HCDR1, HCDR2, and HCDR3 sequences of SEQ ID NOs: 139, 140 and 141, respectively, and the LCDR1, LCDR2, and LCDR3 sequences of SEQ ID NOs: 143, 144 and 145, respectively; and
   (ii) VH3 or VH4 domain framework sequences FR1 to FR4; preferably VH3 domain framework sequences FR1 to FR4; and
   (iii) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
57. The pharmaceutical composition or the kit of item 55 or 56, wherein said MSLN-BD comprises
   a) HCDR1, HCDR2, and HCDR3 sequences of SEQ ID NOs: 139, 140 and 141, respectively,
   b) LCDR1, LCDR2, and LCDR3 sequences of SEQ ID NOs: 143, 144 and 145, respectively,
   c) a VH sequence at least 90, 91, 92, 93, 94, 95, 96, 97, 98 or 99 percent identical to the amino acid sequence SEQ ID NO: 142, and
   d) a VL sequence at least 90, 91, 92, 93, 94, 95, 96, 97, 98 or 99 percent identical to the amino acid sequence SEQ ID NO: 146.
58. The pharmaceutical composition or the kit of any one of items 55 to 57, wherein said MSLN-BD:
   a. binds to human MSLN with a monovalent dissociation constant (K_{D}) of less than 10 nM, particularly of 0.01 to 5 nM, particularly of 0.02 to 1 nM, in particular as measured by SPR, particularly wherein said MSLN-BD is a scFv; and/or
   b. is cross reactive with Macaca fascicularis (cynomolgus) MSLN, in particular binds to cynomolgus MSLN with a monovalent K_{D} of less than 15 nM, particularly of 0.01 to 10 nM, particularly of 0.02 to 5 nM as measured by SPR, particularly wherein said MSLN-BD is an scFv.
59. The pharmaceutical composition or the kit of item 53 or 54, wherein said TAA-BD is a HER2 binding domain (HER2-BD), which specifically binds to HER2 (HER2).
60. The pharmaceutical composition or the kit of item 55, wherein said HER2-BD comprises
   (i) the HCDR1, HCDR2, and HCDR3 sequences of SEQ ID NOs: 165, 166 and 167, respectively, and the LCDR1, LCDR2, and LCDR3 sequences of SEQ ID NOs: 170, 171 and 172, respectively; and
   (ii) VH3 or VH4 domain framework sequences FR1 to FR4; preferably VH3 domain framework sequences FR1 to FR4; and
   (iii) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
61. The pharmaceutical composition or the kit of item 55 or 56, wherein said HER2-BD comprises
   a) HCDR1, HCDR2, and HCDR3 sequences of SEQ ID NOs: 165, 166 and 167, respectively,
   b) LCDR1, LCDR2, and LCDR3 sequences of SEQ ID NOs: 170, 171 and 172, respectively,
   c) a VH sequence at least 90, 91, 92, 93, 94, 95, 96, 97, 98 or 99 percent identical to the amino acid sequence SEQ ID NO: 168 or 169, and
   d) a VL sequence at least 90, 91, 92, 93, 94, 95, 96, 97, 98 or 99 percent identical to the amino acid sequence SEQ ID NO: 173 or 174.
62. The pharmaceutical composition or the kit of any one of the items 1 to 52, wherein the MA2 comprises two TAA-BDs.
63. The pharmaceutical composition or the kit of item 62, wherein the two TAA-BDs in MA2 bind the same antigen.
64. The pharmaceutical composition or the kit of item 62 or 63, wherein the TAA is selected from the group consisting of ROR1, HER2 and tumor cell associated antigens, whose extracellular part also occurs in soluble form in the serum of a patient, in particular wherein the TAA is selected from the group consisting of HER2, MSLN and ROR1, and/or wherein the TAA is selected from the group consisting of ROR1 and tumor cell associated antigens, which are also expressed on the surface of healthy cells, in amounts corresponding to 10 - 75 % of the particular TAA density on the surface of the cancer cells, as determined by antigen binding capacity measurements.
65. The pharmaceutical composition or the kit of any one of the items 62 to 64, wherein said TAA-BD binds to said TAA with a monovalent dissociation constant (K_{D}) in the range of 0.1 to 50 nM, particularly of 0.2 to 30 nM, particularly of 0.3 to 20 nM, particularly of 0.4 to 10 nM, as measured by SPR, particularly wherein said TAA-BD is an scFv.
66. The pharmaceutical composition or the kit of any one of the items 62 to 65, wherein said TAA-BD is a mesothelin binding domain (MSLN-BD), which specifically binds to mesothelin (MSLN), particularly human mesothelin.
67. The pharmaceutical composition or the kit of item 66, wherein said MSLN-BD binds to mesothelin (MSLN) with a monovalent dissociation constant (K_{D}) in the range of from 0.4 to 50 nM, preferably of from 0.4 to 40 nM, more preferably of from 0.5 to 30 nM, even more preferably of from 0.5 to 20 nM, when measured by SPR, particularly wherein said MSLN-BD is an scFv.
68. The pharmaceutical composition or the kit of item 66 or 67, wherein said MSLN-BD is cross-reactive with *Macaca fascicularis* (cynomolgus) MSLN, in particular binds to Cynomolgus MSLN with a monovalent K_{D} in the range of 3 to 75 nM, particularly in the range of 3 to 60 nM, particularly of 4 to 50 nM, particularly of 5 to 40 nM, as measured by SPR, when measured by SPR, in particularly wherein said MSLN-BD is an scFv.
69. The pharmaceutical composition or the kit of any one of the items 66 to 68, wherein said MSLN-BD comprises
   (i) the HCDR1, HCDR2, and HCDR3 sequences of SEQ ID NOs: 147, 148 (or 151) and 149, respectively, and the LCDR1, LCDR2, and LCDR3 sequences of SEQ ID NOs: 153, 154 and 155, respectively; or the HCDR1, HCDR2, and HCDR3 sequences of SEQ ID NOs: 157, 158 and 159, respectively, and the LCDR1, LCDR2, and LCDR3 sequences of SEQ ID NOs: 161, 162 and 163, respectively; and
   (ii) VH3 or VH4 domain framework sequences FR1 to FR4; particularly VH3 domain framework sequences FR1 to FR4; and
   (iii) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vκ FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
70. The pharmaceutical composition or the kit of item 69, wherein said MSLN-BD comprises
   a.1) HCDR1, HCDR2, and HCDR3 sequences of SEQ ID NOs: 147, 148 (or 151) and 149, respectively,
   b.1) LCDR1, LCDR2, and LCDR3 sequences of SEQ ID NOs: 153, 154 and 155, respectively,
   c.1) a VH sequence at least 90, 91, 92, 93, 94, 95, 96, 97, 98 or 99 percent identical to the amino acid sequence SEQ ID NO: 150, and
   d.1) a VL sequence at least 90, 91, 92, 93, 94, 95, 96, 97, 98 or 99 percent identical to the amino acid sequence SEQ ID NO: 156;
   or
   a.2) HCDR1, HCDR2, and HCDR3 sequences of SEQ ID NOs: 147, 148 (or 151) and 149, respectively,
   b.2) LCDR1, LCDR2, and LCDR3 sequences of SEQ ID NOs: 153, 154 and 155, respectively,
   c.2) a VH sequence at least 90, 91, 92, 93, 94, 95, 96, 97, 98 or 99 percent identical to the amino acid sequence SEQ ID NO: 152, and
   d.2) a VL sequence at least 90, 91, 92, 93, 94, 95, 96, 97, 98 or 99 percent identical to the amino acid sequence SEQ ID NO: 156;
   or
   a.3) HCDR1, HCDR2, and HCDR3 sequences of SEQ ID NOs: 157, 158 and 159, respectively,
   b.3) LCDR1, LCDR2, and LCDR3 sequences of SEQ ID NOs: 161, 162 and 163, respectively,
   c.3) a VH sequence at least 90, 91, 92, 93, 94, 95, 96, 97, 98 or 99 percent identical to the amino acid sequence SEQ ID NO: 160, and
   d.3) a VL sequence at least 90, 91, 92, 93, 94, 95, 96, 97, 98 or 99 percent identical to the amino acid sequence SEQ ID NO: 164.
71. The pharmaceutical composition or the kit of item 69 or 70, wherein said VH domain comprises a cysteine at position 51 (AHo numbering) and said VL domain comprises a cysteine at position 141 (AHo numbering).
72. The pharmaceutical composition or the kit of any one of the preceding items, wherein said multispecific antibody MA2 is in a format selected from the group consisting of: scDB; a bispecific T cell engager (BiTE; tandem di-scFv); tandem tri-scFv; Fab-(scFv); scFab-dsscFv; tribody (Fab-(scFv)₂); Fab₂; Fab-Fv₂; diabody; triabody; tetrabody; scDb-scFv; di-diabody; scFv-Fc-scFv fusion (ADAPTIR); DVD-Ig; IgG-scFv fusions, such as CODV-IgG, Morrison (IgG CH₃-scFv fusion (Morrison L) or IgG CL-scFv fusion (Morrison H)), bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain) and Ts2Ab (dsscFv linked to C-terminus of heavy chain); a DART^{™}; a TRIDENT^{™}; a scDb, a tandem tri-scFv, a Fab-(scFv), a scFab-dsscFv, a Fab-(scFv)₂, a Fab₂, a Fab-Fv₂, a diabody or a scDb-scFv fused to the N- and/or the C-terminus of a heterodimerization domain other than heterodimeric Fc domains; a MATCH and DuoBodies.
73. The pharmaceutical composition or the kit of any one of the preceding items, wherein said multispecific antibody MA2 is in a format selected from the group consisting of: a Morrison L, a Morrison H, an scFv-Fc-scFv fusion, and a MATCH format, preferably a Morrison L and a MATCH format; particularly wherein said MA2 is in a MATCH format; in particular where said MA2 is a scMATCH3, MATCH3 or a MATCH4.
74. The pharmaceutical composition or the kit of any one of the preceding items, wherein said multispecific antibody MA2 is a single-chain protein.
75. The pharmaceutical composition or the kit of item 74, wherein said single-chain protein comprises an amino acid sequence consisting of:
   (i) a first VL domain,
   (ii) a first polypeptide linker,
   (iii) a first VH domain,
   (iv) a second polypeptide linker,
   (v) a second VL domain,
   (vi) a third polypeptide linker, and
   (vii) a second VH domain,
      arranged one after another in the stated order,
      wherein said first VL domain associates with said second VH domain to form a first binding domain, and said second VL domain associates with said first VH domain to form a second binding domain,
      and wherein said single-chain protein further comprises
   (viii) a third binding domain, which is formed by a third VL domain and a third VH domain that are connected via a fourth polypeptide linker, where said third binding domain is fused C-terminally or N-terminally via a fifth polypeptide linker to said amino acid sequence,
   wherein said three binding domains have the following specificities:
   a) the first binding domain either specifically binds to a TAA (TAA-BD), particularly to mesothelin (MSLN-BD), or specifically binds to hSA (hSA-BD);
   b) the second binding domain specifically binds to human CD3 (CD3-BD); and
   c) the third binding domain specifically binds to a TAA (TAA-BD), particularly to mesothelin (MSLN-BD).
76. The pharmaceutical composition or the kit of item 74 or 75, wherein said single-chain protein comprises an amino acid sequence having at least 90, 95, 96, 97, 98 or 99 percent identity to the amino acid sequence selected from SEQ ID NO: 175, more particularly comprises the amino acid sequence of SEQ ID NO: 175, especially consists of the amino acid sequence SEQ ID NO: 175.
77. The pharmaceutical composition or the kit of any one of the items 1 to 73, wherein said multispecific antibody MA2 is a hetero-dimeric protein in the MATCH3 or MATCH4 format comprising a first and a second single-chain protein,
   wherein said first single-chain protein comprises a first amino acid sequence consisting of (from the N- to the C-terminus):
   (ia) a first VL domain,
   (iia) a first polypeptide linker, and
   (iiia) a second VL domain, and
   wherein said second single-chain protein comprises a second amino acid sequence consisting of (from the N- to the C-terminus):
   (ib) a first VH domain,
   (iib) a second polypeptide linker, and
   (iiib) a second VH domain, and
      wherein said first VL domain associates with either said first or said second VH domain to form a first binding domain, and said second VL domain associates with the other of said VH domains to form a second binding domain,
      and wherein at least one of said first and said second single-chain proteins further comprises
   (iv) a third binding domain, which is formed by a third VL domain and a third VH domain that are connected via a third polypeptide linker, where said third binding domain is fused via a fourth polypeptide linker to said first or said second amino acid sequence, and
      wherein optionally, in the MATCH4 format, at least one of said first and said second single-chain proteins further comprises
   (v) a fourth binding domain, which is formed by a fourth VL domain and a fourth VH domain that are connected via a fifth polypeptide linker, where said fourth binding domain is fused via a sixth polypeptide linker to said first or said second amino acid sequence,
   wherein said three (MATCH3), or optionally four (MATCH4), binding domains have the following specificities:
   when three binding domains are present (MATCH3),
      a) one of the binding domains specifically binds to a TAA (TAA-BDs), particularly to mesothelin (MSLN-BD);
      b) another binding domain specifically binds to human CD3 (CD3-BD); and,
      c) the remaining binding domain either specifically binds to the same TAA (TAA-BDs), particularly to mesothelin (MSLN-BD), or to human serum albumin (hSA-BD);
   or, when the optional fourth binding domain is present (MATCH4),
      a) two binding domains specifically bind to the same TAA (TAA-BDs), particularly to mesothelin (MSLN-BD);
      b) another binding domain specifically binds to human CD3 (CD3-BD); and,
      c) the remaining binding domain specifically binds to human serum albumin (hSA-BD).
78. The pharmaceutical composition or the kit of item 77, wherein the optional fourth binding domain is absent and wherein one of said first and second binding domains is a CD3-BD and the other one of said first and second binding domains is a TAA-BD.
79. The pharmaceutical composition or the kit of item 77, wherein the optional fourth binding domain is present and wherein the third binding domain is fused to either the first or the second amino acid sequence, and the fourth binding domain is fused to the other one of the said two amino acid sequences.
80. The pharmaceutical composition or the kit of item 77 or 79, wherein the optional fourth binding domain is present and wherein one of said first and second binding domains is a CD3-BD and the other one of said first and second binding domains is a hSA-BD.
81. The pharmaceutical composition or the kit of any one of the items 77 to 80, wherein said hetero-dimeric protein does not comprise a cognate pair of a first and a second proteinaceous interaction domain, other than said first and second VL and VH domains, wherein said first proteinaceous interaction domain is comprised in said first single-chain protein and wherein said second proteinaceous interaction domain is comprised in said second single-chain protein.
82. The pharmaceutical composition or the kit of any one of the items 77 to 81, wherein said first single-chain protein and said second single-chain protein hetero-dimerize in a parallel orientation, *i*.*e*. said first VL domain associates with said first VH domain and said second VL domain associates with said second VH domain.
83. The pharmaceutical composition or the kit of any one of items 77 to 81, wherein said first single-chain protein and said second single-chain protein hetero-dimerize in an anti-parallel orientation, *i*.e. said first VL domain associates with said second VH domain and said second VL domain associates with said first VH domain.
84. The pharmaceutical composition or the kit of any one of items 77 to 83, wherein said first single-chain protein comprises an amino acid sequence having at least 90, 95, 96, 97, 98 or 99 percent identity to the amino acid sequence of SEQ ID NO: 176, more particularly comprises the amino acid sequence of SEQ ID NO: 176, especially consists of the amino acid sequence SEQ ID NO: 176 and said second single-chain protein comprises an amino acid sequence having at least 90, 95, 96, 97, 98 or 99 percent identity to the amino acid sequence of SEQ ID NO: 177, more particularly comprises the amino acid sequence of SEQ ID NO: 177, especially consists of the amino acid sequence SEQ ID NO: 177; or said first single-chain protein comprises an amino acid sequence having at least 90, 95, 96, 97, 98 or 99 percent identity to the amino acid sequence of SEQ ID NO: 178, more particularly comprises the amino acid sequence of SEQ ID NO: 178, especially consists of the amino acid sequence SEQ ID NO: 178 and said second single-chain protein comprises an amino acid sequence having at least 90, 95, 96, 97, 98 or 99 percent identity to the amino acid sequence of SEQ ID NO: 179, more particularly comprises the amino acid sequence of SEQ ID NO: 179, especially consists of the amino acid sequence SEQ ID NO: 179.
85. The pharmaceutical composition or the kit of any one of the preceding items, wherein at least one of said antibody variable domains of MA1 and MA2 comprises CDR regions derived from a parental rabbit antibody.
86. The pharmaceutical composition or the kit of any one of the preceding items, wherein the TAA-BD and the CD3-BD or at least one of the two TAA-BDs and the CD3-BD are capable of binding to their respective antigens simultaneously, particularly wherein the TAA-BD and the CD3-BD or the two TAA-BDs and the CD3-BD are capable of binding to their respective antigens simultaneously.
87. The pharmaceutical composition or the kit of any one of items 1 to 86 for use as a medicament.
88. The pharmaceutical composition or the kit of any one of items 1 to 86 for use in the treatment of a disease, particularly a human disease, more particularly a human disease selected from cancer.
89. The pharmaceutical composition or the kit of any one of items 1 to 86 for use in the treatment of a disease according to item 86, wherein said disease is a proliferative disease, particularly a cancer, particularly a cancer selected from melanoma, mesothelioma, pancreatic cancer, stomach cancer, breast cancer, ovarian cancer and lung cancer.
90. A method for the treatment of a disease, particularly a human disease, more particularly a human disease selected from cancers, comprising the step of administering the pharmaceutical composition or the kit of any one of items 1 to 86.
91. The method of item 90, wherein said disease is a proliferative disease, particularly a cancer, particularly a cancer selected from melanoma, mesothelioma, pancreatic cancer, stomach cancer, breast cancer, ovarian cancer and lung cancer.
92. A method for treating a patient suffering from a disease, comprising the step of administering a first multispecific antibody (MA1) comprising one binding domain, which specifically binds to CD137 (CD137-BD), and one binding domain, which specifically binds to PDL1 (PDL1-BD), and a second multispecific antibody (MA2) comprising at least one binding domain, which specifically binds to a tumor cell associated antigen (TAA-BD), and one binding domain, which specifically binds to CD3 (CD3-BD), wherein said multispecific antibodies MA1 and MA2 are as defined in any of the items 1 to 86.
93. The method of item 92, wherein said disease is a proliferative disease, particularly a cancer, particularly a cancer selected from melanoma, mesothelioma, pancreatic cancer, stomach cancer, breast cancer, ovarian cancer and lung cancer.
94. A multispecific antibody MA1 comprising one binding domain, which specifically binds to CD137 (CD137-BD), and one binding domain, which specifically binds to PDL1 (PDL1-BD) for use in the treatment of a subject suffering from a disease, wherein said multispecific antibody MA1 is administered to said subject in combination with a multispecific antibody MA2 comprising at least one binding domain, which specifically binds to a tumor cell associated antigen (TAA-BD), and one binding domain, which specifically binds to CD3 (CD3-BD), wherein said multispecific antibodies MA1 and MA2 are as defined herein.
95. A multispecific antibody MA2 comprising one binding domain, which specifically binds to a tumor cell associated antigen (TAA-BD), and one binding domain, which specifically binds to CD3 (CD3-BD) for use in the treatment of a subject suffering from a disease, wherein said multispecific antibody MA2 is administered to said subject in combination with a multispecific antibody MA1 comprising at least one binding domain, which specifically binds to CD137 (CD137-BD), and one binding domain, which specifically binds to PDL1 (PDL1-BD), wherein said multispecific antibodies MA1 and MA2 are as defined herein.
96. The multispecific antibody MA1 for use according to item 94, or the multispecific antibody MA2 for use according to item 95, wherein said disease is a proliferative disease, particularly a cancer, particularly a cancer selected from melanoma, mesothelioma, pancreatic cancer, stomach cancer, breast cancer, ovarian cancer and lung cancer.

### BRIEF DESCRIPTION OF THE DRAWINGS

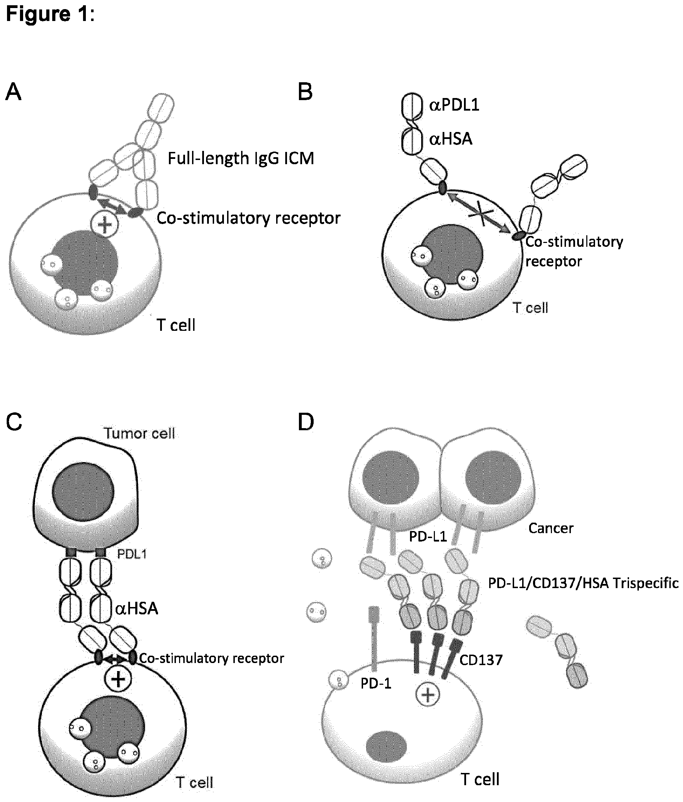

**FIG. 1** shows the target cell dependent activation of T cell co-stimulatory receptors by the multispecific antibodies applied in the present invention. Cross-linking of CD137 with a bivalent monoclonal antibody triggers systemic co-stimulation of T cells that is further boosted by FcyR binding-mediated cross-linking (**A**).The stable bi-/trispecific monovalent molecules used in the present invention cannot cross-link (or, by extension, agonize) co-stimulatory receptors on T cells in the absence of the cell-type being targeted for depletion (**B**). The stable bi-/trispecific monovalent molecules used in the present invention cross-link (or, by extension, agonize) costimulatory receptors on T cells in the presence of the cell-type being targeted for depletion (**C**). Concomitant binding to PDL1 and CD137 triggers selective activation of tumor-reactive T cells and simultaneously blocks PD-1 signaling (**D**).

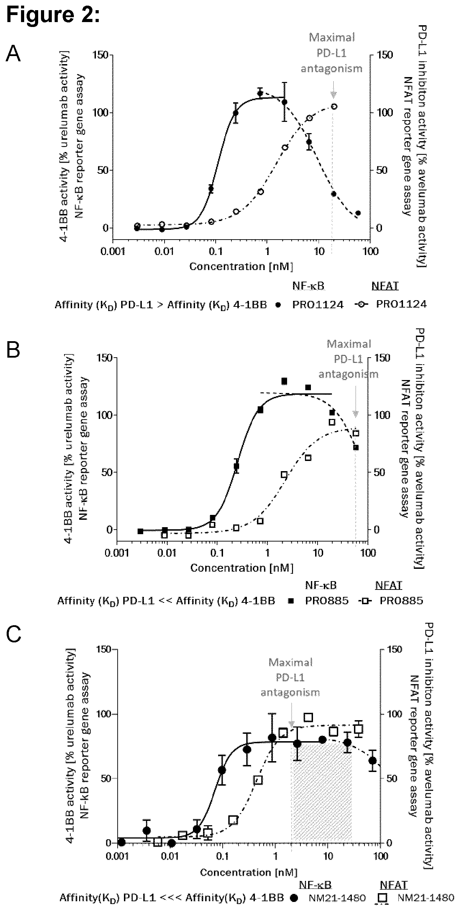

**FIG. 2** shows the concentration-dependent activation of NF-kB signaling in Jurkat reporter cells (filled symbols) and inhibition of PDL1 activity in NFAT reporter cells (open symbols) of: PRO1124 with a PDL1 binding affinity (K_{D}) greater (worse) than that of CD137 (4-1BB) K_{D} (**A**), PRO885 with a PDL1 K_{D} less (better) than that of CD137 (4-1 BB) K_{D} (**B**), and NM21-1480 (PRO1480) with a PDL1 K_{D} considerably less (better) than that of CD137 (4-1 BB) K_{D} (**C**). The grey arrow and the grey dashed line indicate the concentration at which maximal PDL1 antagonism is reached. The corresponding level of CD137 (4-1 BB) agonism at that concentration and the breadth of concentrations, where maximal activity of both PDL1 blockade and CD137 (4-1BB) stimulation is observed, are important for optimal dose finding, as indicated by the shaded grey box in (**C**). By altering the K_{D} of the PDL1-BD to PDL1 to levels significantly lower than the K_{D} of the CD137-BD to CD137 (4-1BB), the EC₅₀ and IC₅₀ values for the respective functional activities become more closely matched and the plateau of CD137 (4-1 BB) stimulation becomes broader. NM21-1480 (PRO1480) has exquisitely optimized PDL1 and CD137 (4-1 BB) binding domains to allow activity of both PDL1 blockade and CD137 (4-1 BB) activation at the same optimal dose and to extend the concentration range of maximal CD137 (4-1 BB) signaling.

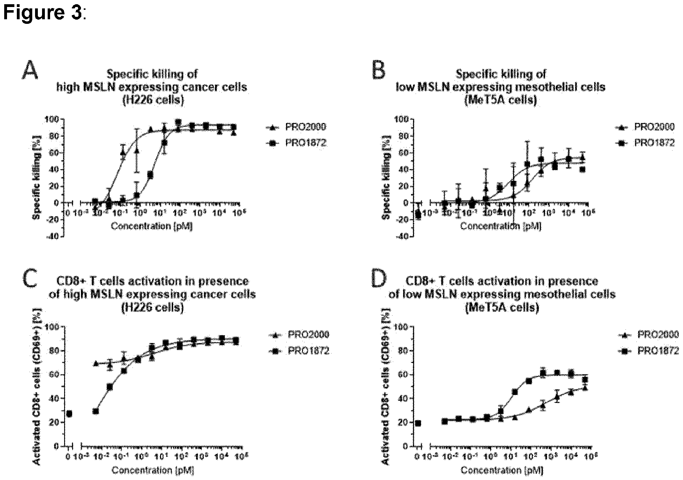

**FIG. 3** shows the cytotoxic activity and effect on CD8+ T cell activation of PRO2000 and PRO1872 in the presence of human serum albumin. (A) Specific killing of high MSLN expressing cancer cells (H226 cells). On cancer cells expressing high levels of mesothelin, the target cell killing potency observed for PRO2000 is 75-fold better than for PRO1872. (B) Specific killing of low MSLN expressing cancer cells (MeT-5A cells). On cells derived from healthy mesothelial tissue (MeT-5A; ATCC CRL-9444) expressing low mesothelin levels the monovalent mesothelin binding protein PRO1872 shows the best killing potency. (C) CD8+ T cell activation in presence of H226 cells, and (D) CD8+ T cell activation in presence of MeT-5A cells. Similar data were observed for CD8+ T cell activation. PBMCs from donor #1 were used. Target cells and CD8+ T cells were analyzed by flow cytometry 40 h after the beginning of their incubation with the respective molecules, and data were fitted using sigmoidal 4PL fit (GraphPad Prism).

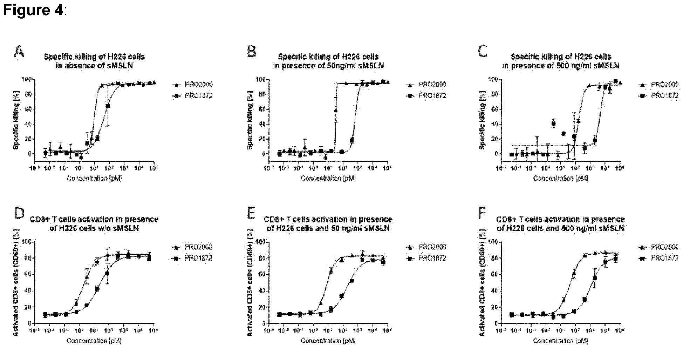

**FIG. 4** shows the cytotoxic activity and effect on CD8+ T cell activation of PRO2000 and PRO1872 in absence or presence of sMSLN. (A to C) Cytotoxic activity of PRO2000 and PRO1872 on H226 target cells. Specific killing of H226 cells in absence of sMSLN (A), in presence of 50 ng/ml sMSLN (B), or in presence of 500 ng/ml sMSLN (C). PRO2000 killing potency is less affected by increasing concentrations of sMSLN as compared to PRO1872 (D to F) Similar data are observed for CD8+ T cell activation in the corresponding conditions. CD8+ T cell activation in presence of H226 cells without sMSLN (D), in presence of 50 ng/ml sMLSN (E), or in presence of 500 ng/ml sMLSN (F). PBMCs from donor #2 were used. Target cells and CD8+ T cells were analyzed by flow cytometry 40 h after the beginning of their incubation with the respective molecules, and data were fitted using sigmoidal 4PL fit (GraphPad Prism).

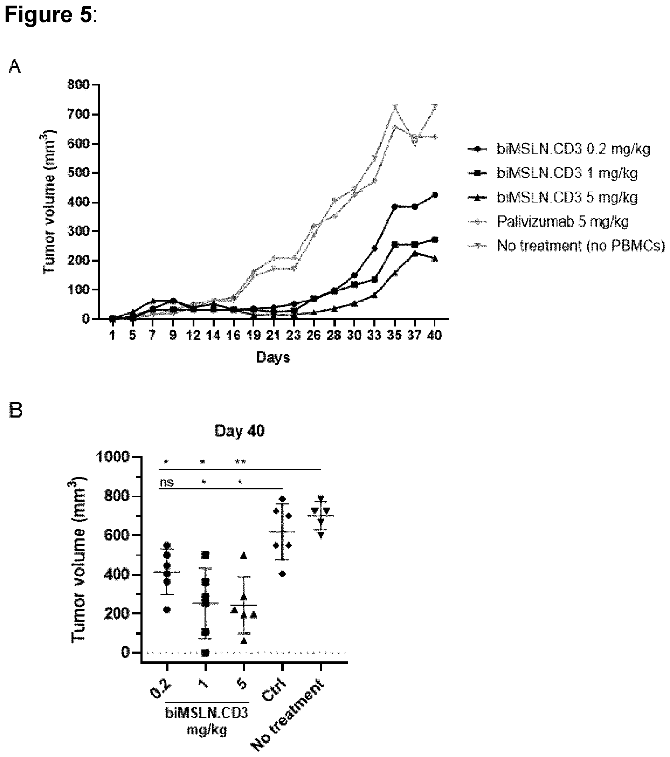

**FIG. 5** shows MSLN-expressing NSCLC tumor cells xenograft experiment with molecule PRO2000 (biMSLN.CD3) revealing tumor growth inhibition for PRO2000 treatment relative to control conditions. (A) Longitudinal analysis of tumor growth in the presence or absence of treatment. The lines depict the median. Animals were subcutaneously co-implanted with 1 x 10⁷ H292 MSLN-expressing NSCLC tumor cells and 1 x 10⁷ PBMCs, and treatment was administered intravenously starting on day 5 and repeated every 5 days until the end of the experiment. (B) Day 40 data displayed as a scatter plot. Each point corresponds to one animal, and the data are displayed with the mean and standard deviation. After a two-way repeated measures ANOVA, the Tukey's multiple comparisons test was performed, and the significance of each data set is depicted relative to palivizumab control (Ctrl, lower line), or no treatment (upper line). ns = not significant; *, p < 0.05; **, p < 0.01, ***, p < 0.001. The spark line in gray indicates 0 on the y-axis.

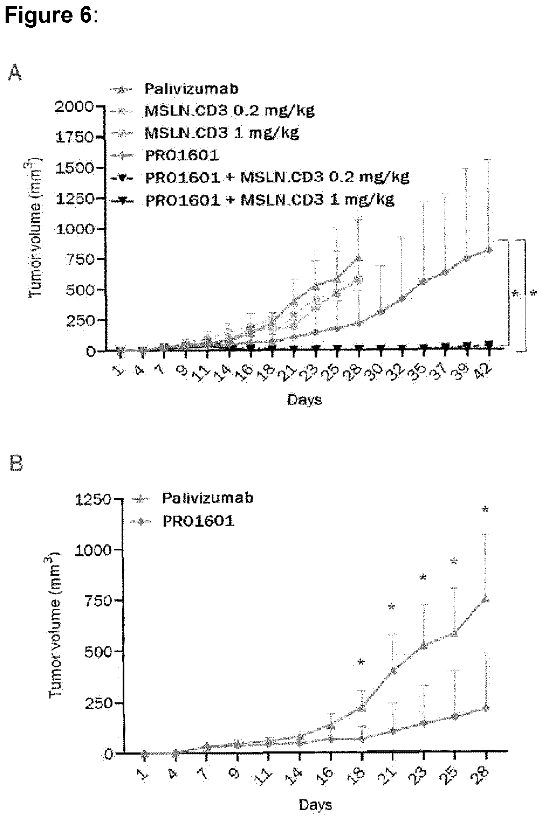

**FIG. 6** shows MSLN-expressing NSCLC tumor cells xenograft experiment with molecules PRO1872 (MSLN.CD3) and PRO1601 (PDL1.CD137) alone as well as with a combination of PRO1601 + PRO1872 (MSLN.CD3), revealing significantly enhanced tumor growth inhibition for the combination treatment relative to the PRO1872 and PRO1601 single treatments. (A) Longitudinal analysis of tumor growth in the presence or absence of treatment. The lines depict the median. Animals were subcutaneously co-implanted with 1 x 10⁷ H292 MSLN-expressing NSCLC tumor cells and 1 x 10⁷ PBMCs, and treatment was administered intravenously starting on day 5 and repeated every 5 days until the end of the experiment. (B) Enlarged representation of the 0 to 28 days section of PRO1601 single treatment revealing significant tumor growth inhibition for PRO1601 alone relative to control conditions. After a two-way repeated measures ANOVA, the Tukey's multiple comparisons test was performed. The significances of the tumor growth inhibition of the PRO1601 + PRO1872 combination relative to PRO1601 alone and PRO1601 alone relative to palivizumab control, respectively, are depicted (*, p < 0.001). The spark line in gray indicates 0 on the y-axis.

### DETAILED DESCRIPTION OF THE INVENTION

Known TAA/CD3 bsAbs-based immunotherapies typically suffer from dose-limiting toxicities and limited *in vivo* efficacy. There is thus a need in the medical field for improved TAA/CD3 bsAbs-based immunotherapies, which have higher efficacy but at the same time a similar or even lower toxicological profile than the currently available approaches.

The present invention provides pharmaceutical compositions or a kit comprising a first multispecific antibody, which comprise one CD137-agonizing binding domain and one PDL1-antagonizing binding domain but no immunoglobulin Fc region, and a second multispecific antibody, which specifically binds to a TAA and CD3. These multispecific antibody combinations exhibit a significantly greater efficacy in TAA-(re)directed target cell killing and tumor growth inhibition than the individual multispecific antibody components alone.

Albeit the multispecific CD137/PDL1 binding antibodies in the pharmaceutical compositions or the kit of the present invention are monovalent for CD137, they are able to cluster and to agonize CD137, however strictly in the presence of PDL1-positive cells, thus avoiding systemic activation of CD137. The monovalent CD137 binding and Fc-less structure of the multispecific antibody ensures that agonism of CD137 on effector cells can only arise when the antibody concomitantly binds to PDL1 on the surface of target cells. At the same time the toxicological profiles of the pharmaceutical compositions or the kit of the present invention are not worse than for the respective anti-TAAxCD3 multispecific antibody components alone that are comprised therein. Accordingly, the application of the pharmaceutical compositions or the kit of the present invention provides a significant risk-to-benefit gain when compared to the single application of the individual anti-TAAxCD3 multispecific antibodies.

When these multispecific CD137/PDL1 binding antibodies are combined with anti-TAAxCD3 multispecific antibodies comprising two TAA-BDs having medium to low binding affinity for the same TAA target, this approach in particular allows TAA-(re)directed target cell killing of tumor cell expressing non-clean TAAs, i.e. TAAs that are also expressed at significantly high amounts in healthy cells, such as Mesothelin (MSLN), EGFR, EpCAM and HER2. The application of these specific variants of the pharmaceutical compositions or the kits of the present invention provide a significant risk-to-benefit gain also for TAA-(re)directed target cell killing of tumor cell expressing non-clean TAAs when compared to single anti-TAAxCD3 antibody therapies.

Furthermore, the optional addition of a half-life-extending anti-hSA domain not only enables convenient dosing but should also promote delivery of the molecule to tumor microenvironments.

The pharmaceutical compositions or the kits of the present invention thus provide distinct therapeutic advantages over conventional compositions and therapies.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

In one aspect, the present invention relates to a pharmaceutical composition comprising:
1) a first multispecific antibody (MA1) comprising
   a) one binding domain, which specifically binds to CD137 (CD137-BD), and
   b) one binding domain, which specifically binds to PDL1 (PDL1-BD),
2) a second multispecific antibody (MA2) comprising
   a) at least one binding domain, which specifically bind(s) to a tumor cell associated antigen (TAA-BD),
   b) one binding domain, which specifically binds to CD3 (CD3-BD),
3) a pharmaceutically acceptable carrier.

In another aspect, the present invention relates to a kit comprising:
1) a first multispecific antibody (MA1) comprising
   a) one binding domain, which specifically binds to CD137 (CD137-BD), and
   b) one binding domain, which specifically binds to PDL1 (PDL1-BD), and
2) a second multispecific antibody (MA2) comprising
   a) at least one binding domain, which specifically binds to a tumor cell associated antigen (TAA-BD), and
   b) one binding domain, which specifically binds to CD3 (CD3-BD).

In particular embodiments, said second multispecific antibody (MA2) comprises one or two TAA-BD(s).

In particular embodiments, said multispecific antibody MA1 does not comprise an immunoglobulin Fc region polypeptide.

In a particular aspect, the present invention relates to a pharmaceutical composition comprising:
1) a first multispecific antibody (MA1) comprising
   a) one binding domain, which specifically binds to CD137 (CD137-BD), and
   b) one binding domain, which specifically binds to PDL1 (PDL1-BD),
2) a second multispecific antibody (MA2) comprising
   a) one or two binding domain(s), which specifically bind(s) to a tumor cell associated antigen (TAA-BD),
   b) one binding domain, which specifically binds to CD3 (CD3-BD),
3) a pharmaceutically acceptable carrier,
wherein said MA1 does not comprise an immunoglobulin Fc region polypeptide.

In another particular aspect, the present invention relates to a kit comprising:
1) a first multispecific antibody (MA1) comprising
   a) one binding domain, which specifically binds to CD137 (CD137-BD), and
   b) one binding domain, which specifically binds to PDL1 (PDL1-BD), and
2) a second multispecific antibody (MA2) comprising
   a) one or two binding domain(s), which specifically bind(s) to a tumor cell associated antigen (TAA-BD), and
   b) one binding domain, which specifically binds to CD3 (CD3-BD),
wherein said MA1 does not comprise an immunoglobulin Fc region polypeptide.

The term "antibody" and the like, as used herein, includes whole antibodies or single chains thereof; and any antigen-binding fragment (*i*.e., "antigen-binding portion") or single chains thereof; and molecules comprising antibody CDRs, VH regions or VL regions (including without limitation multispecific antibodies). A naturally occurring "whole antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDRs), interspersed with regions that are more conserved, termed framework regions (FRs). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e. g., effector cells) and the first component (Clq) of the classical complement system.

The term "immunoglobulin Fc region", as used herein, refers to the CH2 and CH3 domains of the heavy chain constant regions.

The terms "binding domain", "antigen-binding fragment thereof", "antigen-binding portion" of an antibody, and the like, as used herein, refer to one or more fragments of an intact antibody that retain the ability to specifically bind to a given antigen (e. g., PDL1, CD137, TAA, CD3, hSA). Antigen-binding functions of an antibody can be performed by fragments of an intact antibody. In some embodiments, a binding domain of a multispecific antibody applied in the pharmaceutical composition or the kit of the present invention is selected from the group consisting of a Fab fragment, a monovalent fragment consisting of the VL, VH, CL and CH1 domains; a F(ab)2 fragment, a bivalent fragment comprising two Fab fragments linked by a disulfide bridge at the hinge region; an Fd fragment consisting of the VH and CH1 domains; an Fv fragment consisting of the VL and VH domains of a single arm of an antibody; a single domain antibody (dAb) fragment (Ward et al., 1989 Nature 341:544-546), which consists of a VH domain; an isolated complementarity determining region (CDR), a dsFv, a scAb, STAB, and binding domains based on alternative scaffolds including but limited to ankyrin-based domains, fynomers, avimers, anticalins, fibronectins, and binding sites being built into constant regions of antibodies (e. g. f-star technology (F-star's Modular Antibody Technology^{™})). Suitably, the binding domain of an antibody applied in the present invention is a single-chain Fv fragment (scFv) or a single antibody variable domain. In a preferred embodiment, the binding domain of an antibody applied in the present invention is a single-chain Fv fragment (scFv). In particular embodiments, the two variable domains of an antigen-binding fragment, as in an Fv or an scFv fragment, are stabilized by an interdomain disulfide bond, in particular said VH domain comprises a single cysteine residue in position 51 (AHo numbering) and said VL domain comprises a single cysteine residue in position 141 (AHo numbering).

The term "Complementarity Determining Regions" ("CDRs") refers to amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273, 927-948 ("Chothia" numbering scheme); ImMunoGenTics (IMGT) numbering (Lefranc, M.-P., The Immunologist, 7, 132-136 (1999); Lefranc, M.-P. et al., Dev. Comp. Immunol., 27, 55-77 (2003)) ("IMGT" numbering scheme); and the numbering scheme described in Honegger & Pluckthun, J. Mol. Biol. 309 (2001) 657-670 ("AHo" numbering). For example, for classic formats, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 24-34 (LCDR1), 50-56 (LCDR2), and 89-97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align.

In the context of the present invention, the numbering system suggested by Honegger & Plückthun ("AHo") is used (Honegger & Pluckthun, J. Mol. Biol. 309 (2001) 657-670), unless specifically mentioned otherwise. In particular, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H27-H42; HCDR2 (also referred to as CDR-H2): H57-H76; HCDR3 (also referred to as CDR-H3): H108-H138. For the sake of clarity, the numbering system according to Honegger & Plückthun takes the length diversity into account that is found in naturally occurring antibodies, both in the different VH and VL subfamilies and, in particular, in the CDRs, and provides for gaps in the sequences. Thus, in a given antibody variable domain usually not all positions 1 to 149 will be occupied by an amino acid residue.

The term "binding specificity" as used herein refers to the ability of an individual antibody to react with one antigenic determinant and not with a different antigenic determinant. As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In its most general form (and when no defined reference is mentioned), "specific binding" is referring to the ability of the antibody to discriminate between the target of interest and an unrelated molecule, as determined, for example, in accordance with a specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard color development *(e. g.* secondary antibody with horseradish peroxide and tetramethyl benzidine with hydrogen peroxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. In a further example, an SPR assay can be carried out, wherein at least 10-fold, particularly at least 100-fold difference between a background and signal indicates on specific binding. Typically, determination of binding specificity is performed by using not a single reference molecule, but a set of about three to five unrelated molecules, such as milk powder, transferrin or the like.

Suitably, the antibodies applied in the pharmaceutical compositions or the kit of the invention are isolated antibodies. The term "isolated antibody", as used herein, refers to an antibody that is substantially free of other antibodies having different antigenic specificities *(e. g.,* an isolated antibody that specifically binds PDL1 and CD137 is substantially free of antibodies that specifically bind antigens other than PDL1 and CD137, an isolated antibody that specifically binds PDL1, CD137 and hSA is substantially free of antibodies that specifically bind antigens other than PDL1, CD137 and hSA, and an isolated antibody that specifically binds MSLN, CD3 and hSA is substantially free of antibodies that specifically bind antigens other than MSLN, CD3 and hSA). Moreover, an isolated antibody may be substantially free of other cellular material and/or chemicals.

Suitably, the antibodies applied in the pharmaceutical compositions or the kit of the invention are monoclonal antibodies. The term "monoclonal antibody" or "monoclonal antibody composition" as used herein refers to antibodies that are substantially identical to amino acid sequence or are derived from the same genetic source. A monoclonal antibody composition displays a binding specificity and affinity for a particular epitope, or binding specificities and affinities for specific epitopes.

Antibodies applied in the pharmaceutical compositions or the kit of the invention include, but are not limited to, chimeric, human and humanized antibodies.

The term "chimeric antibody" (or antigen-binding fragment thereof) is an antibody molecule (or antigen-binding fragment thereof) in which (a) the constant region, or a portion thereof, is altered, replaced or exchanged so that the antigen-binding site (variable region) is linked to a constant region of a different or altered class, effector function and/or species, or an entirely different molecule which confers new properties to the chimeric antibody, *e. g.,* an enzyme, toxin, hormone, growth factor, drug, etc.; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

The term "human antibody" (or antigen-binding fragment thereof), as used herein, is intended to include antibodies (and antigen-binding fragments thereof) having variable regions in which both the framework and CDR regions are derived from sequences of human origin. Furthermore, if the antibody contains a constant region, the constant region also is derived from such human sequences, *e. g.,* human germline sequences, or mutated versions of human germline sequences. The human antibodies and antigen-binding fragments thereof of the invention may include amino acid residues not encoded by human sequences *(e. g.,* mutations introduced by random or site-specific mutagenesis *in vitro* or by somatic mutation *in vivo).* This definition of a human antibody specifically excludes a humanized antibody comprising non-human antigen-binding residues. Human antibodies can be produced using various techniques known in the art, including phage-display libraries (Hoogenboom and Winter, J. Mol. Biol, 227:381 (1991); Marks et al, J. Mol. Biol, 222:581 (1991)). Also available for the preparation of human monoclonal antibodies are methods described in Cole et al, Monoclonal Antibodies and Cancer Therapy, Alan R. Liss, p. 77 (1985); Boemer et al, J. Immunol, 147(I):86-95 (1991). See also van Dijk and van de Winkel, Curr. Opin. Pharmacol, 5: 368-74 (2001). Human antibodies can be prepared by administering the antigen to a transgenic animal that has been modified to produce such antibodies in response to antigenic challenge, but whose endogenous loci have been disabled, *e. g.,* immunized xenomice (see, *e. g*., U.S. Pat. Nos. 6,075,181 and 6,150,584 regarding XENOMOUSE^{™} technology). See also, for example, Li et al, Proc. Natl. Acad. Sci. USA, 103:3557- 3562 (2006) regarding human antibodies generated via a human B-cell hybridoma technology.

A "humanized" antibody (or antigen-binding fragment thereof), as used herein, is an antibody (or antigen-binding fragment thereof) that retains the reactivity of a non-human antibody while being less immunogenic in humans. This can be achieved, for instance, by retaining the non-human CDR regions and replacing the remaining parts of the antibody with their human counterparts (*i*.*e.,* the constant region as well as the framework portions of the variable region). Additional framework region modifications may be made within the human framework sequences as well as within the CDR sequences derived from the germline of another mammalian species. The humanized antibodies MA1 and MA2 applied in the pharmaceutical composition or the kit of the invention may include amino acid residues not encoded by human sequences *(e. g.,* mutations introduced by random or site-specific mutagenesis *in vitro* or by somatic mutation *in vivo,* or a conservative substitution to promote stability or manufacturing). See, *e. g.,* Morrison et al., Proc. Natl. Acad. Sci. USA, 81:6851-6855, 1984; Morrison and Oi, Adv. Immunol., 44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include but are not limited to the Xoma technology disclosed in U.S. Pat. No. 5,766,886.

The term "recombinant humanized antibody" as used herein, includes all human antibodies that are prepared, expressed, created or isolated by recombinant means, such as antibodies isolated from a host cell transformed to express the humanized antibody, *e. g.,* from a transfectoma, and antibodies prepared, expressed, created or isolated by any other means that involve splicing of all or a portion of a human immunoglobulin gene, sequences to other DNA sequences.

Preferably, the antibodies applied in the pharmaceutical compositions or the kit of the invention are humanized. More preferably, the antibodies applied in the pharmaceutical compositions or the kit of the invention are humanized and comprises rabbit derived CDRs.

The term "multispecific antibody" as used herein, refers to an antibody that binds to two or more different epitopes on at least two or more different targets *(e. g.,* CD137 and PDL1). The term "multispecific antibody" includes bispecific, trispecific, tetraspecific, pentaspecific and hexaspecific. The term "bispecific antibody" as used herein, refers to an antibody that binds to at least two different epitopes on two different targets *(e. g.,* CD137 and PDL1). The term "trispecific antibody" as used herein, refers to an antibody that binds to at least three different epitopes on three different targets *(e. g.,* CD137, PDL1 and hSA).

The term "epitope" means a protein determinant capable of specific binding to an antibody. Epitopes usually consist of chemically active surface groupings of molecules such as amino acids or sugar side chains and usually have specific three-dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein.

The term "linear epitope" refers to an epitope, wherein all points of interaction between the protein and the interacting molecule (such as an antibody) occurring linearly along the primary amino acid sequence of the protein (continuous).

The term "distal epitope" refers to an epitope which is comprised in the region of the extracellular part of a cell-bound antigen that is distant from the cell surface.

The term "proximal epitope" refers to an epitope which is comprised in the region of the extracellular part of a cell-bound antigen that is close to the cell surface.

The term "recognize" as used herein refers to an antibody antigen-binding fragment thereof that finds and interacts *(e. g.,* binds) with its conformational epitope.

The term "avidity" as used herein refers to an informative measure of the overall stability or strength of the antibody-antigen complex. It is controlled by three major factors: antibody epitope affinity; the valency of both the antigen and antibody; and the structural arrangement of the interacting parts. Ultimately these factors define the specificity of the antibody, that is, the likelihood that the particular antibody is binding to a precise antigen epitope.

Suitably, the CD137-BD of MA1 is characterized by one or more of the following parameters:
a. binds to human CD137 with a monovalent dissociation constant (K_{D}) of less than 50nM, particularly with a monovalent K_{D} of 0.005 to 50 nM, more particularly of 0.01 to 30 nM, more particularly of 0.01 to 10 nM, more particularly of 0.1 to 5 nM, as measured by surface plasmon resonance (SPR), particularly wherein said CD137-BD is an scFv;
b. binds to human CD137 with a K_{off} rate of 10⁻² s⁻¹ or less, more particularly of 10⁻² s⁻¹ to 10⁻⁶ s⁻¹ more particularly of 5x10⁻³ s⁻¹ to 10⁻⁵ s⁻¹, more particularly of 10⁻³ s⁻¹ to 5x10⁻⁴ s⁻¹, as measured by SPR;
c. binds to human CD137 with a Kₒₙ rate of at least 10⁴ M⁻¹s⁻¹ or greater, more particularly of 10⁴ M⁻¹s⁻¹ to 10⁷ M⁻¹s⁻¹, more particularly of 10⁵ M⁻¹s⁻¹ to 5x10⁶ M⁻¹s⁻¹, as measured by SPR;
d. does not cross-compete with urelumab and utomilumab;
e. is cross-reactive with Macaca fascicularis (cynomolgus) CD137, in particular binds to cynomolgus CD137 with a monovalent K_{D} of less than 50 nM, particularly with a monovalent K_{D} of 0.005 to 50 nM, more particularly of 0.01 to 30 nM, more particularly of 0.01 to 10 nM, more particularly of 0.1 to 5 nM, as measured by SPR, particularly wherein said CD137-BD is an scFv.

As used herein, the term "affinity" refers to the strength of interaction between antibody and antigen at single antigenic sites. Within each antigenic site, the variable region of the antibody "arm" interacts through weak non-covalent forces with antigen at numerous sites; the more interactions, the stronger the affinity.

"Binding affinity" generally refers to the strength of the total sum of non-covalent interactions between a single binding site of a molecule *(e. g.,* of an antibody) and its binding partner *(e. g.,* an antigen or, more specifically, an epitope on an antigen). Unless indicated otherwise, as used herein, "binding affinity", "bind to", "binds to" or "binding to" refers to intrinsic binding affinity that reflects a 1:1 interaction between members of a binding pair *(e. g.,* an antibody fragment and antigen). The affinity of a molecule X for its partner Y can generally be represented by the dissociation constant (K_{D}). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative and exemplary embodiments for measuring binding affinity, *i*.*e.* binding strength are described in the following.

The term "K_{assoc}", "Kₐ" or "Kₒₙ", as used herein, are intended to refer to the association rate of a particular antibody-antigen interaction, whereas the term "K_{dis}", "K_{d}" or "K_{off}", as used herein, is intended to refer to the dissociation rate of a particular antibody- antigen interaction. In one embodiment, the term "K_{D}", as used herein, is intended to refer to the dissociation constant, which is obtained from the ratio of K_{d} to Kₐ (*i*.*e.* K_{d}/Kₐ) and is expressed as a molar concentration (M). The "K_{D}" or "K_{D} value" or "KD" or "KD value" according to this invention is in one embodiment measured by using surface-plasmon resonance assays. Affinity to CD137 was determined by surface plasmon resonance (SPR) measurements as described in EP 20164913.4 and EP 20177337.1. Affinity to PDL1 was measured by SPR as described in EP 20164913.4 and EP 20177337.1. Affinity to recombinant human mesothelin (human MSLN) and recombinant *Cynomolgus* MSLN *(Cynomolgus* MSLN) was determined by surface plasmon resonance (SPR) measurements as described in EP 20164913.4 and EP 20177337.1. Affinity to recombinant human CD3 was measured by SPR as described EP 20164913.4 and EP 20177337.1.

Suitably, the first multispecific antibody (MA1) is monovalent for CD137 and PDL1 specificity.

Suitably, the MA1 acts as a CD137 agonist in the presence of PDL1-positive cells. An "activator" or "activating antibody" or "agonist" or "agonist antibody" is one that enhances or initiates the innate activity of the antigen to which it binds usually triggered by binding of the antigen to its natural ligand, in particular signaling by the antigen. In the context of the present invention, the term "CD137 agonist" encompasses the MA1 used in the present invention that is capable to activate CD137 signaling upon clustering of CD137-antigen-binding fragments thereof, *e. g.,* wherein binding of at least two of said CD137-antigen-binding fragments allow for multimerization of the bound CD137 molecules and their activation. In some embodiments, agonist antibodies activate signaling without the presence of the natural ligand.

Suitable CD137-BDs for MA1 are binding domains provided in the present disclosure. The CD137-BDs used in the invention include, but are not limited to, the humanized CD137-binding domains whose sequences are listed in Table 1. Additional details regarding the generation and characterization of the MA1 and its binding domains, as described herein, are disclosed in the patent application WO 2019/072868, which is herewith incorporated by reference in its entirety.

The term "multivalent antibody" refers to a single binding molecule with more than one valency, where "valency" is described as the number of antigen-binding moieties that binds to epitopes on target molecules. As such, the single binding molecule can bind to more than one binding site on a target molecule and/or to more than one target molecule due to the presence of more than one copy of the corresponding antigen-binding moieties. Examples of multivalent antibodies include, but are not limited to bivalent antibodies, trivalent antibodies, tetravalent antibodies, pentavalent antibodies, and the like.

The term "monovalent antibody", as used herein, refers to an antibody that binds to a single target molecule, and more specifically to a single epitope on a target molecule, such as CD137. Also, the term "binding domain" or "monovalent binding domain", as used herein, refers to a binding domain that binds to a single epitope on a target molecule such as CD137.

The term "bivalent antibody" as used herein, refers to (i) a bispecific antibody comprising two different antigen-binding moieties that bind to two targets, (ii) an antibody comprising two different antigen-binding moieties that bind to two different epitopes on two identical target molecules, such as CD137 target molecules, or (iii) an antibody comprising two identical antigen-binding moieties that bind to two identical target molecules, such as CD137 target molecules.

The inventors of the present invention have previously found that a multispecific antibody comprising (a) only one CD137 binding domain (CD137-BD) and (b) one PDL1 binding domain (PDL1-BD) is able to effectively activate CD137 signaling in a targeted manner. These multispecific antibodies are not capable of agonizing CD137 on T cells in the absence of another cell-type, which is recognized by PDL1 binding domain (Figure 1A). The effective activation of CD137 takes place only in the presence of PDL1-positive cells due to binding of anti-PDL1 domains of these multispecific antibodies to PDL1 molecules exposed on the surface of PDL1-positive cells (Figure 1B and 1C). This leads to increased density of the multispecific antibodies in a specific location, and thus increased density of CD137 binding domains. The CD137-BDs thus can effectively cluster and agonize CD137. This concomitant binding to PDL1 and CD137 triggers selective activation of tumor-reactive T cells and simultaneously blocks PD-1 signaling (Figure 1C). Due to high overexpression of PDL1 on tumor cells, CD137 signaling is activated only locally in the presence of said tumor cells, which leads to reduced systemic toxicity. These multispecific antibodies are thus expected to have several beneficial effects in comparison to current treatment options, such as (i) lower rate of immune-related adverse events, and (ii) lower rate of dose-limiting toxicities.

The inventors of the present invention have further found that a certain balancing of the affinities of the CD137-BD and PDL1-BD relative to each other, i.e. a significant higher affinity of the PDL1-BD to PDL1 relative to the affinity of the CD137-BD to CD137, is necessary to ensure that the effective concentration, at which the maximum inhibition of the PDL1/PD-1 interaction is achieved (IC₁₀₀), falls well within the concentration range, at which the maximum activation of CD137-driven NF-κB signaling is achieved. Thereby, the concentration window of maximal activity is significantly extended (compare e.g. Figure 2C with Figure 2A), which is predicted to be beneficial for therapeutic applications and allows higher flexibility in dosing the multispecific antibody or a pharmaceutical composition comprising the multispecific antibody. If the affinities of the PDL1-BD and the CD137-BD to their respective binding-targets are similar or the affinity (K_{D}) of the PDL+-BD to PDL1 is only marginally smaller (better) than the affinity (K_{D}) CD137-BD to CD137, the effective concentrations for achieving maximal PDL1-blockage and maximal CD137 mediated signaling are not overlapping (Figure 2A and 2B), which is believed to decrease the overall efficacy and the therapeutic applicability of such antibodies. Thus, in one embodiment, the multispecific antibody used in the present invention comprises at least one CD137-BD and at least one PDL1-BD, wherein said CD137-BD binds to human CD137 with a monovalent dissociation constant (K_{D}) of at least 5 times, preferably at least 10 times, e.g., at least 20, at least 30, at least 50, at least 100 times higher relative to a monovalent dissociation constant (K_{D}) of said PDL1-BD for binding to human PDL1, in particular, wherein said CD137-BD binds to human CD137 with a monovalent dissociation constant (K_{D}) that is 20 to 1000 times, preferably 30 to 800 times, in particular 50 - 500 times higher than the monovalent dissociation constant (K_{D}) of said PDL1-BD for binding to human PDL1, when measured by SPR.

Importantly, the multispecific antibody MA1 used in the present invention that is monovalent for CD137 specificity is not capable of inducing CD137 signaling systemically due to a lack of CD137 activation in the absence of clustering, which is caused by binding of PDL1-BD to its antigen.

In one embodiment, the multispecific antibody MA1 consists of one CD137-BD and one PDL1-BD.

The term "CD137" refers in particular to human CD137 with UniProt ID number Q07011, reproduced herein as SEQ ID NO: 89. Suitably, the CD137-BD of the present invention targets CD137, in particular human CD137 as shown in UniProt ID number Q07011, reproduced herein as SEQ ID NO: 89. Suitably, the multispecific antibody used in the invention comprises a CD137-BD that targets human and cynomolgus (*Macaca fascicularis*) CD137. Preferably, the multispecific antibody used in the invention comprises a CD137-BD that does not block CD137/CD137L interaction.

In one embodiment, the CD137-BD does not cross-compete for binding with urelumab. Thus, the CD137-BD binds to a different epitope than urelumab. Urelumab, also referred to as BMS-663513, is a fully humanized IgG4 mAb from Bristol-Myers Squibb, and is described in WO 2004/010947, US 6,887,673 and US 7,214,493. In another embodiment, the CD137-BD used in the invention cross-competes for binding with urelumab.

In one embodiment, the CD137-BD does not cross-compete for binding with utomilumab. Thus, the CD137-BD binds to a different epitope than utomilumab. Utomilumab, also referred to as PF-05082566, is a fully human IgG2 mAb from Pfizer, and is described in WO 2012/032433 and US 8,821,867. In another embodiment, the CD137-BD used in the invention cross-competes for binding with utomilumab.

In a further embodiment, the CD137-BD does not cross-compete for binding neither with urelumab nor with utomilumab. Thus, the CD137-BD binds to a different epitope than urelumab and utomilumab.

The terms "compete" or "cross-compete" and related terms are used interchangeably herein to mean the ability of an antibody or other binding agent to interfere with the binding of other antibodies or binding agents to CD137 in a standard competitive binding assay.

The term "same epitope", as used herein, refers to individual protein determinants on the same protein capable of specific binding to an antibody, where these individual protein determinants are identical, i.e. consist of identical chemically active surface groupings of molecules such as amino acids or sugar side chains having identical three-dimensional structural characteristics, as well as identical charge characteristics. The term "different epitope", as used herein in connection with a specific protein target, refers to individual protein determinants on the same protein capable of specific binding to an antibody, where these individual protein determinants are not identical, i.e. consist of non-identical chemically active surface groupings of molecules such as amino acids or sugar side chains having different three-dimensional structural characteristics, as well as different charge characteristics. These different epitopes can be overlapping or non-overlapping.

Suitably, the CD137-BD of MA1 is further characterized by one or more of the following parameters:
f. when being in scFv format, has a melting temperature (Tm), determined by differential scanning fluorimetry, of at least 50°C, preferably of at least 55°C, more preferably at least 60°C, in particular wherein said antibody or antigen binding fragment thereof is formulated in phosphate citrate buffer at pH 6.4, 150 mM NaCl, in particular wherein said antibody is formulated in 50 mM phosphate citrate buffer with 150 mM NaCl at pH 6.4;
g. when being in scFv format, has a loss in monomer content, after storage for at least two weeks, particularly for at least four weeks, at 4°C, of less than 7 %, e.g. less than 6 %, less than 5 %, less than 4 %, less than 3 %, less than 2 %, preferably less than 1 %, when said antibody is at a starting concentration of 10 mg/ml, and in particular wherein said antibody is formulated in 50 mM phosphate citrate buffer with 150 mM NaCl at pH 6.4;
h. when being in scFv format, has a loss in monomer content, after storage for at least two weeks, particularly for at least four weeks, at 40°C, of less than 5 %, e.g. less than 4 %, less than 3 %, less than 2 %, preferably less than 1 %, when said antibody is at a starting concentration of 10 mg/ml, and in particular wherein said antibody is formulated in 50 mM phosphate citrate buffer with 150 mM NaCl at pH 6.4.

DSF is described earlier (Egan, et al., MAbs, 9(1) (2017), 68-84; Niesen, et al., Nature Protocols, 2(9) (2007) 2212-2221). The midpoint of transition for the thermal unfolding of the scFv constructs is determined by Differential Scanning Fluorimetry using the fluorescence dye SYPRO^{®} Orange (see Wong & Raleigh, Protein Science 25 (2016) 1834-1840). Samples in phosphate-citrate buffer at pH 6.4 are prepared at a final protein concentration of 50 µg/ml and containing a final concentration of 5x SYPRO^{®} Orange in a total volume of 100 µl. Twenty-five microliters of prepared samples are added in triplicate to white-walled AB gene PCR plates. The assay is performed in a qPCR machine used as a thermal cycler, and the fluorescence emission is detected using the software's custom dye calibration routine. The PCR plate containing the test samples is subjected to a temperature ramp from 25°C to 96°C in increments of 1°C with 30 s pauses after each temperature increment. The total assay time is about 2 h. The Tm is calculated by the software GraphPad Prism using a mathematical second derivative method to calculate the inflection point of the curve. The reported Tm is an average of three measurements.

The loss in monomer content is as determined by area under the curve calculation of SE-HPLC chromatograms. SE-HPLC is a separation technique based on a solid stationary phase and a liquid mobile phase as outlined by the USP chapter 621. This method separates molecules based on their size and shape utilizing a hydrophobic stationary phase and aqueous mobile phase. The separation of molecules is occurring between the void volume (V₀) and the total permeation volume (V_{T}) of a specific column. Measurements by SE-HPLC are performed on a Chromaster HPLC system (Hitachi High-Technologies Corporation) equipped with automated sample injection and a UV detector set to the detection wavelength of 280 nm. The equipment is controlled by the software EZChrom Elite (Agilent Technologies, Version 3.3.2 SP2) which also supports analysis of resulting chromatograms. Protein samples are cleared by centrifugation and kept at a temperature of 4-6°C in the autosampler prior to injection. For the analysis of scFv samples the column Shodex KW403-4F (Showa Denko Inc., #F6989202) is employed with a standardized buffered saline mobile phase (50 mM sodium-phosphate pH 6.5, 300 mM sodium chloride) at the recommended flow rate of 0.35 ml/min. The target sample load per injection was 5 µg. Samples are detected by an UV detector at a wavelength of 280 nm and the data recorded by a suitable software suite. The resulting chromatograms are analyzed in the range of V₀ to V_{T} thereby excluding matrix associated peaks with >10 min elution time.

The multispecific antibody MA1 used in the present invention comprises one PDL1 binding domain (PDL1-BD).

The term "PDL1" refers in particular to human PDL1 with UniProt ID number Q9NZQ7, reproduced herein as SEQ ID NO: 90. Suitably, the PDL1-BD used in the present invention targets PDL1, in particular human PDL1 as shown in UniProt ID number Q9NZQ7, reproduced herein as SEQ ID NO: 90. Suitably, the antibodies MA1 used in the present invention comprise a PDL1-BD that targets human and cynomolgus (*Macaca fascicularis*) PDL1, and preferably does not cross-react with *Mus musculus* PDL1. The PDL1-BD used in the present invention specifically binds to human PDL1 protein.

The PDL1-BD used in the present invention is a PDL1 inhibitor. The term "blocker" or "inhibitor" or "antagonist" refers to an antibody or binding domain thereof that inhibits or reduces a biological activity of the antigen it binds to. The PDL1-BD used in the present invention targets and decreases or inhibits the binding ability of PDL1 to its binding partners, thereby interfering with the PDL1 function. In particular, the PDL1-BD used in the present invention blocks the interaction of PDL1 with its receptor, specifically with PD-1. In particular, the PDL1-BD used in the present invention blocks the interaction of PDL1 with its receptor or receptors, specifically with PD-1 and/or B7-1.

Suitable the PDL1-BDs of MA1 used in the invention are binding domains provided in the present disclosure. The PDL1-BDs used in the invention include, but are not limited to, the humanized PDL1-BDs whose sequences are listed in Table 2.

Suitably, the PDL1-BD used in the present invention is characterized by one or more of the following parameters:
a. binds to human PDL1 with a monovalent dissociation constant (K_{D}) of less than 10 nM, particularly with a monovalent K_{D} of 0.05 pM to 10 nM, more particularly of 0.1 pM to 5 nM, more particularly of 0.2 pM to 1 nM, more particularly 0.5 pM to 500 pM, more particularly of 1 pM to 200 pM, more particularly of 1 pM to 100 pM, as measured by SPR;
b. binds to human PDL1 with a K_{off} rate of 5x10⁻³ s⁻¹ or less, more particularly of 5x10⁻³ s⁻¹ to 10⁻⁷ s⁻¹, more particularly of 10⁻³ s⁻¹ to 5x10⁻⁶ s⁻¹, more particularly of 10-³ s⁻¹ to 10⁻⁶ s⁻¹, as measured by SPR;
c. binds to human PDL1 with a Kₒₙ rate of at least 10⁴ M⁻¹s⁻¹ or greater, more particularly of 10⁴ M⁻¹s⁻¹ to 10⁸ M⁻¹s⁻¹, more particularly of 10⁵ M⁻¹s⁻¹ to 5x10⁷ M⁻¹s⁻¹, more particularly of 5x10⁵ M⁻¹s⁻¹ to 10⁷ M⁻¹s⁻¹, as measured by SPR;
d. is cross-reactive with *Macaca fascicularis* (cynomolgus) PDL1;
e. is non-cross reactive to *Mus musculus* PDL1.

Suitably, the PDL1-BD used in the present invention is further characterized by one or more of the following parameters:
f. when being in scFv format, has a melting temperature (Tm), determined by differential scanning fluorimetry, of at least 55°C, e.g. at least 60°C, preferably at least 65°C, more preferably at least 70°C, in particular wherein said antibody or antigen-binding fragment thereof is formulated in phosphate citrate buffer at pH 6.4, 150 mM NaCl, in particular wherein said antibody is formulated in 50 mM phosphate citrate buffer with 150 mM NaCl at pH 6.4;
g. when being in scFv format, has a loss in monomer content, after five consecutive freeze-thaw cycles, of less than 5 %, preferably less than 3 %, more preferably less than 1 %, when said antibody is at a starting concentration of 10 mg/ml, in particular wherein said antibody is formulated in 50 mM phosphate citrate buffer with 150 mM NaCl at pH 6.4; and/or
h. when being in scFv format, has a loss in monomer content, after storage for at least two weeks, particularly for at least four weeks, at 4°C, of less than 15%, e.g. less than 12 %, less than 10 %, less than 7 %, less than 5 %, less than 4 %, less than 3 %, less than 2 %, preferably less than 1 %, when said antibody is at a starting concentration of 10 mg/ml, and in particular wherein said antibody is formulated in 50 mM phosphate citrate buffer with 150 mM NaCl at pH 6.4.

Suitably, the multispecific antibodies MA1 do not comprise an immunoglobulin Fc region polypeptide. Thus, the multispecific antibodies MA1 used in the invention typically have a compact multi-domain and low molecular weight antibody architecture. The term "Fc region" herein is used to define a C-terminal region of an immunoglobulin heavy chain, including native-sequence Fc regions and variant Fc regions. Native-sequence Fc regions include human IgG1, IgG2 (IgG2A, IgG2B), IgG3 and IgG4. "Fc receptor" or "FcR" describes a receptor that binds to the Fc region of an antibody. Particularly, the FcR is a native sequence human FcR, which binds an IgG antibody (a gamma receptor) and includes receptors of the FcγRI, FcyRII, and FcγRIII subclasses, including allelic variants and alternatively spliced forms of these receptors, FcγRII receptors including FcγRIIA (an "activating receptor") and FcγRI IB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain, (see M. Daeron, Annu. Rev. Immunol. 5:203-234 (1997). FcRs are reviewed in Ravetch and Kinet, Annu. Rev. Immunol. 9: 457-92 (1991); Capet et al, Immunomethods 4: 25-34 (1994); and de Haas et al, J. Lab. Clin. Med. 126: 330-41 (1995). Other FcRs, including those to be identified in the future, are encompassed by the term "FcR" herein. The term "Fc receptor" or "FcR" also includes the neonatal receptor, FcRn, which is responsible for the transfer of maternal IgGs to the fetus. Guyer et al., J. Immunol. 117: 587 (1976) and Kim et al., J. Immunol. 24: 249 (1994). Methods of measuring binding to FcRn are known (see, *e. g*., Ghetie and Ward, Immunol. Today 18: (12): 592-8 (1997); Ghetie et al., Nature Biotechnology 15 (7): 637-40 (1997); Hinton et al., J. Biol. Chem. TJI (8): 6213-6 (2004); WO 2004/92219 (Hinton et al). Binding to FcRn *in vivo* and serum half-life of human FcRn high-affinity binding polypeptides can be assayed, e. g., in transgenic mice or transfected human cell lines expressing human FcRn, or in primates to which the polypeptides having a variant Fc region are administered. WO 2004/42072 (Presta) describes antibody variants which improved or diminished binding to FcRs. See also, e. g., Shields et al., J. Biol. Chem. 9(2): 6591-6604 (2001).

In order to increase the number of specificities/functionalities at the same or lower molecular weight, it is advantageous to use antibodies comprising antibody fragments, such as Fv, scFv, Fab, Fab' and F(ab')2 fragments and other antibody fragments. These smaller molecules retain the antigen-binding activity of the whole antibody and can also exhibit improved tissue penetration and pharmacokinetic properties in comparison to the whole immunoglobulin molecules. Whilst such fragments appear to exhibit a number of advantages over whole immunoglobulins, they also suffer from an increased rate of clearance from serum since they lack the Fc domain that imparts a long half-life *in vivo* (Medasan et al., 1997, J. Immunol. 158:2211-2217). Molecules with lower molecular weights penetrate more efficiently into target tissues (*e. g*. solid cancers) and thus hold the promise for improved efficacy at the same or lower dose.

The inventors have found that an addition of human serum albumin binding domain (hSA-BD) to the multispecific antibodies MA1 applied in the invention does not interfere with the ability of the other binding domains to bind to their respective targets. This finding is insofar surprising as it cannot *a priori* be expected that all three binding domains remain functional without sterically or otherwise inhibiting each other in a complex multi-target, multi-cell *in vivo* situation.

Suitably, the multispecific antibody MA1 applied in the pharmaceutical composition or the kit of the invention may comprise a further binding domain having a specificity to human serum albumin.

In one embodiment, the multispecific antibody MA1 comprises: (i) one CD137-BD; (ii) one PDL1-BD; and (iii) one hSA-BD.

The term "hSA" refers in particular to human serum albumin with UniProt ID number P02768. Human Serum Albumin (hSA) is a 66.4 kDa abundant protein in human serum (50 % of total protein) composed of 585 amino acids (Sugio, Protein Eng, Vol. 12, 1999, 439-446). Multifunctional hSA can transport a number of metabolites such as fatty acids, metal ions, bilirubin and some drugs (Fanali, Molecular Aspects of Medicine, Vol. 33, 2012, 209-290). hSA concentration in serum is around 3.5 - 5 g/dl. Albumin-binding antibodies and fragments thereof may be used for example, for extending the *in vivo* serum half-life of drugs or proteins conjugated thereto.

In some embodiments, the hSA-BD is derived from a monoclonal antibody or antibody fragment.

Suitable hSA-BDs for the multispecific antibodies MA1 are binding domains provided in the present disclosure. The hSA-BDs of the multispecific antibodies MA1 include, but are not limited to, the humanized hSA-binding domains whose sequences are listed in Table 3.

In particular, the hSA-BDs of the multispecific antibodies MA1 specifically bind to human serum albumin.

Another suitable hSA-BD for use in the multispecific antibody MA1 comprises or is derived from a binding domain selected from the group consisting of: (i) polypeptides that bind serum albumin (see, for example, Smith et al., 2001, Bioconjugate Chem. 12:750-756; EP 0 486 525; US 6,267,964; WO 2004/001064; WO 2002/076489; and WO 2001/45746); (ii) anti-serum albumin binding single variable domains described in Holt et al., Protein Engineering, Design & Selection, vol 21, 5, pp283-288, WO 2004/003019, WO 2008/096158, WO 2005/118642, WO 2006/0591056 and WO 2011/006915; (iii) anti-serum albumin antibodies described in WO 2009/040562, WO 2010/035012 and WO 2011/086091.

Suitably, the multispecific antibodies MA2 applied in the pharmaceutical compositions or the kit of the invention are monovalent for CD3 specificity.

Suitable CD3-BDs for use in the multispecific antibodies MA2 are binding domains provided in the present disclosure. The CD3-BDs of the invention include, but are not limited to, the humanized CD3-binding domains whose sequences are listed in Table 6.

Suitably, the multispecific antibodies MA2 applied in the pharmaceutical compositions or the kit of the invention comprise at least one TAA-BD. The MA2, which have two or more TAA-BDs, can have specificity for the same TAA or for two different TAAs.

The term "tumor-associated antigen (TAA)" refers to an antigen that is expressed on the surface of a tumor cell. In particular embodiments, a TAA is an antigen that is preferentially expressed on a tumor cell when compared to non-tumor cells, particularly wherein expression of the TAA on a tumor cell is at least more than 5-fold, at least more than 10-fold, at least more than 20-fold, at least more than 50-fold, or at least more than 100-fold higher than on non-tumor cells from the same organism or patient. In particular, the TAA is taken from the group of: ADRB3, AFP, ALK, BCMA, beta human chorionic gonadotropin, CA-125 (MUC16), CAIX, CD123, CD133, CD135, CD135 (FLT3), CD138, CD171, CD19, CD20, CD22, CD24, CD276, CD33, CD33, CD38, CD44v6, CD79b, CD97, CDH3 (cadherin 3), CEA, CEACAM6, CLDN6, CLEC12A (CLL1), CSPG4, CYP1B1, EGFR, EGFRvIII, EPCAM, EPHA2, Ephrin B2, ERBBs (e. g. ERBB2), FAP, FGFR1, folate receptor alpha, folate receptor beta, Fos-related antigen, GA733, GD2, GD3, GFRalpha4, globoH, GPC3, GPR20, GPRC5D, HAVCR1, Her2/neu (HER2), HLA-A2, HMWMAA, HPV E6 or E7, human telomerase reverse transcriptase, IL-11Ra, IL-13Ra2, intestinal carboxyl esterase, KIT, Legumain, LewisY, LMP2, Ly6k, MAD-CT-1, MAD-CT-2, ML-IAP, MN-CA IX, MSLN, MUC1, mut hsp 70-2, NA-17, NCAM, neutrophil elastase, NY-BR-1, NY-ESO-1, o-acetyl-GD2, OR51E2, PANX3, PDGFR-beta, PLAC1, Polysialic acid, PSCA, PSMA, RAGE1, ROR1, sLe, sperm protein 17, SSEA-4, SSTR2, sTn antigen, sTn-O-Glycopeptides, TAG72, TARP, TEM1/CD248, TEM7R, thyroglobulin, Tn antigen, Tn-O-Glycopeptides, TPBG (5T4), TRP-2, TSHR, UPK2 and VEGFR2.

In particular embodiments, the TAA is selected from the group consisting of CD138, CD79b, TPBG (5T4), HER2, MSLN, MUC1, CA-125 (MUC16), PSMA, BCMA, CD19, EpCAM, CLEC12A (CLL1), CD20, CD22, CEA, CD33, EGFR, GPC3, CD123, CD38, CD33, CD276, CDH3 (cadherin 3), FGFR1, SSTR2, CD133, EPHA2, HLA-A2, IL13RA2, ROR1, CEACAM6, CD135, GD-2, GA733, CD135 (FLT3), CSPG4 and TAG-72, in particular selected from the group consisting of CD138, CD79b, CD123, HER2, MSLN, PSMA, BCMA, CD19, CD20, CEA, CD38, CD33, CLEC12a, and ROR1. In a special embodiment, the TAA is selected from the group consisting of HER2, MSLN and ROR1.

In one embodiment, the multispecific antibodies MA2 applied in the pharmaceutical compositions or the kit comprises one TAA-BD, i.e. is monovalent for TAA specificity.

In particular embodiments, the multispecific antibodies MA2 applied in the pharmaceutical compositions or the kit comprises one TAA-BD that binds to clean TAAs, i.e. TAAs that are almost exclusively expressed on cancer cells, such as oncofetal tumor antigens, such as ROR1.

In the context of the present invention, the term "clean TAA" refers to TAAs which are not expressed on the surface of healthy cells or which are expressed on the surface of healthy cells in amounts corresponding to less than 10 %, particularly less than 5 %, particularly less than 1 %, particularly less than 0.5 %, of the respective TAA density on the surface of the cancer cells.

In other particular embodiments, the multispecific antibodies MA2 applied in the pharmaceutical compositions or the kit comprises one TAA-BD that binds to non-clean TAAs, i.e. TAAs that are also expressed in significant amounts on the surface of healthy cells, such as Mesothelin (MSLN), EGFR, EpCAM and HER2.

In the context of the present invention, the term "clean TAA" refers to TAAs which are expressed on the surface of healthy cells in amounts corresponding to 10 - 90 %, particularly 10 - 80 %, particularly 10 - 75 %, of the respective TAA density on the surface of the cancer cells.

In these embodiment, said single TAA-BD binds to said TAA with a monovalent dissociation constant (K_{D}) of less than 50 nM, particularly less than 20 nM, particularly less than 10 nM, particularly less than 5 nM, particularly of 0.01 to 2 nM, particularly of 0.02 to 1 nM, particularly of 0.03 to 0.5 nM as measured by SPR, particularly wherein said TAA-BD is an scFv.

Specific examples of MA2 comprising one TAA-BD and one CD3-BD are PRO1872 (MSLN_{lowKDX}CD3xhSA), PRO1766 (HER2xCD3xhSA) and PRO1767 (HER2xCD3xhSA), whose sequences are listed in Table 9.

In another embodiment, the multispecific antibodies MA2 applied in the pharmaceutical compositions or the kit comprise two or three TAA-BDs, particularly two TAA-BDs, which have specificity for the same TAA.

In yet another embodiment, the multispecific antibodies MA2 applied in the pharmaceutical compositions or the kit comprise two, three or four TAA-BDs, particularly two TAA-BDs, which have specificity for two different TAAs.

In these embodiments, the TAA-BDs, independently of each other, bind clean and non-clean TAAs.

In particular embodiments, the multispecific antibodies MA2 applied in the pharmaceutical compositions or the kit comprise two TAA-BDs, which have specificity for the same TAA, and wherein the TAA is selected from non-clean TAAs.

In particular embodiments, the multispecific antibodies MA2 applied in the pharmaceutical compositions or the kit comprise two TAA-BDs, which have specificity for the same TAA, and wherein the TAA is selected from tumor cell associated antigens, whose extracellular part also occurs in soluble form in the serum of a patient.

In the context of the present invention, the term "occurs in soluble form in the serum of a patient" means that the concentration of the particular protein of interest, i.e. the extracellular part of the TAA, in the serum is at least 1 ng/ml.

The two TAA-BDs of the multispecific antibodies MA2, which have specificity for the same TAA, either bind the same or different epitopes on the extracellular part of said TAA target molecules. Preferably, the two TAA-BDs of the multispecific antibodies MA2, which have specificity for the same TAA, bind the same epitopes on the TAA target molecules.

In these embodiments, the two TAA-BDs of the multispecific antibodies MA2, which have specificity for the same TAA, bind to said TAA with a monovalent dissociation constant (K_{D}) in the range of 0.1 to 50 nM, particularly of 0.2 to 30 nM, particularly of 0.3 to 20 nM, particularly of 0.4 to 10 nM, as measured by SPR, particularly wherein said TAA-BD is an scFv.

These multispecific antibodies MA2, which comprise two low-affinity TAA-BDs, allows to more safely address non-clean TAAs, such as Mesothelin (MSLN), EGFR, EpCAM and HER2, due to avidity effects, which cause a significant increase in the potency of these multispecific antibodies MA2 towards high TAA expressing cells over healthy low TAA expressing cells. Furthermore, the binding of these multispecific antibodies MA2 to TAAs, whose extracellular part is shed into the serum of cancer patients (soluble TAA), which typically causes a significant reduction of the effective concentration of TAA binding drugs, is not much affected by the presence of significant amounts of such soluble TAAs. Examples of such TAAs are MSLN, PSMA and MUC1.

In a specific embodiment, the two TAA-BDs of the multispecific antibodies MA2 are MSLN-BDs which specifically bind to mesothelin (MSLN), particularly human mesothelin. The two MSLN-BDs bind to any region of the extracellular part of MSLN, e.g. to Region I, Region II and/or Region III of MSLN. Preferably, the two MSLN-BDs of the multispecific antibodies MA2 of this specific embodiment bind to Region I and/or Region II, in particular to Region I of MSLN. Region I is the part of MSLN that is most distal from the cell surface, where MSLN is attached to.

The two MSLN-BDs of the multispecific antibodies MA2 of this specific embodiment either bind the same or different epitopes on the MSLN target molecules. Preferably, the two MSLN-BDs bind the same epitopes on the MSLN target molecules.

Suitable MSLN-BDs for use in the multispecific antibodies MA2 are binding domains provided in the present disclosure. The MSLN-BDs applied in the invention include, but are not limited to, the humanized MSLN-binding domains whose sequences are listed in Table 7.

Suitably, the multispecific antibody MA2 applied in the pharmaceutical composition or the kit of the invention may comprise a further binding domain having specificity to human serum albumin.

Suitable hSA-BDs for the multispecific antibodies MA2 are binding domains provided in the present disclosure, as defined above. The hSA-BDs of the multispecific antibodies MA2 include, but are not limited to, the humanized hSA-binding domains whose sequences are listed in Table 3.

Specific examples of MA2 comprising two low affinity MSLN-BDs and one CD3-BD (e.g. biMSLN_{high KDX}CD3xhSA) are PRO2000 and RPO2100, whose sequences are listed in Table 9. Further details regarding their manufacturing and functional properties are disclosed in the unpublished patent application EP 20177337.1, which is herewith incorporated by reference.

The inventors of the present invention have previously found that the tri-specific molecule PRO2000 (biMSLN_{high KDX}CD3xhSA) is capable of killing target cells, which have an approximately 7-fold higher MSLN expression level than healthy MeT-5A cells (ATCC CRL-9444), as determined by flow cytometry, with high efficiency and with an EC₅₀ that is 2000-fold lower than the EC₅₀ for killing said MeT-5A cells, as determined in a T-cell driven cytotoxicity assay against said target cells and said MeT-5A cells (Figure 3). Thus, although PRO2000 exhibits a very high killing potency for high MSLN expressing target cells, the killing potency of PRO2000 towards healthy cells is much lower, indicating a potentially large therapeutic window for treatments using PRO2000. In contrast thereto, the potencies of a tri-specific reference molecule PRO1872 (MSLN_{lowKD}xCD3xhSA), which comprises one MSLN-BD having a more than 5-fold better binding affinity (K_{D}) than the MSLN-BDs of PRO2000, for killing said high MSLN expressing target cells and said healthy Met-5A cells does not differ significantly. This finding indicates that the therapeutic window for the use of these specific types of multispecific antibodies MA2 is increased. In addition, the inventors of the present invention have previously found that the EC₅₀ of the tri-specific molecules PRO2000 and PRO2100 (biMSLN_{high KDX}CD3xhSA) for killing target cells, which have an approximately 7-fold higher MSLN expression level than said healthy MeT-5A cells, as determined by flow cytometry, does not increase by more than 5-fold in the presence of 100 ng/ml soluble mesothelin (sMSLN), and in the case of PRO2000, by not more than 20-fold in the presence of 500 ng/ml soluble mesothelin (sMSLN), as determined in a T-cell driven cytotoxicity assay against said target cells. On the other hand, the EC₅₀ of the tri-specific reference molecule PRO1872 (MSLN_{low KDX}CD3xhSA) for killing said target cells increased by almost 10-fold in the presence of 100 ng/ml sMSLN and by more than 100-fold in the presence of 500 ng/ml sMSLN (Figure 4). Thus, the high killing potency of these specific types of multispecific antibodies MA2 for high MSLN expressing target cells is only marginally affected by high concentrations of sMSLN.

In one particular embodiment, the multispecific antibody MA2 applied in the pharmaceutical composition or the kit of the invention does not comprise an immunoglobulin Fc region polypeptide.

In another particular embodiment, the multispecific antibody MA2 applied in the pharmaceutical composition or the kit of the invention does comprise an immunoglobulin Fc region polypeptide.

Other variable domains used in the invention include amino acid sequences that have been mutated, yet have at least 80, 85, 90, 91, 92, 93, 94, 95, 96, 97, 98 or 99 percent identity in the CDR regions with the CDR regions depicted in the sequences described in Tables 1 to 3 and 6 to 8. Other variable domains used the invention include mutant amino acid sequences wherein no more than 1, 2, 3, 4 or 5 amino acids have been mutated in the CDR regions when compared with the CDR regions depicted in the sequence described in Tables 1 to 3 and 6 to 8.

Suitably, the VH domains of the binding domains used in the invention belong to a VH3 or VH4 family. In one embodiment, a binding domain used in the invention comprises a VH domain belonging to the VH3 family. In the context of the present invention, the term "belonging to VHx family (or VLx family)" means that the framework sequences FR1 to FR3 show the highest degree of homology to said VHx family (or VLx, respectively). Examples of VH and VL families are given in Knappik et al., J. Mol. Biol. 296 (2000) 57-86, or in WO 2019/057787. A specific example of a VH domain belonging to VH3 family is represented by SEQ ID NO: 91, and a specific example of a VH domain belonging to VH4 family is represented by SEQ ID NO: 92. In particular, framework regions FR1 to FR3 taken from SEQ ID NO: 91 belong to VH3 family (Table 4, regions marked in non-bold). Suitably, a VH belonging to VH3 family, as used herein, is a VH comprising FR1 to FR3 having at least 85 %, particularly at least 90 %, more particularly at least 95 % sequence identity to FR1 to FR3 of SEQ ID NO: 91. Alternative examples of VH3 and VH4 sequences, and examples of other VHx sequences, may be found in Knappik et al., J. Mol. Biol. 296 (2000) 57-86 or in WO 2019/057787. Suitably, the PDL1-BD, the CD137-BD, the hSA-BD, the CD3-BD, the MSLN-BD and the HER2-BD comprised in MA1 and MA2 that are applied in the pharmaceutical composition or the kit of the invention comprise: Vκ frameworks FR1, FR2 and FR3, particularly Vκ1 or Vκ3 frameworks, particularly Vκ1 frameworks FR1 to 3, and a framework FR4, which is selected from a Vκ FR4, and a Vλ FR4, particularly a Vλ FR4. Suitable Vκ1 frameworks FR1 to FR3 as well as exemplary Vλ FR4 are set forth in SEQ ID NO: 93 (Table 4, FR regions are marked in non-bold). Alternative examples of Vκ1 sequences, and examples of Vκ2, Vκ3 or Vκ4 sequences, may be found in Knappik et al., J. Mol. Biol. 296 (2000) 57-86. Suitable Vκ1 frameworks FR1 to 3 comprise the amino acid sequences having at least 70, 80, 90 percent identity to amino acid sequences corresponding to FR1 to FR3 and taken from SEQ ID NO: 93 (Table 4, FR regions are marked in non-bold). Suitable Vλ FR4 are as set forth in SEQ ID NO: 94 to SEQ ID NO: 100 and in SEQ ID NO: 101 comprising a single cysteine residue, particular in a case where a second single cysteine is present in the corresponding VH chain, particularly in position 51 (AHo numbering) of VH, for the formation of an interdomain disulfide bond. In one embodiment, the VL domains used in the present invention comprises Vλ FR4 comprising the amino acid sequence having at least 70, 80, 90 percent identity to an amino acid sequence selected from any of SEQ ID NO: 94 to SEQ ID NO: 101, particularly to SEQ ID NO: 94 or 101.

The binding domains used in the invention comprise a VH domain listed in Tables 1 to 3 and 6 to 8. Suitably, a binding domain used the invention comprises a VH amino acid sequence listed in one of Tables 1 to 3 and 6 to 8, wherein no more than 20 amino acids in a framework sequence (for example, a sequence which is not a CDR) have been mutated (wherein a mutation is, as various non-limiting examples, an addition, substitution or deletion). Suitably, a binding domain used in the present invention comprises a VH amino acid sequence listed in one of Tables 1 to 3 and 6 to 8, wherein no more than 15 amino acids, particularly no more than 10 amino acids, particularly no more than 5 amino acids in a framework sequence (for example, a sequence which is not a CDR) have been mutated (wherein a mutation is, as various non-limiting examples, an addition, substitution or deletion). Other binding domains used in the invention include amino acids that have been mutated, yet have at least 80, 85, 90, 91, 92, 93, 94, 95, 96, 97, 98 or 99 percent identity in the VH regions with the VH regions depicted in the corresponding sequences described in one of Tables 1 to 3 and 6 to 8, including VH domains comprising at least positions 5 to 140 (AHo numbering), particularly at least positions 3 to 145 of one of the sequences shown in Tables 1 to 3 and 6 to 8.

In particular, a binding domain used in the invention comprises a VL domain listed in one of Tables 1 to 3 and 6 to 8. Suitably, a binding domain used in the invention comprises a VL amino acid sequence listed in one of Tables 1 to 3 and 6 to 8, wherein no more than about 10 amino acids in a framework sequence (for example, a sequence which is not a CDR) have been mutated (wherein a mutation is, as various non-limiting examples, an addition, substitution or deletion). Suitably, a binding domain used in the invention comprises a VL amino acid sequence listed in one of Tables 1 to 3 and 6 to 8, wherein no more than about 20 amino acids in a framework sequence (for example, a sequence which is not a CDR) have been mutated (wherein a mutation is, as various non-limiting examples, an addition, substitution or deletion). Other binding domains used in the invention include amino acids that have been mutated, yet have at least 80, 85, 90, 91, 92, 93, 94, 95, 96, 97, 98 or 99 percent identity in the VL regions with a VL region depicted in the sequences described in Tables 1 to 3 and 6 to 8, including VL domains comprising at least positions 5 to 140 (AHo numbering), particularly at least positions 3 to 145 of one of the sequences shown in Tables 1 to 3 and 6 to 8.

In the context of the present invention, the term "binding domain used in the present invention" relates to a binding domain as such, *i. e*. independent of a multispecific context, and, in particular, to a binding domain comprised in a multispecific construct, *e. g*. one of the binding domains comprised in a bispecific, trispecific or tetraspecific construct.

Suitably, the binding domains of the multispecific antibodies MA1 and MA2 are selected from the group consisting of: a Fab, an Fv, an scFv, dsFv, a scAb, and STAB.

Suitably, the binding domains of the multispecific antibodies MA1 and MA2 are operably linked. The binding domains of the multispecific antibodies MA1 and MA2 are capable of binding to their respective antigens or receptors simultaneously. The term "simultaneously", as used in this connection and in connection with the MA1, refers to the simultaneous binding of the CD137-BDs and the PDL1-BD. Similarly, the term "simultaneously", as used in connection with the MA2, refers to the simultaneous binding of at least one of the TAA-BDs and the CD3-BD. In specific cases, e.g. in cases where the applied MA2 has two or more TAA-BDs that are specific for the same TAA and the target cells have a high density of said TAA on the cell surface, it might also be possible that three binding domains, *i. e*. two TAA-BDs and the CD3-BD, bind simultaneously.

The multispecific antibodies MA1 comprises one CD137-BD and one PDL1-BD, wherein said CD137-BDs, and said PDL1-BD are operably linked to each other.

The multispecific antibodies MA1 comprises at least one TAA-BD, and one CD3-BD, wherein said at least one TAA-BD, and said CD3-BD are operably linked to each other.

The term "operably linked", as used herein, indicates that two molecules (*e. g*., polypeptides, domains, binding domains) are attached in a way that each molecule retains functional activity. Two molecules can be "operably linked" whether they are attached directly or indirectly (*e. g*., via a linker, via a moiety, via a linker to a moiety). The term "linker" refers to a peptide or other moiety that is optionally located between binding domains or antibody fragments used in the invention. A number of strategies may be used to covalently link molecules together. These include, but are not limited to, polypeptide linkages between N- and C-termini of proteins or protein domains, linkage via disulfide bonds, and linkage via chemical cross-linking reagents. In one aspect of this embodiment, the linker is a peptide bond, generated by recombinant techniques or peptide synthesis. Choosing a suitable linker for a specific case where two polypeptide chains are to be connected depends on various parameters, including but not limited to the nature of the two polypeptide chains (*e. g*., whether they naturally oligomerize), the distance between the N- and the C-termini to be connected if known, and/or the stability of the linker towards proteolysis and oxidation. Furthermore, the linker may contain amino acid residues that provide flexibility.

In the context of the present invention, the term "polypeptide linker" refers to a linker consisting of a chain of amino acid residues linked by peptide bonds that is connecting two domains, each being attached to one end of the linker. The polypeptide linker should have a length that is adequate to link two molecules in such a way that they assume the correct conformation relative to one another so that they retain the desired activity. In particular embodiments, the polypeptide linker has a continuous chain of between 2 and 30 amino acid residues (*e*. *g.,* 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues). In addition, the amino acid residues selected for inclusion in the polypeptide linker should exhibit properties that do not interfere significantly with the activity of the polypeptide. Thus, the linker peptide on the whole should not exhibit a charge that would be inconsistent with the activity of the polypeptide, or interfere with internal folding, or form bonds or other interactions with amino acid residues in one or more of the monomers that would seriously impede the binding of receptor monomer domains. In particular embodiments, the polypeptide linker is non-structured polypeptide. Useful linkers include glycine-serine, or GS linkers. By "Gly-Ser" or "GS" linkers is meant a polymer of glycines and serines in series (including, for example, (Gly-Ser)ₙ, (GSGGS)ₙ (GGGGS)ₙ and (GGGS)ₙ, where n is an integer of at least one), glycine-alanine polymers, alanine-serine polymers, and other flexible linkers such as the tether for the shaker potassium channel, and a large variety of other flexible linkers, as will be appreciated by those in the art. Glycine-serine polymers are preferred since oligopeptides comprising these amino acids are relatively unstructured, and therefore may be able to serve as a neutral tether between components. Secondly, serine is hydrophilic and therefore able to solubilize what could be a globular glycine chain. Third, similar chains have been shown to be effective in joining subunits of recombinant proteins such as single-chain antibodies.

Suitably, the multispecific antibody MA1 is in a format selected from any suitable multispecific format known in the art, *i.e*. any formats that are at least bispecific and do not comprise immunoglobulin Fc region(s), including, by way of non-limiting example, a single-chain diabody (scDb); a bispecific T cell engager (BiTE; tandem di-scFv); a tandem tri-scFv; Fab-(scFv); scFab-dsscFv; tribody (Fab-(scFv)₂); Fab₂; Fab-Fv₂; diabody; triabody; scDb-scFv; a scDb, a tandem tri-scFv, a Fab-(scFv), a scFab-dsscFv, a Fab-(scFv)₂, a Fab₂, a Fab-Fv₂, a diabody or a scDb-scFv fused to the N- and/or the C-terminus of a heterodimerization domain other than heterodimeric Fc domains; and a MATCH (described in WO 2016/0202457; Egan T., et al., MABS 9 (2017) 68-84) and DuoBodies. Particularly suitable for use herein is a scDb, a scDb-scFv, a scMATCH3 and a MATCH3, especially a scDb-scFv, a scMATCH3 and a MATCH3.

The term "diabodies" refers to antibody fragments with two antigen-binding sites, which fragments comprise a VH connected to VL in the same polypeptide chain (VH-VL). By using a linker that is too short to allow pairing between the two domains on the same chain, the domains are forced to pair with the complementary domains of another chain to create two antigen-binding sites. Diabodies may be bivalent or bispecific. Diabodies are described more fully in, for example, EP 404 097, WO 93/01161, Hudson et al., Nat. Med. 9:129-134 (2003), and Holliger et al., Proc. Natl. Acad. Sci. USA 90: 6444-6448 (1993). Triabodies and tetrabodies are also described in Hudson et al., Nat. Med. 9:129-134 (2003).

The bispecific scDb, in particular the bispecific monomeric scDb, particularly comprises two variable heavy chain domains (VH) or fragments thereof and two variable light chain domains (VL) or fragments thereof connected by linkers L1, L2 and L3 in the order VHA-L1-VLB-L2-VHB-L3-VLA, VHA-L1-VHB-L2-VLB-L3-VLA, VLA-L1-VLB-L2-VHB-L3-VHA, VLA-L1-VHB-L2-VLB-L3-VHA, VHB-L1-VLA-L2-VHA-L3-VLB, VHB-L1-VHA-L2-VLA-L3-VLB, VLB-L1-VLA-L2-VHA-L3-VHB or VLB-L1-VHA-L2-VLA-L3-VHB, wherein the VLA and VHA domains jointly form the antigen-binding site for the first antigen, and VLB and VHB jointly form the antigen-binding site for the second antigen.

The linker L1 particularly is a peptide of 2-10 amino acids, more particularly 3-7 amino acids, and most particularly 5 amino acids, and linker L3 particularly is a peptide of 1-10 amino acids, more particularly 2-7 amino acids, and most particularly 5 amino acids. In particular embodiments, the linker L1 and/or L3 comprises one or two units of four (4) glycine amino acid residues and one (1) serine amino acid residue (GGGGS)ₙ, wherein n=1 or 2, particularly n=1.

The middle linker L2 particularly is a peptide of 10-40 amino acids, more particularly 15-30 amino acids, and most particularly 20-25 amino acids. In particular embodiments, said linker L2 comprises two or more units of four (4) glycine amino acid residues and one (1) serine amino acid residue (GGGGS)ₙ, wherein n= 2, 3, 4, 5, 6, 7 or 8, particularly n=4 or 5.

In one embodiment, the multispecific antibody MA1 is a scDb-scFv. The term "scDb-scFv" refers to an antibody format, wherein a single-chain Fv (scFv) fragment is fused by a flexible Gly-Ser linker to a single-chain diabody (scDb). In one embodiment, said flexible Gly-Ser linker is a peptide of 2-40 amino acids, *e. g*., 2-35, 2-30, 2-25, 2-20, 2-15, 2-10 amino acids, particularly 10 amino acids. In particular embodiments, said linker comprises one or more units of four (4) glycine amino acid residues and one (1) serine amino acid residue (GGGGS)ₙ, wherein n=1, 2, 3, 4, 5, 6, 7 or 8, particularly n=2.

In another embodiment, the multispecific antibody MA1 is in a scMATCH3 or a MATCH3 format described in WO 2016/0202457; Egan T., et al., MABS 9 (2017) 68-84.

In one embodiment, the multispecific antibodies MA1 applied in the pharmaceutical compositions or the kit of the invention do not comprise CH1 and/or CL regions.

Specific but non-limiting examples of multispecific antibodies MA1 that can suitably be applied in the pharmaceutical compositions or the kit of the invention are the scDb-based antibodies PRO885, PRO951, PRO1123, PRO1124, PRO1125, PRO1126, PRO1134, and the scDb-scFv-based antibodies PRO963, PRO966, PRO1057, PRO1058, PRO1175, PRO1186, PRO1430, PRO1479, PRO1482, PRO1431, PRO1432, PRO1473, PRO1476, PRO1480, PRO1481, PRO1480diS, PRO1599, RPO1600, RPO1601, whose sequences are listed in table 5. Particularly suitable as multispecific antibodies MA1 are PRO1480, PRO1481, PRO1480diS, and RPO1601.

These multispecific antibodies MA1, in particular PRO1480, PRO1481, PRO1480diS and PRO1601 exhibit very advantageous safety properties. For example, PRO1480, when tested in female cynomolgus monkeys, after single-dose administration of PRO1480 up to 20 mg/kg, there was:
- no elevation of liver enzymes (ALT/AST/GGT/CK), which are the key clinical pathology indicators of hepatotoxicity caused by urelumab;
- no neutropenia or thrombocytopenia, which has been observed upon application of urelumab in the clinic;
- no atypical liver weights when compared to avelumab;
- no significant expansion of systemic central and effector memory T cells (less than reported values for well tolerated utomilumab), confirming specific activity on 4-1BB exclusively in the TME;
- a prolonged serum half-life of 5 - 5.5 days, corresponding to approximately 2 weeks in humans.

Furthermore, in a second 4-week repeated dose toxicology study with PRO1480 in cynomolgus monkeys, there was:
- no treatment related findings in alterations of organ weights, macroscopic observations and microscopic histophathological evaluation of tissue sections;
- no signs of toxicity and in particular, no elevations of transaminases or liver inflammation, markers for hepatotoxicity;
- no test-article related effects in clinical chemistry, hematology and urinalysis;
- no release of the cytokines GM-CSF, Interleukin 18, IL1B, IFNg, IL-2, IL-4, IL-5 and GM-CSF following the pre-dose, the first dose and the fourth dose of PRO1480;
- only moderate induction of the cytokines IL-6, MCP-1 and IL-10 in some of low- and mid-dose animals following dosing of PRO1480, which is consistent with ADA response.

In particular, the intra-venus infusion of PRO1480 was generally well tolerated and not associated with any signs of toxicity in cynomolgus monkeys up to 140 mg/kg/dose. Such an excellent toxicological profile has not been seen for any corresponding anti-CD137xPDL1 antibody therapeutics of the prior art, due to the presence of the Fc domain.

Suitably, the multispecific antibodies MA2 are in a format selected from any suitable multispecific, *e. g.* at least bispecific, format known in the art, including, by way of non-limiting example, a tandem scDb (Tandab); a bispecific T cell engager (BiTE; tandem di-scFv); a tandem tri-scFv; Fab-(scFv); scFab-dsscFv; tribody (Fab-(scFv)₂); Fab₂; Fab-Fv₂; diabody; triabody; tetrabody; scDb-scFv; di-diabody; scFv-Fc-scFv fusion (ADAPTIR); DVD-Ig; IgG-scFv fusions, such as CODV-IgG, Morrison (IgG CH₃-scFv fusion (Morrison L) or IgG CL-scFv fusion (Morrison H)), bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain) and Ts2Ab (dsscFv linked to C-terminus of heavy chain); a DART^{™}; a TRIDENT^{™}; a scDb, a tandem tri-scFv, a Fab-(scFv), a scFab-dsscFv, a Fab-(scFv)₂, a Fab₂, a Fab-Fv₂, a diabody or a scDb-scFv fused to the N- and/or the C-terminus of a heterodimerization domain other than heterodimeric Fc domains; a MATCH (described in WO 2016/0202457; Egan T. et al., MABS 9 (2017) 68-84) and DuoBodies (bispecific IgGs prepared by the Duobody technology) (MAbs. 2017 Feb/Mar;9(2): 182-212. doi: 10.1080/19420862.2016.1268307).

In one embodiment, the multispecific antibody MA2 is in a MATCH format described in WO 2016/0202457; Egan T., et al., MABS 9 (2017) 68-84. In particular, the multispecific antibody MA2 is in a MATCH3 or a MATCH4 format.

In another embodiment, the multispecific antibodies MA2 are in an IgG-scFv fusion format selected from CODV-IgG, Morrison (IgG CH₃-scFv fusion (Morrison L) or IgG CL-scFv fusion (Morrison H)), bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain), Ts2Ab (dsscFv linked to C-terminus of heavy chain), a DART^{™} and a TRIDENT^{™}.

Specific but non-limiting examples of multispecific antibodies MA2 that can suitably be applied in the pharmaceutical compositions or the kit of the invention are the scMATCH3-based antibodies PRO1766, PRO1767, PRO1872, and the MATCH4-based antibodies PRO2000 and PRO2100, whose sequences are listed in Table 9.

Further antibodies that can suitably be applied as MA2 in the pharmaceutical compositions or the kit of the invention are antibodies described in the prior art that comprise one or two TAA-BDs and one CD3-BD, as defined herein. Non-limiting examples of antibodies described in the prior art that can suitably be applied as MA2 are:
- The bispecific monovalent anti-HER2 x CD3 antibodies based on the knob-into-whole technology disclosed in WO 2015/095418;
- The bispecific 1 Fab-lgG-based anti-HER2 x CD3 antibodies having two low-affinity HER2 binding domains and one anti-CD3ε binding domain disclosed in WO 2019/157308 (to Genentech), in particular BTRC-4017A;
- The bispecific monovalent anti-HER2 x CD3 antibody GBR-1302 from Glenmark;
- The bispecific anti-MSLN x CD3 antibodies MG1122-A, having two low-affinity MSLN binding domains and one anti-CD3ε binding domain, and MG1122-B, having two low-affinity MSLN binding domains and one anti-CD3ε binding domain, as disclosed in Yoon et al. (Biomolecules, 2020,10(3), 399);
- The (scFv)₂-Fc-(scFv)₂ fusion (ADAPTIR) bispecific anti-ROR1 x CD3 antibody APVO-425 from Aptevo;
- The anti-TAA x CD3 (ROR1 x CD3) DART^{™} antibodies DART-1 to DART-33 and DART-A to DART-D disclosed in WO 2017/142928 (to Macrogenics).

The multispecific antibodies MA1 and MA2 that are applied in the pharmaceutical composition or the kit of the invention can be produced using any convenient antibody-manufacturing method known in the art (see, *e. g*., Fischer, N. & Leger, O., Pathobiology 74 (2007) 3-14 with regard to the production of bispecific constructs; Hornig, N. & Farber-Schwarz, A., Methods Mol. Biol. 907 (2012)713-727, and WO 99/57150 with regard to bispecific diabodies and tandem scFvs). Specific examples of suitable methods for the preparation of the bispecific construct further include, inter alia, the Genmab (see Labrijn et al., Proc. Natl. Acad. Sci. USA 110 (2013) 5145-5150) and Merus (see de Kruif et al., Biotechnol. Bioeng. 106 (2010) 741-750) technologies. Methods for production of bispecific antibodies comprising a functional antibody Fc part are also known in the art (see, *e. g*., Zhu et al., Cancer Lett. 86 (1994) 127-134); and Suresh et al., Methods Enzymol. 121 (1986) 210-228).

These methods typically involve the generation of monoclonal antibodies, for example by means of fusing myeloma cells with the spleen cells from a mouse that has been immunized with the desired antigen using the hybridoma technology (see, e. g., Yokoyama et al., Curr. Protoc. Immunol. Chapter 2, Unit 2.5, 2006) or by means of recombinant antibody engineering (repertoire cloning or phage display/yeast display) (see, *e. g*., Chames & Baty, FEMS Microbiol. Letters 189 (2000) 1-8), and the combination of the antigen-binding domains or fragments or parts thereof of two or more different monoclonal antibodies to give a bispecific or multispecific construct using known molecular cloning techniques.

The multispecific molecules MA1 and MA2 used in the invention can be prepared by conjugating the constituent binding specificities, using methods known in the art. For example, each binding specificity of the bispecific molecule can be generated separately and then conjugated to the other(s). When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl)-cyclohaxane-I-carboxylate (sulfo-SMCC) (see e. g., Karpovsky et al., 1984 J. Exp. Med. 160: 1686; Liu, M A et al., 1985 Proc. Natl. Acad. Sci. USA 82:8648). Other methods include those described in Paulus, 1985 Behring Ins. Mitt. No. 78, 118-132; Brennan et al., 1985 Science 229:81-83), and Glennie et al., 1987 J. Immunol. 139: 2367-2375). Conjugating agents are SATA and sulfo-SMCC, both available from Pierce Chemical Co. (Rockford, 111).

When the binding specificities are antibodies, they can be conjugated by sulfhydryl bonding of the C-terminus hinge regions of the two heavy chains. In a particular embodiment, the hinge region is modified to contain an odd number of sulfhydryl residues, for example one, prior to conjugation.

Alternatively, two or more binding specificities can be encoded in the same vector and expressed and assembled in the same host cell. This method is particularly useful where the bispecific molecule is a mAb x mAb, mAb x Fab, Fab x F (ab')₂ or ligand x Fab fusion protein. The multispecific antibody used in the invention can be a single chain multispecific antibody comprising at least two binding determinants. The multispecific antibody use in the invention can also comprise at least two of said single-chain molecules. Methods for preparing multispecific antibodies and molecules are described for example in U.S. Pat. No. 5,260,203; U.S. Pat. No. 5,455,030; U.S. Pat. No. 4,881,175; U.S. Pat. No. 5, 132, 405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the multispecific antibodies to their specific targets can be confirmed by, for example, enzyme-linked immunosorbent assay (ELISA), radioimmunoassay (REA), FACS analysis, bioassay (*e. g*., growth inhibition), or Western Blot assay. Each of these assays generally detects the presence of protein-antibody complexes of particular interest by employing a labeled reagent (*e. g*., an antibody) specific for the complex of interest.

Suitably, the pharmaceutical composition further comprises a pharmaceutically acceptable carrier.

"Pharmaceutically acceptable carrier" means a medium or diluent that does not interfere with the structure of the antibodies. Pharmaceutically acceptable carriers enhance or stabilize the composition, or facilitate preparation of the composition. Certain, of such carries enable pharmaceutical compositions to be formulated as, for example, tablets, pills, dragees, capsules, liquids, gels, syrups, slurries, suspension and lozenges for the oral ingestion by a subject. Certain of such carriers enable pharmaceutical compositions to be formulated for injection, infusion or topical administration. For example, a pharmaceutically acceptable carrier can be a sterile aqueous solution.

Pharmaceutically acceptable carriers include but are not limited to solvents, buffer solutions, dispersion media, coatings, antibacterial and antifungal agents, isotonic and absorption delaying agents, and the like that are physiologically compatible.

The pharmaceutical compositions of the present invention can be prepared in accordance with methods well known and routinely practiced in the art. See, *e. g.*, Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the multispecific antibodies MA1 and MA2 is employed in the pharmaceutical compositions of the invention. The multispecific antibodies MA1 and MA2 are formulated into pharmaceutically acceptable dosage forms by conventional methods known to those of skill in the art. Dosage regimens are adjusted to provide the optimum desired response (*e*. *g.,* a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions or the kit can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions used in the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

The pharmaceutical compositions or the kit of the invention can be administered by a variety of methods known in the art. In the case of administering the multispecific antibody components of the kits, administration may be done concomitantly or sequentially. In the case of a sequential administration, the multispecific antibody components may be administered based on individually adjusted administration schemes and regimens. The route and/or mode of administration vary depending upon the desired results. Administration can be intravenous, intramuscular, intraperitoneal, or subcutaneous, or administered proximal to the site of the target. The pharmaceutically acceptable carrier should be suitable for intravenous, intramuscular, subcutaneous, parenteral, spinal or epidermal administration (*e*. *g.,* by injection or infusion). Depending on the route of administration, the active compound, *i. e*., the multispecific antibodies MA1 and MA2 applied in the pharmaceutical composition of the invention, may be coated in a material to protect the compound from the action of acids and other natural conditions that may inactivate the compound.

The pharmaceutical composition or the kit of the invention is usually administered on multiple occasions. Intervals between single dosages can be weekly, monthly or yearly. Intervals can also be irregular as indicated by measuring blood levels of the multispecific antibody in the patient. Alternatively, the pharmaceutical composition of the invention can be administered as a sustained release formulation, in which case less frequent administration is required. Dosage and frequency vary depending on the half-life of the antibodies MA1 and MA2 in the patient. In general, humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

In one aspect, the present invention relates to the pharmaceutical composition or the kit of the invention for use as a medicament. In a suitable embodiment, the present invention provides the pharmaceutical composition or the kit for use in treatment of a proliferative disease, in particular a cancer in a subject in need thereof.

In another aspect, the present invention provides the pharmaceutical composition for use in a manufacture of a medicament for treatment of a proliferative disease, in particular a cancer.

In another aspect, the present invention relates to the use of the pharmaceutical composition or the kit for treating a proliferative disease, in particular a cancer in a subject in need thereof.

In another aspect, the present invention relates to a method of treating a subject comprising administering to the subject a therapeutically effective amount of the pharmaceutical composition of the present invention. In a suitable embodiment, the present invention relates to a method of treating a proliferative disease, in particular a cancer in a subject comprising administering to the subject a therapeutically effective amount of the pharmaceutical composition of the present invention.

In yet another aspect, the present invention relates to a method for treating a subject suffering from a proliferative disease, particularly a cancer, comprising the step of administering to said subject a first multispecific antibody (MA1) comprising one binding domain, which specifically binds to CD137 (CD137-BD), and one binding domain, which specifically binds to PDL1 (PDL1-BD), and a second multispecific antibody (MA2) comprising at least one binding domain, which specifically binds to a tumor cell associated antigen (TAA-BD), and one binding domain, which specifically binds to CD3 (CD3-BD), wherein said multispecific antibodies MA1 and MA2 are as defined herein.

In another aspect, the present invention relates to (i) a multispecific antibody MA1 comprising one binding domain, which specifically binds to CD137 (CD137-BD), and one binding domain, which specifically binds to PDL1 (PDL1-BD), for use in the treatment of a subject suffering from a proliferative disease, particularly a cancer, wherein said multispecific antibody MA1 is administered to said subject in combination with a second multispecific antibody MA2 comprising at least one binding domain, which specifically binds to a tumor cell associated antigen (TAA-BD), and one binding domain, which specifically binds to CD3 (CD3-BD), or (ii) a multispecific antibody MA2 comprising one binding domain, which specifically binds to a tumor cell associated antigen (TAA-BD), and one binding domain, which specifically binds to CD3 (CD3-BD), for use in the treatment of a subject suffering from a proliferative disease, particularly a cancer, wherein said multispecific antibody MA2 is administered to said subject in combination with a second multispecific antibody MA1 comprising at least one binding domain, which specifically binds to CD137 (CD137-BD), and one binding domain, which specifically binds to PDL1 (PDL-BD); wherein said multispecific antibodies MA1 and MA2 are as defined herein.

The term "subject" includes human and non-human animals.

The term "animals" include all vertebrates, *e. g*., non-human mammals and non-mammals, such as non-human primates, sheep, dog, cow, chickens, amphibians, and reptiles. Except when noted, the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, *e. g*., in a human, and includes: (a) inhibiting the disease, *i. e*., arresting its development; and (b) relieving the disease, *i*. *e*., causing regression of the disease.

The term "therapeutically effective amount" or "efficacious amount" refers to the amount of an agent that, when administered to a mammal or other subject for treating a disease, is sufficient to affect such treatment for the disease. The "therapeutically effective amount" will vary depending on the agent, the disease and its severity and the age, weight, etc., of the subject to be treated.

In one embodiment, the proliferative disease is a cancer. The term "cancer" refers to a disease characterized by the rapid and uncontrolled growth of aberrant cells. Cancer cells can spread locally or through the bloodstream and lymphatic system to other parts of the body. The terms "tumor" and "cancer" are used interchangeably herein, *e. g*., both terms encompass solid and liquid, *e. g*., diffuse or circulating, tumors. As used herein, the term "cancer" or "tumor" includes premalignant, as well as malignant cancers and tumors. The term "cancer" is used herein to mean a broad spectrum of tumors, including all solid and hematological malignancies. Examples of such tumors include, but are not limited to: a benign or especially malignant tumor, solid tumors, mesothelioma, brain cancer, kidney cancer, liver cancer, adrenal gland cancer, bladder cancer, breast cancer, stomach cancer (*e. g*., gastric tumors), esophageal cancer, ovarian cancer, cervical cancer, colon cancer, rectum cancer, prostate cancer, pancreatic cancer, lung cancer (*e. g*. non-small cell lung cancer and small cell lung cancer), vaginal cancer, thyroid cancer, melanoma (*e. g*., unresectable or metastatic melanoma), renal cell carcinoma, sarcoma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (*e. g*., Philadelphia chromosome-positive chronic myelogenous leukemia), acute lymphoblastic leukemia (*e. g*., Philadelphia chromosome-positive acute lymphoblastic leukemia), non-Hodgkin's lymphoma, plasma cell myeloma, Hodgkin's lymphoma, a leukemia, and any combination thereof. In a preferred embodiment, the cancer is a cancer selected from melanoma, mesothelioma, pancreatic cancer, stomach cancer, breast cancer, ovarian cancer and lung cancer.

The pharmaceutical composition or the kit of the present invention inhibits the growth of solid tumors, but also liquid tumors. In a further embodiment, the proliferative disease is a solid tumor. The term "solid tumor" especially means a mesothelioma, breast cancer, ovarian cancer, colon cancer, rectum cancer, prostate cancer, stomach cancer (especially gastric cancer), cervical cancer, lung cancer (*e*. *g*., non-small cell lung cancer and small cell lung cancer), pancreatic cancer and a tumor of the head and neck. Further, depending on the tumor type and the particular combination used, a decrease of the tumor volume can be obtained. The pharmaceutical composition or the kit of the present invention is also suited to prevent the metastatic spread of tumors and the growth or development of micro-metastases in a subject having a cancer.

**Sequence listing (mutations designated according to AHo numbering scheme; the CDRs defined according to Numab CDR definition, unless specified otherwise)**

**Table 1. Examples of CD137 binding domains as used in the present invention (CDR residues shown in bold and italic letters).**

| **SEQ ID NUMBER** | **Ab region** | **Sequence** |
|---|---|---|
| **38-02-A04** | | |
| SEQ ID NO: 1 | **HCDR1** (H27-H42; AHo numbering) | GFSFSNSYWIC |
| SEQ ID NO: 2 | **HCDR2** (H57-H76; AHo numbering) | CTFVGSSDSTYYANWAKG |
| SEQ ID NO: 3 | **HCDR3** (H108-H138; AHo numbering) | RHPSDAVYGYANNL |
| SEQ ID NO: 4 | **VH** (VH4) (38-02-A04 sc01) | QVQLQESGPGLVKPSETLSLTCKVSGFSFSNSYWICWIRQPPGKGLEWIGCTFV GSSDSTYYANWAKGRVTISVDSSKNQFSLKLSSVTAADTAVYYCARHPSDAVY GYANNLWGQGTLVTVSS |
| SEQ ID NO: 5 | **VH** (VH4) (38-02-A04 sc05 IF) Mutations VH: I44V; F89V; Y105F. | QVQLQESGPGLVKPSETLSLTCKVSGFSFSNSYWICWVRQPPGKGLEWIGCTF VGSSDSTYYANWAKGRVTISVDSSKNQVSLKLSSVTAADTAVYFCARHPSDAV YGYANNLWGQGTLVTVSS |
| SEQ ID NO: 6 | **VH** (VH4) (38-02-A04 sc06 Full) Mutations VH: V25A; I44V; V82K; F89V; Y105F | QVQLQESGPGLVKPSETLSLTCKASGFSFSNSYWICWVRQPPGKGLEWIGCTF VGSSDSTYYANWAKGRVTISKDSSKNQVSLKLSSVTAADTAVYFCARHPSDAV YGYANNLWGQGTLVTVSS |
| SEQ ID NO: 7 | **VH** (VH3) (38-02-A04 sc13) Mutations VH: G51C (AHo numbering) | EVQLVESGGGLVQPGGSLRLSCAASGFSFSNSYWICWVRQAPGKCLEWIGCTF VGSSDSTYYANWAKGRFTISRDNSKNTVYLQMNSLRAEDTAVYYCARHPSDA VYGYANNLWGQGTLVTVSS |
| SEQ ID NO: 8 | **LCDR1** (L24-L42; AHo numbering) (Kabat definition) | QASQSINNVLA |
| SEQ ID NO: 9 | **LCDR2** (L58-L72; AHo numbering) (Kabat definition) | RASTLAS |
| SEQ ID NO: 10 | **LCDR3** (L107-L138; AHo numbering) (Kabat definition) | QSSYGNYGD |
| SEQ ID NO: 11 | **VL** (Vk1-sk17) (38-02-A04 sc01) | DIQMTQSPSSLSASVGDRVTITCQASQSINNVLAWYQQKPGKAPKLLIYRASTL ASGVPSRFSGSGSGTDFTLTISSLQPEDFATYYCQSSYGNYGDFGTGTKVTVLG |
| SEQ ID NO: 12 | **VL** (Vk1-sk17) (38-02-A04 sc05 IF) Mutations VL: A51P | DIQMTQSPSSLSASVGDRVTITCQASQSINNVLAWYQQKPGKPPKLLIYRASTL ASGVPSRFSGSGSGTDFTLTISSLQPEDFATYYCQSSYGNYGDFGTGTKVTVLG |
| SEQ ID NO: 13 | **VL** (Vk1-sk17) (38-02-A04 sc06 Full) Mutations VL: I2L; A51P | DLQMTQSPSSLSASVGDRVTITCQASQSINNVLAWYQQKPGKPPKLLIYRASTL ASGVPSRFSGSGSGTDFTLTISSLQPEDFATYYCQSSYGNYGDFGTGTKVTVLG |
| SEQ ID NO: 14 | **VL** (Vk1-sk17) (38-02-A04 sc13) Mutations VL: T141C (AHo numbering) | DIQMTQSPSSLSASVGDRVTITCQASQSINNVLAWYQQKPGKAPKLLIYRASTL ASGVPSRFSGSGSGTDFTLTISSLQPEDFATYYCQSSYGNYGDFGCGTKVTVLG |
| SEQ ID NO: 15 | **scFv** (VL-linker-VH) (38-02-A04 sc01) | DIQMTQSPSSLSASVGDRVTITCQASQSINNVLAWYQQKPGKAPKLLIYRASTL ASGVPSRFSGSGSGTDFTLTISSLQPEDFATYYCQSSYGNYGDFGTGTKVTVLG GGGGSGGGGSGGGGSGGGGSQVQLQESGPGLVKPSETLSLTCKVSGFSFSNSY WICWIRQPPGKGLEWIGCTFVGSSDSTYYANWAKGRVTISVDSSKNQFSLKLSS VTAADTAVYYCARHPSDAVYGYANNLWGQGTLVTVSS |
| SEQ ID NO: 16 | **scFv** (VL-linker-VH) (38-02-A04 sc05 IF) | DIQMTQSPSSLSASVGDRVTITCQASQSINNVLAWYQQKPGKPPKLLIYRASTL ASGVPSRFSGSGSGTDFTLTISSLQPEDFATYYCQSSYGNYGDFGTGTKVTVLG GGGGSGGGGSGGGGSGGGGSQVQLQESGPGLVKPSETLSLTCKVSGFSFSNSY WICWVRQPPGKGLEWIGCTFVGSSDSTYYANWAKGRVTISVDSSKNQVSLKLS SVTAADTAVYFCARHPSDAVYGYANNLWGQGTLVTVSS |
| SEQ ID NO: 17 | **scFv** (VL-linker-VH)(38-02-A04 sc06 Full) | DLQMTQSPSSLSASVGDRVTITCQASQSINNVLAWYQQKPGKPPKLLIYRASTL ASGVPSRFSGSGSGTDFTLTISSLQPEDFATYYCQSSYGNYGDFGTGTKVTVLG GGGGSGGGGSGGGGSGGGGSQVQLQESGPGLVKPSETLSLTCKASGFSFSNSY WICWVRQPPGKGLEWIGCTFVGSSDSTYYANWAKGRVTISKDSSKNQVSLKLS SVTAADTAVYFCARHPSDAVYGYANNLWGQGTLVTVSS |
| SEQ ID NO: 18 | **scFv** (VL-linker-VH) (38-02-A04 sc13) | DIQMTQSPSSLSASVGDRVTITCQASQSINNVLAWYQQKPGKAPKLLIYRASTL ASGVPSRFSGSGSGTDFTLTISSLQPEDFATYYCQSSYGNYGDFGCGTKVTVLG GGGGSGGGGSGGGGSGGGGSEVQLVESGGGLVQPGGSLRLSCAASGFSFSNS YWICWVRQAPGKCLEWIGCTFVGSSDSTYYANWAKGRFTISRDNSKNTVYLQ MNSLRAEDTAVYYCARHPSDAVYGYANNLWGQGTLVTVSS |

| **38-27-C05 sc01** | | |
|---|---|---|
| SEQ ID NO: 19 | **HCDR1** (H27-H42; AHo numbering) | GFSFNNDYDMC |
| SEQ ID NO: 20 | **HCDR2** (H57-H76; AHo numbering) | CIDTGDGSTYY ASW AKG |
| SEQ ID NO: 21 | **HCDR3** (H108-H138; AHo numbering) | REAASSSGYGMGYFDL |
| SEQ ID NO: 22 | **VH** (VH4) | QVQLQESGPGLVKPSETLSLTCKVSGFSFNNDYDMCWIRQPPGKGLEWIGCID TGDGSTYYASWAKGRVTISVDSSKNQFSLKLSSVTAADTAVYYCAREAASSSG YGMGYFDLWGQGTLVTVSS |
| SEQ ID NO: 23 | **LCDR1** (L24-L42; AHo numbering) (Kabat definition) | QSSQSVYDNNWLA |
| SEQ ID NO: 24 | **LCDR2** (L58-L72; AHo numbering) (Kabat definition) | RASNLAS |
| SEQ ID NO: 25 | **LCDR3** (L107-L138; AHo numbering) (Kabat definition) | QGTYLSSNWYWA |
| SEQ ID NO: 26 | **VL** (Vk1-sk17) | DIQMTQSPSSLSASVGDRVTITCQSSQSVYDNNWLAWYQQKPGKAPKLLIYRA SNLASGVPSRFSGSGSGTDFTLTISSLQPEDFATYYCQGTYLSSNWYWAFGTGT KVTVLG |
| SEQ ID NO: 27 | **scFv** (VL-linker-VH) | DIQMTQSPSSLSASVGDRVTITCQSSQSVYDNNWLAWYQQKPGKAPKLLIYRA SNLASGVPSRFSGSGSGTDFTLTISSLQPEDFATYYCQGTYLSSNWYWAFGTGT KVTVLGGGGGSGGGGSGGGGSGGGGSQVQLQESGPGLVKPSETLSLTCKVSG FSFNNDYDMCWIRQPPGKGLEWIGCIDTGDGSTYYASWAKGRVTISVDSSKNQ FSLKLSSVTAADTAVYYCAREAASSSGYGMGYFDLWGQGTLVTVSS |

| **38-27-A11** | | |
|---|---|---|
| SEQ ID NO: 28 | **HCDR1** (H27-H42; AHo numbering) | GFSFSANYYPC |
| SEQ ID NO: 29 | **HCDR2** (H57-H76; AHo numbering) | CIYGGSSDITYDANWTK |
| SEQ ID NO: 30 | **HCDR3** (H108-H138; AHo numbering) | RSAWYSGWGGDL |
| SEQ ID NO: 31 | **VH** (VH3) (38-27-A11 sc02) | EVQLVESGGGLVQPGGSLRLSCAASGFSFSANYYPCWVRQAPGKGLEWIGCIY GGSSDITYDANWTKGRFTISRDNSKNTVYLQMNSLRAEDTAVYYCARSAWYS GWGGDLWGQGTLVTVSS |
| SEQ ID NO: 32 | **VH** (VH3) (38-27-A11 sc03) | ESQLVESGGGLVQPGGSLRLSCAASGFSFSANYYPCWVRQAPGKGLEWIGCIY GGSSDITYDANWTKGRFTISRDNSKNTVYLQMNSLRAEDTAVYFCARSAWYSG WGGDLWGPGTLVTVSS |
| SEQ ID NO: 33 | **VH** (VH3) (38-27-A11 sc07) (G51C) | EVQLVESGGGLVQPGGSLRLSCAASGFSFSANYYPCWVRQAPGKCLEWIGCIY GGSSDITYDANWTKGRFTISRDNSKNTVYLQMNSLRAEDTAVYYCARSAWYS GWGGDLWGQGTLVTVSS |
| SEQ ID NO: 34 | **LCDR1** (L24-L42; AHo numbering) (Kabat definition) | QASQSISNRLA |
| SEQ ID NO: 35 | **LCDR2** (L58-L72; AHo numbering) (Kabat definition) | SASTLAS |
| SEQ ID NO: 36 | **LCDR3** (L107-L138; AHo numbering) (Kabat definition) | QSTYYGNDGNA |
| SEQ ID NO: 37 | **VL** (Vk1-sk17) (38-27-A11 sc02) | DIQMTQSPSSLSASVGDRVTITCQASQSISNRLAWYQQKPGKAPKLLIYSASTLA SGVPSRFSGSGSGTDFTLTISSLQPEDFATYYCQSTYYGNDGNAFGTGTKVTVL G |
| SEQ ID NO: 38 | **VL** (Vk1-sk17) (38-27-A11 sc03) | DFQLTQSPSSLSASVGDRVTITCQASQSISNRLAWYQQKPGKPPKLLIYSASTLA SGVPSRFSGSGSGTDFTLTISSLQPEDFATYYCQSTYYGNDGNAFGTGTKVTVL G |
| SEQ ID NO: 39 | **VL** (Vk1-sk17) (38-27-A11 sc07) (T141C) | DIQMTQSPSSLSASVGDRVTITCQASQSISNRLAWYQQKPGKAPKLLIYSASTLA SGVPSRFSGSGSGTDFTLTISSLQPEDFATYYCQSTYYGNDGNAFGCGTKVTVL G |
| SEQ ID NO: 40 | **scFv** (VL-linker-VH) (38-27-A11 sc02) (PRO1359) | DIQMTQSPSSLSASVGDRVTITCQASQSISNRLAWYQQKPGKAPKLLIYSASTLA SGVPSRFSGSGSGTDFTLTISSLQPEDFATYYCQSTYYGNDGNAFGTGTKVTVL GGGGSGGGGSGGGGSGGGGSEVQLVESGGGLVQPGGSLRLSCAASGFSFSA NYYPCWVRQAPGKGLEWIGCIYGGSSDITYDANWTKGRFTISRDNSKNTVYLQ MNSLRAEDTAVYYCARSAWYSGWGGDLWGQGTLVTVSS |
| SEQ ID NO: 41 | **scFv** (VL-linker-VH) (38-27-A11 sc03) (PRO 1360) | DFQLTQSPSSLSASVGDRVTITCQASQSISNRLAWYQQKPGKPPKLLIYSASTLA SGVPSRFSGSGSGTDFTLTISSLQPEDFATYYCQSTYYGNDGNAFGTGTKVTVL GGGGSGGGGSGGGGSGGGGSESQLVESGGGLVQPGGSLRLSCAASGFSFSA NYYPCWVRQAPGKGLEWIGCIYGGSSDITYDANWTKGRFTISRDNSKNTVYLQ MNSLRAEDTAVYFCARSAWYSGWGGDLWGPGTLVTVSS |
| SEQ ID NO: 42 | **scFv** (VL-linker-VH) (38-27-A11 sc07) (VL-T141C; VH-G51C) (PRO 1704) | DIQMTQSPSSLSASVGDRVTITCQASQSISNRLAWYQQKPGKAPKLLIYSASTLA SGVPSRFSGSGSGTDFTLTISSLQPEDFATYYCQSTYYGNDGNAFGCGTKVTVL GGGGSGGGGSGGGGSGGGGSEVQLVESGGGLVQPGGSLRLSCAASGFSFSA NYYPCWVRQAPGKCLEWIGCIYGGSSDITYDANWTKGRFTISRDNSKNTVYLQ MNSLRAEDTAVYYCARSAWYSGWGGDLWGQGTLVTVSS |

**Table 2. Examples of PDL1 binding domains as used in the present invention (CDR residues shown in bold and italic letters).**

| **SEQ ID NUMBER** | **Ab region** | **Sequence** |
|---|---|---|
| **37-20-B03** | | |
| SEQ ID NO: 43 | **HCDR1** (H27-H42; AHo numbering) | GFSFNSDYWIY |
| SEQ ID NO: 44 | **HCDR2** (H57-H76; AHo numbering) | SIYGGSSGNTQYASWAQG |
| SEQ ID NO: 45 | **HCDR3** (H108-H138; AHo numbering) | RGYVDYGGATDL |
| SEQ ID NO: 46 | **VH** (VH4) (37-20-B03 sc01) | QVQLQESGPGLVKPSETLSLTCKVSGFSFNSDYWIYWIRQPPGKGLEWIGSIYG GSSGNTQYASWAQGRVTISVDSSKNQFSLKLSSVTAADTAVYYCARGYVDYGG ATDLWGQGTLVTVSS |
| SEQ ID NO: 47 | **VH** (VH1) (37-20-B03 sc02) | QVQLVQSGAEVKKPGASVKVSCKASGFSFNSDYWIYWVRQAPGQGLEWMGS IYGGSSGNTQYASWAQGRVTMTRDTSISTAYMELSSLRSEDTAVYYCARGYVD YGGATDLWGQGTLVTVSS |
| SEQ ID NO: 48 | **VH** (VH3) (37-20-B03 sc09.1) Mutations: G56A; Y105F | EVQLVESGGGLVQPGGSLRLSCAASGFSFNSDYWIYWVRQAPGKGLEWIASIY GGSSGNTQYASWAQGRFTISRDNSKNTVYLQMNSLRAEDTAVYFCARGYVDY GGATDLWGQGTLVTVSS |
| SEQ ID NO: 49 | **LCDR1** (L24-L42; AHo numbering) (Kabat definition) | QASQSIGTYLA |
| SEQ ID NO: 50 | **LCDR2** (L58-L72; AHo numbering) (Kabat definition) | RAFILAS |
| SEQ ID NO: 51 | **LCDR3** (L107-L138; AHo numbering) (Kabat definition) | QSNFYSDSTTIGPNA |
| SEQ ID NO: 52 | **VL** (Vk1-sk17) | DIQMTQSPSSLSASVGDRVTITCQASQSIGTYLAWYQQKPGKAPKLLIYRAFILA SGVPSRFSGSGSGTDFTLTISSLQPEDFATYYCQSNFYSDSTTIGPNAFGTGTKV |
| | (37-20-B03 sc01) (37-20-B03 sc02) | TVLG |
| SEQ ID NO: 53 | **VL** (Vk1-sk17) (37-20-B03 sc09.1) Mutations: S9A; A51P | DIQMTQSPASLSASVGDRVTITCQASQSIGTYLAWYQQKPGKPPKLLIYRAFILA SGVPSRFSGSGSGTDFTLTISSLQPEDFATYYCQSNFYSDSTTIGPNAFGTGTKV TVLG |
| SEQ ID NO: 54 | **scFv (VL-linker-VH)** (37-20-B03 sc01) (PRO997) | DIQMTQSPSSLSASVGDRVTITCQASQSIGTYLAWYQQKPGKAPKLLIYRAFILA SGVPSRFSGSGSGTDFTLTISSLQPEDFATYYCQSNFYSDSTTIGPNAFGTGTKV TVLGGGGSGGGGSGGGGSGGGGSQVQLQESGPGLVKPSETLSLTCKVSGFS FNSDYWIYWIRQPPGKGLEWIGSIYGGSSGNTQYASWAQGRVTISVDSSKNQFS LKLSSVTAADTAVYYCARGYVDYGGATDLWGQGTLVTVSS |
| SEQ ID NO: 55 | **scFv (VL-linker-VH)** (37-20-B03 sc02) (PRO1013) | DIQMTQSPSSLSASVGDRVTITC*QASQSIGTYLA*WYQQKPGKAPKLLIY*RAFILA* *S*GVPSRFSGSGSGTDFTLTISSLQPEDFATYYC*QSNFYSDSTTIGPNA*FGTGTKV TVLGGGGGSGGGGSGGGGSGGGGSQVQLVQSGAEVKKPGASVKVSCKAS*GF* *SFNSDYWIY*WVRQAPGQGLEWMG*SIYGGSSGNTQYASWAQG*RVTMTRDTSIS TAYMELSSLRSEDTAVYYCA*RGYVDYGGATDL*WGQGTLVTVSS |
| SEQ ID NO: 56 | **scFv (VL-linker-VH)** (37-20-B03 sc09.1) | DIQMTQSPASLSASVGDRVTITCQASQSIGTYLAWYQQKPGKPPKLLIYRAFIL ASGVPSRFSGSGSGTDFTLTISSLQPEDFATYYCQSNFYSDSTTIGPNAFGTGTK VTVLGGGGGSGGGGSGGGGSGGGGSEVQLVESGGGLVQPGGSLRLSCAASGF SFNSDYWIYWVRQAPGKGLEWIASIYGGSSGNTQYASWAQGRFTISRDNSKN TVYLQMNSLRAEDTAVYFCARGYVDYGGATDLWGQGTLVTVSS |

| **33-03-G02** | | |
|---|---|---|
| SEQ ID NO: 57 | **HCDR1** (H27-H42; AHo numbering) | GFSFSSGYDMC |
| SEQ ID NO: 58 | **HCDR2** (H57-H76; AHo numbering) | CVVAGSVDITYYASWAKG |
| SEQ ID NO: 59 | **HCDR3** (H108-H138; AHo numbering) | RKDAYSDAFNL |
| SEQ ID NO: 60 | **VH** (VH4) (33-03-G02 sc01) | QVQLQESGPGLVKPSETLSLTCKVSGFSFSSGYDMCWIRQPPGKGLEWIGCVV AGSVDITYYASWAKGRVTISVDSSKNQFSLKLSSVTAADTAVYYCARKDAYSD AFNLWGQGTLVTVSS |
| SEQ ID NO: 61 | **VH** (VH4) (33-03-G02 sc03 Full) (Mutations: V2S; V25A; 144V; G56A; V82K; F89V; Y105F) | QSQLQESGPGLVKPSETLSLTCKASGFSFSSGYDMCWVRQPPGKGLEWIACVV AGSVDITYYASWAKGRVTISKDSSKNQVSLKLSSVTAADTAVYFCARKDAYSD AFNLWGQGTLVTVSS |
| SEQ ID NO: 62 | **VH** (VH4) (33-03-G02 sc18) Mutations VH: V25A; 144; G56A; V82K; F89V (AHo numbering) | QVQLQESGPGLVKPSETLSLTCKASGFSFSSGYDMCWVRQPPGKGLEWIACVV AGSVDITYYASWAKGRVTISKDSSKNQVSLKLSSVTAADTAVYYCARKDAYSD AFNLWGQGTLVTVSS |
| SEQ ID NO: 63 | **LCDR1** (L24-L42; AHo numbering) (Kabat definition) | QASQSINDYLA |
| SEQ ID NO: 64 | **LCDR2** (L58-L72; AHo numbering) (Kabat definition) | KASTLAS |
| SEQ ID NO: 65 | **LCDR3** (L107-L138; AHo numbering) (Kabat definition) | QQGYIITDIDNV |
| SEQ ID NO: 66 | **VL** (Vk1-sk17) (33-03-G02 sc01) (33-03-G02 sc18) | DIQMTQSPSSLSASVGDRVTITCQASQSINDYLAWYQQKPGKAPKLLIYKASTL ASGVPSRFSGSGSGTDFTLTISSLQPEDFATYYCQQGYIITDIDNVFGTGTKVTV LG |
| SEQ ID NO: 67 | **VL** (Vk1-sk17) (33-03-G02 sc03 Full) (Mutations VL: I2F; M4L; A51P) | DFQLTQSPSSLSASVGDRVTITCQASQSINDYLAWYQQKPGKSPKLLIYKASTLA SGVPSRFSGSGSGTDFTLTISSLQPEDFATYYCQQGYIITDIDNVFGTGTKVTVL G |
| SEQ ID NO: 68 | **scFv (VL-linker-VH)** (33-03-G02 sc01) (PRO830) | DIQMTQSPSSLSASVGDRVTITCQASQSINDYLAWYQQKPGKAPKLLIYKASTL ASGVPSRFSGSGSGTDFTLTISSLQPEDFATYYCQQGYIITDIDNVFGTGTKVTV LGGGGGSGGGGSGGGGSGGGGSQVQLQESGPGLVKPSETLSLTCKVSGFSFSS |
| | | GYDMCWIRQPPGKGLEWIGCVVAGSVDITYYASWAKGRVTISVDSSKNQFSLK LSSVTAADTAVYYCARKDAYSDAFNLWGQGTLVTVSS |
| SEQ ID NO: 69 | **scFv (VL-linker-VH)** (33-03-G02 sc03 Full) | DFQLTQSPSSLSASVGDRVTITCQASQSINDYLAWYQQKPGKSPKLLIYKASTLA SGVPSRFSGSGSGTDFTLTISSLQPEDFATYYCQQGYIITDIDNVFGTGTKVTVL GGGGSGGGGSGGGGSGGGGSQSQLQESGPGLVKPSETLSLTCKASGFSFSSG YDMCWVRQPPGKGLEWIACVVAGSVDITYYASWAKGRVTISKDSSKNQVSLKL SSVTAADTAVYFCARKDAYSDAFNLWGQGTLVTVSS |
| SEQ ID NO: 70 | **scFv (VL-linker-VH)** (33-03-G02 sc18) | DIQMTQSPSSLSASVGDRVTITCQASQSINDYLAWYQQKPGKAPKLLIYKASTL ASGVPSRFSGSGSGTDFTLTISSLQPEDFATYYCQQGYIITDIDNVFGTGTKVTV LGGGGSGGGGSGGGGSGGGGSQVQLQESGPGLVKPSETLSLTCKASGFSFSS GYDMCWVRQPPGKGLEWIACVVAGSVDITYYASWAKGRVTISKDSSKNQVSLK LSSVTAADTAVYYCARKDAYSDAFNLWGQGTLVTVSS |

**Table 3. Examples of human serum albumin binding domains as used in the present invention (CDR residues shown in bold and italic letters).**

| **SEQ ID NUMBER** | **Ab region** | **Sequence** |
|---|---|---|
| **19-01-H04 sc03** | | |
| SEQ ID NO: 71 | **HCDR1** (H27-H42; AHo numbering) | GFSLSSNAMG |
| SEQ ID NO: 72 | **HCDR2** (H57-H76; AHo numbering) | IISVGGFTYYASWAKG |
| SEQ ID NO: 73 | **HCDR3** (H108-H138; AHo numbering) | RDRHGGDSSGAFYL |
| SEQ ID NO: 74 | **VH** | EVQLVESGGGLVQPGGSLRLSCAASGFSLSSNAMGWVRQAPGKGLEYIGIISV GGFTYYASWAKGRFTISRDNSKNTVYLQMNSLRAEDTATYFCARDRHGGDSS GAFYLWGQGTLVTVSS |
| SEQ ID NO: 75 | **LCDR1** (L24-L42; AHo numbering) (Kabat definition) | QSSESVYSNNQLS |
| SEQ ID NO: 76 | **LCDR2** | DASDLAS |
| | (L58-L72; AHo numbering) (Kabat definition) | |
| SEQ ID NO: 77 | **LCDR3** (L107-L138; AHo numbering) (Kabat definition) | AGGFSSSSDTA |
| SEQ ID NO: 78 | **VL** | DIQMTQSPSSLSASVGDRVTITCQSSESVYSNNQLSWYQQKPGQPPKLLIYDAS DLASGVPSRFSGSGSGTDFTLTISSLQPEDFATYYCAGGFSSSSDTAFGGGTKLT VLG |
| SEQ ID NO: 79 | **scFv** (VL-linker-VH) | DIQMTQSPSSLSASVGDRVTITCQSSESVYSNNQLSWYQQKPGQPPKLLIYDAS DLASGVPSRFSGSGSGTDFTLTISSLQPEDFATYYCAGGFSSSSDTAFGGGTKLT VLGGGGGSGGGGSGGGGSGGGGSEVQLVESGGGLVQPGGSLRLSCAASGFSL SSNAMGWVRQAPGKGLEYIGIISVGGFTYYASWAKGRFTISRDNSKNTVYLQM NSLRAEDTATYFCARDRHGGDSSGAFYLWGQGTLVTVSS |

| **23-13-A01 sc03** | | |
|---|---|---|
| SEQ ID NO: 80 | **HCDR1** (H27-H42; AHo numbering) | GFSFSSSYWIC |
| SEQ ID NO: 81 | **HCDR2** (H57-H76; AHo numbering) | CVFTGDGTTYYASWAKG |
| SEQ ID NO: 82 | **HCDR3** (H108-H138; AHo numbering) | RPVSVYYYGMDL |
| SEQ ID NO: 83 | **VH** | EVQLVESGGGLVQPGGSLRLSCAASGFSFSSSYWICWVRQAPGKGLEWVGCV FTGDGTTYYASWAKGRFTISRDNSKNTVYLQMNSLRAEDTATYFCARPVSVYY YGMDLWGQGTLVTVSS |
| SEQ ID NO: 84 | **LCDR1** (L24-L42; AHo numbering) (Kabat definition) | QASQIISSRSA |
| SEQ ID NO: 85 | **LCDR2** (L58-L72; AHo numbering) (Kabat definition) | QASKLAS |
| SEQ ID NO: 86 | **LCDR3** (L107-L138; AHo numbering) (Kabat definition) | QCTYIDSNFGA |
| SEQ ID NO: 87 | **VL** | DVVMTQSPSSLSASVGDRVTITCQASQIISSRSAWYQQKPGQPPKLLIYQASKLA SGVPSRFSGSGSGTDFTLTISSLQPEDFATYYCQCTYIDSNFGAFGGGTKLTVLG |
| SEQ ID NO: 88 | **scFv** (VL-linker-VH) | DVVMTQSPSSLSASVGDRVTITCQASQIISSRSAWYQQKPGQPPKLLIYQASKLA SGVPSRFSGSGSGTDFTLTISSLQPEDFATYYCQCTYIDSNFGAFGGGTKLTVLG GGGGSGGGGSGGGGSGGGGSEVQLVESGGGLVQPGGSLRLSCAASGFSFSSSY WICWVRQAPGKGLEWVGCVFTGDGTTYYASWAKGRFTISRDNSKNTVYLQM NSLRAEDTATYFCARPVSVYYYGMDLWGQGTLVTVSS |

**Table 4. Other sequences related to the present invention.**

| **SEQ ID NUMBER** | **Ab region** | **Sequence** |
|---|---|---|
| SEQ ID NO: 89 | Human CD137 | MGNSCYNIVATLLLVLNFERTRSLQDPCSNCPAGTFCDNNRNQICSPCPPNSFSS AGGQRTCDICRQCKGVFRTRKECSSTSNAECDCTPGFHCLGAGCSMCEQDCK QGQELTKKGCKDCCFGTFNDQKRGICRPWTNCSLDGKSVLVNGTKERDVVCG PSPADLSPGASSVTPPAPAREPGHSPQIISFFLALTSTALLFLLFFLTLRFSVVKRG RKKLLYIFKQPFMRPVQTTQEEDGCSCRFPEEEEGGCEL |
| SEQ ID NO: 90 | Human PDL1 | MRIFAVFIFMTYWHLLNAFTVTVPKDLYVVEYGSNMTIECKFPVEKQLDLAAL IVYWEMEDKNIIQFVHGEEDLKVQHSSYRQRARLLKDQLSLGNAALQITDVKL QDAGVYRCMISYGGADYKRITVKVNAPYNKINQRILVVDPVTSEHELTCQAEG YPKAEVIWTSSDHQVLSGKTTTTNSKREEKLFNVTSTLRINTTTNEIFYCTFRRL DPEENHTAELVIPELPLAHPPNERTHLVILGAILLCLGVALTFIFRLRKGRMMDV KKCGIQDTNSKKQSDTHLEET |
| SEQ ID NO: 91 | VH3 | EVQLVESGGGLVQPGGSLRLSCAASGFSFSANYYPCWVRQAPGKGLEWIGCIY GGSSDITYDANWTKGRFTISRDNSKNTVYLQMNSLRAEDTAVYYCARSAWYS GWGGDLWGQGTLVTVSS |
| SEQ ID NO: 92 | VH4 | QVQLQESGPGLVKPSETLSLTCKVSGFSFSNSYWICWIRQPPGKGLEWIGCTFV GSSDSTYYANWAKGRVTISVDSSKNQFSLKLSSVTAADTAVYYCARHPSDAVY GYANNLWGQGTLVTVSS |
| SEQ ID NO: 93 | Vkappa1 | DIQMTQSPSSLSASVGDRVTITCQASQSINNVLAWYQQKPGKAPKLLIYRASTL ASGVPSRFSGSGSGTDFTLTISSLQPEDFATYYCQSSYGNYGDFGTGTKVTVLG |
| SEQ ID NO: 94 | Vλ germline-based FR4 (Sk17) | FGTGTKVTVLG |
| SEQ ID NO: 95 | Vλ germline-based FR4 (Sk12) | FGGGTKLTVLG |
| SEQ ID NO: 96 | Vλ germline-based FR4 | FGGGTQLIILG |
| SEQ ID NO: 97 | Vλ germline-based FR4 | FGEGTELTVLG |
| SEQ ID NO: 98 | Vλ germline-based FR4 | FGSGTKVTVLG |
| SEQ ID NO: 99 | Vλ germline-based FR4 | FGGGTQLTVLG |
| SEQ ID NO: 100 | Vλ germline-based FR4 | FGGGTQLTALG |
| SEQ ID NO: 101 | Vλ germline-based FR4 G141C | FGCGTKVTVLG |
| SEQ ID NO: 102 | **Linker** | GGGGSGGGGSGGGGSGGGGS |
| SEQ ID NO: 103 | **Linker** | GGGGS |
| SEQ ID NO: 104 | **Linker** | GGGGSGGGGS |

**Table 5. Examples of multispecific antibodies (MA1) as used in the present invention (Linkers are shown in bold).**

| **SEQ ID NUMBER** | **Ab Format** | **Sequence** |
|---|---|---|
| **PRO885** (38-02-A04 sc01 seDb-i/33-03-G02 sc01 scDb-o) | | |
| SEQ ID NO: 105 | scDb | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKVSGFSFSNSYWICWIRQPPGKGLEWIGCTF VGSSDSTYYANWAKGRVTISVDSSKNQFSLKLSSVTAADTAVYYCARHPSDAVY GYANNLWGQGTLVTVSSGGGGSGGGGSGGGGSGGGGSIQMTQSPSSLSASVG DRVTITCQASQSINNVLAWYQQKPGKAPKLLIYRASTLASGVPSRFSGSGSGTDFT LTISSLQPEDFATYYCQSSYGNYGDFGTGTKVTVLGGGGGSQVQLQESGPGLVK PSETLSLTCKVSGFSFSSGYDMCWIRQPPGKGLEWIGCVVAGSVDITYYASWAKG RVTISVDSSKNQFSLKLSSVTAADTAVYYCARKDAYSDAFNLWGQGTLVTVSS |

| **PRO951** (38-27-C05 sc02 scDb-i/33-03-G02 sc0 | | |
|---|---|---|
| SEQ ID NO: 106 | scDb-0 | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG |
| | | GGSEVQLVESGGGLVQPGGSLRLSCAASGFSFNNDYDMCWVRQAPGKGLEWIG CIDTGDGSTYYASWAKGRFTISRDNSKNTVYLQMNSLRAEDTAVYYCAREAASS SGYGMGYFDLWGQGTLVTVSSGGGGSGGGGSGGGGSGGGGSIQMTQSPSSLS ASVGDRVTITCQSSQSVYDNNWLAWYQQKPGKAPKLLIYRASNLASGVPSRFSG SGSGTDFTLTISSLQPEDFATYYCQGTYLSSNWYWAFGTGTKVTVLGGGGGSQV QLQESGPGLVKPSETLSLTCKVSGFSFSSGYDMCWIRQPPGKGLEWIGCVVAGSV DITYYASWAKGRVTISVDSSKNQFSLKLSSVTAADTAVYYCARKDAYSDAFNLW GQGTLVTVSS |

| **PRO1123** (38-02-A04 sc05 | IF scDb-i/33-03-G02 | 2sc01 scDb-o) |
|---|---|---|
| SEQ ID NO: 107 | scDb | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKVSGFSFSNSYWICWVRQPPGKGLEWIGCT FVGSSDSTYYANWAKGRVTISVDSSKNQVSLKLSSVTAADTAVYFCARHPSDAV YGYANNLWGQGTLVTVSSGGGGSGGGGSGGGGSGGGGSIQMTQSPSSLSASV GDRVTITCQASQSINNVLAWYQQKPGKPPKLLIYRASTLASGVPSRFSGSGSGTDF TLTISSLQPEDFATYYCQSSYGNYGDFGTGTKVTVLGGGGSQVQLQESGPGLV KPSETLSLTCKVSGFSFSSGYDMCWIRQPPGKGLEWIGCVVAGSVDITYYASWAK GRVTISVDSSKNQFSLKLSSVTAADTAVYYCARKDAYSDAFNLWGQGTLVTVSS |

| **PRO1124** (38-02-A04 sc06 | Full scDb-i/33-03-G02 | sc01 scDb-o) |
|---|---|---|
| SEQ ID NO: 108 | scDb | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKASGFSFSNSYWICWVRQPPGKGLEWIGCT FVGSSDSTYYANWAKGRVTISKDSSKNQVSLKLSSVTAADTAVYFCARHPSDAV YGYANNLWGQGTLVTVSSGGGGSGGGGSGGGGSGGGGSLQMTQSPSSLSASV GDRVTITCQASQSINNVLAWYQQKPGKPPKLLIYRASTLASGVPSRFSGSGSGTDF TLTISSLQPEDFATYYCQSSYGNYGDFGTGTKVTVLGGGGGSQVQLQESGPGLV KPSETLSLTCKVSGFSFSSGYDMCWIRQPPGKGLEWIGCVVAGSVDITYYASWAK GRVTISVDSSKNQFSLKLSSVTAADTAVYYCARKDAYSDAFNLWGQGTLVTVSS |

| **PR01125** (38-02-A04 sc01 | scDb-i/33-03-G02 scc02 IF scDb-o) | |
|---|---|---|
| SEQ ID NO: 109 | scDb | DIQMTQSPSSLSASVGDRVTITCQASQSINDYLAWYQQKPGKSPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKVSGFSFSNSYWICWIRQPPGKGLEWIGCTF |
| | | VGSSDSTYYANWAKGRVTISVDSSKNQFSLKLSSVTAADTAVYYCARHPSDAVY GYANNLWGQGTLVTVSSGGGGSGGGGSGGGGSGGGGSIQMTQSPSSLSASVG DRVTITCQASQSINNVLAWYQQKPGKAPKLLIYRASTLASGVPSRFSGSGSGTDFT LTISSLQPEDFATYYCQSSYGNYGDFGTGTKVTVLGGGGGSQVQLQESGPGLVK PSETLSLTCKVSGFSFSSGYDMCWVRQPPGKGLEWIACVVAGSVDITYYASWAK GRVTISVDSSKNQFSLKLSSVTAADTAVYFCARKDAYSDAFNLWGQGTLVTVSS |

| **PRO1126** (38-02-A04 sc01 scDb-i/33-03-G02 sc c03 Full scDb-o) | | |
|---|---|---|
| SEQ ID NO: 110 | scDb | DFQLTQSPSSLSASVGDRVTITCQASQSINDYLAWYQQKPGKSPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKVSGFSFSNSYWICWIRQPPGKGLEWIGCTF VGSSDSTYYANWAKGRVTISVDSSKNQFSLKLSSVTAADTAVYYCARHPSDAVY GYANNLWGQGTLVTVSSGGGGSGGGGSGGGGSGGGGSIQMTQSPSSLSASVG DRVTITCQASQSINNVLAWYQQKPGKAPKLLIYRASTLASGVPSRFSGSGSGTDFT LTISSLQPEDFATYYCQSSYGNYGDFGTGTKVTVLGGGGGSQSLQESGPGLVKP SETLSLTCKASGFSFSSGYDMCWVRQPPGKGLEWIACVVAGSVDITYYASWAKG RVTISKDSSKNQVSLKLSSVTAADTAVYFCARKDAYSDAFNLWGQGTLVTVSS |

| **PRO1134** (38-02-A04 sc01 scDb-i/33-03-G02 sc sc07GL VH3 scDb-o) | | |
|---|---|---|
| SEQ ID NO: 111 | scDb | DIQMTQSPSSLSASVGDAVTITCQASQSINDYLAWYQQKPGKSPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKVSGFSFSNSYWICWIRQPPGKGLEWIGCTF VGSSDSTYYANWAKGRVTISVDSSKNQFSLKLSSVTAADTAVYYCARHPSDAVY GYANNLWGQGTLVTVSSGGGGSGGGGSGGGGSGGGGSIQMTQSPSSLSASVG DRVTITCQASQSINNVLAWYQQKPGKAPKLLIYRASTLASGVPSRFSGSGSGTDFT LTISSLQPEDFATYYCQSSYGNYGDFGTGTKVTVLGGGGGSEVQLVESGGGLVQ PGGSLRLSCAASGFSFSSGYDMCWVRQAPGKGLEWVGCVVAGSVDITYYASWA KGRFTISRDNSKNTVYLQMNSLRAEDTATYYCARKDAYSDAFNLWGPGTLVTVS S |

| **PRO963 (= PRO1051)** (38--02-A04 sc01 scDb-i /33-03-G02 sc01 scDb-o/19-01-H04-sc03 scFv) | | |
|---|---|---|
| SEQ ID NO: 112 | scDb-scFv | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKVSGFSFSNSYWICWIRQPPGKGLEWIGCTF VGSSDSTYYANWAKGRVTISVDSSKNQFSLKLSSVTAADTAVYYCARHPSDAVY |
| | | GYANNLWGQGTLVTVSSGGGGSGGGGSGGGGSGGGGSIQMTQSPSSLSASVG DRVTITCQASQSINNVLAWYQQKPGKAPKLLIYRASTLASGVPSRFSGSGSGTDFT LTISSLQPEDFATYYCQSSYGNYGDFGTGTKVTVLGGGGGSQVQLQESGPGLVK PSETLSLTCKVSGFSFSSGYDMCWIRQPPGKGLEWIGCVVAGSVDITYYASWAKG RVTISVDSSKNQFSLKLSSVTAADTAVYYCARKDAYSDAFNLWGQGTLVTVSSG GGGSGGGGSIQMTQSPSSLSASVGDRVTITCQSSESVYSNNQLSWYQQKPGQPPK LLIYDASDLASGVPSRFSGSGSGTDFTLTISSLQPEDFATYYCAGGFSSSSDTAFGG GTKLTVLGGGGGSGGGGSGGGGSGGGGSEVQLVESGGGLVQPGGSLRLSCAA SGFSLSSNAMGWVRQAPGKGLEYIGIISVGGFTYYASWAKGRFTISRDNSKNTVY LQMNSLRAEDTATYFCARDRHGGDSSGAFYLWGQGTLVTVSS |

| **PRO966** (= **PRO1052**) (38-27-C05 sc01 scDb-i/33-03-G02 sc01 scDb-o/19-01-H04-sc03 scFv) | | |
|---|---|---|
| SEQ ID NO: 113 | scDb-scFv | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKVSGFSFNNDYDMCWIRQPPGKGLEWIGCI DTGDGSTYYASWAKGRVTISVDSSKNQFSLKLSSVTAADTAVYYCAREAASSSG YGMGYFDLWGQGTLVTVSSGGGGSGGGGSGGGGSGGGGSIQMTQSPSSLSAS VGDRVTITCQSSQSVYDNNWLAWYQQKPGKAPKLLIYRASNLASGVPSRFSGSG SGTDFTLTISSLQPEDFATYYCQGTYLSSNWYWAFGTGTKVTVLGGGGGSQVQL QESGPGLVKPSETLSLTCKVSGFSFSSGYDMCWIRQPPGKGLEWIGCVVAGSVDIT YYASWAKGRVTISVDSSKNQFSLKLSSVTAADTAVYYCARKDAYSDAFNLWGQ GTLVTVSSGGGGSGGGGSIQMTQSPSSLSASVGDRVTITCQSSESVYSNNQLSWY QQKPGQPPKLLIYDASDLASGVPSRFSGSGSGTDFTLTISSLQPEDFATYYCAGGFS SSSDTAFGGGTKLTVLGGGGGSGGGGSGGGGSGGGGSEVQLVESGGGLVQPG GSLRLSCAASGFSLSSNAMGWVRQAPGKGLEYIGIISVGGFTYYASWAKGRFTISR DNSKNTVYLQMNSLRAEDTATYFCARDRHGGDSSGAFYLWGQGTLVTVSS |

| **PRO1057** (38-02-A04 sc01 scDb-i/33-03-G02 sc01 scDb-o/23-12-A01-sc03, skl7sh4) | | |
|---|---|---|
| SEQ ID NO: 114 | scDb-scFv | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKVSGFSFSNSYWICWIRQPPGKGLEWIGCTF VGSSDSTYYANWAKGRVTISVDSSKNQFSLKLSSVTAADTAVYYCARHPSDAVY GYANNLWGQGTLVTVSSGGGGSGGGGSGGGGSGGGGSIQMTQSPSSLSASVG DRVTITCQASQSINNVLAWYQQKPGKAPKLLIYRASTLASGVPSRFSGSGSGTDFT |
| | | LTISSLQPEDFATYYCQSSYGNYGDFGTGTKVTVLGGGGGSQVQLQESGPGLVK PSETLSLTCKVSGFSFSSGYDMCWIRQPPGKGLEWIGCVVAGSVDITYYASWAKG RVTISVDSSKNQFSLKLSSVTAADTAVYYCARKDAYSDAFNLWGQGTLVTVSSG GGGSGGGGSVVMTQSPSSLSASVGDRVTITCQASQIISSRSAWYQQKPGQPPKLL IYQASKLASGVPSRFSGSGSGTDFTLTISSLQPEDFATYYCQCTYIDSNFGAFGGGT KLTVLGGGGGSGGGGSGGGGSGGGGSEVQLVESGGGLVQPGGSLRLSCAASG FSFSSSYWICWVRQAPGKGLEWVGCVFTGDGTTYYASWAKGRFTISRDNSKNTV YLQMNSLRAEDTATYFCARPVSVYYYGMDLWGQGTLVTVSS |

| **PRO1058** (38-27-C05 sc01 scDb-i/33-03-G02 sc 01 scDb-o/23-13-A01-sc03, sk17sh4) | | |
|---|---|---|
| SEQ ID NO: 115 | scDb-scFv | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG GGSQVQLQESGPGLVKPSETLSLTCKVSGFSFNNDYDMCWIRQPPGKGLEWIGCI DTGDGSTYYASWAKGRVTISVDSSKNQFSLKLSSVTAADTAVYYCAREAASSSG YGMGYFDLWGQGTLVTVSSGGGGSGGGGSGGGGSGGGGSIQMTQSPSSLSAS VGDRVTITCQSSQSVYDNNWLAWYQQKPGKAPKLLIYRASNLASGVPSRFSGSG SGTDFTLTISSLQPEDFATYYCQGTYLSSNWYWAFGTGTKVTVLGGGGGSQVQL QESGPGLVKPSETLSLTCKVSGFSFSSGYDMCWIRQPPGKGLEWIGCVVAGSVDIT YYASWAKGRVTISVDSSKNQFSLKLSSVTAADTAVYYCARKDAYSDAFNLWGQ GTLVTVSSGGGGSGGGGSVVMTQSPSSLSASVGDRVTITCQASQIISSRSAWYQQ KPGQPPKLLIYQASKLASGVPSRFSGSGSGTDFTLTISSLQPEDFATYYCQCTYIDS NFGAFGGGTKLTVLGGGGGSGGGGSGGGGSGGGGSEVQLVESGGGLVQPGGS LRLSCAASGFSFSSSYWICWVRQAPGKGLEWVGCVFTGDGTTYYASWAKGRFTI SRDNSKNTVYLQMNSLRAEDTATYFCARPVSVYYYGMDLWGQGTLVTVSS |

| **PRO1175** (37-20-B03-sc01-o/38-02-A04 sc01-i /19-01-H04sc03 scFv) | | |
|---|---|---|
| SEQ ID NO: 116 | scDb-scFv | DIQMTQSPSSLSASVGDRVTITCQASQSIGTYLAWYQQKPGKAPKLLIYRAFILAS GVPSRFSGSGSGTDFTLTISSLQPEDFATYYCQSNFYSDSTTIGPNAFGTGTKVTVL GGGGSQVQLQESGPGLVKPSETLSLTCKVSGFSFSNSYWICWIRQPPGKGLEWI GCTFVGSSDSTYYANWAKGRVTISVDSSKNQFSLKLSSVTAADTAVYYCARHPS DAVYGYANNLWGQGTLVTVSSGGGGSGGGGSGGGGSGGGGSIQMTQSPSSLS ASVGDRVTITCQASQSINNVLAWYQQKPGKAPKLLIYRASTLASGVPSRFSGSGS GTDFTLTISSLQPEDFATYYCQSSYGNYGDFGTGTKVTVLGGGGSQVQLQESGP GLVKPSETLSLTCKVSGFSFNSDYWIYWIRQPPGKGLEWIGSIYGGSSGNTQYAS |
| | | WAQGRVTISVDSSKNQFSLKLSSVTAADTAVYYCARGYVDYGGATDLWGQGTL VTVSSGGGGSGGGGSIQMTQSPSSLSASVGDRVTITCQSSESVYSNNQLSWYQQ KPGQPPKLLIYDASDLASGVPSRFSGSGSGTDFTLTISSLQPEDFATYYCAGGFSSS SDTAFGGGTKLTVLGGGGGSGGGGSGGGGSGGGGSEVQLVESGGGLVQPGGS LRLSCAASGFSLSSNAMGWVRQAPGKGLEYIGIISVGGFTYYASWAKGRFTISRD NSKNTVYLQMNSLRAEDTATYFCARDRHGGDSSGAFYLWGQGTLVTVSS |

| **PR01186** (38-02-A04 sc01 scDb-i/37-20-B03sc 01 scDb-o/23-13-A01-sc03 scFv) | | |
|---|---|---|
| SEQ ID NO: 117 | scDb-scFv | DIQMTQSPSSLSASVGDRVTITCQASQSIGTYLAWYQQKPGKAPKLLIYRAFILAS GVPSRFSGSGSGTDFTLTISSLQPEDFATYYCQSNFYSDSTTIGPNAFGTGTKVTVL GGGGGSQVQLQESGPGLVKPSETLSLTCKVSGFSFSNSYWICWIRQPPGKGLEWI GCTFVGSSDSTYYANWAKGRVTISVDSSKNQFSLKLSSVTAADTAVYYCARHPS DAVYGYANNLWGQGTLVTVSSGGGGSGGGGSGGGGSGGGGSDIQMTQSPSSL SASVGDRVTITCQASQSINNVLAWYQQKPGKAPKLLIYRASTLASGVPSRFSGSGS GTDFTLTISSLQPEDFATYYCQSSYGNYGDFGTGTKVTVLGGGGGSQVQLQESGP GLVKPSETLSLTCKVSGFSFNSDYWIYWIRQPPGKGLEWIGSIYGGSSGNTQYAS WAQGRVTISVDSSKNQFSLKLSSVTAADTAVYYCARGYVDYGGATDLWGQGTL VTVSSGGGGSGGGGSVVMTQSPSSLSASVGDRVTITCQASQIISSRSAWYQQKPG QPPKLLIYQASKLASGVPSRFSGSGSGTDFTLTISSLQPEDFATYYCQCTYIDSNFG AFGGGTKLTVLGGGGGSGGGGSGGGGSGGGGSEVQLVESGGGLVQPGGSLRL SCAASGFSFSSSYWICWVRQAPGKGLEWVGCVFTGDGTTYYASWAKGRFTISRD NSKNTVYLQMNSLRAEDTATYFCARPVSVYYYGMDLWGQGTLVTVSS |

| **PRO1430** (38-02-A04 sc13 scDb-i/37-20-B03 sc 01 scDb-o/19-01-H04 sc03 scFv) | | |
|---|---|---|
| SEQ ID NO: 118 | scDb-scFv | DIQMTQSPSSLSASVGDRVTITCQASQSIGTYLAWYQQKPGKAPKLLIYRAFILAS GVPSRFSGSGSGTDFTLTISSLQPEDFATYYCQSNFYSDSTTIGPNAFGTGTKVTVL GGGGGSEVQLVESGGGLVQPGGSLRLSCAASGFSFSNSYWICWVRQAPGKCLE WIGCTFVGSSDSTYYANWAKGRFTISRDNSKNTVYLQMNSLRAEDTAVYYCARH PSDAVYGYANNLWGQGTLVTVSSGGGGSGGGGSGGGGSGGGGSIQMTQSPSS LSASVGDRVTITCQASQSINNVLAWYQQKPGKAPKLLIYRASTLASGVPSRFSGSG SGTDFTLTISSLQPEDFATYYCQSSYGNYGDFGCGTKVTVLGGGGGSQVQLQESG PGLVKPSETLSLTCKVSGFSFNSDYWIYWIRQPPGKGLEWIGSIYGGSSGNTQYAS WAQGRVTISVDSSKNQFSLKLSSVTAADTAVYYCARGYVDYGGATDLWGQGTL VTVSSGGGGSGGGGSIQMTQSPSSLSASVGDRVTITCQSSESVYSNNQLSWYQQ |
| | | KPGQPPKLLIYDASDLASGVPSRFSGSGSGTDFTLTISSLQPEDFATYYCAGGFSSS SDTAFGGGTKLTVLGGGGGSGGGGSGGGGSGGGGSEVQLVESGGGLVQPGGS LRLSCAASGFSLSSNAMGWVRQAPGKGLEYIGIISVGGFTYYASWAKGRFTISRD NSKNTVYLQMNSLRAEDTATYFCARDRHGGDSSGAFYLWGQGTLVTVSS |

| **PRO1479** (38-02-A04 sc13 scDb-i/37-20-B03 sc09.1 scDb-o/19-01-H04 sc03 scFv) | | |
|---|---|---|
| SEQ ID NO: 119 | scDb-scFv | DIQMTQSPASLSASVGDRVTITCQASQSIGTYLAWYQQKPGKPPKLLIYRAFILAS GVPSRFSGSGSGTDFTLTISSLQPEDFATYYCQSNFYSDSTTIGPNAFGTGTKVTVL GGGGGSEVQLVESGGGLVQPGGSLRLSCAASGFSFSNSYWICWVRQAPGKCLE WIGCTFVGSSDSTYYANWAKGRFTISRDNSKNTVYLQMNSLRAEDTAVYYCARH PSDAVYGYANNLWGQGTLVTVSSGGGGSGGGGSGGGGSGGGGSIQMTQSPSS LSASVGDRVTITCQASQSINNVLAWYQQKPGKAPKLLIYRASTLASGVPSRFSGSG SGTDFTLTISSLQPEDFATYYCQSSYGNYGDFGCGTKVTVLGGGGGSEVQLVESG GGLVQPGGSLRLSCAASGFSFNSDYWIYWVRQAPGKGLEWIASIYGGSSGNTQY ASWAQGRFTISRDNSKNTVYLQMNSLRAEDTAVYFCARGYVDYGGATDLWGQ GTLVTVSSGGGGSGGGGSIQMTQSPSSLSASVGDRVTITCQSSESVYSNNQLSWY QQKPGQPPKLLIYDASDLASGVPSRFSGSGSGTDFTLTISSLQPEDFATYYCAGGFS SSSDTAFGGGTKLTVLGGGGGSGGGGSGGGGSGGGGSEVQLVESGGGLVQPG GSLRLSCAASGFSLSSNAMGWVRQAPGKGLEYIGIISVGGFTYYASWAKGRFTISR DNSKNTVYLQMNSLRAEDTATYFCARDRHGGDSSGAFYLWGQGTLVTVSS |

| **PRO1482** (37-20-B03 sc09.1 scDb-i/38-02-A04 sc13 scDb-o//19-01-H04 sc03 scFv) | | |
|---|---|---|
| SEQ ID NO: 120 | scDb-scFv | DIQMTQSPSSLSASVGDRVTITCQASQSINNVLAWYQQKPGKAPKLLIYRASTLAS GVPSRFSGSGSGTDFTLTISSLQPEDFATYYCQSSYGNYGDFGCGTKVTVLGGGG GSEVQLVESGGGLVQPGGSLRLSCAASGFSFNSDYWIYWVRQAPGKGLEWIASIY GGSSGNTQYASWAQGRFTISRDNSKNTVYLQMNSLRAEDTAVYFCARGYVDYG GATDLWGQGTLVTVSSGGGGSGGGGSGGGGSGGGGSIQMTQSPASLSASVGD RVTITCQASQSIGTYLAWYQQKPGKPPKLLIYRAFILASGVPSRFSGSGSGTDFTLT ISSLQPEDFATYYCQSNFYSDSTTIGPNAFGTGTKVTVLGGGGGSEVQLVESGGG LVQPGGSLRLSCAASGFSFSNSYWICWVRQAPGKCLEWIGCTFVGSSDSTYYAN WAKGRFTISRDNSKNTVYLQMNSLRAEDTAVYYCARHPSDAVYGYANNLWGQ GTLVTVSSGGGGSGGGGSIQMTQSPSSLSASVGDRVTITCQSSESVYSNNQLSWY QQKPGQPPKLLIYDASDLASGVPSRFSGSGSGTDFTLTISSLQPEDFATYYCAGGFS SSSDTAFGGGTKLTVLGGGGGSGGGGSGGGGSGGGGSEVQLVESGGGLVQPG |
| | | GSLRLSCAASGFSLSSNAMGWVRQAPGKGLEYIGIISVGGFTYYASWAKGRFTISR DNSKNTVYLQMNSLRAEDTATYFCARDRHGGDSSGAFYLWGQGTLVTVSS |

| **PR01431** (38-02-A04 sc13 seDb-i/33-03-G02 sc 18 scDb-o/19-01-H04 sc03 scFv) | | |
|---|---|---|
| SEQ ID NO: 121 | scDb-scFv | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG GGSEVQLVESGGGLVQPGGSLRLSCAASGFSFSNSYWICWVRQAPGKCLEWIGC TFVGSSDSTYYANWAKGRFTISRDNSKNTVYLQMNSLRAEDTAVYYCARHPSDA VYGYANNLWGQGTLVTVSSGGGGSGGGGSGGGGSGGGGSIQMTQSPSSLSAS VGDRVTITCQASQSINNVLAWYQQKPGKAPKLLIYRASTLASGVPSRFSGSGSGT DFTLTISSLQPEDFATYYCQSSYGNYGDFGCGTKVTVLGGGGGSQVQLQESGPGL VKPSETLSLTCKASGFSFSSGYDMCWVRQPPGKGLEWIACVVAGSVDITYYASW AKGRVTISKDSSKNQVSLKLSSVTAADTAVYYCARKDAYSDAFNLWGQGTLVT VSSGGGGSGGGGSIQMTQSPSSLSASVGDRVTITCQSSESVYSNNQLSWYQQKP GQPPKLLIYDASDLASGVPSRFSGSGSGTDFTLTISSLQPEDFATYYCAGGFSSSSD TAFGGGTKLTVLGGGGGSGGGGSGGGGSGGGGSEVQLVESGGGLVQPGGSLR LSCAASGFSLSSNAMGWVRQAPGKGLEYIGIISVGGFTYYASWAKGRFTISRDNS KNTVYLQMNSLRAEDTATYFCARDRHGGDSSGAFYLWGQGTLVTVSS |

| **PR01473** (38-02-A04 sc13 scDb-i/33-03-G02 sc c03 scDb-o/19-01-H04 sc03 scFv) | | |
|---|---|---|
| SEQ ID NO: 122 | scDb-scFv | DFQLTQSPSSLSASVGDRVTITCQASQSINDYLAWYQQKPGKSPKLLIYKASTLAS GVPSRFSGSGSGTDFTLTISSLQPEDFATYYCQQGYIITDIDNVFGTGTKVTVLGGG GGSEVQLVESGGGLVQPGGSLRLSCAASGFSFSNSYWICWVRQAPGKCLEWIGC TFVGSSDSTYYANWAKGRFTISRDNSKNTVYLQMNSLRAEDTAVYYCARHPSDA VYGYANNLWGQGTLVTVSSGGGGSGGGGSGGGGSGGGGSIQMTQSPSSLSAS VGDRVTITCQASQSINNVLAWYQQKPGKAPKLLIYRASTLASGVPSRFSGSGSGT DFTLTISSLQPEDFATYYCQSSYGNYGDFGCGTKVTVLGGGGGSQSQLQESGPGL VKPSETLSLTCKASGFSFSSGYDMCWVRQPPGKGLEWIACVVAGSVDITYYASW AKGRVTISKDSSKNQVSLKLSSVTAADTAVYFCARKDAYSDAFNLWGQGTLVTV SSGGGGSGGGGSIQMTQSPSSLSASVGDRVTITCQSSESVYSNNQLSWYQQKPG QPPKLLIYDASDLASGVPSRFSGSGSGTDFTLTISSLQPEDFATYYCAGGFSSSSDT AFGGGTKLTVLGGGGGSGGGGSGGGGSGGGGSEVQLVESGGGLVQPGGSLRL SCAASGFSLSSNAMGWVRQAPGKGLEYIGIISVGGFTYYASWAKGRFTISRDNSK NTVYLQMNSLRAEDTATYFCARDRHGGDSSGAFYLWGQGTLVTVSS |

| **PRO1476** (33-03-G02 sc03 scDb-i/38-02-A04 sc c13 scDb-o/19-01-H04 sc03 scFv) | | |
|---|---|---|
| SEQ ID NO: 123 | scDb-scFv | DIQMTQSPSSLSASVGDRVTITCQASQSINNVLAWYQQKPGKAPKLLIYRASTLAS GVPSRFSGSGSGTDFTLTISSLQPEDFATYYCQSSYGNYGDFGCGTKVTVLGGGG GSQSQLQESGPGLVKPSETLSLTCKASGFSFSSGYDMCWVRQPPGKGLEWIACVV AGSVDITYYASWAKGRVTISKDSSKNQVSLKLSSVTAADTAVYFCARKDAYSDA FNLWGQGTLVTVSSGGGGSGGGGSGGGGSGGGGSFQLTQSPSSLSASVGDRVT ITCQASQSINDYLAWYQQKPGKSPKLLIYKASTLASGVPSRFSGSGSGTDFTLTISS LQPEDFATYYCQQGYIITDIDNVFGTGTKVTVLGGGGGSEVQLVESGGGLVQPG GSLRLSCAASGFSFSNSYWICWVRQAPGKCLEWIGCTFVGSSDSTYYANWAKGR FTISRDNSKNTVYLQMNSLRAEDTAVYYCARHPSDAVYGYANNLWGQGTLVTV SSGGGGSGGGGSIQMTQSPSSLSASVGDRVTITCQSSESVYSNNQLSWYQQKPG QPPKLLIYDASDLASGVPSRFSGSGSGTDFTLTISSLQPEDFATYYCAGGFSSSSDT AFGGGTKLTVLGGGGGSGGGGSGGGGSGGGGSEVQLVESGGGLVQPGGSLRL SCAASGFSLSSNAMGWVRQAPGKGLEYIGIISVGGFTYYASWAKGRFTISRDNSK NTVYLQMNSLRAEDTATYFCARDRHGGDSSGAFYLWGQGTLVTVSS |

| **PRO1432** (33-03-G02 sc18 scDb-i138-02-A04 sc 13 scDb-o/19-01-H04 sc03 scFv) | | |
|---|---|---|
| SEQ ID NO: 124 | scDb-scFv | DIQMTQSPSSLSASVGDRVTITCQASQSINNVLAWYQQKPGKAPKLLIYRASTLAS GVPSRFSGSGSGTDFTLTISSLQPEDFATYYCQSSYGNYGDFGCGTKVTVLGGGG GSQVQLQESGPGLVKPSETLSLTCKASGFSFSSGYDMCWVRQPPGKGLEWIACV VAGSVDITYYASWAKGRVTISKDSSKNQVSLKLSSVTAADTAVYYCARKDAYSD AFNLWGQGTLVTVSSGGGGSGGGGSGGGGSGGGGSIQMTQSPSSLSASVGDRV TITCQASQSINDYLAWYQQKPGKAPKLLIYKASTLASGVPSRFSGSGSGTDFTLTIS SLQPEDFATYYCQQGYIITDIDNVFGTGTKVTVLGGGGGSEVQLVESGGGLVQPG GSLRLSCAASGFSFSNSYWICWVRQAPGKCLEWIGCTFVGSSDSTYYANWAKGR FTISRDNSKNTVYLQMNSLRAEDTAVYYCARHPSDAVYGYANNLWGQGTLVTV SSGGGGSGGGGSIQMTQSPSSLSASVGDRVTITCQSSESVYSNNQLSWYQQKPG QPPKLLIYDASDLASGVPSRFSGSGSGTDFTLTISSLQPEDFATYYCAGGFSSSSDT AFGGGTKLTVLGGGGGSGGGGSGGGGSGGGGSEVQLVESGGGLVQPGGSLRL SCAASGFSLSSNAMGWVRQAPGKGLEYIGIISVGGFTYYASWAKGRFTISRDNSK NTVYLQMNSLRAEDTATYFCARDRHGGDSSGAFYLWGQGTLVTVSS |

| **PRO1480** (38-27-A11 sc02 scDb-i/37-20-B03 sc 09.1 scDb-o/19-01-H04 sc03 scFv) | | |
|---|---|---|
| SEQ ID NO: 125 | scDb-scFv | DIQMTQSPASLSASVGDRVTITCQASQSIGTYLAWYQQKPGKPPKLLIYRAFILAS |
| | | GVPSRFSGSGSGTDFTLTISSLQPEDFATYYCQSNFYSDSTTIGPNAFGTGTKVTVL GGGGGSEVQLVESGGGLVQPGGSLRLSCAASGFSFSANYYPCWVRQAPGKGLE WIGCIYGGSSDITYDANWTKGRFTISRDNSKNTVYLQMNSLRAEDTAVYYCARS AWYSGWGGDLWGQGTLVTVSSGGGGSGGGGSGGGGSGGGGSIQMTQSPSSLS ASVGDRVTITCQASQSISNRLAWYQQKPGKAPKLLIYSASTLASGVPSRFSGSGSG TDFTLTISSLQPEDFATYYCQSTYYGNDGNAFGTGTKVTVLGGGGGSEVQLVES GGGLVQPGGSLRLSCAASGFSFNSDYWIYWVRQAPGKGLEWIASIYGGSSGNTQ YASWAQGRFTISRDNSKNTVYLQMNSLRAEDTAVYFCARGYVDYGGATDLWG QGTLVTVSSGGGGSGGGGSIQMTQSPSSLSASVGDRVTITCQSSESVYSNNQLSW YQQKPGQPPKLLIYDASDLASGVPSRFSGSGSGTDFTLTISSLQPEDFATYYCAGG FSSSSDTAFGGGTKLTVLGGGGGSGGGGSGGGGSGGGGSEVQLVESGGGLVQP GGSLRLSCAASGFSLSSNAMGWVRQAPGKGLEYIGIISVGGFTYYASWAKGRFTI SRDNSKNTVYLQMNSLRAEDTATYFCARDRHGGDSSGAFYLWGQGTLVTVSS |

| **PRO1481** (38-27-A11 sc03 scDb-i/37-20-B03 sc 09.1 scDb-o/19-01-H04 sc03 scFv) | | |
|---|---|---|
| SEQ ID NO: 126 | scDb-scFv | DIQMTQSPASLSASVGDRVTITCQASQSIGTYLAWYQQKPGKPPKLLIYRAFILAS GVPSRFSGSGSGTDFTLTISSLQPEDFATYYCQSNFYSDSTTIGPNAFGTGTKVTVL GGGGGSESQLVESGGGLVQPGGSLRLSCAASGFSFSANYYPCWVRQAPGKGLE WIGCIYGGSSDITYDANWTKGRFTISRDNSKNTVYLQMNSLRAEDTAVYFCARSA WYSGWGGDLWGPGTLVTVSSGGGGSGGGGSGGGGSGGGGSFQLTQSPSSLSA SVGDRVTITCQASQSISNRLAWYQQKPGKPPKLLIYSASTLASGVPSRFSGSGSGT DFTLTISSLQPEDFATYYCQSTYYGNDGNAFGTGTKVTVLGGGGGSEVQLVESG GGLVQPGGSLRLSCAASGFSFNSDYWIYWVRQAPGKGLEWIASIYGGSSGNTQY ASWAQGRFTISRDNSKNTVYLQMNSLRAEDTAVYFCARGYVDYGGATDLWGQ GTLVTVSSGGGGSGGGGSIQMTQSPSSLSASVGDRVTITCQSSESVYSNNQLSWY QQKPGQPPKLLIYDASDLASGVPSRFSGSGSGTDFTLTISSLQPEDFATYYCAGGFS SSSDTAFGGGTKLTVLGGGGGSGGGGSGGGGSGGGGSEVQLVESGGGLVQPG GSLRLSCAASGFSLSSNAMGWVRQAPGKGLEYIGIISVGGFTYYASWAKGRFTISR DNSKNTVYLQMNSLRAEDTATYFCARDRHGGDSSGAFYLWGQGTLVTVSS |

| **PRO1480dis** (38-27-A11 sc 07/scDb-i/37-20-/-B03 sc09.1 scDB-o/19-01-H04 sc03 scFv) | | |
|---|---|---|
| SEQ ID NO: 127 | scDb-scFv | DIQMTQSPASLSASVGDRVTITCQASQSIGTYLAWYQQKPGKPPKLLIYRAFILAS GVPSRFSGSGSGTDFTLTISSLQPEDFATYYCQSNFYSDSTTIGPNAFGTGTKVTVL GGGGGSEVQLVESGGGLVQPGGSLRLSCAASGFSFSANYYPCWVRQAPGKCLE |
| | | WIGCIYGGSSDITYDANWTKGRFTISRDNSKNTVYLQMNSLRAEDTAVYYCARS AWYSGWGGDLWGQGTLVTVSSGGGGSGGGGSGGGGSGGGGSIQMTQSPSSLS ASVGDRVTITCQASQSISNRLAWYQQKPGKAPKLLIYSASTLASGVPSRFSGSGSG TDFTLTISSLQPEDFATYYCQSTYYGNDGNAFGCGTKVTVLGGGGGSEVQLVES GGGLVQPGGSLRLSCAASGFSFNSDYWIYWVRQAPGKGLEWIASIYGGSSGNTQ YASWAQGRFTISRDNSKNTVYLQMNSLRAEDTAVYFCARGYVDYGGATDLWG QGTLVTVSSGGGGSGGGGSIQMTQSPSSLSASVGDRVTITCQSSESVYSNNQLSW YQQKPGQPPKLLIYDASDLASGVPSRFSGSGSGTDFTLTISSLQPEDFATYYCAGG FSSSSDTAFGGGTKLTVLGGGGGSGGGGSGGGGSGGGGSEVQLVESGGGLVQP GGSLRLSCAASGFSLSSNAMGWVRQAPGKGLEYIGIISVGGFTYYASWAKGRFTI SRDNSKNTVYLQMNSLRAEDTATYFCARDRHGGDSSGAFYLWGQGTLVTVSS |

| **PRO1599** (38-02-A04 sc13 scDb-i/37-20-B03 sc 01 scDb-o/23-13-A01 sc02 scFv) | | |
|---|---|---|
| SEQ ID NO: 128 | scDb-scFv | MTQSPSSLSASVGDRVTITCQASQSIGTYLAWYQQKPGKAPKLLIYRAFILASGVP SRFSGSGSGTDFTLTISSLQPEDFATYYCQSNFYSDSTTIGPNAFGTGTKVTVLGGG GGSEVQLVESGGGLVQPGGSLRLSCAASGFSFSNSYWICWVRQAPGKCLEWIGCT FVGSSDSTYYANWAKGRFTISRDNSKNTVYLQMNSLRAEDTAVYYCARHPSDAV YGYANNLWGQGTLVTVSSGGGGSGGGGSGGGGSGGGGSIQMTQSPSSLSASVG DRVTITCQASQSINNVLAWYQQKPGKAPKLLIYRASTLASGVPSRFSGSGSGTDFT LTISSLQPEDFATYYCQSSYGNYGDFGCGTKVTVLGGGGGSQVQLQESGPGLVKP SETLSLTCKVSGFSFNSDYWIYWIRQPPGKGLEWIGSIYGGSSGNTQYASWAQGR VTISVDSSKNQFSLKLSSVTAADTAVYYCARGYVDYGGATDLWGQGTLVTVSSG GGGSGGGGSVVMTQSPSSLSASVGDRVTITCQASQIISSRSAWYQQKPGQPPKLLI YQASKLASGVPSRFSGSGSGTDFTLTISSLQPEDFATYYCQCTYIDSNFGAFGGGT KLTVLGGGGGSGGGGSGGGGSGGGGSEVQLVESGGGLVQPGGSLRLSCAASGFS FSSSYWICWVRQAPGKGLEWVGCVFTGDGTTYYASWAKGRFTISRDNSKNTVYL QMNSLRAEDTATYFCARPVSVYYYGMDLWGQGTLVTVSSLESRGPV |

| **PR01600** (38-02-A04 sc13 scDb-i/37-20-B03 sc 09.1 scDb-o/23-13-A01 sc02 scFv) | | |
|---|---|---|
| SEQ ID NO: 129 | scDb-scFv | dIQMTQSPSSLSASVGDRVTITCQASQSIGTYLAWYQQKPGKAPKLLIYRAFILASG VPSRFSGSGSGTDFTLTISSLQPEDFATYYCQSNFYSDSTTIGPNAFGTGTKVTVLG GGGGSEVQLVESGGGLVQPGGSLRLSCAASGFSFSNSYWICWVRQAPGKCLEWI GCTFVGSSDSTYYANWAKGRFTISRDNSKNTVYLQMNSLRAEDTAVYYCARHPS DAVYGYANNLWGQGTLVTVSSGGGGSGGGGSGGGGSGGGGSIQMTQSPSSLSA |
| | | SVGDRVTITCQASQSINNVLAWYQQKPGKAPKLLIYRASTLASGVPSRFSGSGSGT DFTLTISSLQPEDFATYYCQSSYGNYGDFGCGTKVTVLGGGGGSQVQLQESGPGL VKPSETLSLTCKVSGFSFNSDYWIYWIRQPPGKGLEWIGSIYGGSSGNTQYASWA QGRVTISVDSSKNQFSLKLSSVTAADTAVYYCARGYVDYGGATDLWGQGTLVT VSSGGGGSGGGGSVVMTQSPSSLSASVGDRVTITCQASQIISSRSAWYQQKPGQPP KLLIYQASKLASGVPSRFSGSGSGTDFTLTISSLQPEDFATYYCQCTYIDSNFGAFG GGTKLTVLGGGGGSGGGGSGGGGSGGGGSEVQLVESGGGLVQPGGSLRLSCAAS GFSFSSSYWICWVRQAPGKGLEWVGCVFTGDGTTYYASWAKGRFTISRDNSKNT VYLQMNSLRAEDTATYFCARPVSVYYYGMDLWGQGTLVTVSS |

| **PRO1601** (38-27-A11 sc02 scDb-i/37-20-B03 sc 09.1 scDb-o/23-13-A01 sc02 scFv) | | |
|---|---|---|
| SEQ ID NO: 130 | scDb-scFv | DIQMTQSPASLSASVGDRVTITCQASQSIGTYLAWYQQKPGKPPKLLIYRAFILAS GVPSRFSGSGSGTDFTLTISSLQPEDFATYYCQSNFYSDSTTIGPNAFGTGTKVTVL GGGGGSEVQLVESGGGLVQPGGSLRLSCAASGFSFSANYYPCWVRQAPGKGLE WIGCIYGGSSDITYDANWTKGRFTISRDNSKNTVYLQMNSLRAEDTAVYYCARS AWYSGWGGDLWGQGTLVTVSSGGGGSGGGGSGGGGSGGGGSIQMTQSPSSLSA SVGDRVTITCQASQSISNRLAWYQQKPGKAPKLLIYSASTLASGVPSRFSGSGSGT DFTLTISSLQPEDFATYYCQSTYYGNDGNAFGTGTKVTVLGGGGGSEVQLVESGG GLVQPGGSLRLSCAASGFSFNSDYWIYWVRQAPGKGLEWIASIYGGSSGNTQYAS WAQGRFTISRDNSKNTVYLQMNSLRAEDTAVYFCARGYVDYGGATDLWGQGTL VTVSSGGGGSGGGGSVVMTQSPSSLSASVGDRVTITCQASQIISSRSAWYQQKPG QPPKLLIYQASKLASGVPSRFSGSGSGTDFTLTISSLQPEDFATYYCQCTYIDSNFG AFGGGTKLTVLGGGGGSGGGGSGGGGSGGGGSEVQLVESGGGLVQPGGSLRLSC AASGFSFSSSYWICWVRQAPGKGLEWVGCVFTGDGTTYYASWAKGRFTISRDNS KNTVYLQMNSLRAEDTATYFCARPVSVYYYGMDLWGQGTLVTVSS |

**Table 6. Examples of CD3 binding domains as used in the present invention (CDR residues shown in bold and italic letters).**

| **SEQ ID NUMBER:** | **Ab region** | **Sequence** |
|---|---|---|
| **28-21-D09 sc04** | | |
| SEQ ID NO: 131 | **HCDR1** (H27-H42; AHo numbering) | GFSLSSYDMS |
| SEQ ID NO: 132 | **HCDR2** (H57-H76; AHo numbering) | ASYASGPTYYASWAKG |
| SEQ ID NO: 133 | **HCDR3** (H108-H138; AHo numbering) | RGGWTGTSHSNI |
| SEQ ID NO: 134 | **VH** | EVQLVESGGGLVQPGGSLRLSCAASGFSLSSYDMSWVRQAPGKGLAWIGAS YASGPTYYASWAKGRFTISRDNSKNTVYLQMNSLRAEDTATYFCARGGWTGT SHSNIWGQGTLVTVSS |
| SEQ ID NO: 135 | **LCDR1** (L24-L42; AHo numbering) (Kabat definition) | QSSQSVFSNNYLA |
| SEQ ID NO: 136 | **LCDR2** (L58-L72; AHo numbering) (Kabat definition) | SASTLAS |
| SEQ ID NO: 137 | **LCDR3** (L107-L138; AHo numbering) (Kabat definition) | LGSYACSSADCYV |
| SEQ ID NO: 138 | **VL** (28-21-D09 sc04) | DIQMTQSPSSLSASVGDRVTITCQSSQSVFSNNYLAWFQQKPGQSPKRLIYSAS TLASGVPSRFSGSGSGTDFTLTISSLQPEDFATYYCLGSYACSSADCYVFGTGT KVTVLG |

**Table 7. Examples of MSLN binding domains of the present invention (CDR residues shown in bold and italic letters).**

| **SEQ ID NUMBER:** | **Description** | **Sequence** |
|---|---|---|
| **54-22-H03-sc01 (h igh affinity)** | | |
| SEQ ID NO: 139 | **HCDR1** (H27-H42; AHo numbering) | GISVSNDYYMC |
| SEQ ID NO: 140 | **HCDR2** (H57-H76; AHo numbering) | CISTYIGNTHYASWAKG |
| SEQ ID NO: 141 | **HCDR3** (H108-H138; AHo numbering) | KNAGYPGYRYAIDL |
| SEQ ID NO: 142 | **VH** (PRO 1795) | EVQLVESGGGLVQPGGSLRLSCAASGISVSNDYYMCWVRQAPGKGL |
| | | EWIGCISTYIGNTHYASWAKGRFTISRDNSKNTVYLQMNSLRAEDTA VYYCAKNAGYPGYRYAIDLWGQGTLVTVSS |
| SEQ ID NO: 143 | **LCDR1** (L24-L42; AHo numbering) (Kabat definition) | QASESIGNYLA |
| SEQ ID NO: 144 | **LCDR2** (L58-L72; AHo numbering) (Kabat definition) | SASTLAS |
| SEQ ID NO: 145 | **LCDR3** (L107-L138; AHo numbering) (Kabat definition) | QSTDYGDSYI |
| SEQ ID NO: 146 | **VL** (PRO 1795) | DIQMTQSPSSLSASVGDRVTITCQASESIGNYLAWYQQKPGKAPKLLI YSASTLASGVPSRFSGSGSGTDFTLTISSLQPEDFATYYCQSTDYGDSY IFGTGTKVTVLG |

| **54-01-G02 (low affinity)** | | |
|---|---|---|
| SEQ ID NO: 147 | **HCDR1** (H27-H42; AHo numbering) | GFSLSSYAMG |
| SEQ ID NO: 148 | **HCDR2** (H57-H76; AHo numbering) | YISTINNTYYASWAKG |
| SEQ ID NO: 149 | **HCDR3** (H108-H138; AHo numbering) | REIRSGWVDYGFSI |
| SEQ ID NO: 150 | **VH** 54-01-G02-sc01 (PRO1783) | QVQLVESGGGLVQPGGSLRLSCAASGFSLSSYAMGWVRQAPGKGLE WIGYISTINNTYYASWAKGRFTISRDNSKNTVYLQMNSLRAEDTAVY YCAREIRSGWVDYGFSIWGQGTLVTVSS |
| SEQ ID NO: 151 | **HCDR2** 54-01-G02 N62A (H57-H76; AHo numbering) | YISTIANTYYASWAKG |
| SEQ ID NO: 152 | **VH** 54-01-G02-sc03; mutation: N62A (PRO2197) | QVQLVESGGGLVQPGGSLRLSCAASGFSLSSYAMGWVRQAPGKGLE WIGYISTIANTYYASWAKGRFTISRDNSKNTVYLQMNSLRAEDTAVY YCAREIRSGWVDYGFSIWGQGTLVTVSS |
| SEQ ID NO: 153 | **LCDR1** (L24-L42; AHo numbering) (Kabat definition) | QASQNIYSNLA |
| SEQ ID NO: 154 | **LCDR2** (L58-L72; AHo numbering) (Kabat definition) | DASDLAS |
| SEQ ID NO: 155 | **LCDR3** (L107-L138; AHo numbering) (Kabat definition) | QQVRSSSDIDNP |
| SEQ ID NO: 156 | **VL** 54-01-G02-sc01 (PRO1783 and PRO2197) | DIQMTQSPSSLSASVGDRVTITCQASQNIYSNLAWYQQKPGKAPKLLI YDASDLASGVPSRFSGSGSGTDFTLTISSLQPEDFATYYCQQVRSSSDI DNPFGTGTKVTVLG |

| **54-32-A07 (low affinity)** | | |
|---|---|---|
| SEQ ID NO: 157 | **HCDR1** (H27-H42; AHo numbering) | GFSLSSYAMG |
| SEQ ID NO: 158 | **HCDR2** (H57-H76; AHo numbering) | YISKIGTTYYASWAKG |
| SEQ ID NO: 159 | **HCDR3** (H108-H138; AHo numbering) | RGSSSGGYLDDGFDP |
| SEQ ID NO: 160 | **VH** 54-32-A07-sc02 (PRO 1925) | QSQLVESGGGLVQPGGSLRLSCAVSGFSLSSYAMGWVRQAPGKGLE YIGYISKIGTTYYASWAKGRFTISKDNSKNTVYLQMNSLRAEDTAVY FCARGSSSGGYLDDGFDPWGQGTLVTVSS |
| SEQ ID NO: 161 | **LCDR1** (L24-L42; AHo numbering) (Kabat definition) | QASQSISNYLA |
| SEQ ID NO: 162 | **LCDR2** (L58-L72; AHo numbering) (Kabat definition) | DASDLAS |
| SEQ ID NO: 163 | **LCDR3** (L107-L138; AHo numbering) (Kabat definition) | QQVYDSNNVENV |
| SEQ ID NO: 164 | **VL** 54-32-A07-sc02 (PRO 1925) | ALQMTQSPSSLSASVGDRVTITCQASQSISNYLAWYQQKPGKPPKFLI YDASDLASGVSSRFSGSGSGTDFTLTISSLQPEDFATYYCQQVYDSNN VENVFGTGTKVTVLG |

**Table 8. Examples of HER2 binding domains as used in the present invention (CDR residues shown in bold and italic letters).**

| **SEQ ID NUMBER:** | **Ab region** | **Sequence** |
|---|---|---|
| **HER2-Trastuzum** | **ab** | |
| SEQ ID NO: 165 | **HCDR1 _{Trastuzumab}** (H27-H42; AHo numbering) | GFNIKDTYIH |
| SEQ ID NO: 166 | **HCDR2 _{Trastuzumab}** (H57-H76; AHo numbering) | RIYPTNGYTRYADSVKG |
| SEQ ID NO: 167 | **HCDR3 _{Trastuzumab}** (H108-H138; AHo numbering) | RWGGDGFYAMDY |
| SEQ ID NO: 168 | **VH _{Trastuzumab}** HER2-Trastuzumab scFv (PRO 1705) | |
| SEQ ID NO: 169 | **VH _{Trastuzumab (G51C)}** Her2-Trastuzumab-diS scFv (PRO 1706) | EVQLVESGGGLVQPGGSLRLSCAASGFNIKDTYIHWVRQAPGKCLEWVARI YPTNGYTRYADSVKGRFTISADTSKNTAYLQMNSLRAEDTAVYYCSRWGG DGFYAMDYWGQGTLVTVSS |
| SEQ ID NO: 170 | **LCDR1 _{Trastuzumab}** (L24-L42; AHo numbering) (Kabat definition) | RASQDVNTAVA |
| SEQ ID NO: 171 | **LCDR2 _{Trastuzumab}** (L58-L72; AHo numbering) (Kabat definition) | SASFLYS |
| SEQ ID NO: 172 | **LCDR3 _{Trastuzumab}** (L107-L138; AHo numbering) (Kabat definition) | QQHYTTPPT |
| SEQ ID NO: 173 | **VL _{Trastuzumab (λ-capped)}** HER2-Trastuzumab scFv (PRO 1705) | DIQMTQSPSSLSASVGDRVTITCRASQDVNTAVAWYQQKPGKAPKLLIYSAS FLYSGVPSRFSGSRSGTDFTLTISSLQPEDFATYYCQQHYTTPPTFGGGTKLT |
| | | VLG |
| SEQ ID NO: 174 | **VL _{Trastuzumab (G141C) (λ-capped)}** Her2-Trastuzumab-diS scFv (PRO 1706) | DIQMTQSPSSLSASVGDRVTITCRASQDVNTAVAWYQQKPGKAPKLLIYSAS FLYSGVPSRFSGSRSGTDFTLTISSLQPEDFATYYCQQHYTTPPTFGCGTKLT VLG |

**Table 9. Examples of multispecific antibodies (MA2) as used in the present invention (Linkers are shown in bold).**

| **SEQ ID NUMBER** | **Ab Format** | **Sequence** |
|---|---|---|
| **PRO1872 *(MSLNx C D3 x hSA)*** (54-22-H03-s c01 scFv/28-21-D09-sc04 scDb-i/23-13-A01-sc02 diS scDb-o) | | |
| SEQ ID NO: 175 | scDb-scFv | DVVMTQSPSSLSASVGDRVTITCQASQIISSRSAWYQQKPGQPPKLLIYQAS KLASGVPSRFSGSGSGTDFTLTISSLQPEDFATYYCQCTYIDSNFGAFGCGTK LTVLGGGGGSEVQLVESGGGLVQPGGSLRLSCAASGFSLSSYDMSWVRQA PGKGLAWIGASYASGPTYYASWAKGRFTISRDNSKNTVYLQMNSLRAEDT ATYFCARGGWTGTSHSNIWGQGTLVTVSSGGGGSGGGGSGGGGSGGGGSD IQMTQSPSSLSASVGDRVTITCQSSQSVFSNNYLAWFQQKPGQSPKRLIYSAS TLASGVPSRFSGSGSGTDFTLTISSLQPEDFATYYCLGSYACSSADCYVFGTG TKVTVLGGGGGSEVQLVESGGGLVQPGGSLRLSCAASGFSFSSSYWICWVR QAPGKCLEWVGCVFTGDGTTYYASWAKGRFTISRDNSKNTVYLQMNSLRA EDTATYFCARPVSVYYYGMDLWGQGTLVTVSSGGGGSGGGGSDIQMTQSP SSLSASVGDRVTITCQASESIGNYLAWYQQKPGKAPKLLIYSASTLASGVPS RFSGSGSGTDFTLTISSLQPEDFATYYCQSTDYGDSYIFGTGTKVTVLGGGG GSGGGGSGGGGSGGGGSEVQLVESGGGLVQPGGSLRLSCAASGISVSNDYY MCWVRQAPGKGLEWIGCISTYIGNTHYASWAKGRFTISRDNSKNTVYLQM NSLRAEDTAVYYCAKNAGYPGYRYAIDLWGQGTLVTVSS |

| **PR02000 (*biMSLN_{low affinity} x CD3 x hSA)*** (54-01-G02-sc01_(G₄S)₂_28-21-D09-sc04 _(G₃S)₂_23-13-A01-sc02 diS VH/54-01-G02-sc01 (G₄S)₂ 23-13-A01-sc02 diS (G₂S)₂ 28-21-D09-sc04 VL) | | |
|---|---|---|
| SEQ ID NO: 176 | MATCH4 (CHAIN_1) | DIQMTQSPSSLSASVGDRVTITCQASQNIYSNLAWYQQKPGKAPKLLIYDAS DLASGVPSRFSGSGSGTDFTLTISSLQPEDFATYYCQQVRSSSDIDNPFGTGT KVTVLGGGGGSGGGGSGGGGSGGGGSQVQLVESGGGLVQPGGSLRLSCAA SGFSLSSYAMGWVRQAPGKGLEWIGYISTINNTYYASWAKGRFTISRDNSK NTVYLQMNSLRAEDTAVYYCAREIRSGWVDYGFSIWGQGTLVTVSSGGGG SGGGGSEVQLVESGGGLVQPGGSLRLSCAASGFSLSSYDMSWVRQAPGKG |
| | | LAWIGASYASGPTYYASWAKGRFTISRDNSKNTVYLQMNSLRAEDTATYFC ARGGWTGTSHSNIWGQGTLVTVSSGGGSGGGSEVQLVESGGGLVQPGGSL RLSCAASGFSFSSSYWICWVRQAPGKCLEWVGCVFTGDGTTYYASWAKGR FTISRDNSKNTVYLQMNSLRAEDTATYFCARPVSVYYYGMDLWGQGTLVT VSS |
| SEQ ID NO: 177 | MATCH4 (CHAIN_2) | DIQMTQSPSSLSASVGDRVTITCQASQNIYSNLAWYQQKPGKAPKLLIYDAS DLASGVPSRFSGSGSGTDFTLTISSLQPEDFATYYCQQVRSSSDIDNPFGTGT KVTVLGGGGGSGGGGSGGGGSGGGGSQVQLVESGGGLVQPGGSLRLSCAA SGFSLSSYAMGWVRQAPGKGLEWIGYISTINNTYYASWAKGRFTISRDNSK NTVYLQMNSLRAEDTAVYYCAREIRSGWVDYGFSIWGQGTLVTVSSGGGG SGGGGSDVVMTQSPSSLSASVGDRVTITCQASQIISSRSAWYQQKPGQPPKL LIYQASKLASGVPSRFSGSGSGTDFTLTISSLQPEDFATYYCQCTYIDSNFGAF GCGTKLTVLGGGSGGSDIQMTQSPSSLSASVGDRVTITCQSSQSVFSNNYLA WFQQKPGQSPKRLIYSASTLASGVPSRFSGSGSGTDFTLTISSLQPEDFATYY CLGSYACSSADCYVFGTGTKVTVLG |

| **PRO2100 (*biMSLN_{low affinity} x CD3 x hSA)*** (54-01-G02-sc03_(G₄S)₂_28-21-D09-sc04_(G₃S)₂_23-13-A01-sc02 diS VH/54-01-G02-sc03 (G₄S)₂ 23-13-A01-sc02 diS (G₂S)₂ 28-21-D09-sc04 VL) | | |
|---|---|---|
| SEQ ID NO: 178 | MATCH4 (CHAIN_1) | DIQMTQSPSSLSASVGDRVTITCQASQNIYSNLAWYQQKPGKAPKLLIYDAS DLASGVPSRFSGSGSGTDFTLTISSLQPEDFATYYCQQVRSSSDIDNPFGTGT KVTVLGGGGGSGGGGSGGGGSGGGGSQVQLVESGGGLVQPGGSLRLSCAA SGFSLSSYAMGWVRQAPGKGLEWIGYISTIANTYYASWAKGRFTISRDNSK NTVYLQMNSLRAEDTAVYYCAREIRSGWVDYGFSIWGQGTLVTVSSGGGG SGGGGSEVQLVESGGGLVQPGGSLRLSCAASGFSLSSYDMSWVRQAPGKG LAWIGASYASGPTYYASWAKGRFTISRDNSKNTVYLQMNSLRAEDTATYFC ARGGWTGTSHSNIWGQGTLVTVSSGGGSGGGSEVQLVESGGGLVQPGGSL RLSCAASGFSFSSSYWICWVRQAPGKCLEWVGCVFTGDGTTYYASWAKGR FTISRDNSKNTVYLQMNSLRAEDTATYFCARPVSVYYYGMDLWGQGTLVT VSS |
| SEQ ID NO: 179 | MATCH4 (CHAIN_2) | DIQMTQSPSSLSASVGDRVTITCQASQNIYSNLAWYQQKPGKAPKLLIYDAS DLASGVPSRFSGSGSGTDFTLTISSLQPEDFATYYCQQVRSSSDIDNPFGTGT KVTVLGGGGGSGGGGSGGGGSGGGGSQVQLVESGGGLVQPGGSLRLSCAA SGFSLSSYAMGWVRQAPGKGLEWIGYISTIANTYYASWAKGRFTISRDNSK |
| | | NTVYLQMNSLRAEDTAVYYCAREIRSGWVDYGFSIWGQGTLVTVSSGGGG SGGGGSDVVMTQSPSSLSASVGDRVTITCQASQIISSRSAWYQQKPGQPPKL LIYQASKLASGVPSRFSGSGSGTDFTLTISSLQPEDFATYYCQCTYIDSNFGAF GCGTKLTVLGGGSGGSDIQMTQSPSSLSASVGDRVTITCQSSQSVFSNNYLA WFQQKPGQSPKRLIYSASTLASGVPSRFSGSGSGTDFTLTISSLQPEDFATYY CLGSYACSSADCYVFGTGTKVTVLG |

| **PRO1766 *(HER2 x CD3 x hSA)*** (28-21-D09-sc04-scDb-o/19-01-H04-sc03-scDb-i/Her2-diS scFv) | | |
|---|---|---|
| SEQ ID NO: 180 | scDb-scFv | DIQMTQSPSSLSASVGDRVTITCQSSQSVFSNNYLAWFQQKPGQSPKRLIYS ASTLASGVPSRFSGSGSGTDFTLTISSLQPEDFATYYCLGSYACSSADCYVFG TGTKVTVLGGGGGSEVQLVESGGGLVQPGGSLRLSCAASGFSLSSNAMGW VRQAPGKGLEYIGIISVGGFTYYASWAKGRFTISRDNSKNTVYLQMNSLRAE DTATYFCARDRHGGDSSGAFYLWGQGTLVTVSSGGGGSGGGGSGGGGSG GGGSIQMTQSPSSLSASVGDRVTITCQSSESVYSNNQLSWYQQKPGQPPKLL IYDASDLASGVPSRFSGSGSGTDFTLTISSLQPEDFATYYCAGGFSSSSDTAF GGGTKLTVLGGGGGSEVQLVESGGGLVQPGGSLRLSCAASGFSLSSYDMS WVRQAPGKGLAWIGASYASGPTYYASWAKGRFTISRDNSKNTVYLQMNSL RAEDTATYFCARGGWTGTSHSNIWGQGTLVTVSSGGGGSGGGGSIQMTQS PSSLSASVGDRVTITCRASQDVNTAVAWYQQKPGKAPKLLIYSASFLYSGVP SRFSGSRSGTDFTLTISSLQPEDFATYYCQQHYTTPPTFGCGTKLTVLGGGG GSGGGGSGGGGSGGGGSEVQLVESGGGLVQPGGSLRLSCAASGFNIKDTYI HWVRQAPGKCLEWVARIYPTNGYTRYADSVKGRFTISADTSKNTAYLQMN SLRAEDTAVYYCSRWGGDGFYAMDYWGQGTLVTVSS |

| **PRO1767 (*HER2 x CD3_{dis} x hSA)*** (28-21-D09-sc04-diS-scDb-o/19-01-H04-sc03-scDb-i/Her2-diS scFv) | | |
|---|---|---|
| SEQ ID NO: 181 | scDb-scFv | DIQMTQSPSSLSASVGDRVTITCQSSQSVFSNNYLAWFQQKPGQSPKRLIYS ASTLASGVPSRFSGSGSGTDFTLTISSLQPEDFATYYCLGSYACSSADCYVFG CGTKVTVLGGGGGSEVQLVESGGGLVQPGGSLRLSCAASGFSLSSNAMGW VRQAPGKGLEYIGIISVGGFTYYASWAKGRFTISRDNSKNTVYLQMNSLRAE DTATYFCARDRHGGDSSGAFYLWGQGTLVTVSSGGGGSGGGGSGGGGSG GGGSIQMTQSPSSLSASVGDRVTITCQSSESVYSNNQLSWYQQKPGQPPKLL IYDASDLASGVPSRFSGSGSGTDFTLTISSLQPEDFATYYCAGGFSSSSDTAF GGGTKLTVLGGGGGSEVQLVESGGGLVQPGGSLRLSCAASGFSLSSYDMS WVRQAPGKCLAWIGASYASGPTYYASWAKGRFTISRDNSKNTVYLQMNSL |
| | | RAEDTATYFCARGGWTGTSHSNIWGQGTLVTVSSGGGGSGGGGSIQMTQS PSSLSASVGDRVTITCRASQDVNTAVAWYQQKPGKAPKLLIYSASFLYSGVP SRFSGSRSGTDFTLTISSLQPEDFATYYCQQHYTTPPTFGCGTKLTVLGGGG GSGGGGSGGGGSGGGGSEVQLVESGGGLVQPGGSLRLSCAASGFNIKDTYI HWVRQAPGKCLEWVARIYPTNGYTRYADSVKGRFTISADTSKNTAYLQMN SLRAEDTAVYYCSRWGGDGFYAMDYWGQGTLVTVSS |

Throughout the text of this application, should there be a discrepancy between the text of the specification (e. g., Tables 1 to 9) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

### Examples

### Example 1: Assessment of the CD137 agonistic effect of anti-PDL1xCD137 molecules (MA1) by using a cell-based assay of transgenic NF-kB Jurkat reporter cell line expressing CD137:

The ability of the bispecific scDb-based antibodies PRO885, PRO951, PRO1123, PRO1124, PRO1125, PRO1126, PRO1134, and the trispecific scDb-scFv-based antibodies PRO963, PRO966, PRO1057, PRO1058, PRO1175, PRO1186, PRO1430, PRO1479, PRO1482, PRO1431, PRO1432, PRO1473, PRO1476, PRO1480, PRO1481, PRO1480diS, PRO1599, RPO1600, RPO1601, to activate CD137 signaling in Jurkat cells was assessed by using a cell-based assay of transgenic NF-kB Jurkat reporter cell line expressing CD137. The measurements were performed in the presence of PDL1 expressing CHO (clone A2) and HCC827 cells and, as a negative control, in the presence of CHO WT cells without PDL1 expression.

The measurements demonstrated that the bispecific scDb-based antibodies as well as trispecific scDb-scFv-based antibodies activated CD137 signaling more efficient in the presence of PDL1 expressing CHO cells than urelumab. In the absence of PDL1, neither of these antibodies could activate CD137 in reporter cells while urelumab showed activation of CD137 signaling independently of PDL1. The same results could be obtained when the assessment was repeated in the presence of cells expressing lower amounts of PDL1.

Further information and details on the multispecific antibodies MA1 applied in the invention as well as on the binding domains comprised therein, such as:
- their general description,
- their manufacturing,
- their biophysical characterization, such as stability studies and the evaluation of the monomeric content, or
- their complete functional characterization, including the assessment of their antitumor efficacy in the human cell line-derived lung cancer xenograft model HCC827,
are disclosed in detail in the patent application WO 2019/072868, which are herewith incorporated by reference in its entirety, in particular the above-mentioned additional information.

### Example 2: Generation, pharmacodynamic characterization and functional characterization of exemplary anti-MSLN multispecific antibodies MA2:

The Generation, pharmacodynamic characterization and functional characterization of the exemplary anti-MSLN multispecific antibodies PRO1872, PR02000 and PRO2100 are disclosed in detail in the unpublished patent applications EP 20164913.4 (PRO1872) and EP 20177337.1 (PRO2000, PRO2100), which are herewith incorporated by reference. Besides, the cytotoxicity assay (T-cell driven target cell depletion) data disclosed in EP 20177337.1 demonstrate that the bivalent anti-MSLN antibodies PR02000 and PRO2100, which comprise two low affinity MSLN-BDs and one CD3-BD (e.g. biMSLN_{high KD}xCD3xhSA), are capable of killing target cells in an antigen density-dependent manner by taking advantage of avidity effects, which the monovalent anti-MSLN antibody PRO1872 (MSLN_{low KD}xCD3xhSA) could not (Figure 3). Furthermore, the data demonstrates that the potency of PRO2000 and PRO2100 to initiate T-cell driven target cell killing is not much affected, i.e. decreases only by 17-folds, in the presence of 500 ng/ml soluble MSLN (sMSLN) (Figure 4).

### Example 3: Generation, biophysical characterization of exemplary anti-HER2 multispecific antibodies MA2:

The HER2-BD used in the exemplary anti-HER2 multispecific antibodies PRO1766 and PRO1767 are based on the VH and VL CDR sequences of the anti-HER2-BDs of trastuzumab, wherein FW4 of the VL sequence was replaced by the Vλ germline-based FR4 of SEQ-ID NO: 95 (AHo numbering). Furthermore, to generate a scFv construct a linker sequence was introduced between said VH and VL domains. In addition, the glycine residues at positions 51 of the VH sequence and at position 141 in the Vλ-FR4 of the VL sequence (AHo numbering) were replaced by cysteines to allow the formation of an internal disulfide bridge in the final scFv format. Finally, this trastuzumab-based HER2 scFv was combined with the scDB, comprising the CD3-BD (28-21-D09-sc04 or the disulfide stabilized variant 28-21-D09-sc04-diS) and the hSA-BD (19-01-H04-sc03), through a further linker to generate a scMATCH3 format. The sequences of the final anti-(HER2 x CD3diS x hSA) scMATCH3 molecules PRO1766 and PRO1767 are listed in table 9.

The MATCH is a format invented by Numab that consists solely of variable domains connected by different linkers that allow for the specific pairing of matching domain pairs only (Egan TJ et al. Novel multi-specific heterodimeric antibody format allowing modular assembly of variable domain fragments. MAbs. 2016 Oct 27:0. [http://dx.doi.org/10.1080/19420862.2016.1248012]). This format is particularly well suited for the convenient screening of different combinations of antigen binding domains for optimal cooperativity. The MATCH, such as the scMATCH3 and MATCH4, can be expressed recombinantly from mammalian cells. For the purification, a conventional affinity chromatography step can be used.

Anti-(HER2 x CD3diS x hSA) scMATCH3 molecules PRO1766 and PRO1767 were produced from mammalian cells using standard protein production methods, as for example described in the unpublished patent application EP20164913.4.

PRO1766 and in particular PRO1767, when formulated at a concentration of 10 mg/ml in 50 mM phosphate-citrate buffer at pH 6.4 with 150 mM NaCl, exhibit a high midpoint of the unfolding transition (Tm) of above 55°C, as determined by DSF, and also showed excellent stability upon storage over four weeks at 4°C as well as at 25°C with virtually no loss in protein content as well as monomeric content.

### Example 4: In vivo tumor growth inhibition with PR02000 (biMSLN_{hiah KD}xCD3xhSA):

### Introduction

An *in vivo,* mesothelin-expressing cell line xenograft experiment was performed at Charles River Laboratories in order to determine the ability of PRO2000 (biMSLN_{high KD}xCD3xhSA) to effectively control tumor growth relative to control animals.

### Methods

### Animals

Female NCG mice from Charles River Laboratories were bred and housed under conditions suitable for humanized mouse work. Animals were used between 8-12 weeks of age.

### Study design

Animals in treatment groups (n = 5-6 per group) were subcutaneously co-implanted with 1 x 10⁷ H292 NSCLC tumor cells and 1 x 10⁷ PBMCs in the flank. After 5 days, animals were dosed intravenously with molecules of interest, with additional doses every 5 days until the end of the experiment. During the experiment, animals were monitored at regular intervals for tumor growth using caliper measurements and for weight loss. Animals were euthanized either when the mean tumor volume in the control group was 800 mm³ or at 40 days, whichever came first. Animals were monitored and euthanized according to animal health and welfare regulations at Charles River Laboratories.

### Results

We assessed the efficacy of **PRO2000** (biMSLN_{high KD}xCD3xhSA) in promoting tumor growth inhibition using a PBMC/H292 co-implantation model, as described in the methods. H292 cells express moderate levels of MSLN and are established from non-small cell lung carcinoma. Multiple dose levels of PRO2000 were administered intravenously, as shown in Figure 5. As comparisons, we used palivizumab (anti-RSV antibody) as a control IgG treatment, as well as tumor cells engrafted in the absence of PBMCs (no treatment). We observed that control conditions resulted in tumor outgrowth (light gray lines, Figure 5A). Treatment with the PRO2000 (biMSLN_{high KD}xCD3xhSA) molecule resulted in tumor growth inhibition at 1 and 5 mg/kg relative to controls (black lines and dark gray lines in Figure 5A, respectively). We examined the significance of the treatments using two-way ANOVA, followed by Tukey's multiple comparisons test; day 40 data are shown in Figure 5B, with each point representing an individual animal. The two higher doses (1 mg/kg and 5 mg/kg) resulted in significantly lower tumor volumes relative to palivizumab-treated animals (ctrl) and untreated animals. The lowest dose (0.2 mg/kg) appeared to be suboptimal, as these comparisons were not significant as compared to palivizumab-treated animals. There appeared to be no adverse effects on overall animal health, as animal weights were relatively stable throughout the experiment (data not shown). Taken together, these data indicate that the multispecific antibody PRO2000 (biMSLN_{high KD}xCD3xhSA) has tumor growth inhibition activity *in vivo* and is a suitable MA2 for the pharmaceutical compositions or the kit of the present invention.

### Example 5: In vivo combination study: Comparing the effects of PRO1601 alone or in combination with a mesothelin-targeting molecule:

### Experimental description

An *in vivo,* mesothelin-expressing cell line xenograft experiment was performed at Charles River Laboratories in order to determine the abilities of PRO1601 (anti-PDL1xCD137xhSA), PRO1872 (anti-MSLNxCD3xhSA) and a combination of PRO1601 with PRO1872 to effectively control tumor growth relative to control animals.

### Methods

### Animals

Female NCG mice from Charles River Laboratories were bred and housed under conditions suitable for humanized mouse work. Animals were used between 8-12 weeks of age.

### Study design

Animals in treatment groups (n=7-8 per group) were subcutaneously co-implanted with 1 x 10⁷ H292 NSCLC tumor cells and 1 x 10⁷ PBMCs in the flank. After 5 days, animals were dosed intravenously with molecules of interest, with additional doses every 5 days until the end of the experiment. During the experiment, animals were monitored at regular intervals for tumor growth using caliper measurements and for weight loss. Animals were euthanized either when the mean tumor volume in the control group was 1000 mm³ or at 50 days, whichever came first. Animals were monitored and euthanized according to animal health and welfare regulations at Charles River Laboratories.

### Data analysis

Data analysis was performed using GraphPad Prism. Groups were compared using two-way ANOVA and the Tukey's multiple comparisons post-hoc test. A value of p < 0.05 was considered statistically significant. * indicates p < 0.05.

### Results and discussion

We assessed the efficacy of the 4-1BB (CD137) and PDL1 targeting molecule PRO1601, as well as the MSLN- and CD3-specific multispecific molecule PRO1872 in promoting tumor growth inhibition using a PBMC/H292 co-implantation model, as described in the methods. H292 cells express moderate levels of MSLN and are established from non-small cell lung carcinoma. This cell line intermediate levels of PDL1, making it a potential target for PRO1601 as well. PRO1601 (1 mg/kg) and PRO1872 (0.2 mg/kg or 1 mg/kg), as well as a combination of the two molecules were administered intravenously, as shown in Figure 6A. As comparison, we used palivizumab (anti-RSV antibody) as a control IgG treatment.

We observed that the control condition resulted in tumor outgrowth (medium gray line with triangles, Figure 6A). Treatment with the PRO1601 molecule resulted in significant tumor growth inhibition at multiple time points relative to palivizumab, as shown in Figure 6B (dark gray line with diamonds vs. medium gray line with triangles). Treatment with the combination of PRO1601 + PRO1872 resulted in significant tumor growth inhibition relative to PRO1601 alone by the end of the experiment (black lines vs. dark gray lines with diamonds, Figure 6A). Treatment with 0.2 mg/kg of PRO1872 alone (light gray dashed line with circles) resulted in a significantly poorer tumor growth inhibition compared to PRO1601 + PRO1872 at day 28 (both doses, black lines, p < 0.001).

There appeared to be no adverse effects on overall animal health related to the treatments, as animal weights were relatively stable throughout the experiment (data not shown). Taken together, these data indicate the promise of combination therapy using PRO1601 and PRO1872 to inhibit tumor growth activity in vivo and is a promising conceptual candidate for cancer immunotherapy.

## Claims

1. A pharmaceutical composition comprising:
1) a first multispecific antibody (MA1) comprising
a) one binding domain, which specifically binds to CD137 (CD137-BD), and
b) one binding domain, which specifically binds to PDL1 (PDL1-BD),
2) a second multispecific antibody (MA2) comprising
a) one or two binding domain(s), which specifically bind(s) to a tumor cell associated antigen (TAA-BD),
b) one binding domain, which specifically binds to CD3 (CD3-BD),
3) a pharmaceutically acceptable carrier,
wherein said MA1 does not comprise an immunoglobulin Fc region polypeptide.

2. A kit comprising:
1) a first multispecific antibody (MA1) comprising
a) one binding domain, which specifically binds to CD137 (CD137-BD), and
b) one binding domain, which specifically binds to PDL1 (PDL1-BD), and
2) a second multispecific antibody (MA2) comprising
a) one or two binding domain(s), which specifically bind(s) to a tumor cell associated antigen (TAA-BD),
b) one binding domain, which specifically binds to CD3 (CD3-BD),
wherein said MA1 does not comprise an immunoglobulin Fc region polypeptide.

3. The pharmaceutical composition of claim 1, or the kit of claim 2, wherein said MA1 or both antibodies MA1 and MA2, further comprises one human serum albumin binding domain (hSA-BD).

4. The pharmaceutical composition or the kit of any one of the preceding claims, wherein said CD137-BD comprises
(a) a VH sequence of SEQ ID NO: 4 and a VL sequence of SEQ ID NO:11;
(b) a VH sequence of SEQ ID NO: 5 and a VL sequence of SEQ ID NO: 12;
(c) a VH sequence of SEQ ID NO: 6 and a VL sequence of SEQ ID NO: 13;
(d) a VH sequence of SEQ ID NO: 7 and a VL sequence of SEQ ID NO: 14;
(e) a VH sequence of SEQ ID NO: 22 and a VL sequence of SEQ ID NO: 26;
(f) a VH sequence of SEQ ID NO: 31 and a VL sequence of SEQ ID NO: 37;
(g) a VH sequence of SEQ ID NO: 32 and a VL sequence of SEQ ID NO: 38; or
(h) a VH sequence of SEQ ID NO: 33 and a VL sequence of SEQ ID NO: 39;
and/or said PDL1-BD comprises
(a) a VH sequence of SEQ ID NO: 46 and a VL sequence of SEQ ID NO: 52;
(b) a VH sequence of SEQ ID NO: 47 and a VL sequence of SEQ ID NO: 52;
(c) a VH sequence of SEQ ID NO: 48 and a VL sequence of SEQ ID NO: 53;
(d) a VH sequence of SEQ ID NO: 60 and a VL sequence of SEQ ID NO: 66;
(e) a VH sequence of SEQ ID NO: 61 and a VL sequence of SEQ ID NO: 67; or
(f) a VH sequence of SEQ ID NO: 62 and a VL sequence of SEQ ID NO: 66;
and said hSA-BD, if present, comprises
(a) a VH sequence of SEQ ID NO: 74 and a VL sequence of SEQ ID NO: 78;
(b) a VH sequence of SEQ ID NO: 83 and a VL sequence of SEQ ID NO: 87; in particular wherein said CD137-BD comprises
(f) a VH sequence of SEQ ID NO: 31 and a VL sequence of SEQ ID NO: 37;
(g) a VH sequence of SEQ ID NO: 32 and a VL sequence of SEQ ID NO: 38; or
(h) a VH sequence of SEQ ID NO: 33 and a VL sequence of SEQ ID NO: 39;
said PDL1-BD comprises
(a) a VH sequence of SEQ ID NO: 46 and a VL sequence of SEQ ID NO: 52;
(b) a VH sequence of SEQ ID NO: 47 and a VL sequence of SEQ ID NO: 52; or
(c) a VH sequence of SEQ ID NO: 48 and a VL sequence of SEQ ID NO: 53;
and said hSA-BD, if present, comprises
(a) a VH sequence of SEQ ID NO: 74 and a VL sequence of SEQ ID NO: 78;
(b)a VH sequence of SEQ ID NO: 83 and a VL sequence of SEQ ID NO: 87.

5. The pharmaceutical composition or the kit of any one of claims 1 to 4, wherein said MA1 is selected from the scDbs of SEQ ID NOs: 105, 106, 107, 108, 109, 110, and 111 and from the scDb-scFvs of SEQ ID NOs: 112, 113, 114, 115, 116, 117, 118, 119, 120, 121, 122, 123, 124, 125, 126, 127, 128, 129 and 130, preferably wherein said MA1 is selected from the scDb-scFvs of SEQ ID NOs: 112, 113, 114, 115, 116, 117, 118, 119, 120, 121, 122, 123, 124, 125, 126, 127, 128, 129 and 130, more preferably wherein said MA1 is selected from the scDb-scFvs of SEQ ID NO: 125, 126, 127 and 130, in particularly wherein said MA1 is selected from the scDb-scFvs of SEQ ID NO: 125, 127 and 130.

6. The pharmaceutical composition or the kit of any one of the preceding claims, wherein the tumor cell associated antigen (TAA) is selected from the group consisting of CD138, CD79b, TPBG (5T4), HER2, MSLN, MUC1, CA-125 (MUC16), PSMA, BCMA, CD19, EpCAM, CLEC12A (CLL1), CD20, CD22, CEA, CD33, EGFR, GPC3, CD123, CD38, CD33, CD276, CDH3 (cadherin 3), FGFR1, SSTR2, CD133, EPHA2, HLA-A2, IL13RA2, ROR1, CEACAM6, CD135, GD-2, GA733, CD135 (FLT3), CSPG4 and TAG-72,
preferably, wherein the TAA is selected from the group consisting of CD138, CD79b, CD123, HER2, MSLN, PSMA, BCMA, CD19, CD20, CEA, CD38, CD33, CLEC12a and ROR1,
in particular wherein the TAA is selected from the group consisting of HER2, MSLN and ROR1.

7. The pharmaceutical composition or the kit of any one of the preceding claims, wherein said MA2 comprises one TAA-BD.

8. The pharmaceutical composition or the kit of claim 7, wherein the TAA-BD is either
a mesothelin binding domain (MSLN-BD), which specifically binds to mesothelin (MSLN), particularly a MSLN-BD comprising
(i) the HCDR1, HCDR2, and HCDR3 sequences of SEQ ID NOs: 139, 140 and 141, respectively, and the LCDR1, LCDR2, and LCDR3 sequences of SEQ ID NOs: 143, 144 and 145, respectively; and
(ii) VH3 or VH4 domain framework sequences FR1 to FR4; preferably VH3 domain framework sequences FR1 to FR4; and
(iii) a VL domain comprising a VL framework comprising framework regions FR1, FR2 and FR3, which are selected from V_{K} subtypes, particularly from the V_{K}1 and V_{K}3 subtypes, particularly are of the V_{K}1 subtype, and a framework FR4, which is selected from a V_{K} FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101,
particularly a MSLN-BD comprising
a) HCDR1, HCDR2, and HCDR3 sequences of SEQ ID NOs: 139, 140 and 141, respectively,
b) LCDR1, LCDR2, and LCDR3 sequences of SEQ ID NOs: 143, 144 and 145, respectively,
c) a VH sequence at least 90, 91, 92, 93, 94, 95, 96, 97, 98 or 99 percent identical to the amino acid sequence SEQ ID NO: 142, and
d) a VL sequence at least 90, 91, 92, 93, 94, 95, 96, 97, 98 or 99 percent identical to the amino acid sequence SEQ ID NO: 146;
or
a HER2 binding domain (HER2-BD), which specifically binds to HER2 (HER2), particularly a HER2-BD comprising
(i) the HCDR1, HCDR2, and HCDR3 sequences of SEQ ID NOs: 165, 166 and 167, respectively, and the LCDR1, LCDR2, and LCDR3 sequences of SEQ ID NOs: 170, 171 and 172, respectively; and
(ii) VH3 or VH4 domain framework sequences FR1 to FR4; preferably VH3 domain framework sequences FR1 to FR4; and
(iii) a VL domain comprising a VL framework comprising framework regions FR1, FR2 and FR3, which are selected from V_{K} subtypes, particularly from the V_{K}1 and V_{K}3 subtypes, particularly are of the V_{K}1 subtype, and a framework FR4, which is selected from a V_{K} FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101.
particularly a HER2-BD comprising
a) HCDR1, HCDR2, and HCDR3 sequences of SEQ ID NOs: 165, 166 and 167, respectively,
b) LCDR1, LCDR2, and LCDR3 sequences of SEQ ID NOs: 170, 171 and 172, respectively,
c) a VH sequence at least 90, 91, 92, 93, 94, 95, 96, 97, 98 or 99 percent identical to the amino acid sequence SEQ ID NO: 168 or 169, and
d) a VL sequence at least 90, 91, 92, 93, 94, 95, 96, 97, 98 or 99 percent identical to the amino acid sequence SEQ ID NO: 173 or 174.

9. The pharmaceutical composition or the kit of any one of claims 1 to 6, wherein the MA2 comprises two TAA-BDs, particularly two TAA-BDs, which bind to the same antigen.

10. The pharmaceutical composition or the kit of claim 9, wherein said TAA-BDs are mesothelin binding domains (MSLN-BDs), which specifically bind to mesothelin (MSLN), particularly MSLN-BDs comprising
(i) the HCDR1, HCDR2, and HCDR3 sequences of SEQ ID NOs: 147, 148 (or 151) and 149, respectively, and the LCDR1, LCDR2, and LCDR3 sequences of SEQ ID NOs: 153, 154 and 155, respectively; or the HCDR1, HCDR2, and HCDR3 sequences of SEQ ID NOs: 157, 158 and 159, respectively, and the LCDR1, LCDR2, and LCDR3 sequences of SEQ ID NOs: 161, 162 and 163, respectively; and
(ii) VH3 or VH4 domain framework sequences FR1 to FR4; particularly VH3 domain framework sequences FR1 to FR4; and
(iii) a VL domain comprising a VL framework comprising framework regions FR1, FR2 and FR3, which are selected from V_{K} subtypes, particularly from the V_{K}1 and V_{K}3 subtypes, particularly are of the V_{K}1 subtype, and a framework FR4, which is selected from a V_{K} FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101;
particularly MSLN-BDs comprising
a.1) HCDR1, HCDR2, and HCDR3 sequences of SEQ ID NOs: 147, 148 (or 151) and 149, respectively,
b.1) LCDR1, LCDR2, and LCDR3 sequences of SEQ ID NOs: 153, 154 and 155, respectively,
c.1) a VH sequence at least 90, 91, 92, 93, 94, 95, 96, 97, 98 or 99 percent identical to the amino acid sequence SEQ ID NO: 150, and
d.1) a VL sequence at least 90, 91, 92, 93, 94, 95, 96, 97, 98 or 99 percent identical to the amino acid sequence SEQ ID NO: 156;
or
a.2) HCDR1, HCDR2, and HCDR3 sequences of SEQ ID NOs: 147, 148 (or 151) and 149, respectively,
b.2) LCDR1, LCDR2, and LCDR3 sequences of SEQ ID NOs: 153, 154 and 155, respectively,
c.2) a VH sequence at least 90, 91, 92, 93, 94, 95, 96, 97, 98 or 99 percent identical to the amino acid sequence SEQ ID NO: 152, and
d.2) a VL sequence at least 90, 91, 92, 93, 94, 95, 96, 97, 98 or 99 percent identical to the amino acid sequence SEQ ID NO: 156;
or
a.3) HCDR1, HCDR2, and HCDR3 sequences of SEQ ID NOs: 157, 158 and 159, respectively,
b.3) LCDR1, LCDR2, and LCDR3 sequences of SEQ ID NOs: 161, 162 and 163, respectively,
c.3) a VH sequence at least 90, 91, 92, 93, 94, 95, 96, 97, 98 or 99 percent identical to the amino acid sequence SEQ ID NO: 160, and
d.3) a VL sequence at least 90, 91, 92, 93, 94, 95, 96, 97, 98 or 99 percent identical to the amino acid sequence SEQ ID NO: 164.

11. The pharmaceutical composition or the kit of any of the preceding claims, wherein said CD3-BD comprises
(i) HCDR1, HCDR2, and HCDR3 sequences of SEQ ID NOs: 131, 132 and 133, embedded in an antibody VH framework, particularly in a human antibody VH framework, particularly in a human VH3 framework, and
(ii) LCDR1, LCDR2, and LCDR3 sequences of SEQ ID NOs: 135, 136 and 137, embedded in an antibody VH framework, particularly in a human antibody VL framework, wherein the VL framework comprises framework regions FR1, FR2 and FR3, which are selected from V_{K} subtypes, particularly from the V_{K}1 and V_{K}3 subtypes, particularly are of the V_{K}1 subtype, and a framework FR4, which is selected from a V_{K} FR4 and a Vλ FR4, particularly is a Vλ FR4 comprising an amino acid sequence having at least 70, 80, 90 percent identity, particularly at least 90 percent identity, to any of SEQ ID NO: 94 to SEQ ID NO: 101, more particularly a Vλ FR4 selected from any of SEQ ID NO: 94 to SEQ ID NO: 101, particularly a Vλ FR4 according to SEQ ID NO: 94 or 101;
particularly wherein said CD3-BD comprises
(i) a VH domain comprising the amino acid sequence of SEQ ID NO: 134, and
(ii) a VL domain comprising the amino acid sequence of SEQ ID NO: 138.

12. The pharmaceutical composition or the kit of any one of the preceding claims, wherein said MA2 is selected from the scMATCH3-based antibodies of SEQ ID NOs: 175, 180 and 181 and from the MATCH4-based heterodimeric antibodies of SEQ ID NOs: 176 and 177, and 178 and 179.

13. The pharmaceutical composition or the kit of any one of claims 1 to 12 for use in the treatment of a disease, particularly a human disease, more particularly a human disease selected from cancer, particularly a cancer selected from mesothelioma, pancreatic cancer, gastric cancer, lung cancer, breast cancer and ovarian cancer, an inflammatory and an autoimmune disease.

14. A method for the treatment of a disease, particularly a human disease, more particularly a human disease selected from cancers, particularly a cancer selected from mesothelioma, pancreatic cancer, gastric cancer, lung cancer, breast cancer and ovarian cancer, comprising the step of administering the pharmaceutical composition or the kit of any one of claim 1 to 12.

15. A method for treating a patient suffering from cancer, particularly a cancer selected from melanoma, mesothelioma, pancreatic cancer, stomach cancer, breast cancer, ovarian cancer and lung cancer, comprising the step of administering a first multispecific antibody (MA1) comprising one binding domain, which specifically binds to CD137 (CD137-BD), and one binding domain, which specifically binds to PDL1 (PDL1-BD), and a second multispecific antibody (MA2) comprising at least one binding domain, which specifically binds to a tumor cell associated antigen (TAA-BD), and one binding domain, which specifically binds to CD3 (CD3-BD), to said patient, wherein said MA1 and MA2 are as defined in claims 1 to 12.
